# EUROPEAN PATENT APPLICATION

(11) **EP 2 860 260 A1**
(43) Date of publication of application: **15.04.2015**
(21) Application number: 14181574.6
(22) Date of filing: 10.04.2009
(51) Int. Cl.: C12P 21/04

(54) **Human serum albumin linkers and conjugates thereof**

(30) Priority: 11.04.2008 US 123942 P; 18.11.2008 US 115797 P
(62) Divisional of application: 09730096.6
(71) Applicant: Merrimack Pharmaceuticals, Inc., Cambridge, MA 02139 (US)
(72) Inventor: McDonagh, Charlotte, Winchester, MA 01890 (US); Feldhaus, Michael, Grantham, NH 03753 (US); Huhalov, Alexandra, Lexington, MA 02420-3708 (US)
(74) Representative: Taylor, Kate Laura

(57) **Abstract**

The present invention relates to a human serum albumin (HSA) linker and binding, diagnostic, and therapeutic agents conjugated thereto. Also disclosed are methods and kits for the diagnostic and therapeutic application of an HSA linker conjugate.

## Description

### FIELD OF THE INVENTION

The invention provides a human serum albumin (HSA) linker and binding, diagnostic, and therapeutic conjugates thereof. In one embodiment, the HSA linker includes two amino acid substitutions. The invention further provides methods for the manufacture and administration of diagnostic and therapeutic HSA linker conjugates.

### BACKGROUND OF THE INVENTION

The serum albumins belong to a family of proteins that includes alpha-fetoprotein and human group-specific component, also known as vitamin-D binding protein. The scrum albumins are the major soluble proteins of the circulatory system and contribute to many vital physiological processes. Serum albumin generally comprises about 50% of the total blood component by dry weight. The albumins and their related blood proteins also play an important role in the transport, distribution, and metabolism of many endogenous and exogenous ligands in the human body, including a variety of chemically diverse molecules, such as fatty acids, amino acids, steroids, calcium, metals such as copper and zinc, and various pharmaceutical agents. The albumin family of molecules are generally thought to facilitate transfer of many of these ligands across organ-circulatory interfaces, such as the liver, intestines, kidneys, and the brain. The albumins are thus involved in a wide range of circulatory and metabolic functions.

Human serum albumin (HSA) is a protein of about 66,500 kD and is comprised of 585 amino acids including at least 17 disulphide bridges. As with many of the members of the albumin family, human serum albumin plays an important role in human physiology and is located in virtually every human tissue and bodily secretion. As indicated above, HSA has the ability to bind and transport a wide spectrum of ligands throughout the circulatory system, including the long-chain fatty acids, which are otherwise insoluble in circulating plasma.

### SUMMARY OF THE INVENTION

In a first aspect, the invention features an agent that includes a human serum albumin (HSA) linker having an amino acid sequence set forth in any one of SEQ ID NOS:6-10 and first and second binding moieties selected from antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, receptors (e.g., cell surface receptors), ligands, aptamers, and biologically-active fragments thereof, in which the first binding moiety is bonded to the amino terminus of the HSA linker and the second binding moiety is bonded to the carboxy terminus of the HSA linker. In one embodiment, the first binding moiety specifically binds ErbB3 and the second binding moiety specifically binds ErbB2. In other embodiments, the HSA linker has the amino acid sequences set forth in SEQ ID NOS:9 or 10.

In a second aspect, the invention features an agent that includes an HSA linker that has an amino acid sequence set forth in any one SEQ ID NOS:11-15 and at least first and second binding moieties, which can be selected from antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, receptors (e.g., cell surface receptors), ligands, aptamers, and biologically-active fragments thereof, wherein the first binding moiety is bonded to the amino terminus and the second binding moiety is bonded to the carboxy terminus of the sequence. In an embodiment, three or more binding moieties (e.g., 4, 5, 6, 7, 8, 9, 10, or more) can be included in the agent; these additional binding moities can be added to the agent, e.g., in tandem (e.g., 2, 3, 4, or 5 or more in tandem) with the first or second binding moiety. In one embodiment, the first binding moiety specifically binds ErbB3 and the second binding moiety specifically binds ErbB2. In other embodiments, the HSA linker has the amino acid sequences set forth in SEQ ID NOS:14 or 15.

In a third aspect, the invention provides an HSA linker that has an amino acid sequence having at least 90% sequence identity to the sequence set forth in SEQ ID NO:1, and a serine residue at position 34 and a glutamine residue at position 503 of the amino acid sequence set forth in SEQ ID NO:1. In one embodiment, the amino acid sequence has at least 95% sequence identity to the sequence set forth in SEQ ID NO:1. In another embodiment, the HSA linker has the amino acid sequence set forth in SEQ ID NO:1.

In a fourth aspect, the invention features an agent that includes an IISA linker having at least 90% amino acid sequence identity to the sequence set forth in SEQ ID NO:1 and at least a first binding moiety. In one embodiment, the agent includes a first polypeptide connector that binds the first binding moiety to the HSA linker.

In a fifth aspect, the invention features an agent that includes an HSA linker having an amino acid sequence set forth in any one of SEQ ID NOS:11-15, or a fragment or variant of any one of these sequences, and at least a first binding moiety.

In either the fourth or fifth aspect of the invention, the agent further includes a first polypeptide connector (e.g., AAS, AAQ, or AAAL) that binds the first binding moiety to the amino or carboxy terminus of the HSA linker. In an embodiment, the first connector covalently binds the first binding moiety to the HSA linker.

In either the fourth or fifth aspect of the invention, the agent further includes at least a second binding moiety. In an embodiment, the agent further includes a second polypeptide connector (e.g., AAS, AAQ, or AAAL) that binds the second binding moiety to the HSA linker. In other embodiments, the second connector binds the second binding moiety to the amino or carboxy terminus of the HSA linker. In a further embodiment, the second connector covalently binds the second binding moiety to the HSA linker. In other embodiments, the agent further includes three or more binding moieties which are included in tandem with the first or second binding moiety; the three or more binding moieties can further include a connector sequence that joins the three or more binding moieties to the first or second binding moiety and to each other.

In either the fourth or fifth aspect of the invention, the agent includes a first polypeptide connector that covalently binds a first binding moiety to the amino terminus of the HSA linker and a second polypeptide connector that covalently binds a second binding moiety to the carboxy terminus of the HSA linker. In one embodiment, the first connector has the amino acid sequence AAS or AAQ and the second connector has the amino acid sequence set forth in SEQ ID NO:5,

In either the fourth or fifth aspect of the invention, the first or second binding moiety (or third or more binding moiety) is an antibody, single-chain Fv molecule, bispecific single chain Fv ((scFv')₂) molecule, domain antibody, diabody, triabody, hormone, Fab fragment, F(ab')₂ molecule, tandem scFv (taFv) fragment, receptor (e.g., cell surface receptor), ligand, aptamer, or biologically-active fragment thereof. In other embodiments, the agents of the invention include combinations of these different types of binding moities. In one embodiment, at least the first or second binding moiety is a human or humanized single-chain Fv molecule.

In an embodiment of any one of the first, second, third, fourth, or fifth aspects of the invention, one or more of the first or second binding moiety (or, if present, the third or further binding moiety) is or specifically binds to a protein selected from the group consisting of an insulin-like growth factor 1 receptor (IGF1R), IGF2R, insulin-like growth factor (IGF), mesenchymal epithelial transition factor receptor (c-met; also known as hepatocyte growth factor receptor (HGFR)), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, an integrin (e.g., an α4 integrin or a β-1 integrin), P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD 106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, MART-1, gp100, MAGE-1, cphrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof. In a further embodiment, one or more of the first or second binding moiety (or, if present, the third or further binding moiety) is or specifically binds to erythroblastic leukemia viral oncogene homolog (ErbB) receptor (e.g., ErbB1 receptor; ErbB2 receptor; ErbB3 receptor; and ErbB4 receptor). In another embodiment, one or more of the first or second binding moiety (or, if present, the third or further binding moiety) is or specifically binds to alpha-fetoprotein (AFP) or an interferon, or a biologically-active fragment thereof. In a further embodiment, one or more of the first or second binding moiety (or, if present, the third or further binding moiety) is natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, or anakinra.

In any one of the first, second, third, fourth, or fifth aspects of the invention, the agent is conjoined to a diagnostic, a therapeutic agent, or both. In one embodiment, the diagnostic agent is a detectable label, such as a radioactive, fluorescent, or heavy metal label. In another embodiment, the therapeutic agent is a cytotoxic agent, cytostatic, or immunomodulatory agent. Cytotoxic agents include alkylating agents, antibiotics, antineoplastic agents, antiproliferative agents, antimetabolites, tubulin inhibitors, topoisomerase I or II inhibitors, hormonal agonists or antagonists, immunomodulators, DNA minor groove binders, and radioactive agents, or any agent capable of binding to and killing a tumor cell or inhibiting tumor cell proliferation. Antineoplastic agents include cyclophosphamide, camptothecin, homocamptothecin, colchicine, combrestatin, combrestatin, rhizoxin, dolistalin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and their derivatives.

In any one of the first, second, third, fourth, or fifth aspects of the invention, the agent is admixed with a pharmaceutically acceptable carrier, excipient, or diluent. In one embodiment, the agent exhibits an *in vivo* half-life of between 6 hours and 7 days. In another embodiment, the agent exhibits an *in vivo* half-life greater than 8 hours.

In a sixth aspect, the invention features a method for treating a mammal having a disease or disorder by administering any one of the HSA agents described in the first five aspects. In one embodiment, the disease or disorder is associated with cellular signaling through a cell surface receptor. In another embodiment, the mammal is a human. In a further embodiment, the disease or disorder is a proliferative or autoimmune disease. Proliferative diseases include melanoma, clear cell sarcoma, head and neck cancer, bladder cancer, breast cancer, colon cancer, ovarian cancer, endometrial cancer, gastric cancer, pancreatic cancer, renal cancer, prostate cancer, salivary gland cancer, lung cancer, liver cancer, skin cancer, and brain cancer. Autoimmune diseases include multiple sclerosis, psoriasis, myasthenia gravis, uveitis, systemic lupus erythematosus, and rheumatoid arthritis. In one embodiment, the HSA agent is administered in combination with one or more therapeutic agents, such as an antineoplastic agent.

In a seventh aspect, the invention features a method for making an HSA linker agent by bonding at least a first binding moiety to the amino terminus and a second binding moiety to the carboxy terminus of the amino acid sequence set forth in any one of SEQ ID NOS:1, 3, or 6-15, or a sequence having at least 90%, 95%, 97%, or 100% sequence identity to a sequence set forth in any one of SEQ ID NOS:1, 3, or 6-15. In one embodiment, the first or second binding moiety is covalently joined to the amino or carboxy terminus of an HSA linker. In other embodiments, a third or additional binding moiety (e.g., a fourth, fifth, sixth, seventh, eighth, ninth, or tenth binding moiety) is covalently joined in tandem with the first or second binding moiety to the amino or carboxy terminus of the HSA linker. In another embodiment, one or more of the first or second binding moiety (or, if present, the third or further binding moiety) is an antibody, single-chain Fv molecule, bispecific single chain Fv ((scFv')₂) molecule, domain antibody, diabody, triabody, hormone, Fab fragment, F(ab')₂ molecule, tandem scFv (taFv) fragment, receptor (e.g., cell surface receptor), ligand, or aptamer. In another embodiment, the first or second binding moiety (or, if present, the third or further binding moiety) is a human or humanized single-chain Fv molecule. In yet another embodiment, one or more of the first or second binding moiety (or, if present, the third or further binding moiety) is or specifically binds to insulin-like growth factor 1 receptor (IGF1R), IGF2R, insulin-like growth factor (IGF), mesenchymal epithelial transition factor receptor (c-met; also known as hepatocyte growth factor receptor (HGFR)), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, an integrin (e.g., an α4 integrin or a β-1 integrin), P-selectin, sphingosine-1-phosphate receptor-1 (S1PR), hyaluronate receptor, leukocyte function antigen-1 (LFA-1), CD4, CD11, CD 18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD 178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof. In a further embodiment, one or more of the first or second binding moiety (or, if present, the third or further binding moiety) is or specifically binds to erythroblastic leukemia viral oncogene homolog (ErbB) receptor (e.g., ErbB1 receptor; ErbB2 receptor; ErbB3 receptor; and ErbB4 receptor). In another embodiment, one or more of the first or second binding moiety (or, if present, the third or further binding moiety) is or specifically binds to alpha-fetoprotein (AFP) or an interferon, or a biologically-active fragment thereof. In a further embodiment, one or more of the first or second binding moiety (or, if present, the third or further binding moiety) is natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, or anakinra. In another embodiment, the agent is conjoined to a diagnostic or therapeutic agent. In one embodiment, the diagnostic agent is a detectable label, such as a radioactive, bioluminescent, fluorescent, heavy metal, or epitope tag. In another embodiment, the therapeutic agent is a cytotoxic agent, cytostatic, or immunomodulatory agent. Cytotoxic agents include alkylating agents, antibiotics, antineoplastic agents, antiproliferative agents, antimetabolites, tubulin inhibitors, topoisomerase I and II inhibitors, hormonal agonists or antagonists, immunomodulators, DNA minor groove binders, and radioactive agents, or any agent capable of binding to and killing a tumor cell or inhibiting tumor cell proliferation. Antineoplastic agents include cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and their derivatives. In a further embodiment, the agent is admixed with a pharmaceutically acceptable carrier, excipient, or diluent.

In a eighth aspect, the invention features a method for making an HSA agent by substituting one or more surface-exposed amino acid residues in the amino acid sequences set forth in any one of SEQ ID NOS:1, 3, and 6-15 with a substitute amino acid capable of chemical modification that allows conjugation of a diagnostic or therapeutic agent. In one embodiment, the substitute amino acid is cysteine and the surface exposed amino acid residues are serine or threonine. In another embodiment, the chemical modification results in a covalent bond between the substitute amino acid and the diagnostic or therapeutic agent. In a further embodiment, the surface-exposed amino acid residues is threonine at position 496, serine at position 58, threonine at position 76, threonine at position 79, threonine at position 83, threonine at position 125, threonine at position 236, serine at position 270, serine at position 273, serine at position 304, serine at position 435, threonine at position 478, threonine at position 506, or threonine at position 508.

In a ninth aspect, the invention features a method for making an HSA agent by substituting one or more of the residues in the amino acid sequences set forth in any one of SEQ ID NOS:1, 3, and 6-15 with an asparagine, serine, or threonine, thereby incorporating a glycosylation site within the HSA agent.

In a tenth aspect, the invention features a method for making an HSA agent by substituting one or more of the asparagine, serine, or threonine residues in the amino acid sequences set forth in any one of SEQ ID NOS:1, 3, and 6-15 with any amino acid other than asparagine, serine, or threonine, thereby removing a glycosylation site from the HSA agent.

In an eleventh aspect, the invention features an agent that has at least 90% sequence identity to one of the amino acid sequences set forth in SEQ ID NOS:16-25. In one embodiment, the agent has at least 95% sequence identity to one of the amino acid sequences set forth in SEQ ID NOS:16-25. In another embodiment, the agent has one of the amino acid sequences set forth in SEQ ID NOS:16-25. In a further embodiment, the agent is conjoined to a diagnostic or therapeutic agent. Diagnostic agents include detectable labels, such as a radioactive, bioluminescent, fluorescent, or heavy metal labels, or epitope tags. Fluorescent molecules that can serve as detectable labels include green fluorescent protein (GFP), enhanced GFP (eGFP), yellow fluorescent protein (YFP), cyan fluorescent protein (CFP), red fluorescent protein (RFP), and dsRed. In one embodiment, the bioluminescent molecule is luciferase. In another embodiment, the epitope tag is c-myc, hemagglutinin, or a histidine tag. In a further embodiment, the therapeutic agent is a cytotoxic polypeptide such as cytochrome c, caspase 1-10, granzyme A or B, tumor necrosis factor-alpha (TNF-α), TNF-β, Fas, Fas ligand, Fas-associated death doman-like IL-1β converting enzyme (FLICE), TRAIL/APO2L, TWEAK/APO3L, Bax, Bid, Bik, Bad, Bak, RICK, vascular apoptosis inducing proteins 1 and 2 (VAP1 and VAP2), pierisin, apoptosis-inducing protein (AIP), IL-1α propiece polypeptide, apoptin, apoptin-associated protein 1 (AAP-1), endostatin, angiostatin, and biologically-active fragments thereof. An agent of the invention can be combined with one or more therapeutic agents such as cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxcl, cisplatin, carboplatin, tamoxifen, raloxifene, letrozole, epirubicin, bevacizumab, pertuzumab, trastuzumab, and derivatives thereof.

In an embodiment of any aspect of the invention, the first and second binding moieties (and, if present, one or more of the third or further binding moiety) specifically bind the same target molecule. In another embodiment of any aspect of the invention, the first and second binding moieties (and, if present, one or more of the third or further binding moiety) specifically bind different target molecules. In a further embodiment of any aspect of the invention, the first and second binding moieties (and, if present, one or more of the third or further binding moiety) specifically bind different epitopes on the same target molecule.

In a twelfth aspect, the invention features a polypeptide linker that has amino acid residues 25-44 and 494-513 of the amino acid sequence set forth in SEQ ID NO:1. In one embodiment, the polypeptide linker has amino acid residues 25-70 and 450-513 of the amino acid sequence set forth in SEQ ID NO:1. In another embodiment, the polypeptide linker has amino acid residues 15-100 and 400-520 of the amino acid sequence set forth in SEQ ID NO:1. In a further embodiment, the polypeptide linker has amino acid residues 10-200 and 300-575 of the amino acid sequence set forth in SEQ ID NO:1. In another embodiment, the polypeptide linker has amino acid residues 5-250 and 275-580 of the amino acid sequence set forth in SEQ ID NO:1.

In the twelfth aspect of the invention, the polypeptide linker includes at least a first binding moiety. In one embodiment, the polypeptide linker includes at least a first polypeptide connector that binds the first binding moiety to the amino or carboxy terminus of the polypeptide linker. In another embodiment, the first polypeptide connector covalently binds the first binding moiety to the polypeptide linker. In a further embodiment, the polypeptide linker includes a second binding moiety. In one embodiment, the polypeptide linker includes a second polypeptide connector that binds the second binding moiety to the polypeptide linker. In other embodiments, the second connector binds the second binding moiety to the amino or carboxy terminus of the polypeptide linker. In a further embodiment, the second connector covalently binds the second binding moiety to the polypeptide linker. In other embodiments, the polypeptide linker includes a third, fourth, fifth, sixth, seventh, eighth, ninth, or tenth binding moiety. In other embodiments, these additional binding moieties are present in tandem with one or both of the first or second binding moiety. In yet other embodiments, a polypeptide connector (e.g., AAS, AAQ, or AAAL) separates one or more of these additional binding moities from each other, the first or second binding moiety, or the polypeptide linker.

In the twelfth aspect of the invention, the polypeptide linker includes a first polypeptide connector that covalently binds a first binding moiety to the amino terminus of the polypeptide linker and a second polypeptide connector that covalently binds a second binding moiety to the carboxy terminus of the polypeptide linker. In one embodiment, the first connector has the amino acid sequence AAS or AAQ and the second connector has the amino acid sequence set forth in SEQ ID NO:5.

In the twelfth aspect of the invention, one or more of the first or second binding moiety (or, if present, the third or further binding moiety) is an antibody, single-chain Fv molecule, bispecific single chain Fv ((scFv')₂) molecule, domain antibody, diabody, triabody, hormone, Fab fragment, F(ab')₂ molecule, tandem scFv (taFv) fragment, receptor (e.g., cell surface receptor), ligand, aptamer, or biologically-active fragment thereof. In one embodiment, one or more of the first or second binding moiety (or, if present, the third or further binding moiety) is a human or humanized single-chain Fv molecule.

In the twelfth aspect of the invention, one or more of the first or second binding moiety (or, if present, the third or further binding moiety) is or specifically binds to a protein selected from the group consisting of an insulin-like growth factor 1 receptor (IGF1R), IGF2R, insulin-like growth factor (IGF), mesenchymal epithelial transition factor receptor (c-met; also known as hepatocyte growth factor receptor (HGFR)), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, an integrin (e.g., an α4 integrin or a β-1 integrin), P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof. In a further embodiment, the first or second binding moiety is or specifically binds to erythroblastic leukemia viral oncogene homolog (ErbB) receptor (e.g., ErbB1 receptor; ErbB2 receptor; ErbB3 receptor; and ErbB4 receptor). In another embodiment, one or more of the first or second binding moiety (or, if present, the third or further binding moiety) is or specifically binds to alpha-fetoprotein (AFP) or an interferon, or a biologically-active fragment thereof. In a further embodiment, one or more of the first or second binding moiety (or, if present, the third or further binding moiety) is natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, or anakinra.

In the twelfth aspect of the invention, the polypeptide linker is conjoined to a diagnostic, a therapeutic agent, or both. In one embodiment, the diagnostic agent is a detectable label, such as a radioactive, fluorescent, or heavy metal label. In another embodiment, the therapeutic agent is a cytotoxic agent, cytostatic, or immunomodulatory agent. Cytotoxic agents include alkylating agents, antibiotics, antineoplastic agents, antiproliferative agents, antimetabolites, tubulin inhibitors, topoisomerase I or II inhibitors, hormonal agonists or antagonists, immunomodulators, DNA minor groove binders, and radioactive agents, or any agent capable of binding to and killing a tumor cell or inhibiting tumor cell proliferation. Antineoplastic agents include cyclophosphamide, camptothecin, homocamptothccin, colchicine, combrestatin, combrestatin, rhizoxin, dolistatin, ansamitocin p3, maytansinoid, auristatin, calcachimicin, methotrexate, 5-fluorouracil (5-FU), doxorubicin, paclitaxcl, docetaxel, cisplatin, carboplatin, tamoxifen, raloxifene, letrozole, epirubicin, bevacizumab, pertuzumab, trastuzumab, and their derivatives. In one embodiment, the polypeptide linker is admixed with a pharmaceutically acceptable carrier, excipient, or diluent. In another embodiment, the polypeptide linker exhibits an *in vivo* half-life of between 6 hours and 7 days. In a further embodiment, the polypeptide linker exhibits an *in vivo* half-life greater than 8 hours.

A thirteenth aspect of the invention features an agent of any of the prior aspects of the invention (one through twelve), in which the HSA polypeptide linker sequence is replaced by another polypeptide linker sequence. For example, the polypeptide linker sequence could be a mammalian, non-human serum albumin polypeptide sequence, such as, e.g., a bovine, murine, feline, and canine serum albumin (BSA) polypeptide sequence. In other embodiments this polypeptide linker sequence is between 5 and 1,000 amino acids in length, e.g., 10, 20, 30, 40, 50, 60, 70, 80, 90, 100, 200, 300, 400, 500, 600, 700, 800, or 900 amino acids in length, or any number of amino acids within this range. In other embodiments, the polypeptide linker sequence includes a single amino acid (including, but not limited to, e.g., glycine, alanine, serine, glutamine, leucine, and valine), or combinations of amino acids.

In another embodiment, the HSA polypeptide linker sequence is replaced by an alpha-fetoprotein polypeptide (AFP) polypeptide sequence, e.g., mammalian AFP polypeptide sequence, such as a human, murine, bovine, or canine AFP polypeptide sequence. In an embodiment, the AFP linker sequence of the agent can correspond to the full-length human AFP polypeptide sequence (a.a. 1-609; SEQ ID NO: 58), the mature human AFP polypeptide sequence lacking amino acids 1-18 of the signal sequence (a.a., 19-609 of SEQ ID NO: 58), or fragments thereof. In other embodiments, the AFP polypeptide linker sequence contains at least 5 to 8 contiguous amino acids, preferably at least 10, 20, or 50 contiguous amino acids, more preferably at least 100 contiguous amino acids, and most preferably at least 200, 300, 400, or more contiguous amino acids of SEQ ID NO: 58, or has at least 90% sequence identity (e.g., at least 95%, 97%, 99%, or more sequence identity) to a continguous polypeptide sequence of SEQ ID NO: 58 having one or more of these lengths. For example, an AFP polypeptide linker sequence having 90% sequence identity to a 34-mer human AFP peptide corresponding to amino acids 446-479 of SEQ ID NO: 58 (LSEDKLLACGEGAADIIIGHLCIRHEMTPVNPGV; SEQ ID NO: 59) may contain up to 3 amino acids altered from the 446-479 segment of SEQ ID NO: 58. One such example of sequence deviation in biologically active human AFP fragments is found in, e.g., U.S. Patent No. 5,707,963 (incorporated by reference herein), which discloses a 34-amino acid fragment of human AFP (SEQ ID NO: 59) with flexibility at two amino acid residues (amino acid 9 and 22 of SEQ ID NO: 59). Other examples of AFP polypeptide linker sequences include, e.g., amino acids 19-198 of SEQ ID NO: 58 (human AFP Domain I), amino acids 217-408 of SEQ ID NO: 58 (human AFP Domain II), amino acids 409-609 of SEQ ID NO: 58 (human AFP Domain III), amino acids 19-408 of SEQ ID NO: 58 (human AFP Domain I+II), amino acids 217-609 of SEQ ID NO: 58 (human AFP Domain II+III), and amino acids 285-609 of SEQ ID NO: 58 (human AFP Fragment I). In another embodiment, the human AFP polypeptide linker sequence is an 8-amino acid sequence that includes amino acids 489-496 (i.e., EMTPVNPG) of SEQ ID NO: 58.

A fourteenth aspect of the invention features kits that include any of the HSA linkers, HSA linker agents, or any other agents described in the first, second, third, fourth, fifth, eleventh, twelfth, and thirteenth aspects of the invention. The kits further include instructions to allow a practitioner (e.g., a physician, nurse, or patient) to administer the compositions and agents contained therein. In an embodiment, the kits include multiple packages of a single- or multi-dose pharmaceutical composition containing an effective amount of an agent of the invention, e.g,. an HSA linker of the invention that includes, e.g., one or more binding moieties (e.g., antibodies or antibody fragments (e.g., scFv)), diagnostic agents (e.g., radionuclide or chelating agents), and/or therapeutic agents (e.g., cytotoxic or immunomodulatory agents). Optionally, instruments or devices necessary for administering the pharmaceutical composition(s) may be included in the kits. For instance, a kit of this invention may provide one or more pre-filled syringes containing an effective amount of an HSA linker of the invention, or any binding, diagnostic, and/or therapeutic agent conjugated thereto. Furlhermore, the kits may also include additional components such as instructions or administration schedules for a patient suffering from a disease or condition (e.g., a cancer, autoimmune disease, or cardiovascular disease) to use the pharmaceutical composition(s) containing, e.g., an HSA linker of the invention, or any binding, diagnostic, and/or therapeutic agent conjugated thereto.

### DEFINITIONS

The term "antibody" as used interchangeably herein, includes whole antibodies or immunoglobulins and any antigen-binding fragment or single chains thereof. Antibodies, as used herein, can be mammalian (e.g., human or mouse), humanized, chimeric, recombinant, synthetically produced, or naturally isolated. In most mammals, including humans, antibodies have at least two heavy (H) chains and two light (L) chains connected by disulfide bonds. Each heavy chain consists of a heavy chain variable region (abbreviated herein as V_{H}) and a heavy chain constant region. The heavy chain constant region consists of three domains, C_{H}1, C_{H}2, and C_{H}3 and a hinge region between C_{H}1 and C_{H}2. Each light chain consists of a light chain variable region (abbreviated herein as V_{L}) and a light chain constant region. The light chain constant region consists of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (Clq) of the classical complement system. Antibodies of the present invention include all known forms of antibodies and other protein scaffolds with antibody-like properties. For example, the antibody can be a human antibody, a humanized antibody, a bispecific antibody, a chimeric antibody, or a protein scaffold with antibody-like properties, such as fibronectin or ankyrin repeats. The antibody also can be a Fab, Fab'2, scFv, SMIP, diabody, nanobody, aptamers, or a domain antibody. The antibody can have any of the following isotypes: IgG (e.g., IgG1, IgG2, IgG3, and IgG4), IgM, IgA (e.g., IgA1, IgA2, and IgAsec), IgD, or IgE. Antibodies that can be used as binding moieties, as defined herein, in combination with an HSA linker of the invention include, but are not limited to, natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, and panitumumab.

The term "antibody fragment," as used herein, refers to one or more fragments of an antibody that retain the ability to specifically bind to an antigen (e.g., ErbB2). The antigen-binding function of an antibody can be performed by fragments of a full-length antibody. Examples of binding fragments encompassed within the term "antigen-binding portion" of an antibody include but are not limited to: (i) a Fab fragment, a monovalent fragment consisting of the V_{L}, V_{H}, C_{L}, and C_{H}1 domains; (ii) a F(ab')₂ fragment, a bivalent fragment comprising two Fab fragments linked by a disulfide bridge at the hinge region; (iii) a Fd fragment consisting of the V_{H} and C_{H}1 domains; (iv) a Fv fragment consisting of the V_{L} and V_{H} domains of a single arm of an antibody, (v) a dAb including V_{H} and V_{L} domains; (vi) a dAb fragment (Ward et al., Nature 341:544-546 (1989)), which consists of a V_{H} domain; (vii) a dAb which consists of a V_{H} or a V_{L} domain; (viii) an isolated complementarity determining region (CDR); and (ix) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g.,* Bird et al., Science 242:423-426 (1988) and Huston et al., Proc. Natl. Acad. Sci. USA 85:5879-5883 (1988)). These antibody fragments are obtained using conventional techniques known to those with skill in the art, and the fragments are screened for utility in the same manner as are intact antibodies. Antibody fragments can be produced by recombinant DNA techniques, or by enzymatic or chemical cleavage of intact immunoglobulins.

By "autoimmune disease" is meant a disease in which an immune system response is generated against self epitopes or antigens. Examples of autoimmune diseases include, but are not limited to, alopecia areata, ankylosing spondylitis, antiphospholipid syndrome, autoimmune Addison's disease, autoimmune hemolytic anemia, autoimmune hepatitis, Behcet's disease, bullous pemphigoid, cardiomyopathy, celiac Sprue-dermatitis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, Crohn's disease, discoid lupus, essential mixed cryoglobulinemia, fibromyalgia-fibromyositis, Grave's disease, Guillain-Barré syndrome, Hashimoto's thyroiditis, hypothyroidism, idiopathic pulmonary fibrosis, idiopathic thrombocytopenia purpura (ITP), IgA nephropathy, insulin dependent diabetes, juvenile arthritis, lichen planus, lupus, Ménière's disease, mixed connective tissue disease, multiple sclerosis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arthritis, sarcoidosis, scleroderma, Sjögren's syndrome, Stiff-Man syndrome, systemic lupus erythematosus (SLE), Takayasu arteritis, temporal arteritis/giant cell arteritis, ulcerative colitis, uveitis (e.g., birdshot retinochoroidopathy uveitis and sarcoid uveitis), vasculitis, vitiligo, Wegener's granulomatosis, and myasthenia gravis.

By "binding moiety" is meant any molecule that specifically binds to a target epitope, antigen, ligand, or receptor. Binding moieties include but are not limited to antibodies (e.g., monoclonal, polyclonal, recombinant, humanized, and chimeric antibodies), antibody fragments (e.g., Fab fragments, Fab'2, scFv antibodies, SMIP, domain antibodies, diabodics, minibodies, scFv-Fc, affibodies, nanobodies, and domain antibodies), receptors, ligands, aptamers, and other molecules having a known binding partner.

By "biologically-active" is meant that a molecule, including biological molecules, such as nucleic acids, peptides, polypeptides, and proteins, exerts a physical or chemical activity on itself or other molecule. For example, a "biologically-active" molecule may possess, e.g., enzymatic activity, protein binding activity (e.g., antibody interactions), or cytotoxic activities are "biologically-active."

The term "chimeric antibody" refers to an immunoglobulin or antibody whose variable regions derive from a first species and whose constant regions derive from a second species. Chimeric antibodies can be constructed, for example, by genetic engineering, from immunoglobulin gene segments belonging to different species (e.g., from a mouse and a human).

By "connector" or "polypeptide connector" is meant an amino acid sequence of 2 to 20 residues in length that is covalently attached to one or both of the amino or carboxy termini of an HSA linker of the invention, or is covalently attached to one or more residues of an HSA linker of the invention (e.g., a residue between the amino and carboxy terminal residues). In preferred embodiments, the polypeptide connector attached to the amino terminus of an HSA linker has the amino acid sequence "AAS" or "AAQ" and the connector attached to the carboxy terminus has the amino acid sequence "AAAL" (SEQ ID NO:5).

The terms "effective amount" or "amount effective to" or "therapeutically effective amount" means an amount of an agent of the invention (e.g., an HSA linker bonded with one or more binding moieties or diagnostic or therapeutic agents with or without a connector sequence) sufficient to produce a desired result, for example, killing a cancer cell, reducing tumor cell proliferation, reducing inflammation in a diseased tissue or organ, or labeling a specific population of cells in a tissue, organ, or organism (e.g., a human).

The term "human antibody," as used herein, is intended to include antibodies, or fragments thereof, having variable regions in which both the framework and CDR regions arc derived from human germline immunoglobulin sequences as described, for example, by Kabat et al., (Sequences of proteins of Immunological Interest, Fifth Edition, U.S. Department of Health and Human Services, NIH Publication No. 91-3242 (1991)). Furthermore, if the antibody contains a constant region, the constant region also is derived from human germline immunoglobulin sequences. The human antibodies may include amino acid residues not encoded by human germline immunoglobulin sequences (e.g., mutations introduced by random or site-specific mutagenesis *in vitro* or by somatic mutation *in vivo).* However, the term "human antibody", as used herein, is not intended to include antibodies in which CDR sequences derived from the germline of another mammalian species, such as a mouse, have been grafted onto human framework sequences (i.e., a humanized antibody or antibody fragment).

The term "humanized antibody" refers to any antibody or antibody fragment that includes at least one immunoglobulin domain having a variable region that includes a variable framework region substantially derived from a human immunoglobulin or antibody and complementarity determining regions (e.g., at least one CDR) substantially derived from a non-human immunoglobulin or antibody.

As used herein, an "inflammatory signaling inhibitor" or "ISI" is an agent that decreases the binding between a pro-inflammatory cytokine (e.g., TNF-alpha, TNF-beta, or IL-1) and its receptor (e.g., TNF receptor 1 or 2, or IL-1 receptor, respectively); decreases the binding of activating molecules to pro-inflammatory cell surface signaling molecules (e.g., CD20, CD25, CTLA-4, CD80/CD86, or CD28); or decreases the downstream activation of, or activity of, intracellular signaling molecules that are activated following the binding of pro-inflammatory cytokines to their receptors or the binding of activating molecules to pro-inflammatory cell surface signaling molecules (e.g., an agent that decreases the activation of, or activity of, signaling molecules in the p38 MAPK signaling pathway). Preferably, the decrease in binding between a pro-inflammatory cytokine and its receptor, the decrease in binding of an activating molecule to a pro-inflammatory cell surface signaling molecule, or the decrease in intracellular signaling which occurs following the binding of pro-inflammatory cytokines to their receptors or activating molecules to pro-inflammatory cell surface signaling molecules is by at least about 10%, preferably by 20%, 30%, 40%, or 50%, more preferably by 60%, 70%, 80%, 90% or more (up to 100%). An ISI may act by reducing the amount of pro-inflammatory cytokine (e.g., TNF-alpha, TNF-beta, or IL-1) freely available to bind the receptor. For example, an ISI may be a soluble pro-inflammatory cytokine receptor protein (e.g., a soluble TNF receptor fusion protein such as etancrcept (ENBREL®) or lenercept), or a soluble pro-inflammatory cell surface signaling molecule (e.g., a soluble CTLA-4 (abatacept)), or an antibody directed against a pro-inflammatory cytokine or a pro-inflammatory cell surface signaling molecule (e.g., an anti-TNF antibody, such as adalimumab, certolizumab, inflixamab, or golimumab; an anti-CD20 antibody, such as rituximab; or TRU-015 (TRUBION®)). In addition, an ISI may act by disrupting the ability of the endogenous wild-type pro-inflammatory cytokine or the pro-inflammatory cell surface signaling molecule to bind to its receptor (e.g., TNF receptor 1 or 2, IL-1 receptor, or CD11a (e.g., efalizumab (RAPTIVA®, Genentech))). Examples of dominant-negative TNF-alpha variants are XENP345 (a pegylated version of TNF variant A145R/I97T) and XproTM1595, and further variants disclosed in U.S. Patent Application Publication Nos. 20030166559 and 20050265962, herein incorporated by reference. An example of a dominant negative IL-1 variant is anakinra (KINERET®), which is a soluble form of IL-1 that binds to the IL-1 receptor without activating intracellular signaling pathways. Inflammatory signaling inhibitors, which can be used in the present invention, are also small molecules which inhibit or reduce the signaling pathways downstream of pro-inflammatory cytokine or pro-inflammatory cell surface signaling molecules (e.g., DE 096). Examples of ISIs of this kind include inhibitors of p38 MAP kinase, e.g., 5-amino-2-carbonylthiopene derivatives (as described in WO 04/089929, herein incorporated); ARRY-797; BIRB 796 BS, (1-5-tert-butyl-2-p-tolyl-2H-pyrazol-3-yl)-3-[4-2(morpholin-4-yl-ethoxy)-naphtalen-1-yl]-urea); CHR-3620; CNI-1493; FR-167653 (Fujisawa Pharmaceutical, Osaka, Japan); ISIS 101757 (Isis Pharmaceuticals); ML3404; NPC31145; PD169316; PHZ1112; RJW67657, (4-(4-(4-fluorophenyl)-1-(3-phenylpropyl)-5-(4-pyridinyl)-1H-imidazol-2-yl)-3-butyn-1-ol; SCIO-469; SB202190; SB203580, (4-(4-fluorophenyl)-2-(4-methylsulfinylphenyl)-5-(4-pyridyl)1H-imidazole); SB239063, trans-1-(4-hydroxycyclohexyl)-4-(4-fluorophenyl-methoxypyridimidin-4-yl)imidazole; SB242235; SD-282; SKF-86002; TAK 715; VX702; and VX745. Furthermore, an ISI may interfere with the processing of a pro-inflammatory cytokine (e.g., TNF-alpha and TNF-beta) from its membrane bound form to its soluble form. Inhibitors of TACE are ISIs of this class. Examples of inhibitors of TACE include BB-1101, BB-3103, BMS-561392, butynyloxyphenyl β-sulfone piperidine hydroxomates, CH4474, DPC333, DPH-067517, GM6001, GW3333, Ro 32-7315, TAPI-1, TAPI-2, and TMI 005. Additional examples of ISIs include short peptides derived from the *E. coli* heat shock proteins engineered for disease-specific immunomodulatory activity (e.g., dnaJP1).

By "integrin antagonist" is meant any agent that reduces or inhibits the biological activity of an integrin molecule (e.g., a reduction or inhibition of at least 10%, 20%, 30%, 40%, 50%, 60%, 70%, 80%, 90%, 95%, 99%, or more relative to the biological activity in the absence of the integrin antagonist), such as the α4 subunit of an integrin molecule. The agent may act directly or indirectly on the α4 integrin subunit (NCBI Accession No. P13612; Takada et al., EMBO J. 8:1361-1368 (1989)) by inhibiting the activity or expression of the α4 integrin subunit, or may act on a target to which the intact integrin containing an α4 subunit binds. For example, an antibody or blocking peptide that binds to vascular cell adhesion molecule-1 (VCAM-1), thus preventing the binding of α4β1 integrin to VCAM-1 is considered an integrin antagonist for purposes of the present invention. Non-limiting exemplary integrin antagonists suitable for use with the present invention may include proteins, blocking peptides, antibodies, such as natalizumab (TYSABRI®), and small molecule inhibitors. Examples of α4 integrin antagonists include, but are not limited to, natalizumab (Elan/Biogen Idec; see, e.g., U.S. Patent Nos. 5,840,299; 6,033,665; 6,602,503; 5,168,062; 5,385,839; and 5,730,978; incorporated by reference herein), oMEPUPA-V (Biogen; U.S. Patent No. 6,495,525; incorporated by reference herein), alefacept, CDP-323 (Celltech); firategrast (SB-68399; GlaxoSmithKline); TR-9109 (Pfizer); ISIS-107248 (Antisense Therapeutics); R-1295 (Roche); and TBC-4746 (Schering-Plough). Additional non-limiting examples of α4 integrin antagonists include the small molecules described in U.S. Patent Nos. 5,821,231; 5,869,448; 5,936,065; 6,265,572; 6,288,267; 6,365,619; 6,423,728; 6,426,348; 6,458,844; 6.479,666; 6,482,849; 6,596,752; 6,667,331; 6,668,527; 6,685,617; 6,903,128; and 7,015,216 (each herein incorporated by reference); in U.S. Patent Application Publication Nos. 2002/0049236; 2003/0004196; 2003/0018016; 2003/0078249; 2003/0083267; 2003/0100585; 2004/0039040; 2004/0053907; 2004/0087574; 2004/0102496; 2004/0132809; 2004/0229858; 2006/0014966; 2006/0030553; 2006/0166866; 2006/0166961; 2006/0241132; 2007/0054909; and 2007/0232601 (each herein incorporated by reference); in European Patent Nos. EP 0842943; EP 0842944; EP 0842945; EP 0903353; and EP 0918059; and in PCT Publication Nos. WO 95/15973; WO 96/06108: WO 96/40781; WO 98/04247; WO 98/04913; WO 98/42656; WO 98/53814; WO 98/53817; WO 98/53818; WO 98/54207; WO 98/58902; WO 99/06390; WO 99/06431; WO 99/06432; WO 99/06433; WO 99/06434; WO 99/06435; WO 99/06436; WO 99/06437; WO 99/10312; WO 99/10313; WO 99/20272; WO 99/23063; WO 99/24398; WO 99/25685; WO 99/26615; WO 99/26921; WO 99/26922; WO 99/26923; WO 99/35163; WO 99/36393; WO 99/37605; WO 99/37618; WO 99/43642; WO 01/42215; and WO 02/28830; all of which are incorporated by reference herein. Additional examples of α4 integrin antagonists include the phenylalanine derivatives described in: U.S. Patent Nos. 6,197,794; 6,229,011; 6,329,372; 6,388,084; 6,348,463; 6,362,204; 6,380,387; 6,445,550; 6,806,365; 6,835,738; 6,855,706; 6,872,719; 6,878,718; 6,911,451; 6,916,933; 7,105,520; 7,153,963; 7,160,874; 7,193,108; 7,250,516; and 7,291,645 (each herein incorporated by reference). Additional amino acid derivatives that are α4 integrin antagonists include those described in, e.g., U.S. Patent Application Publication Nos. 2004/0229859 and 2006/0211630 (herein incorporated by reference), and PCT Publication Nos. WO 01/36376; WO 01/47868; and WO 01/70670; all of which are incorporated by reference herein. Other examples of α4 integrin antagonists include the peptides, and the peptide and semi-peptide compounds described in, e.g., PCT Publication Nos. WO 94/15958; WO 95/15973; WO 96/00581; WO 96/06108; WO 96/22966 (Leu-Asp-Val tripeptide; Biogen, Inc.); WO 97/02289; WO 97/03094; and WO 97/49731. An additional example of an α4 integrin antagonist is the pegylated molecule described in U.S. Patent Application Publication No. 2007/066533 (herein incorporated by reference). Examples of antibodies that arc α4 integrin antagonists include those described in, e.g., PCT Publication Nos. WO 93/13798; WO 93/15764; WO 94/16094; and WO 95/19790. Additional examples of α4 integrin antagonists are described herein.

By "interferon" is meant a mammalian (e.g., a human) interferon -alpha, -beta, -gamma, ortau polypeptide, or biologically-active fragment thereof, e.g., IFN-α (e.g., IFN-α-1a; see U.S. Patent Application No. 20070274950, incorporated herein by reference in its entirety), IFN-α-1b, IFN-α-2a (see PCT Application No. WO 07/044083, herein incorporated by reference in its entirety), and IFN-α-2b), IFN-β (e.g., described in U.S. Patent No. 7,238,344, incorporated by reference in its entirety; IFN-b-1a (AVONEX® and REBIF®), as described in U.S. Patent No. 6,962,979, incorporated by reference in its entirety, and IFN-β-1b (BETASERON®, as described in U.S. Patent Nos. 4,588,585; 4,959,314; 4,737,462; and 4,450,103; incorporated by reference in their entirety), IFN-g, and IFN-t (as described in U.S. Patent No. 5,738,845 and U.S. Patent Application Publication Nos. 20040247565 and 20070243163; incorporated by reference in their entirety).

By "HSA linker conjugate" is meant a human serum albumin (HSA) linker protein of the invention in combination with one or more binding moieties, polypeptide connectors, diagnostic agents, or therapeutic agents.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. Monoclonal antibodies can be prepared using any art recognized technique and those described herein such as, for example, a hybridoma method, as described by Kohler et al., Nature 256:495 (1975), a transgenic animal *(e.g.,* Lonberg et al., Nature 368(6474):856-859 (1994)), recombinant DNA methods *(e.g.,* U.S. Pat. No. 4,816,567), or using phage, yeast, or synthetic scaffold antibody libraries using the techniques described in, for example, Clackson et al., Nature 352:624-628 (1991) and Marks et al., J. Mol. Biol. 222:581-597 (1991).

By "pharmaceutically acceptable carrier" is meant a carrier which is physiologically acceptable to the treated mammal while retaining the therapeutic properties of the compound with which it is administered. One exemplary pharmaceutically acceptable carrier is physiological saline. Other physiologically acceptable carriers and their formulations are known to one skilled in the art and described, for example, in Remington's Pharmaceutical Sciences, (18th edition), ed. A. Gennaro, 1990, Mack Publishing Company, Easton, PA incorporated herein by reference.

By "proliferative disease" or "cancer" is meant any condition characterized by abnormal or unregulated cell growth. Examples of proliferative diseases include, for example, solid tumors such as: sarcomas (e.g., clear cell sarcoma), carcinomas (e.g., renal cell carcinoma), and lymphomas; tumors of the breast, colon, rectum, lung, oropharynx, hypopharynx, esophagus, stomach, pancreas, liver, bilecyst, bile duct, small intestine, urinary system (including the kidney, bladder, and epithelium of the urinary tract), female genital system (including the uterine neck, uterus, ovary, chorioma, and gestational trophoblast), male genital system (including the prostate, seminal vesicle, and testicles), endocrine glands (including the thyroid gland, adrenal gland, and pituitary body), skin (including angioma, melanoma, sarcoma originating from bone or soft tissue, and Kaposi's sarcoma), brain and meninges (including astrocytoma, neuroastrocytoma, spongioblastoma, retinoblastoma, neuroma, neuroblastoma, neurinoma and neuroblastoma), nerves, eyes, hemopoietic system (including chloroleukemia, plasmacytoma and dermal T lymphoma/leukemia), and immune system (including lymphoma, e.g., Hodgkin's lymphoma and non-Hodgkin's lymphoma). An example of a non-solid tumor proliferative disease is leukemia (e.g., acute lymphoblastic leukemia).

The term "recombinant antibody," refers to an antibody prepared, expressed, created, or isolated by recombinant means, such as (a) antibodies isolated from an animal (e.g., a mouse) that is transgenic or transchromosomal for immunoglobulin genes (e.g., human immunoglobulin genes) or a hybridoma prepared therefrom, (b) antibodies isolated from a host cell transformed to express the antibody, e.g., from a transfcctoma, (c) antibodies isolated from a recombinant, combinatorial antibody library (e.g., containing human antibody sequences) using phage, yeast, or synthetic scaffold display, and (d) antibodies prepared, expressed, created, or isolated by any other means that involve splicing of immunoglobulin gene sequences (e.g., human immunoglobulin genes) to other DNA sequences.

By "specifically bind" is meant the preferential association of a binding moiety (e.g., an antibody, antibody fragment, receptor, ligand, or small molecule portion of an agent of the invention) to a target molecule (e.g., a secreted target molecule, such as a cytokine, chemokine, hormone, receptor, or ligand) or to a cell or tissue bearing the target molecule (e.g., a cell surface antigen, such as a receptor or ligand) and not to non-target cells or tissues lacking the target molecule. It is recognized that a certain degree of non-specific interaction may occur between a binding moiety and a non-target molecule (present alone or in combination with a cell or tissue). Nevertheless, specific binding may be distinguished as mediated through specific recognition of the target molecule. Specific binding results in a stronger association between the binding moiety (e.g., an antibody) and e.g., cells bearing the target molecule (e.g., an antigen) than between the binding moiety and e.g., cells lacking the target molecule. Specific binding typically results in greater than 2-fold, preferably greater than 5-fold, more preferably greater than 10-fold and most preferably greater than 100-fold increase in amount of bound binding moiety (per unit time) to e.g., a cell or tissue bearing the target molecule or marker as compared to a cell or tissue lacking that target molecule or marker. Binding moieties bind to the target molecule or marker with a dissociation constant of e.g., less than 10⁻⁶M, more preferably less than 10⁻⁷M, 10⁻⁸M, 10⁻⁹M, 10⁻¹⁰M, 10⁻¹¹M, or 10⁻¹²M, and most preferably less than 10⁻¹³M, 10⁻¹⁴M, or 10⁻¹⁵M. Specific binding to a protein under such conditions requires a binding moiety that is selected for its specificity for that particular protein. A variety of assay formats are appropriate for selecting binding moieties (e.g., antibodies) capable of specifically binding to a particular target molecule. For example, solid-phase ELISA immunoassays are routinely used to select monoclonal antibodies specifically immunoreactive with a protein. See Harlow & Lane, Antibodies, A Laboratory Manual, Cold Spring Harbor Publications, New York (1988), for a description of immunoassay formats and conditions that can be used to determine specific immunoreactivity.

The term "substantial identity" or "substantially identical," when used in the context of comparing a polynucleotide or polypeptide sequence to a reference sequence, means that the polynucleotide or polypeptide sequence is the same as the reference sequence or has a specified percentage of nucleotides or amino acid residues that are the same at the corresponding locations within the reference sequence when the two sequences are optimally aligned. For instance, an amino acid sequence that is "substantially identical" to a reference sequence has at least about 60% identity, preferably 65%, 70%, 75%, 80%, 85%, 90%, 91%, 92%, 93%, 94%, 95%, 96%, 97%, 98%, 99%, or higher percentage identity (up to 100%) to the reference sequence (e.g., the HSA amino acid sequence as set forth in SEQ ID NO:1, or a fragment thereof), when compared and aligned for maximum correspondence over the full length of the reference sequence as measured using a BLAST or BLAST 2.0 sequence comparison algorithms with default parameters, or by manual alignment and visual inspection (see, e.g., NCBI web site).

By "surface-exposed amino acid residue" or "surface-exposed" is meant an amino acid residue that is present on the exterior face of the folded and conformationally-correct tertiary structure of a HSA polypeptide. Such residues can be substituted with e.g., other, chemically-reactive, amino acids (e.g., cysteine) to allow for site-specific conjugation of diagnostic or therapeutic agents. Additionally, surface-exposed amino acid residues can be substituted to allow (e.g., by addition of serine, threonine, or asparagine residues, or glycosylation motifs) or prevent (e.g., by removal of serine, threonine, or asparagine residues, or glycosylation motifs) glycosylation. Surface-exposed amino acid residues include, but are not limited to, threonine at position 496, serine at position 58, threonine at position 76, threonine at position 79, threonine at position 83, threonine at position 125, threonine at position 236, serine at position 270, serine at position 273, serine at position 304, serine at position 435, threonine at position 478, threonine at position 506, and threonine at position 508 (amino acid numbering is relative to e.g., the sequence of the HSA linker set forth in SEQ ID NO:1). Other surface-exposed residues can be identified by the skilled artisan using the HSA crystal structure (Sugio et al., "Crystal structure of human serum albumin at 2.5 A resolution," Protein Eng. 12:439-446 (1999)).

A "target molecule" or "target cell" is meant a molecule (e.g., a protein, epitope, antigen, receptor, or ligand) or cell to which a binding moiety (e.g., an antibody), or an HSA conjugate that contains one or more binding moieties (e.g., an HSA linker bonded to one or more antibodies or antibody fragments) can specifically bind. A target molecule can be present on the exterior of a target cell (e.g., a cell-surface or secreted protein) or in the interior of a target cell.

By "treating" is meant the reduction (e.g., by at least 10%, 15%, 20%, 25%, 30%, 35%, 40%, 45%, 50%, 60%, 70%, 80%, 90%, 95%, 99%, or even 100%) in the progression or severity of a human disease or disorder (e.g., an autoimmune or proliferative disease), or in the progression, severity, or frequency of one or more symptoms of the human disease or disorder in a human patient.

### BRIEF DESCRIPTION OF THE DRAWINGS

**Figure 1** is an illustration showing the schematic representation of an exemplary HSA linker conjugate. The connector between the amino terminal binding moiety and the HSA linker has a sequence of alanine, alanine and serine. The connector between the HSA linker and the carboxy terminal binding moiety has a sequence of alanine, alanine, alanine, leucine (SEQ ID NO:5).
**Figure 2** is a graph showing that B2B3 variants inhibit HRG-induced pErbB3 in ZR75-1 breast cancer cells.
**Figures 3A-D** arc graphs showing the inhibition of phosphorylated ErbB3 in BT474 breast cancer cells following 24 hour pre-treatment with the B2B3 variants A5-HSA-B1D2 (Figure 3A), H3-HSA-B1D2 (Figure 3B), H3-HSA-F5B6H2 (Figure 3C), and F4-HSA-F5B6H2 (Figure 3D).
**Figures 4A-D** are graphs showing the inhibition of phosphorylated AKT in BT474 breast cancer cells following 24 hour pre-treatment with the B2B3 variants A5-HSA-B1D2 (Figure 4A), H3-HSA-B1D2 (Figure 4B), H3-HSA-F5B6H2 (Figure 4C), and F4-HSA-F5B6H2 (Figure 4D).
**Figures 5A-D** are graphs showing that inhibition of phosphorylated ERK in BT474 breast cancer cells following 24 hour pre-treatment with the B2B3 variants A5-HSA-B1D2 (Figure 5A), H3-HSA-B1D2 (Figure 5B), H3-HSA-F5B6H2 (Figure 5C), and F4-HSA-F5B6H2 (Figure 5D).
**Figure 6** is a graph showing that treatment of BT474 breast cancer cells with B2B3-1 variants causes G1 arrest and a decrease in the number of cells in S phase.
**Figure 7** is a flow cytometry histogram showing that pre-incubation of BT-474-M3 cells with 1 µM B2B3-1 substantially blocks binding of HRG.
**Figures 8A-D** are graphs showing that B2B3-1 inhibits ErbB3 and AKT phosphorylation in B-T474-M3 and ZR75-30 cell lines. Breast cancer cell lines BT-474-M3 (Figures 8A and 8C) and ZR75-3 0 (Figures 8B and 8D) were pre-treated with a dose titration of B2B3-1 for 24 hours and then stimulated for 10 minutes with 5 nM of HRG 1β EGF domain. The phosphorylation status of ErbB3 and AKT was then examined using an ELISA assay.
**Figure 9** is a photograph of a Western blot that shows the effect of B2B3-1 treatment on signaling proteins in BT474 breast cancer cells.
**Figure 10** is a photograph of a Western blot that shows the immunoprecipitation of B2B3-1 treated BT474 breast cancer cells.
**Figures 11A**-**C** are graphs showing B2B3-1 treatment of BT-474 cell line causes G1 arrest and a decrease in the population of cells in S phase (Figure 11A), and inhibits colony formation in both BT-474 and SKBr3 cells compared to untreated cells (Figure 11B). In addition, B2B3-1 inhibits proliferation of BT-474-M3 cells in a cell impedance assay (Figure 11C).
**Figure 12** is a graph showing that B2B3-1 does not stimulate ErbB3 phosphorylation in ZR75-1 cells.
**Figures 13A-B** are graphs showing that B2B3-1 binds specifically to ErbB3 (Figure 13A) and ErbB2 (Figure 13B).
**Figure 14** is a graph showing that avidity binding of B2B3-1 to MALME-3 cells results in a significant increase in apparent binding affinity compared to ErbB2-only binding variant, SKO-3, and ErbB3-only binding variant, SKO-2.
**Figures 15A-C** are graphs showing the stability of B2B3-1 in mouse, Cynomolgus monkey, and human serum. 100 nM B2B3-1 was incubated in mouse (Figure 15A), Cynomolgus monkey (Figure 15B), or human (Figure 15C) serum at 37°C for a period of 120 hours. Samples were removed at 0, 24, 48, 72, 96 and 120 hours and the ability of B2B3-1 to bind both ErbB2 and ErbB3 was measured by ELISA (RLU = relative light units).
**Figures 16** is a graph showing B2B3-1 dose response in a BT-474-M3 breast cancer xenograft model. The relationship of B2B3-1 dose on tumor response was assessed in the BT-474-M3 breast tumor line at the doses indicated. HSA was given at 52.5 mg/kg, which is an equimolar dose to the 90 mg/kg B2B3-1 dose.
**Figures 17A-E** are graphs showing that B2B3-1 is effective in multiple xenograft models in an ErbB2 dependent manner. Calu-3 (human lung adenocarcinoma; Figure 17A), SKOV-3 (human ovarian adenocarcinoma; Figure 17B), NCI-N87 (human gastric carcinoma; Figure 17C), ACHN (human kidney adenocarcinoma; Figure 17D), and MDA-MB-361 (human breast adenocarcinoma; Figure 17E) xenograft model arc shown. Mice were treated with 30 mg/kg of B2B3-1 every 3 days or HAS control at an equimolar dose to B2B3-1.
**Figures 18A-B** are graphs showing that the over-expression of ErbB2 converts B2B3-1 non-responder ADRr breast cancer xenograft model into a responder. ErbB2 was over-expressed in wild type ADRr xenografts (Figure 18A) and ADRr-E2 xenografts (Figure 18B) using a retroviral expression system.
**Figures 19A-B** are graphs showing that B2B3-1 activity correlates positively with ErbB2 expression levels *in vitro* (Figure 19A) and *in vivo* (Figure 19B).
**Figure 20A-B** show that B2B3-1 treatment modifies tumor cell cycling. Figure 20A includes fluorescent micrographs showing that B2B3-1 treatment of BT474-M3 breast tumor cells for 6 hours results in translocation of cell cycle inhibitor p27^{kip1} to the nucleus. Hoechst stain was used to identify the nucleus. Figure 20B is a Western blot of BT-474-M3 cells treated with B2B3-1 for 72 hours, which resulted in a decrease in the levels of the cell cycle regulator Cyclin DI. The cytoskeleton protein vinculin was probed as a protein loading control in this experiment.
**Figures 21A-B** are micrographs showing that B2B3-1 treatment of BT474 breast tumor xenografts results in translocation of p27^{kip1} to the nucleus. BT474 breast tumor xenografts were treated with B2B3-1 (Figure 21A) at a dose of 30 mg/kg or an equimolar dose of HSA (Figure 21B) every 3 days for a total of 4 doses and stained for p27^{kip1}.
**Figure 22A-B** are fluorescent micrographs showing that B2B3-1 treatment results in a reduction of the proliferation marker Ki67 in BT474-M3 breast cancer xenograft. BT474-M3 breast tumor xenografts were treated with B2B3-1 (Figure 22A) at a dose of 30 mg/kg or an equimolar dose of HSA (Figure 22B) every 3 days for a total of 4 doses.
**Figures 23A-B** are fluorescent micrographs showing that B2B3-1 treatment results in a reduction of vessel density in BT474-M3 breast cancer xenograft tumors (as determined by a reduction in CD31 staining). BT474-M3 breast tumor xenografts were treated with B2B3-1 (Figure 23A) at a dose of 30 mg/kg or an equimolar dose of HSA (Figure 23B) every 3 days for a total of 4 doses.
**Figures 24A-B** are graphs showing that B2B3-1 inhibits phosphorylation *of ErbB3 in vivo.* Lysates from individual BT-474-M3 xenograft tumors treated with B2B3-1 (M1-M5) or control HSA (H1-H2) were subjected to SDS-PAGE and probed for pErbB3 and beta tubulin using Western blot analysis (Figure 24A). Normalization of the mean pErbB3 signal to the mean beta tubulin signal demonstrated that B2B3-1 treated tumors contained significantly less pErbB3 than HSA tumors (Figure 24B).

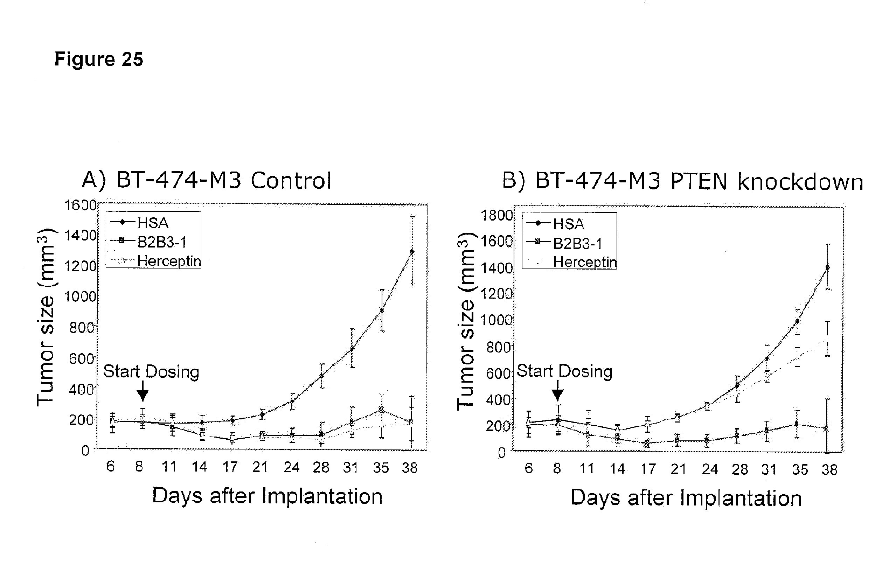

**Figures 25A and B** are graphs showing the *in vivo* activity of B2B3-1 in BT-474-M3 xenografts which have reduced PTEN activity. Cultured BT-474-M3 tumor cells were transfected with a control vector (Figure 25A) or with a retroviral vector encoding shPTEN (Figure 25B), which knocks out PTEN activity. BT-474-M3 breast cancer cells engineered to knock out PTEN activity were injected into the right flank of mice, while cells transfected with control vector were injected into the left flank of the same mouse. Mice were treated with 30mg/kg B2B3-1 every 3 days or 10mg/kg Herceptin every week and HSA was injected as a control at an equimolar dose to B2B3-1. B2B3-1 and Herceptin promoted a reduction in the size of tumors formed by control BT-474-M3 breast cancer cells (Figure 25A), whereas only B2B3-1 (and not Herceptin) promoted a reduction in the size of tumors formed by BT-474-M3 breast cancer cells lacking expression of PTEN (Figure 25B).
**Figures 26A-B** show that B2B3-1 inhibits phosphorylation of AKT in BT-474-M3 xenografts that have reduced PTEN activity. Tumors were lysed following the completion of treatment (q3dx11) and tested for PTEN, pErbB3, and pAKT expression levels by Western blot analysis (Figure 26A). Densitometry on the band intensity for pAKT normalized to total AKT and total protein demonstrate that B2B3-1 was able to inhibit phosphorylation of this protein, when Herceptin did not (Figure 26B).
**Figures 27A-D** are graphs showing the single dose pharmacokinetic properties of 5 (Figure 27A), 15 (Figure 27B), 30 (Figure 27C), and 45 (Figure 27D) mg/kg bolus dose of B2B3-1 in nu/nu mice. B2B3-1 serum concentrations are comparable measured by the HSA assay or ErbB2/ErbB3 assay, indicating that the antigen binding activity of B2B3-1 is retained in circulation.
**Figure 28** is a graph showing the dose-exposure relationship for 5, 15, 30, and 45 mg/kg bolus doses of B2B3-1 in nude mice. Increases in dose result in a linear increase in overall exposure to B2B3-1.
**Figure 29** is a graph showing the B2B3-1 serum concentrations measured in Cynomolgus monkeys dosed every three days for 4 doses with 4 mg/kg (n=2), 20 mg/kg (n=2) and 200 mg/kg (up to 336 hour n=4, for 384, 552 and 672 hour time points n=2).
**Figure 30** is an illustration of the B2B3-1 expression plasmid pMP10k4H3-mHSA-B1D2.
**Figure 31** is an illustration of the neomycin resistance plasmid pSV2-neo.
**Figure 32** is an illustration of the hygromycin resistance plasmid pTK-IIyg.

### DETAILED DESCRIPTION OF THE INVENTION

This invention provides a human serum albumin (HSA) linker which, when used to produce an agent of the invention (e.g., a binding, diagnostic, or therapeutic agent) exhibits, for example, an increased *in vivo* half-life of between 6 hours and 7 days and which does not induce significant humoral or cell-mediated immune responses when administered *in vivo* to a mammal (e.g., a human). In one aspect, the invention provides a mutated HSA linker that has two defined amino acid substitutions (i.e., the "C34S" and "N503Q" substitutions, as set forth in SEQ ID NO:1). In another aspect, the invention provides an HSA linker bonded to one or more binding moieties (e.g., antibodies, antibody fragments, receptor/ligands, or small molecules) for diagnostic or therapeutic applications in a mammal (e.g., a human) *in vivo* or for use *in vitro* in connection with mammalian cells, tissues, or organs. In a further aspect, the HSA linker may be coupled to one or more immunomodulatory agents, cytotoxic or cytostatic agents, detectable labels, or radioactive agents for diagnostic or therapeutic applications in a mammal (or in connection with a mammalian cell, tissue, or organ). An agent of the invention, which includes the HSA linker, can be optionally combined with one or more pharmaceutically acceptable carriers or excipients and can be formulated to be administered intravenously, intramuscularly, orally, by inhalation, parenterally, intraperitoneally, intraarterially, transdermally, sublingually, nasally, through use of suppositories, transbuccally, liposomally, adiposally, opthalmically, intraocularly, subcutaneously, intrathecally, topically, or locally. An agent of the invention which includes the HSA linker can be combined or coadministered with one or more biologically-active agents (e.g., biological or chemical agents, such as chemotherapeutics and antineoplastic agents). In a further aspect, the invention provides a kit, with instructions, for the conjugation of binding moieties (e.g., antibodies, antibody fragments, receptors or ligands), immunomodulatory agents, cytotoxic or cytostatic agents, detectable labels, or radioactive agents to the HSA linker of the invention to prepare agents of the invention that can be used for diagnostic or therapeutic applications.

### A Mutated Human Serum Albumin (HSA) Linker

A mutated HSA linker of the invention contains two amino acid mutations, at positions 34 and 503, relative to the wild-type HSA amino acid sequence set forth in SEQ ID NO:3. The cysteine residue at position 34 (i.e., C34) can be mutated to any amino acid residue other than cysteine (e.g., serine, threonine, or alanine). Likewise, the asparagine residue at position 503 (i.e., N503) can be mutated to any amino acid residue other than asparagine (e.g., glutamine, serine, histidine, or alanine). In one embodiment, the HSA linker of the invention has the the amino acid and corresponding nucleotide sequence set forth in SEQ ID NOS:1 and 2, respectively. This mutated HSA linker of the invention contains two amino acid substitutions (i.e., serine for cysteine at amino acid residue 34 ("C34S") and glutamine for asparagine at amino acid residue 503 ("N503Q")). The HSA linker, when bonded to one or more binding moieties (e.g., antibodies, antibody fragments (e.g., single chain antibodies), or other targeting or biologically active agents (e.g., receptors and ligands)), confers several beneficial pharmacologic properties to those conjugates and to additional diagnostic or therapeutic agents also conjoined (e.g., immunomodulatory agents, cytotoxic or cytostatic agents, detectable labels, or radioactive agents)) relative to the pharmacologic properties of these agents in the absence of the HSA linker of the invention. These benefits can include decreased immunogenicity (e.g., decreased host antibody neutralization of linker-antibody conjugates), increased detection of HSA linker conjugates (e.g., by mass spectroscopy) and increased pharmacologic half-life (e.g., a half-life greater than 6 hours, 8 hours, 12 hours, 24 hours, 36 hours, 2 days, 3 days, 4 days, 5 days, 6 days, or 7 days) when administered to a mammal (e.g., a human). Specifically, the substitution of serine for cysteine at amino acid residue 34 results in reduced oxidation and protein heterogeneity of the HSA linker. In wild-type HSA, the asparagine at amino acid residue 503 is sensitive to deamination, likely resulting in reduced pharmacologic half-life. The substitution of glutamine for asparagine at amino acid residue 503 can result in increased pharmacologic half-life of the HSA linker of the invention, and correspondingly, of conjugate agents that include the HSA linker when administered to a mammal (e.g., a human) or cells, tissues, or organs thereof.

In other embodiments, the mutated HSA linker of the invention includes domain I of HSA (SEQ ID NO:53; residues 1-197 of SEQ ID NO:1), domain III of HSA (SEQ ID NO:55; residues 381-585 of (SEQ ID NO:1), combination of domains I and III of HSA, or a combination of domain I or III of HSA with domain II of HSA (SEQ ID NO:54; residues 189-385 of SEQ ID NO:1). For example, an HSA linker of the invention can include domains I and IT, I and III, or 11 and III. In addition, the cysteine residue at position 34 (i.e., C34) of domain I (SEQ ID NO:53) can be mutated to any amino acid residue other than cysteine (e.g., serine, threonine, or alanine). Likewise, the asparagine residue at position 503 (i.e., N503) of domain III (SEQ ID NO:55) can be mutated to any amino acid residue other than asparagine (e.g., glutamine, serine, histidine, or alanine). These HSA linkers can be incorporated into an HSA linker conjugate of the invention, which includes one or more of a polypeptide connector, a binding moiety, and therapeutic or diagnostic agents, each of which is described in detail below.

### Polypeptide Connectors

To facilitate the conjugation of binding moieties, as defined herein, to the HSA linker of the invention, short (e.g., 2-20 amino acids in length) polypeptide connectors that can be bonded (e.g, covalently (e.g., a peptidic bond), ionically, or hydrophobically bonded, or via a high-affinity protein-protein binding interaction (e.g., biotin and avidin)) to the amino or carboxy termini of an HSA linker. These connectors provide flexible tethers to which any of the binding moieties described herein can be attached. A polypeptide connector may be 2, 3, 4, 5, 6, 7, 8, 9, 10, 11, 12, 13, 14, 15, 16, 17, 18, 19, 20 or more amino acids in length. In one embodiment, the connector is a sequence of, e.g., glycine, alanine, serine, glutamine, leucine, or valine residues. Although not specifically enumerated herein, the connector of the invention can be solely glycine, alanine, serine, glutamine, leucine, or valine residues, or it may be any combination of these residues up to about 20 amino acids in length. In a preferred embodiment, the connector attached to the amino terminus of an HSA linker has the amino acid sequence "AAS" or "AAQ" and the connector attached to the carboxy terminus has the amino acid sequence "AAAL" (SEQ ID NO:5). The connector can be covalently bound to the amino or carboxy terminal residue of the HSA linker, to an amino acid residue within the HSA linker, or can be included between one or more binding moieties, if present.

### HSA Linker Manufacture

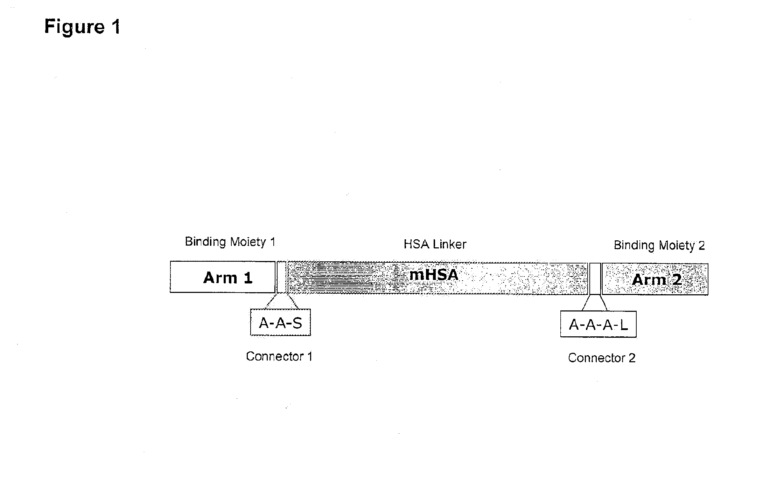

The HSA linker of the invention, which may optionally include the peptide connectors described above, one or more of the binding moieties described below, polypeptide-based detectable labels, and other polypeptide-based therapeutic agents, can be produced recombinantly. For example, a nucleotide sequence encoding the HSA linker (and one or more of the optional elements) may be expressed (e.g., in a plasmid, viral vector, or transgenically) *in* a bacterial (e.g., *E. coli),* insect, yeast, or mammalian cell (e.g., a CHO cell), or a mammalian tissue, organ, or organism (e.g., a transgenic rodent, ungulate (e.g., a goat), or non-human primate). After expression of the HSA linker in the host cell, tissue, or organ, the skilled artisan may isolate and purify the HSA linker using standard protein purification methods (e.g., FPLC or affinity chromatography). A recombinant expression system for the production of an HSA linker in combination with two binding moieties is illustrated in Figure 1.

The HSA linker of the invention, which may include one or more of the optional elements described above, can be synthetically produced. For example, the HSA linker can be prepared by techniques generally established in the art of peptide synthesis, such as the solid-phase approach. Solid-phase synthesis involves the stepwise addition of amino acid residues to a growing peptide chain that is linked to an insoluble support or matrix, such as polystyrene. The C-terminus residue of the peptide is first anchored to a commercially available support with its amino group protected with an N-protecting agent such as a t-butyloxycarbonyl group (tBoc) or a fluorenylmethoxycarbonyl (FMOC) group. The amino-protecting group is removed with suitable deprotecting agents such as TFA in the case of tBOC or piperidine for FMOC and the next amino acid residue (in N-protected form) is added with a coupling agent such as dicyclocarbodiimide (DCC). Upon formation of a peptide bond, the reagents are washed from the support. After addition of the final residue, the agent is cleaved from the support with a suitable reagent, such as trifluoroacetic acid (TFA) or hydrogen fluoride (HF). If desired, the HSA linker, which may include one or more of the optional elements described above, can be manufactured in one, two, three, or more segments, which can then be ligated to form the whole HSA linker construct.

### HSA Linker Binding Moieties

The HSA linker of the invention can also be prepared as a construct that includes one or more binding moieties, such as antibodies, antibody fragments (as defined herein, e.g., a single chain Fv (scFv)) or receptor/ligands (i.e., protein or glycoprotein ligands or receptors) that allow selective and specific binding of the HSA linker construct to a target cell, tissue, or organ. The binding moieties can be bonded to the HSA linker (e.g., via a covalent (e.g., a peptide bond), ionic, or hydrophobic bond, or via a high-affinity protein-protein binding interaction (e.g., biotin and avidin)). As is discussed above, an HSA linker of the invention that includes one or more polypeptide connectors or binding moieties can be recombinantly or synthetically produced.

One or more binding moieties can be bonded to an HSA linker of the invention. In one embodiment, two or more identical binding moieties (i.e., moieties having the same structure and binding affinities) are bonded to an HSA linker, one or more (e.g., in tandem) each at the amino and carboxy termini, the HSA linker thereby affording improved avidity of the binding moieties for their target antigen. Alternatively, two or more different binding moieties (e.g., an antibody, such as a scFv, with binding affinities for two or more different target molecules, or scFv with binding affinities for two or more different epitopes on the same target molecule) can be bonded to an HSA linker (e.g., a bispecific HSA linker conjugate) to allow multiple target antigens or epitopes to be bound by the HSA linker conjugate. In another embodiment, different species of binding moieties can also be bonded to an HSA linker to bestow, for example, two or more different binding specificities or agonistic/antagonistic biological properties on the linker conjugate. Useful combinations of binding moiety pairs for use in the preparation of bispecific HSA linker conjugates are disclosed in, e.g., International Patent Application Publications WO 2006/091209 and WO 2005/117973, herein incorporated by reference. In other embodiments, more than two binding moieties (e.g., the same or different binding moieties) can be bonded to an HSA linker to form an agent of the invention.

The invention features an agent having at least first and second binding moieties, each of which can be bound at either the amino or carboxy terminus of the HSA linker protein, or to polypeptide connectors, as defined herein, present at either or both termini. Figure 1 illustrates an exemplary mutated HSA linker of the invention in which two binding moieties ("arm 1" and "arm 2") are bonded to the mutated HSA linker by the amino terminal polypeptide connector AAS and carboxy terminal polypeptide connector AAAL (SEQ ID NO:5). Binding moieties (e.g., an antibody or scFv) can also be bound to other loci (e.g., internal amino acid residues of the HSA linker), for example, covalently or ionically, e.g., using biotin-avidin interactions. Biotinylation of amine (e.g., lysine residues) and sulfhydryl (e.g., cysteine residues) amino acid side chains is known in the art and can be used to attach binding moieties to the HSA linker.

Binding moieties that can be included in an HSA linker conjugate of the invention include antibodies, antibody fragments, receptors, and ligands. Binding moieties bound to an HSA linker of the invention may be recombinant (e.g., human, murine, chimeric, or humanized), synthetic, or natural. The invention features complete antibodies, domain antibodies, diabodies, bi-specific antibodies, antibody fragments, Fab fragments, F(ab')2 molecules, single chain Fv (scFv) molecules, tandem scFv molecules, antibody fusion proteins, and aptamers.

### Antibodies

Antibodies of the invention include the TgG, IgA, IgM, IgD, and IgE isotypes. Antibodies or antibody fragments of the invention, as used herein, contain one or more complementarity determining regions (CDR) or binding peptides that bind to target proteins, glycoproteins, or epitopes present on the exterior or in the interior of target cells.

Many of the antibodies, or fragments thereof, described herein can undergo non-critical amino-acid substitutions, additions or deletions in both the variable and constant regions without loss of binding specificity or effector functions, or intolerable reduction of binding affinity (e.g., below about 10⁻⁷ M). Usually, an antibody or antibody fragment incorporating such alterations exhibits substantial sequence identity to a reference antibody or antibody fragment from which it is derived. Occasionally, a mutated antibody or antibody fragment can be selected having the same specificity and increased affinity compared with a reference antibody or antibody fragment from which it was derived. Phage-display technology offers powerful techniques for selecting such antibodies. See, e.g., Dower et al., WO 91/17271 McCafferty et al., WO 92/01047; and Huse, WO 92/06204, incorporated by reference herein.

The HSA linker of the invention can also be bonded to one or more fragments of an antibody that retain the ability to bind with specificity to a target antigen. Antibody fragments include separate variable heavy chains, variable light chains, Fab, Fab', F(ab')₂, Fabc, and scFv. Fragments can be produced by enzymatic or chemical separation of intact immunoglobulins. For example, a F(ab')₂ fragment can be obtained from an IgG molecule by proteolytic digestion with pepsin at pH 3.0-3.5 using standard methods such as those described in Harlow and Lane, Antibodies: A Laboratory Manual, Cold Spring Harbor Pubs., N.Y. (1988). Fab fragments may be obtained from F(ab') ₂ fragments by limited reduction, or from whole antibody by digestion with papain in the presence of reducing agents. Fragments can also be produced by recombinant DNA techniques. Segments of nucleic acids encoding selected fragments are produced by digestion of full-length coding sequences with restriction enzymes, or by *de novo* synthesis. Often fragments are expressed in the form of phage-coat fusion proteins. This manner of expression is advantageous for affinity-sharpening of antibodies.

### Humanized Antibodies

The invention further provides for humanized antibodies in combination with the HSA linker of the invention, in which one or more of the antibody CDRs are derived from a non-human antibody sequence, and one or more, but preferably all, of the CDRs bind specifically to an antigen (e.g., a protein, glycoprotein, or other suitable epitope).

A humanized antibody contains constant framework regions derived substantially from a human antibody (termed an acceptor antibody), as well as, in some instances, a majority of the variable region derived from a human antibody. One or more of the CDRs (all or a portion thereof, as well as discreet amino acids surrounding one or more of the CDRs) are provided from a non-human antibody, such as a mouse antibody. The constant region(s) of the antibody, may or may not be present.

The substitution of one or more mouse CDRs into a human variable domain framework is most likely to result in retention of their correct spatial orientation if the human variable domain framework adopts the same or a similar conformation as the mouse variable framework from which the CDRs originated. This is achieved by obtaining the human variable domains from human antibodies whose framework sequences exhibit a high degree of sequence and structural identity with the murine variable framework domains from which the CDRs were derived. The heavy and light chain variable framework regions can be derived from the same or different human antibody sequences. The human antibody sequences can be the sequences of naturally occurring human antibodies, consensus sequences of several human antibodies, or can be human germline variable domain sequences. See, e.g., Kettleborough et al., Protein Engineering 4:773 (1991); Kolbinger et al., Protein Engineering 6:971 (1993).

Suitable human antibody sequences are identified by alignments of the amino acid sequences of the mouse variable regions with the sequences of known human antibodies. The comparison is performed separately for heavy and light chains but the principles are similar for each.

Methods of preparing chimeric and humanized antibodies and antibody fragments are described in, e.g., U.S. Patent Nos. 4,816,567, 5,530,101, 5,622,701, 5,800,815, 5,874,540, 5,914,110, 5,928,904, 6,210,670, 6,677,436, and 7,067,313 and U.S. Patent Application Nos. 2002/0031508, 2004/0265311, and 2005/0226876. Preparation of antibody or fragments thereof is further described in, e.g., U.S. Patent Nos. 6,331,415, 6,818,216, and 7,067,313.

### Receptors and Ligands

The invention provides for protein or glycoprotein receptors or ligands bound to an HSA linker of the invention. HSA linkers bonded with a receptor or ligand can be used, for example, to specifically target a secreted protein, a cell (e.g., a cancer cell), tissue, or organ. Furthermore, the specific binding of the HSA linker-receptor or ligand conjugate to cognate target receptors or ligands can cause agonistic or antagonistic biological activity in intracellular or intercellular signaling pathways. As with the other binding moieties described herein, receptors and ligands, or fragments thereof, can be conjoined to the amino and/or carboxy termini of an HSA linker of the invention, or to an amino acid residue within the HSA linker.

Exemplary receptors and ligands that can be joined to an HSA linker of the invention include, but are not limited to, insulin-like growth factor 1 receptor (IGF1R), IGF2R, insulin-like growth factor (IGF), mesenchymal epithelial transition factor receptor (c-met; also known as hepatocyte growth factor receptor (HGFR)), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, α4 integrin, P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-relatied apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, receptors alpha and beta, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and biologically-active fragments thereof.

Receptors and ligands can be expressed, isolated, or joined to an HSA linker of the invention using any of the methods described *supra* relating to antibodies or antibody fragments.

### Diagnostic Agents

The HSA linker of the invention, or any binding moiety conjugated thereto (e.g., antibody, antibody fragment, receptor, or ligand), can be coupled to a chelating agent or to a detectable label to form a diagnostic agent of the invention. Also contemplated are HSA linker conjugates that include a detectable label, as described herein, as well as one or more of the therapeutic agents or binding moieties described herein.

The HSA linker and chelator components can be coupled to form a conjugate by reacting the free amino group of a threonine residue of the HSA linker with an appropriate functional group of the chelator, such as a carboxyl group or activated ester. For example, a conjugate may be formed by incorporating the chelator ethylenediaminetetraacetic acid (EDTA), which is common in the art of coordination chemistry, when functionalized with a carboxyl substituent on the ethylene chain. Synthesis of EDTA derivatives of this type are reported in Arya et al. (Bioconjugate Chemistry 2:323 (1991)), which describes blocking each of the four coordinating carboxyl groups with a t-butyl group while the carboxyl substituent on the ethylene chain is free to react with the amino group of a peptide portion of the agent of the invention, thereby forming a conjugate.

An HSA linker or an HSA linker conjugate may incorporate a metal chelator component that is peptidic, i.e., compatible with solid-phase peptide synthesis. In this case, the chelator may be coupled to the HSA linker of the invention in the same manner as EDTA described above or, more conveniently, the chelator and HSA linker or HSA linker conjugate of the invention are synthesized *in toto* starting from the C-terminal residue of the HSA linker or HSA linker conjugate and ending with the N-terminal residue of the chelator.

An HSA linker or an HSA linker conjugate may further incorporate a linking group component that serves to couple the HSA linker of the invention to the chelator while not adversely affecting the biological properties of the HSA linker, the targeting function of the binding moiety portion(s) of the HSA linker conjugate, or the metal binding function of the chelator. Suitable linking groups include amino acid chains and alkyl chains functionalized with reactive groups for coupling to the HSA linker or the HSA linker conjugate and to the chelator. An amino acid chain is the preferred linking group when the chelator is peptidic so that the HSA linker or HSA linker conjugate can be synthesized *in toto* by solid-phase techniques. An alkyl chain-linking group may be incorporated in the HSA linker or HSA linker conjugate by reacting the amino group of a threonine residue of a peptide portion of an HSA linker of the invention with a first functional group on the alkyl chain, such as a carboxyl group or an activated ester. Subsequently the chelator is attached to the alkyl chain to complete the formation of the HSA linker or HSA linker conjugate by reacting a second functional group on the alkyl chain with an appropriate group on the chelator. The second functional group on the alkyl chain is selected from substituents that are reactive with a functional group on the chelator while not being reactive with a threonine residue of the mutated HSA linker protein. For example, when the chelator incorporates a functional group such as a carboxyl group or an activated ester, the second functional group of the alkyl chain-linking group can be an amino group. It will be appreciated that formation of the HSA linker or HSA linker conjugate may require protection and deprotection of the functional groups present in order to avoid formation of undesired products. Protection and deprotection are accomplished using protecting groups, reagents, and protocols common in the art of organic synthesis. Particularly, protection and deprotection techniques employed in solid phase peptide synthesis described above may be used.

An alternative chemical linking group to an alkyl chain is polyethylene glycol (PEG), which is functionalized in the same manner as the alkyl chain described above for incorporation in the HSA linker or HSA linker conjugate. It will be appreciated that linking groups may alternatively be coupled first to the chelator and then to the HSA linker or HSA linker conjugate of the invention.

In accordance with one aspect of the invention, HSA linker-chelator conjugates of the invention incorporate a diagnostically useful metal capable of forming a complex. Suitable metals include, e.g., radionuclides, such as technetium and rhenium in their various forms (e.g., ^{99*m*}TcO³⁺, ⁹⁹) *^{m}*TcO₂⁺, ReO³⁺, and ReO₂⁺). Incorporation of the metal within the HSA linker or HSA linker conjugate can be achieved by various methods common in the art of coordination chemistry. When the metal is technetium-99 m, the following general procedure may be used to form a technetium complex. An HSA linker-chelator conjugate solution is formed initially by dissolving the HSA, linker or HSA linker conjugate in aqueous alcohol such as ethanol. The solution is then degassed to remove oxygen then thiol protecting groups are removed with a suitable reagent, for example, with sodium hydroxide, and then neutralized with an organic acid, such as acetic acid (pH 6.0-6.5). In the labeling step, a stoichiometric excess of sodium pertechnetate, obtained from a molybdenum generator, is added to a solution of the conjugate with an amount of a reducing agent such as stannous chloride sufficient to reduce technetium and heated. The labeled HSA linker or HSA linker conjugate may be separated from contaminants ^{99*m*}TcO₄⁻ and colloidal ^{99*m*}TcO₂ chromatographically, for example, with a C-18 Sep Pak cartridge.

In an alternative method, labeling of an HSA linker of the invention can be accomplished by a transchelation reaction. The technetium source is a solution of technetium complexed with labile ligands facilitating ligand exchange with the selected chelator. Suitable ligands for transchelation include tartarate, citrate, and heptagluconate. In this instance the preferred reducing reagent is sodium dithionite. It will be appreciated that the HSA linker or HSA linker conjugate may be labeled using the techniques described above, or alternatively the chelator itself may be labeled and subsequently coupled to an HSA linker protein of the invention to form an HSA linker-chelator conjugate; a process referred to as the "prelabeled ligand" method.

Another approach for labeling an HSA linker of the invention, or any agent conjugated thereto, involves immobilizing the HSA linker-chelator conjugate on a solid-phase support through a linkage that is cleaved upon metal chelation. This is achieved when the chelator is coupled to a functional group of the support by one of the complexing atoms. Preferably, a complexing sulfur atom is coupled to the support which is functionalized with a sulfur protecting group such as maleimide.

When labeled with a diagnostically useful metal, an agent of the invention that includes an HSA linker-chelator conjugate can be used to detect tissue at risk of developing cancer (e.g., lung cancer, breast cancer, colon cancer, and prostate cancer), age-related diseases (e.g., cardiovascular disease, cerebrovascular disease, or Alzheimer's disease), tobacco-related diseases (e.g., emphysema, aortic aneurysms, esophageal cancer, or squamous cell cancer of the head and neck) by procedures established in the art of diagnostic imaging. An agent of the invention that incorporates an HSA linker labeled with a radionuclide metal, such as technetium-99 m, may be administered to a mammal (e.g., a human) by intravenous injection in a pharmaceutically acceptable solution, such as isotonic saline, or by other methods described herein. The amount of a labeled agent of the invention appropriate for administration is dependent upon the distribution profile of the chosen HSA linker or HSA linker conjugate in the sense that an agent of the invention that incorporates a rapidly cleared HSA linker or HSA linker conjugate may be administered at higher doses than an agent that incorporates an HSA linker or HSA linker conjugate that clears less rapidly. Unit doses acceptable for imaging tissues are in the range of about 5-40 mCi for a 70 kg individual. The *in vivo* distribution and localization of an agent of the invention that incorporates a labeled HSA linker or HSA linker conjugate can be tracked by standard techniques described herein at an appropriate time subsequent to administration, typically between 30 minutes and 180 minutes and up to about 5 days depending upon the rate of accumulation at the target site with respect to the rate of clearance at non-target tissue.

An HSA linker of the invention, or any molecule or moiety conjugated thereto, can also be modified or labeled to facilitate diagnostic or therapeutic uses. Detectable labels such as a radioactive, fluorescent, heavy metal, or other molecules may be bound to any of the agents of the invention. Single, dual, or multiple labeling of an agent may be advantageous. For example, dual labeling with radioactive iodination of one or more residues combined with the additional coupling of, for example, ⁹⁰Y via a chelating group to amine-containing side or reactive groups, would allow combination labeling. This may be useful for specialized diagnostic needs such as identification of widely dispersed small neoplastic cell masses.

An HSA linker of the invention, or any molecule or moiety conjugated thereto, can also be modified, for example, by halogenation of the tyrosine residues of the peptide component. Halogens include fluorine, chlorine, bromine, iodine, and astatine. Such halogenated agents may be detectably labeled, e.g., if the halogen is a radioisotope, such as, for example, ¹⁸F, ⁷⁵Br, ⁷⁷Br, ¹²²I, ¹²³I, ¹²⁴I, ¹²⁵I, ¹²⁹I, ¹³¹I, or ²¹¹At. Halogenated agents of the invention contain a halogen covalently bound to at least one amino acid, and preferably to D-Tyr residues in each agent molecule. Other suitable detectable modifications include binding of other compounds (e.g., a fluorochrome such as fluorescein) to a lysine residue of the agent of the invention, or analog, particularly an agent or analog having a linker including lysines.

Radioisotopes for radiolabeling an HSA linker of the invention, or any molecule or moiety conjugated thereto, include any radioisotope that can be covalently bound to a residue of the peptide component of the agent of the invention or analog thereof. The radioisotopes can also be selected from radioisotopes that emit either beta or gamma radiation, or alternatively, any of the agents of the invention can be modified to contain chelating groups that, for example, can be covalently bonded to lysine residue(s) of the HSA linker or any peptidic agent conjugated thereto. The chelating groups can then be modified to contain any of a variety of radioisotopes, such as gallium, indium, technetium, ytterbium, rhenium, or thallium (e.g., ¹²⁵I, ⁶⁷Ga, ¹¹¹In, ⁹⁹mTc, ¹⁶⁹Yb, ¹⁸⁶Rc).

An HSA linker of the invention, or any molecule or moiety conjugated thereto, can be modified by attachment of a radioisotope. Preferable radioisotopes are those having a radioactive half-life corresponding to, or longer than, the biological half-life of the HSA conjugate used. More preferably, the radioisotope is a radioisotope of a halogen atom (e.g. a radioisotope of fluorine, chlorine bromine, iodine, and astatine), even more preferably ⁷⁵Br, ⁷⁷Br, ⁷⁶Br, ¹²²I, ¹²³I, ¹²⁴I, ¹²⁵I, ¹²⁹I, ¹³¹I, or ²¹¹At.

An agent of the invention that incorporates an HSA linker, or any molecule or moiety conjugated thereto, can be coupled to radioactive metals and used in radiographic imaging or radiotherapy. Preferred radioisotopes also include ^{99m}Tc, ⁵¹Cr, ⁶⁷Ga, ⁶⁸Ga, ¹¹¹In, ¹⁶⁸Yb, ¹⁴⁰La, ⁹⁰Y, ⁸⁸Y, ¹⁵³Sm, ¹⁵⁶Ho, ¹⁶⁵Dy, ⁶⁴Cu, ⁹⁷Ru, ¹⁰³Ru, ¹⁸⁶Re, ¹⁸⁸Re, ²⁰³Pb, ²¹¹Bi, ¹¹²Bi, ²¹³Bi, and ²¹⁴Bi. The choice of metal is determined based on the desired therapeutic or diagnostic application.

An HSA linker of the invention, or any molecule or moiety conjugated thereto, can be coupled to a metal component, to produce agent of the invention that can be used as a diagnostic or therapeutic agent. A detectable label may be a metal ion from heavy elements or rare earth ions, such as Gd³⁺, Fe³⁺, Mn³⁺, or Cr²⁺. An agent of the invention that incorporates an HSA linker having paramagnetic or superparamagnetic metals conjoined thereto are useful as diagnostic agents in MRI imaging applications. Paramagnetic metals that can be coupled to the agents of the invention include, but are not limited to, chromium (III), manganese (II), iron (II), iron (III), cobalt (II), nickel (II), copper (II), praseodymium (III), neodymium (III), samarium (III), gadolinium (III), terbium (III), dysprosium (III), holmium (III), erbium (III), and ytterbium (III). Chelating groups may be used to indirectly couple detectable labels or other molecules to an HSA linker of the invention or to an agent conjugated thereto. Chelating groups can link agents of the invention with radiolabels, such as a bifunctional stable chelator, or can be linked to one or more terminal or internal amino acid reactive groups. An HSA linker of the invention, or any molecule or moeity conjugated thereto, can be linked via an isothiocyanate β-Ala or appropriate non-α-amino acid linker which prevents Edman degradation. Examples of chelators known in the art include, for example, the ininocarboxylic and polyaminopolycarboxylic reactive groups, ininocarboxylic and polyaminopolycarboxylic reactive groups, diethylenetriaminepentaacetic acid (DTPA), and 1,4,7,10-tetraazacyclododecane-1,4,7,10-tetraacetic acid (DOTA).

An HSA linker of the invention, when expressed recombinantly, can be joined to a peptidic detectable label or diagnostic agent. Peptides and proteins that can be used as a detectable label with an HSA linker include, but are not limited to, fluorescent proteins, bioluminescent proteins, and epitope tags, each of which is discussed in detail below. One or more of these detectable labels can also be incorporated into an HSA linker of the invention that also includes a therapeutic, cytotoxic, or cytostatic agent.

Fluorescent proteins or fluorochromes, such as green fluorescent protein (GFP; SEQ ID NO:47), enhanced GFP (eGFP), yellow fluorescent protein (SEQ ID NO: 48; YFP), cyan fluorescent protein (SEQ ID NO: 49; CFP), and red fluorescent protein (SEQ ID NO: 50; RFP or DsRed), can be used as detectable label joined to an HSA linker of the invention. Fluorescent proteins can be recombinantly expressed in a cell (e.g., a blood cell, such as a lymphocyte) following transfection or transduction of the cell with an expression vector that encodes the nucleotide sequence of the fluorescent protein. Upon exposure of the fluorescent protein to a stimulating frequency of light, the fluorescent protein will emit light at a low, medium, or high intensity that can be observed by eye under a microscope or by an optical imaging device. Exemplary fluorescent proteins suitable for use as the diagnostic sequence in agents of the invention are described in, e.g., U.S. Patent Nos. 7,417,131 and 7,413,874, each of which is herein incorporated by reference.

Bioluminescent proteins can also be used as a detectable label incorporated into an HSA linker of the invention. Bioluminescent proteins, such as luciferase (e.g., firefly (SEQ ID NO:51), *Renilla* (SEQ ID NO:52), and *Omphalotus* luciferase) and aequorin, emit light as part of a chemical reaction with a substrate (e.g., luciferin and coelenterazine). In one embodiment of the invention, a vector encoding a luciferase gene provides for the *in vivo, in vitro,* or *ex vivo* detection of cells (e.g., blood cells, such as lymphocytes) that have been transduced or transfected according to the methods of the invention. Exemplary bioluminescent proteins suitable for use as diagnostic sequence of the invention and methods for their use are described in, e.g., U.S. Patent Nos. 5,292,658, 5,670,356, 6,171,809, and 7,183,092, each of which is herein incorporated by reference.

Epitope tags are short amino acid sequences, e.g., 5-20 amino acid residues in length, that can be incorporated into an HSA linker of the invention as a detectable label to facilitate detection once expressed in a cell, secreted from the cell, or bound to a target cell. An agent of the invention that incorporates an epitope tag as a diagnostic sequence can be detected by virtue of its interaction with an antibody, antibody fragment, or other binding molecule specific for the epitope tag. Nucleotide sequences encoding the epitope tag are produced either by cloning appropriate portions of natural genes or by synthesizing a polynucleotide that encodes the epitope tag. An antibody, antibody fragment, or other binding molecule that binds an epitope tag can directly incorporate a detectable label (e.g., a fluorochrome, radiolabel, heavy metal, or enzyme such as horseradish peroxidase) or serve itself as a target for a secondary antibody, antibody fragment, or other binding molecule that incorporates such a label. Exemplary epitope tags that can be used as a diagnostic sequence include c-myc (SEQ ID NO:33), hemagglutinin (HA; SEQ ID NO:34), and histidine tag (His₆; SEQ ID NO:35). Furthermore, fluorescent (e.g., GFP) and bioluminescent proteins can also serve as epitope tags, as antibodies, antibody fragments, and other binding molecules are commercially available for the detection of these proteins.

The *in vivo, in vitro,* or *ex vivo* detection, imaging, or tracking of a an HSA linker of the invention that incorporates a diagnostic sequence (e.g., a fluorescent protein, bioluminescent protein, or epitope tag) or any cell expressing or bound thereto can be accomplished using a microscope, flow cytometer, luminometer, or other state of the art optical imaging device, such as an IVIS^{®} Imaging System (Caliper LifeSciences, Hopkinton, MA).

### Therapeutic or Cytotoxic Agents Coupled to the HSA Linker of the Invention

An HSA linker protein of the invention, or any molecule or moiety conjugated thereto, can be coupled to any known cytotoxic or therapeutic moiety to form an agent of the invention that can be administered to treat, inhibit, reduce, or ameliorate disease (e.g., a cancer, autoimmune disease, or cardiovascular disease) or one or more symptoms of disease. Examples include but are not limited to antineoplastic agents such as: Acivicin; Aclarubicin; Acodazole Hydrochloride; Acronine; Adozelesin; Adriamycin; Aldesleukin; Altretamine; Ambomycin; A. metantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Camptothecin; Caracemide; Carbetimer; Carboplatin; Carmustine; Carubicin Hydrochloride; Carzelesin; Cedefingol; Chlorambucil; Cirolcmycin; Cisplatin; Cladribine; Combretestatin A-4; Crisnatol Mesylate; Cyclophosphamide; Cytarabine; Dacarbazine; DACA (N-[2- (Dimethyl-amino) ethyl] acridine-4-carboxamide); Dactinomycin; Daunorubicin Hydrochloride; Daunomycin; Decitabine; Dexormaplatin; Dezaguanine; Dezaguaninc Mesylate; Diaziquone; Docetaxel; Dolasatins; Doxorubicin; Doxorubicin Hydrochloride; Droloxifene; Droloxifene Citrate; Dromostanolone Propionate; Duazomycin; Edatrexate; Eflornithine Hydrochloride; Ellipticine; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; Gemcitabine; Gemcitabine Hydrochloride; Gold Au 198; Homocamptothecin; Hydroxyurea; Idarubicin Hydrochloride; Ifosfamide; Ilmofosine; Interferon Alfa-2a; Interferon Alfa-2b; Interferon Alfa-nl; Interferon Alfa-n3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin;Ormaplatin; Oxisuran; Paclitaxel; Pegaspargase; Peliomycin; Pentamustine; PeploycinSulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Rhizoxin; Rhizoxin D; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Thymitaq; Tiazofurin; Tirapazamine; Tomudex; TOP53; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2-Chlorodeoxyadenosine; 2' Deoxyformycin; 9-aminocamptothecin; raltitrexed; N-propargyl-5,8-dideazafolic acid; 2chloro-2'-arabino-fluoro-2'-deoxyadenosine; 2-chloro-2'-deoxyadenosine; anisomycin; trichostatin A; hPRL-G129R; CEP-751; linomide; sulfur mustard; nitrogen mustard (mechlor ethamine); cyclophosphamide; melphalan; chlorambucil; ifosfamide; busulfan; N-methyl-Nnitrosourca (MNU); N, N'-Bis (2-chloroethyl)-N-nitrosourea (BCNU); N- (2-chloroethyl)-N' cyclohexyl-N-nitrosourea (CCNU); N- (2-chloroethyl)-N'- (trans-4-methylcyclohexyl-N-nitrosourea (MeCCNU); N- (2-chloroethyl)-N'- (diethyl) ethylphosphonate-N-nitrosourea (fotemustine); streptozotocin; diacarbazine (DTIC); mitozolomide; tcmozolomide; thiotepa; mitomycin C; AZQ; adozelesin; Cisplatin; Carboplatin; Ormaplatin; Oxaliplatin;C1-973; DWA 2114R; JM216; JM335; Bis (platinum); tomudex; azacitidine; cytarabine; gemcitabine; 6-Mercaptopurine; 6-Thioguanine; Hypoxanthine; teniposide 9-amino camptothecin; Topotecan; CPT-11; Doxorubicin; Daunomycin; Epirubicin; darubicin; mitoxantrone; losoxantrone; Dactinomycin (Actinomycin D); amsacrine; pyrazoloacridine; all-trans retinol; 14-hydroxy-retro-retinol; all-trans retinoic acid; N- (4-Hydroxyphenyl) rctinamide; 13-cis retinoic acid; 3-Methyl TTNEB; 9-cis retinoic acid; fludarabine (2-F-ara-AMP); or 2-chlorodeoxyadenosine (2-Cda).

Other therapeutic compounds include, but are not limited to, 20-pi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; argininedeaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bleomycin A2; bleomycin B2; bropirimine; budotitane; buthionine sulfoximine; calcipotriol; calphostin C; camptothecin derivatives (e.g., 10-hydroxy-camptothecin); canarypox IL-2; capecitabine; carboxamide-amino-triazole; carboxyamidotriazole; CaRest M3; CARN 700; cartilage derived inhibitor; carzelesin; casein kinase inhibitors (ICOS); castanospcrmine; cecropin B; cetrorelix; chlorins; chloroquinoxaline sulfonamide; cicaprost; cis-porphyrin; cladribine; clomifene analogues; clotrimazole; collismycin A ; collismycin B; combretastatin A4; combretastatin analogue; conagenin; crambescidin 816 ; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; 2'deoxycoformycin (DCF); deslorelin; dexifosfamide; dexrazoxane; dexverapamil; diaziquone; didemnin B; didox; diethylnorspermine; dihydro-5-azacytidine; dihydrotaxol, 9- ; dioxamycin; diphenyl spiromustine; discodermolide; docosanol; dolasetron; doxifluridine; droloxifene; dronabinol; duocarmycin SA; ebselen; ecomustine; edelfosine; edrecolomab; eflornithine; elemene; emitefur; epirubicin; epothilones (A, R = H; B, R = Me); epithilones; epristeride; estramustine analogue; estrogen agonists; estrogen antagonists; etanidazole; ctoposide; etoposide 4'-phosphate (etopofos); exemestane; fadrozole; fazarabine; fenretinide; filgrastim; finasteride; flavopiridol; flezelastine; fluasterone; fludarabine; fluorodaunorunicin hydrochloride; forfenimex; formcstane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; homoharringtonine (HHT); hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- ; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantronc; lovastatin; loxoribine; lurtotecan; lutetium texaphyrin; lysofylline; lytic peptides; maytansine; mannostatin A; marimastat; masoprocol; maspin; matrilysin inhibitors; matrix metalloproteinase inhibitors; menogaril; rnerbarone; meterelin; methioninase; metoclopramide; MIF inhibitor; ifepristone; miltefosine; mirimostim; mismatched double stranded RNA; mithracin; mitoguazone; mitolactol; mitomycin analogues; mitonafide; milotoxin fibroblast growth factor-saporin; mitoxantrone; mofarotene; molgramostim; monoclonal antibody, human chorionic gonadotrophin; monophosphoryl lipid A + myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone + pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; podophyllotoxin; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B 1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazominc; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene dichloride; topotecan; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

The HSA linker agents of the invention can also include site-specifically conjugated molecules and moieties. Site-specific conjugation allows for the controlled stoichiometric attachment to specific residues in the HSA linker of cytotoxic, immunomodulatory, or cytostatic agents including, e.g., anti tubulin agents, DNA minor groove binders, DNA replication inhibitors, alkylating agents, anthracyclines, antibiotics, antifolates, antimetabolites, chemotherapy or radiation sensitizer, duocarmycins, etoposides, fluorinated pyrimidines, ionophores, lexitropsins, nitrosoureas, platinols, purine antimetabolites, puromycins, steroids, taxanes, topoisomerase inhibitors, and vinca alkaloids or any other molecules or moieties described herein.

Techniques for conjugating therapeutic agents to proteins, and in particular to antibodies, are well-known (e.g., Arnon et al., "Monoclonal Antibodies For Immunotargeting Of Drugs In Cancer Therapy," in Monoclonal Antibodies And Cancer Therapy (Reisfeld et al., eds., Alan R. Liss, Inc., 1985); Hellstrom et al., "Antibodies For Drug Delivery," in Controlled Drug Delivery (Robinson et al., eds., Marcel Dekker, Inc., 2nd cd. 1987); Thorpe, "Antibody Carriers Of Cytotoxic Agents In Cancer Therapy: A Review," in Monoclonal Antibodies '84: Biological And Clinical Applications (Pinchera et al., cds., 1985); "Analysis, Results, and Future Prospective of the Therapeutic Use of Radiolabeled Antibody In Cancer Therapy," in Monoclonal Antibodies For Cancer Detection And Therapy (Baldwin et al., eds., Academic Press, 1985); Thorpe et al., Immunol. Rev. 62:119-58 (1982); and Doronina et al., "Development of potent monoclonal antibody auristatin conjugates for cancer therapy," Nature Biotech. 21:(7)778-784 (2003)). See also, e.g., PCT publication WO 89/12624.

An HSA linker of the invention, or any molecule or moiety conjugated thereto, can also be coupled to a lytic peptide to form an agent of the invention. Such lytic peptides induce cell death and include, but are not limited to, streptolysin O; stoichactis toxin; phallolysin; staphylococcus alpha toxin; holothurin A; digitonin; melittin; lysolecithin; cardiotoxin; and cerebratulus A toxin (Kem et al., J. Biol. Chem. 253(16):5752-5757, 1978). An agent of the invention can also be formed by conjugating an IISA linker of the invention, or any molecule or moiety conjugated thereto (e.g., antibody or antibody fragment conjugates), to a synthetic peptide that shares some sequence homology or chemical characteristics with any of the naturally occurring peptide lysins; such characteristics include, but are not limited to, linearity, positive charge, amphipathicity, and formation of alpha-helical structures in a hydrophobic environment (Leuschner et al., Biology of Reproduction 73:860-865, 2005). An HSA linker of the invention, or any molecule or moiety conjugated thereto, can also be coupled to an agent that induces complement-mediated cell lysis such as, for example, the immunoglobulin F_{c} subunit. An HSA linker of the invention, or any molecule or moiety conjugated thereto, can also coupled to any member of the phospholipase family of enzymes (including phospholipase A, phospholipase B, phospholipase C, or phospholipase D) or to a catalytically-active subunit thereof.

An HSA linker of the invention, or any molecule or moiety conjugated thereto, can also be coupled to a radioactive agent to form an agent that can be used for detection or therapeutic applications. Radioactive agents that can be used include but are not limited to Fibrinogen ¹²⁵I; Fludeoxyglucose ¹⁸F; Fluorodopa ¹⁸F; Insulin ¹²⁵I; Insulin ¹³¹I; lobenguane ¹²³I; Iodipamide Sodium ¹³¹I; Iodoantipyrine ¹³¹I; Iodocholesterol ¹³¹I; lodohippurate Sodium ¹²³I; Iodohippurate Sodium ¹²⁵I; Iodohippurate Sodium ¹³¹I; Iodopyracet ¹²⁵I; Iodopyracet ¹³¹I; Iofetamine Hydrochloride **¹²³I;** Iomethin ¹²⁵I; lomethin ¹³¹I; Iothalamate Sodium ¹²⁵I; Iothalamate Sodium ¹³¹I; tyrosine ¹³¹I; Liothyronine ¹²⁵I; Liothyronine ¹³¹I; Merisoprol Acetate ¹⁹⁷Hg; Merisoprol Acetate ²⁰³Hg; Merisoprol ¹⁹⁷Hg; Selenomethionine ⁷⁵Se; Technetium ^{99m}Tc Antimony Trisulfide Colloid; Technetium ^{99m}Tc Bicisate; Technetium ^{99m}Tc Disofenin; Technetium ^{99m}Tc Etidronate; Technetium ^{99m}Tc Exametazime; Technetium ^{99m}Tc Furifosmin; Technetium ^{99m}Tc Gluceptate; Technetium ^{99m}Tc Lidofenin; Technetium ^{99m}Tc Mebrofenin; Technetium ^{99m}Tc Medronate; Technetium ^{99m}Tc Medronate Disodium; Technetium ^{99m}Tc Mertiatide; Technetium ^{99m}Tc Oxidronate; Technetium ^{99m}Tc Pentetate; Technetium ^{99m}Tc Pentetate Calcium Trisodium; Technetium ^{99m}Tc Sestamibi; Technetium ^{99m}Tc Siboroxime; Technetium ^{99m}Tc; Succimer; Technetium ^{99m}Tc Sulfur Colloid; Technetium ^{99m}Tc Teboroxime; Technetium ^{99m}Tc Tetrofosmin; Technetium ^{99m}Tc Tiatide; Thyroxine ¹²⁵I; Thyroxine ¹³¹I; Tolpovidone ¹³¹I; Triolein ¹²⁵I; or Triolein ¹³¹I.

Additionally, a radioisotope could be site-specifically conjoined to an HSA linker or HSA linker conjugate. The available reactive groups could be used to conjugate site-specific bifunctional chelating agents for labeling of radioisotopes, including ¹²³I, ¹²⁴I, ¹²⁵I, ¹³¹I, ^{99m}Tc, ¹¹¹In, ⁶¹⁴Cu, ⁶⁷Cu, ¹⁸⁶Re, ¹⁸⁸Re, ¹⁷⁷Lu, ⁹⁰Y, ⁷⁷As, ⁷²As, ⁸⁶Y, ⁸⁹Zr, ²¹¹At, ²¹²Bi, ²¹³Bi, or ²²⁵Ac.

Therapeutic or cytotoxic agents incorporated into or coupled with an HSA linker or an HSA linker conjugate may further include, for example, anti-cancer Supplementary Potentiating Agents, including, but not limited to: tricyclic anti-depressant drugs (e.g., imipramine, desipramine, amitryptyline, clomipramine, trimipramine, doxepin, nortriptyline, protriptyline, amoxapine, and maprotiline); non-tricyclic anti-depressant drugs (e.g., sertraline, trazodone, and citalopram); Ca²⁺ antagonists (e.g., verapamil, nifcdipinc, nitrcndipine, and caroverine); Calmodulin inhibitors (e.g., prenylamine, trifluoroperazine, and clomipramine); Amphotericin B; Triparanol analogs (e.g., tamoxifen); antiarrhythmic drugs (e.g., quinidine); antihypertensive drugs (e.g., reserpine); Thiol depleters (e.g., buthionine and sulfoximine) and Multiple Drug Resistance reducing agents such as Crcmaphor EL.

An agent of the invention that includes an HSA linker, or any molecule or moiety conjugated thereto, can also be coupled to or administered with cytokines (e.g., granulocyte colony stimulating factor, interferon-alpha, and tumor necrosis factor-alpha). An HSA linker of the invention, or any molecule or moiety conjugated thereto, can also be coupled to an antimetabolic agent. Antimetabolic agents include, but are not limited to, the following compounds and their derivatives: azathioprine, cladribine, cytarabine, dacarbazine, fludarabine phosphate, fluorouracil, gencitabinc chlorhydrate, mercaptopurine, methotrexate, mitobronitol, mitotane, proguanil chlorohydrate, pyrimethamine, raltitrexed, trimetrexate glucuronate, urethane, vinblastine sulfate, vincristine sulfate, etc. More preferably, an HSA linker or conjugate of the invention can be coupled to a folic acid-type antimetabolite, a class of agents that includes, for example, methotrexate, proguanil chlorhydrate, pyrimethanime, trimethoprime, or trimetrexate glucuronate, or derivatives of these compounds.

An HSA linker of the invention, or any molecule or moiety conjugated thereto, can also be coupled to a member of the anthracycline family of neoplastic agents, including but not limited to aclarubicine chlorhydrate, daunorubicine chlorhydrate, doxorubicine chlorhydrate, epirubicine chlorhydrate, idarubicine chlorhydrate, pirarubicine, or zorubicine chlorhydrate; a camptothecin, or its derivatives or related compounds, such as 10, 11 methylenedioxycamptothecin; or a member of the maytansinoid family of compounds, which includes a variety of structurally-related compounds, e.g., ansamitocin P3, maytansine, 2'-N-demethylmaytanbutine, and maytanbicyclinol.

An HSA linker of the invention, or any molecule or moiety conjugated thereto, can also be coupled directly to a cytotoxic or therapeutic agent using known chemical methods, or coupled indirectly to a cytotoxic or therapeutic agent via an indirect linkage. For example, an HSA linker can be attached to a chelating group that is attached to the cytotoxic or therapeutic agent. Chelating groups include, but are not limited to, ininocarboxylic and polyaminopolycarboxylic reactive groups, diethylenetriaminepentaacetic acid (DTPA), and 1,4,7,10-tetraazacyclododecane-1,4,7,10-tetraacetic acid (DOTA). For general methods, see, e.g., Liu et al., Bioconjugate Chem, 12(4):653, 2001; Cheng et al., WO 89/12631; Kieffer et al., WO 93/12112; Albert et al., U.S.P.N. 5,753,627; and WO 91/01144 (each of which are hereby incorporated by reference).

An agent of the invention that includes, e.g., an HSA linker, one or more binding moieties (with or without intervening polypeptide connectors, as defined herein), and a therapeutic or cytotoxic agent, can be specifically targeted by the binding moiety (e.g., antibody, antibody fragment, or receptor/ligand) to a cell or tissue, thereby allowing selective destruction of the target cell or tissue to which the binding moiety is directed. For example, an agent of the invention can be used to target and destroy cancer cells of the lung, breast, prostate, and colon in order to prevent, stabilize, inhibit the progression of, or treat cancers originating in these organs when the agent includes a binding moiety that specifically binds to the cancer cells in these organs. Also, for example, the agents of the invention can be used to target and destroy cells of the vasculature, brain, liver, kidney, heart, lung, prostate, colon, nasopharynx, oropharynx, larynx, bronchus, and skin in order to prevent, stabilize, inhibit the progression of, or treat age-related, tobacco-related, or autoimmune diseases or conditions relating to these organs by targeting, in the case of autoimmune disease for example, autoreactive T cells (e.g., by binding to and agonizing tumor necrosis factor receptor 2 (TNFR2) present on the autoreactive T cells).

An HSA linker of the invention, when expressed recombinantly, can be joined to a cytotoxic polypeptide. Cytotoxic polypeptides, when brought into contact with a target cell (e.g., a cancer cell), exerts cytotoxic or cytostatic effects on the cell. For example, a cytotoxic polypeptide, when joined with an HSA linker of the invention, can induce events in a target cell upon binding of the target cell that leads to cell death through, for example, apoptosis, necrosis, or senescence. Alternatively, a cytotoxic polypeptide joined with an HSA linker of the invention can interfere or inhibit normal cellular biological activities, such as division, metabolism, and growth, or abnormal cellular biological activities, such as metastasis.

For example, an HSA linker of the invention joined to caspase 3 will bind a target cell (e.g., a cancer cell) and undergoe endocytosis. Once internalized by the target cell, the caspase portion of the HSA linker conjugate can initiate the pro-apoptotic caspase cascade, ultimately resulting in the apoptosis of the target cell.

In a preferred embodiment, an HSA linker of the invention includes a cytotoxic polypeptide capable of killing a cancer cell. In another embodiment of the invention, the cytotoxic polypeptide inhibits the growth or metastasis of a cancer cell. The cytotoxic polypeptide joined with an HSA linker of the invention can also be used to kill or inhibit the growth of cells associated with, necessary for, or beneficial to cancer growth, such as endothelial cells that form blood vessels that perfuse solid tumors.

In an embodiment, an HSA linker of the invention can include two or more cytotoxic polypeptides so as to modulate (e.g., increase) the specificity, intensity, or duration of the cytotoxic or cytostatic effect on a target cell (e.g., a cancer cell).

In another embodiment, the HSA linker of the invention is joined to an activatable form of cytotoxic polypeptide (e.g., a biologically-inactive pro-agent that is capable of activation upon cleavage by an enzyme or drug). In this embodiment, texposure (e.g., *in vivo*) of the cytotoxic polypeptide pro-agent to an enzyme or drug capable of cleaving the cytotoxic polypeptide, renders the cytotoxic polypeptide biologically-active (e.g., cytotoxic or cytostatic). An example of a biologically-inactive cytotoxic polypeptide that can be converted to a biologically-active form for use with an HSA linker of the invention is a procaspase (e.g., procaspase 8 or 3). For example, the procaspase 8 domain of an HSA linker of the invention can be cleaved by TRAIL or FasL upon internalization by a target cell (e.g., a cancer cell). Once cleaved, the biologically active caspase 8 can promote apoptosis of the target cell.

In one embodiment of the invention, the cytotoxic polypeptide joined to an HSA linker of the invention can include a full-length peptide, polypeptide, or protein, or biologically-active fragment thereof (e.g., a "death domain"), known to have cytotoxic or cytostatic properties. Peptides, polypeptides, or proteins with cytotoxic or cytostatic properties can be altered (e.g., by making amino acid substitutions, mutations, truncations, or additions) to facilitate incorporation of the cytotoxic sequence into an agent of the invention. Desirable alterations include, for example, changes to the amino acid sequence that facilitate protein expression, longevity, cell secretion, and target cell toxicity.

The invention also features a nucleic acid molecule encoding a cytotoxic polypeptide as a fusion protein with an HSA linker of the invention, optionally including binding moieities and polypeptide connectors. The nucleic acid molecule can be incorporated into a vector (e.g., an expression vector), such that, upon expression of the HSA linker of the invention in a cell transfected or transduced with the vector, the cytotoxic polypeptide, HSA linker, and binding moieties, if present, are operably linked (e.g., fused, contiguously-joined, or tethered together). Examples of peptides, polypeptides, and proteins that can be used as a cytotoxic polypeptide of the present invention include, but are not limited to, apoptosis-inducing proteins such as cytochrome c (SEQ ID NO:39); caspases (e.g., caspase 3 (SEQ ID NO:36) and caspase 8 (SEQ ID NO:37)); procaspases, granzymes (e.g., granzymes A and B (SEQ ID NO: 38)); tumor necrosis factor (TNF) and TNF receptor family members, including TNF-alpha (TNFα; SEQ ID NO:40)), TNF-beta, Fas (SEQ ID NO:41) and Fas ligand; Fas-associated death domain-like IL-1β converting enzyme (FLICE); TRAIL/APO2L (SEQ ID NO:45) and TWEAK/APO3L (see, e.g., U.S. Patent Application Publication No. 2005/0187177, herein incorporated by reference); pro-apoptotic members of the Bcl-2 family, including Bax (SEQ ID NO:46), Bid, Bik, Bad (SEQ ID NO:42), Bak, and RICK (see, e.g., U.S. Patent Application Publication No. 2004/0224389, herein incorporate by reference); vascular apoptosis inducing proteins 1 and 2 (VAP1 and VAP2; Masuda et al., Biochem. Biophys. Res. Commun. 278:197-204 (2000)); pierisin (SEQ ID NO:44; Watanabe et al., Biochemistry 96:10608-10613 (1999)); apoptosis-inducing protein (SEQ ID NO:43; AIP; Murawaka et al., Nature 8:298-307 (2001)); IL-1 α propiece polypeptide (see, e.g., U.S. Patent 6,191,269, herein incorporated by reference); apoptin and apoptin-associated proteins such as AAP-1 (see, e.g., European Patent Application Publication No. EP 1083224, herein incorporated by reference); anti-angiogenic factors such as endostatin and angiostatin; and other apoptosis-inducing proteins, including those described in the following International and U.S. Patent Application Publications, each herein incorporated by reference: U.S. 2003/0054994, U.S. 2003/0086919, U.S. 2007/0031423, WO 2004/078112, WO 2007/012430, and WO 2006/0125001 (intracellular domain of delta 1 and jagged 1).

### Wild-type HSA Linker Conjugates

The present invention also encompasses a naturally-occurring wild-type HSA linker, the amino acid and nucleotide sequences of which are provided in SEQ ID NOS:3 and 4, respectively, in the formation of binding, diagnostic, or therapeutic agents of the invention. In all embodiments of the invention utilizing an HSA linker with the amino acid sequence listed in SEQ ID NO:3, one or more polypeptide connectors, as described above, are covalently attached to the amino and/or carboxy termini of the HSA linker, or to an amino acid residue within the HSA linker sequence, to facilitate conjugation of one or more binding moieties.

### Truncations

The invention further features a conjugate that is formed using a truncated wild-type IISA polypeptide, which can be combined with one or more polypeptide connectors or binding moieties. A wild-type HSA polypeptide lacking 1, 2, 3, 4, 5, 10, 15, 20, 50, 100, 200 or more amino acids of the full-length wild-type HSA amino acid sequence (i.e., SEQ ID NO:3) can be conjoined to any of the binding moieties or diagnostic or therapeutic agents described herein. Truncations can occur at one or both ends of the HSA linker, or can include a deletion of internal residues. Truncation of more than one amino acid residue need not be linear. Examples of wild-type HSA linkers of the invention include those having, in combination with one or more polypeptide connectors or binding moieties, one or more of domain I (SEQ ID NO:56; residues 1-197 of SEQ ID NO:3), domain II (SEQ ID NO:54; residues 189-385 of SEQ ID NO:3), or domain III (SEQ ID NO:57; residues 381-585 of SEQ ID NO:3), or combinations thereof, e.g., domains I and II, I and III, and II and III.

Serum clearance rates of a conjugate of the invention (e.g., a bispecific HSA-drug or radioisotope-containing agent), can be optimized by testing conjugates containing a truncated wild-type HSA linker, as describe above.

### Additional HSA Linker Modifications

The invention further provides site-specific chemical modification of amino acid residues in the HSA linker polypeptide. The correctly-folded tertiary structure of HSA displays certain amino acid residues on the external face of the protein. Chemically-reactive amino acid residues (e.g., cysteine) can be substituted for these surface-exposed residues to allow site-specific conjugation of a diagnostic or therapeutic agent.

The invention also provides for the addition or removal of asparagine, serine, or threonine residues from an HSA linker polypeptide sequence to alter glycosylation of these amino acid residues. Glycosylation sites added to an HSA linker are preferably surface-exposed, as discussed herein. Glycosyl or other carbohydrate moieties introduced to an HSA linker can be directly conjugated to diagnostic, therapeutic, or cytotoic agents.

### Cysteine (Thiol) Conjugation

The invention features the substitution of surface-exposed amino acid residues of the HSA linker polypeptide with cysteine residues to allow for chemical conjugation of diagnostic, therapeutic, or cytotoxic agents. Cysteine residues exposed on the surface of the HSA linker polypeptide (when folded into its native tertiary structure) allow the specific conjugation of a diagnostic, therapeutic, or cytotoxic agent to a thiol reactive group such as maleimide or haloacetyl. The nucleophilic reactivity of the thiol functionality of a cysteine residue to a maleimide group is about 1000 times higher compared to any other amino acid functionality in a protein, such as the amino group of a lysine residue or the N-terminal amino group. Thiol specific functionality in iodoacetyl and maleimide reagents may react with amine groups, but higher pH (>9.0) and longer reaction times are required (Garman, 1997, Non-Radioactive Labelling: A Practical Approach, Academic Press, London). The amount of free thiol in a protein may be estimated using the standard Ellman's assay. In some instances, reduction of the disulfide bonds with a reagent such as dithiothreitol (DTT) or selenol (Singh et al., Anal. Biochem. 304:147-156 (2002)) is required to generate the reactive free thiol.

Sites for cysteine substitution can be identified by analysis of surface accessibility of the HSA linker (e.g., the identification of serine and threonine residues as suitable for substitution are described in Example 1 below). The surface accessibility can be expressed as the surface area (e.g., square angstroms) that can be contacted by a solvent molecule, e.g., water. The occupied space of water is approximated as a sphere with a 1.4 angstrom radius. Software for calculating the surface accessibility of each amino acid of a protein is freely available or licensable. For example, the CCP4 Suite of crystallography programs which employ algorithms to calculate the surface accessibility of each amino acid of a protein with known x-ray crystallography derived coordinates ("The CCP4 Suite: Programs for Protein Crystallography" Acta. Cryst. D50:760-763 (1994); www.ccp4.ac.uk/dist/html/INDEX.html). Solvent accessibility may also be assessed using the free software DeepView Swiss PDB Viewer downloaded from the Swiss Institute ofBioinformatics. The substitution of cysteines at surface-exposed sites allows for conjugation of the reactive cysteine to a thiol reactive group linked to the diagnostic or therapeutic agent.

### Glycosylation

In addition, altered serum clearance rates can be achieved by engineering glycosylation sites into the HSA linker. In certain embodiments, an HSA linker of the invention is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X represents any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the HSA linker is conveniently accomplished by altering the amino acid sequence such that one or more of the above-described tripeptide sequences (for N-linked glycosylation sites) is created. The alteration may also be made by the addition, deletion, or substitution of one or more serine or threonine residues to the sequence of the original HSA linker (for O-linked glycosylation sites). The resulting carbohydrate structures on HSA can also be used for site-specific conjugation of cytotoxic, immunomodulatory or cytostatic agents as described above.

### HSA Linker Agents in Combination with Therapeutic Agents

The invention further provides for the administration of the HSA linker agents described herein with one or more of the therapeutic, cytotoxic, or cytostatic agents described herein. For example, a patient suffering from breast cancer can be administered an HSA linker containing ErbB2 and ErbD3 scFvs (e.g., B2B3-1) can be co-administered with, e.g., doxorubicin, cyclophosphamide, and paclitaxel, a common chemotherapeutic regimen for the treatment of breast cancer. Additional biological and chemical agents useful for the treatment of cancer are listed in Appendix C.

### HSA Linker Agents in Combination with Radiotherapy or Surgery

The invention provides for the administration of an HSA linker agent of the invention prior to, concurrent with, or following radiotherapy or surgery. For example, a patient suffering from a proliferative disorder (e.g., breast cancer) can receive an HSA linker agent, alone or in combination with other therapeutic, cytotoxic, or cytotoxic agents as described herein concurrent with targeted radiotherapy or surgical intervention (e.g., lumpectomy or mastectomy) at the site of the cancerous tissue. Radiotherapies suitable for use in combination with HSA linker agents of the invention include brachytherapy and targeted intraoperative radiotherapy (TARGIT).

### Pharmaceutical Compositions

Pharmaceutical compositions of the invention contain a therapeutically or diagnostically effective amount of an HSA linker conjugate of the invention that includes one or more of a binding moiety (e.g., antibodies or antibody fragments), diagnostic agent (e.g., radionuclide or chelating agents), or a therapeutic agent (e.g., cytotoxic or immunomodulatory agents) agent. The active ingredients, an HSA linker conjugate of the invention (prepared with one or more of a binding moiety, diagnostic agent, or therapeutic agent) can be formulated for use in a variety of drug delivery systems. One or more physiologically acceptable excipients or carriers can also be included in the compositions for proper formulation. Suitable formulations for use in the present invention are found in Remington's Pharmaceutical Sciences, Mack Publishing Company, Philadelphia, PA, 17th ed. (1985). For a brief review of methods for drug delivery, see, Langer Science 249:1527-1533 (1990).

The pharmaceutical compositions arc intended for parenteral, intranasal, topical, oral, or local administration, such as by a transdermal means, for prophylactic and/or therapeutic treatment. Commonly, the pharmaceutical compositions are administered parenterally (e.g., by intravenous, intramuscular, or subcutaneous injection), or by oral ingestion, or by topical application. Thus, the invention provides compositions for parenteral administration that include an HSA linker of the invention, with or without one or more binding, diagnostic, and/or therapeutic agent conjugated thereto, dissolved or suspended in an acceptable carrier, preferably an aqueous carrier, e.g., water, buffered water, saline, PBS, and the like. The compositions may contain pharmaceutically acceptable auxiliary substances as required to approximate physiological conditions, such as pH adjusting and buffering agents, tonicity adjusting agents, wetting agents, detergents and the like. The invention also provides compositions for oral delivery, which may contain inert ingredients such as binders or fillers for the formulation of a tablet, a capsule, and the like. Furthermore, this invention provides compositions for local administration, which may contain inert ingredients such as solvents or emulsifiers for the formulation of a cream, an ointment, and the like.

These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of an HSA linker conjugate (prepared with one or more of a binding, diagnostic, and/or therapeutic agent) in a scaled package of tablets or capsules, for example. The composition in solid form can also be packaged in a container for a flexible quantity, such as in a squeezable tube designed for a topically applicable cream or ointment.

The compositions containing an effective amount of an HSA linker conjugate (prepared with one or more of a binding, diagnostic, and/or therapeutic agent) can be administered to a mammal (e.g., a human) for prophylactic and/or therapeutic treatments. In prophylactic applications, compositions of the invention containing an HSA linker conjugate (prepared with one or more of a binding, diagnostic, and/or therapeutic agent) are administered to a patient susceptible to or otherwise at risk of developing a disease or condition (e.g., a cancer, autoimmune disease, or cardiovascular disease). Such an amount is defined to be a "prophylactically effective dose." In this use, the precise amounts again depend on the patient's state of health, but generally range from about 0.5 mg to about 400 mg of an HSA linker conjugate (prepared with one or more of a binding, diagnostic, and/or therapeutic agent) per dose (e.g., 10 mg, 50 mg, 100 mg, 200 mg, 300 mg, or 400 mg per dose) and from about 0.1 µg to about 300 mg of one or more immunomodulatory agents per dose (e.g., 10 µg, 30 µg, 50 µg, 0.1 mg, 10 mg, 50 mg, 100 mg, or 200 mg per dose). A dose of an HSA linker conjugate (prepared with one or more of a binding, diagnostic, and/or therapeutic agent) can be administered prophylactically to a patient one or more times per hour, day, week, month, or year (e.g., 2, 4, 5, 6, 7, 8, 9, 10, 11, or 12 times per hour, day, week, month, or year). More commonly, a single dose per week of an HSA linker conjugate (prepared with one or more of a binding, diagnostic, and/or therapeutic agent) is administered.

In therapeutic applications, a dose of an HSA linker conjugate (prepared with one or more of a binding, diagnostic, and/or therapeutic agent) is administered to a mammal (e.g., a human) already suffering from a disease or condition (e.g., a cancer, autoimmune disease, or cardiovascular disease) in an amount sufficient to cure or at least partially arrest or alleviate one or more of the symptoms of the disease or condition and its complications. An amount adequate to accomplish this purpose is defined as a "therapeutically effective dose." Amounts effective for this use may depend on the severity of the disease or condition and general state of the patient, but generally range from about 0.5 mg to about 400 mg of an HSA linker conjugate (prepared with one or more of a binding, diagnostic, and/or therapeutic agent) per dose (e.g., 10 mg, 50 mg, 100 mg, 200 mg, 300 mg, or 400 mg per dose). A dose of an HSA linker conjugate (prepared with one or more of a binding, diagnostic, and/or therapeutic agent) can be administered therapeutically to a patient one or more times per hour, day, week, month, or year (e.g., 2, 4, 5, 6, 7, 8, 9, 10, 11, or 12 times per hour, day, week, month, or year). More commonly, a single dose per week of an HSA linker conjugate (prepared with one or more of a binding, diagnostic, and/or therapeutic agent) is administered.

In several embodiments, the patient may receive an HSA linker conjugate (prepared with one or more of a binding, diagnostic, and/or therapeutic agent) in the range of about 0.5 to about 400 mg per dose one or more times per week (e.g., 2, 3, 4, 5, 6, or 7 or more per week), preferably about 5 mg to about 300 mg per dose one or more times per week, and even more preferably about 5 mg to about 200 mg per dose one or more times per week. The patient may also receive a biweekly dose of an HSA linker conjugate (prepared with one or more of a binding, diagnostic, and/or therapeutic agent) in the range of about 50 mg to about 800 mg or a monthly dose of an HSA linker of the invention, or any binding, diagnostic, and/or therapeutic agent conjugated thereto, in the range of about 50 mg to about 1,200 mg.

In other embodiments, an HSA linker conjugate (prepared with one or more of a binding, diagnostic, and/or therapeutic agent) may be administered to a patient in a typical dosage range of about 0.5 mg per week to about 400 mg per week, about 1.0 mg per week to about 300 mg per week, about 5 mg per week to about 200 mg per week, about 10 mg per week to about 100 mg per week, about 20 mg per week to about 80 mg per week, about 100 mg per week to about 300 mg per week, or about 100 mg per week to about 200 mg per week. An HSA linker conjugate (prepared with one or more of a binding, diagnostic, and/or therapeutic agent) may be administered in the range of about 0.5 mg every other day to about 100 mg every other day, preferably about 5 mg every other day to about 75 mg every other day, more preferably about 10 mg every other day to about 50 mg every other day, and even more preferably 20 mg every other day to about 40 mg every other day. An HSA linker conjugate (prepared with one or more of a binding, diagnostic, and/or therapeutic agent) may also be administered in the range of about 0.5 mg three times per week to about 100 mg three times per week, preferably about 5 mg three times per week to about 75 mg three times per week, more preferably about 10 mg three times per week to about 50 mg three times per week, and even more preferably about 20 mg three times per week to about 40 mg three times per week.

In non-limiting embodiments of the methods of the present invention, an HSA linker conjugate (prepared with one or more of a binding, diagnostic, and/or therapeutic agent) is administered to a mammal (e.g., a human) continuously for 1, 2, 3, or 4 hours; 1, 2, 3, or 4 times a day; every other day or every third, fourth, fifth, or sixth day; 1, 2, 3, 4, 5, 6, 7, 8, 9, or 10 times a week; biweekly; 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 times a month; bimonthly; 1, 2, 3, 4, 5, 6, 7, 8, 9, or 10 times every six months; 1, 2, 3, 4, 5, 6, 7, 8, 9, 10, 11, 12, 13, 14, 15, 16, 17, 18, 19, or 20 times a year; or biannually. An HSA linker conjugate (prepared with one or more of a binding, diagnostic, and/or therapeutic agent) may be administered at different frequencies during a therapeutic regime. In additional embodiments, an HSA linker conjugate (prepared with one or more of a binding, diagnostic, and/or therapeutic agent) may be administered to a patient at the same frequency or at a different frequency.

The amount of one or more diagnostic or therapeutic agents and an HSA linker, or any agent conjugated thereto, required to achieve the desired therapeutic effect depends on a number of factors, such as the specific diagnostic or therapeutic agent(s) chosen, the mode of administration, and clinical condition of the recipient. A skilled artisan will be able to determine the appropriate dosages of one or more diagnostic or therapeutic agents and an HSA linker, or any agent conjugated thereto, to achieve the desired results.

Single or multiple administrations of the compositions comprising an effective amount of an HSA linker conjugate (prepared with one or more of a binding, diagnostic, and/or therapeutic agent) can be carried out with dose levels and pattern being selected by the treating physician. The dose and administration schedule can be determined and adjusted based on the severity of the disease or condition in a mammal (e.g., a human), which may be monitored throughout the course of treatment according to the methods commonly practiced by clinicians or those described herein.

An HSA linker conjugate (prepared with one or more of a binding, diagnostic, and/or therapeutic agent) can be administered to a mammalian subject, such as a human, directly or in combination with any pharmaceutically acceptable carrier or salt known in the art. Pharmaceutically acceptable salts may include non-toxic acid addition salts or metal complexes that are commonly used in the pharmaceutical industry. Examples of acid addition salts include organic acids such as acetic, lactic, pamoic, maleic, citric, malic, ascorbic, succinic, benzoic, palmitic, suberic, salicylic, tartaric, methanesulfonic, toluenesulfonic, or trifluoroacetic acids or the like; polymeric acids such as tannic acid, carboxymethyl cellulose, or the like; and inorganic acids such as hydrochloric acid, hydrobromic acid, sulfuric acid phosphoric acid, or the like. Metal complexes include zinc, iron, and the like. One exemplary pharmaceutically acceptable carrier is physiological saline. Other physiologically acceptable carriers and their formulations are known to one skilled in the art and described, for example, in Remington's Pharmaceutical Sciences, (18th edition), ed. A. Gennaro, 1990, Mack Publishing Company, Easton, PA.

### Diagnostic and Therapeutic Applications

The HSA linker conjugates of the invention, as described herein, can be used for diagnostic and therapeutic applications in a human, including, for example, the diagnosis or treatment of proliferative diseases (e.g., cancers, such as melanoma, clear cell sarcoma, and renal cancer) and autoimmune diseases (e.g., multiple sclerosis, rheumatoid arthritis, and uveitis). The following discussion of human proliferative and autoimmune diseases is meant to provide the skilled practitioner with a general understanding of how HSA linker conjugates of the invention can be applied in diagnostic and therapeutic applications and is not meant to limit the scope of the present invention.

### Proliferative Diseases (Cancer)

An HSA linker conjugate of the invention can be used to diagnose, treat, prevent, or eliminate proliferative diseases such as, but not limited to, breast cancer, melanoma, clear cell sarcoma, renal cancer (e.g., renal cell carcinoma), prostate cancer, lung cancer, gastric cancer, and ovarian cancer. Binding moieties to be conjoined with an HSA linker of the invention for diagnostic or therapeutic application in a patient suspected of having or suffering from a proliferative disease may be chosen based on its ability to specifically bind, agonize, activate, antagonize, or inhibit target molecules (e.g., cell surface receptors such as tyrosine kinase receptors) associated with a proliferative disease. Binding moieties that target, for example, insulin-like growth factor receptor (IGFR, e.g., IGF1R and IGF2R), fibroblast growth factor receptor (FGFR), platelet-derived growth factor receptor (PDGFR), vascular endothelial growth factor receptor (VEGFR), tumor necrosis factor receptor (TNFR), epidermal growth factor receptor (EGFR, e.g., ErbB2 (HER2/neu)), Fc receptor, c-kit receptor, or mesenchymal epithelial transition factor receptor (c-mct; also known as hepatocyte growth factor receptor (HGFR)) can be conjoined to an HSA linker of the invention to diagnose or treat a proliferative disease. Specific binding of a cancer cell by an HSA linker conjugate can allow for detection (e.g., an HSA linker conjoined to a detectable label, as defined herein) or destruction (e.g., an HSA linker conjoined to a cytotoxic agent) of the bound cancer cell. Specific application of HSA linker conjugates of the invention for the treatment of breast and renal cancer is described below.

### Breast Cancer

Common forms of breast cancer include invasive ductal carcinoma, a malignant cancer in the breast's ducts, and invasive lobular carcinoma, a malignant cancer in the breast's lobules. Some types of breast cancer cells are known to highly express high levels of epidermal growth factor receptors, especially ErbB2 (i.e., HER2/neu). Aberrant signaling or unregulated activation of EGFRs has been linked to the development and progression of many cancers, including breast cancer. Uncontrolled cellular proliferation mediated via dysfunctional EGFR pathways can be found in a wide variety of solid tumors of epithelial origin and data have linked tumor EGFR expression, overexpression, and dysregulation to advanced disease, metastatic phenotype, resistance to chemotherapy, and an overall poorer prognosis.

An HSA linker of the invention conjoined to one or more binding moieties specific for an EGFR (e.g., anti-ErbB2; trastuzumab) can be used with a diagnostic (e.g., a detectable label) or cytotoxic, cytostatic, or therapeutic agent, as described herein, to diagnose or treat breast cancer. Alternatively, a bispecific HSA linker conjugate that consists of binding moieties specific for ErbB2 and ErbB3, such as "B2B3-1," described further herein, can be employed to diagnose or treat cancers, e.g., breast, kidney, ovarian, and lung cancers.

As described above, an HSA linker conjugate of the invention used to treat breast cancer can be administered prior to (e.g., neoadjuvant chemotherapy), concurrent with, or following (e.g., adjuvant chemotherapy) radiotherapy or surgical intervention. An HSA linker conjugate can also be co-administered with other compounds (e.g., antineoplastic agents, such as biological or chemical therapeutics) useful for the treatment of breast cancer. For example, the antineoplastic agents listed in Table 1, including mitotic inhibitors (e.g., taxanes), topoisomerase inhibitors, alkylating agents (including, e.g., platinum-based agents), selective estrogen modulators (SERM), aromatase inhibitors, antimetabolites, antitumor antibiotics (e.g., anthracycline antibiotics), anti-VEGF agents, anti-ErbB2 (HER2/neu) agents, and anti-ErbB3 agents, are known to be particularly useful for the treatment of breast cancer. An HSA linker conjugate of the invention can be administered by a clinician in combination with any compound, including those listed in Appendix C, known or thought to be beneficial for the treatment of breast cancer.

**Table 1: Exemplary antineoplastic agents for treatment of breast cancer in combination with HSA linker conjugates of the invention.**

| **Therapeutic Class** | **Exemplary Agent (Generic/Tradename)** | **Exemplary Dose** |
|---|---|---|
| Mitotic Inhibitors | paclitaxel (TAXOL®; ABRAXANE®) | 175 mg/m² |
| | docetaxel (TAXOTERE®) | 60-100 mg/m² |
| Topoisomerase Inhibitors | camptothecin | |
| | topotecan hydrochloride (HYCAMTIN®) | |
| | etoposide (EPOSIN®) | |
| Alkylating Agents | cyclophosphamide (CYTOXAN®) | 600 mg/m² |
| Platinum-Based Agents | cisplatin | 20-100 mg/m² |
| | carboplatin (PARAPLATIN®) | 300 mg/m² |
| | nedaplatin (AQUPLA®) | |
| | oxaliplatin (ELOXATIN®) | 65-85 mg/m² |
| | satraplatin (SPERA®) | |
| | triplatin tetranitrate | |
| Selective Estrogen Modulators (SERM) | tamoxifen (NOLVADEX®) | 20-40 mg/day |
| | raloxifene (EVISTA®) | 60 mg/day |
| | toremifene (FARESTON®) | |
| Antimetabolites | methotrexate | 40 mg/m² |
| | fluorouracil (5-FU) | 500 mg/m² |
| | raltitrexed | |
| Antitumor Antibiotics | doxorubicin (ADRIAMYCIN®) | 40-75 mg/m² |
| | epirubicin (ELLENCE®) | 60-120 mg/m² |
| Aromatase Inhibitors | aminoglutethimide (CYTADREN®) | 250-2000 mg/day |
| | anastrozole (ARIMIDEX®) | 1 mg/day |
| | letrozole (FEMARA®) | 2.5 mg/day |
| | vorozole | |
| | exemestane (AROMASIN®) | 25-50 mg/day |
| | testolactone | |
| | fadrozole (AFEMA®) | |
| Anti-VEGF Agents | bevacizumab (AVASTIN®) | 10 mg/kg |
| Anti-ErbB2 (HER2/neu) Agents | trastuzumab (HERCEPTIN®) | 2-8 mg/kg |
| | pertuzumab (OMNITARG®) | |
| Anti-ErbB3 (HER3) Agents | U3-1287 (AMG 888) | |

### Renal Cancer

Kidney cancers, such as renal cell carcinoma, are particularly resistant to traditional radiological and chemical therapies. As such, the application of biological therapeutics, conjoined with an HSA linker of the invention represents an attractive option for patients suffering from these cancers. For example, an HSA linker conjoined with binding moieties that agonize type I interferon or interleukin 2 receptors can be used to treat a renal cancer. As a solid tumor, binding moieties that target and inhibit tumor vascularization (e.g., anti-vascular endothelial growth factor (VEGF) antibodies such as bevacizumab) can also be used for therapeutic effect.

### Autoimmune Diseases

An HSA linker conjugate of the invention can be used to diagnose, treat, prevent, or stabilize autoimmune diseases and disorders in e.g., a human patient, such as, e.g., multiple sclerosis (MS), insulin-dependent diabetes mellitus (IDDM), rheumatoid arthritis (RA), uveitis, Sjögren's syndrome, Grave's disease, psoriasis, and myasthenia gravis. Autoimmune diseases and disorders are caused by self-reactive elements of the immune system (e.g., T cells, B cells, and self-reactive antibodies). As such, binding moieties that inhibit, block, antagonize, or deplete (e.g., anti-lymphocyte or anti-thymocyte globulins; basiliximab, daclizumab, or muromonab-CD3 monoclonal antibodies) self-reactive immune cells and antibodies can be conjoined with an HSA linker of the invention for therapeutic use. Binding moieties that function as inflammatory signaling inhibitors (ISI), as defined herein, can be conjoined to an HSA linker of the invention for the treatment of autoimmunity. In addition, binding moieties that inhibit or antagonize integrin function (e.g., an integrin antagonist, as defined herein) can ameliorate or halt disease progression.

In other embodiments of the invention, the binding moiety is a soluble TNF receptor, such as etanercept or lenercept; an antibody directed against a pro-inflammatory cytokine or a pro-inflammatory cell surface signaling molecule, such as adalimumab, certolizumab, inflixamab, golimumab, and rituxan; a dominant-negative pro-inflammatory cytokine variant, such as XENP345, XPROTM1595, anakinra, and variants disclosed in U.S. Patent Application Publication Nos. 20030166559 and 20050265962; an inhibitor of the signaling pathways downstream of pro-inflammatory cytokine or pro-inflammatory cell surface signaling molecules, such as DE 096, 5-amino-2-carbonylthiopene derivatives (as described in WO2004089929), ARRY-797, BIRB 796 BS, (1-5-tert-butyl-2-p-tolyl-2H-pyrazol-3-yl)-3-[4-2(morpholin-4-yl-ethoxy)-naphtalen-1-yl]-urea, CHR-3620, CNI-1493, FR-167653 (Fujisawa Pharmaceutical, Osaka, Japan), ISIS 101757 (Isis Pharmaceuticals), ML3404, NPC31145, PD169316, PHZ1112, RJW67657, 4-(4-(4-fluorophenyl)-1-(3-phenylpropyl)-5-(4-pyridinyl)-1H-imidazol-2-yl)-3-butyn-1-ol, SCIO-469, SB202190, SB203580, (4-(4-fluorophenyl)-2-(4-methylsulfinylphenyl)-5-(4-pyridyl)1H-imidazole), SB239063, trans-1-(4-hydroxycyclohexyl)-4-(4-fluorophenyl-methoxypyridimidin-4-yl)imidazole, SB242235, SD-282, SKF-86002, TAK 715, VX702, and VX745; or an inhibitor of TNF-alpha converting enzyme (TACE), such as BB-1101, BB-3103, BMS-561392, butynyloxyphenyl β-sulfone piperidine hydroxomates, CH4474, DPC333, DPH-067517, GM6001, GW3333, Ro 32-7315, TAPI-1, TAPI-2, and TMI 005); or an anti-idiotypic agent, such as monoclonal antibodies, LJP 394 (abetimus, RIQUENT®, La Jolla Pharmaceuticals).

In other embodiments, the binding moiety is an interferon, as described herein. Binding moieties that can be conjoined to an HSA linker of the invention include, e.g., interferon-beta (REBIF® (IFN-β-1a), AVONEX® (TFN-β-1a), and BETASERON® (IFN-β-1b)), interferon-t (TAUFERONTM), interferon-alpha (e.g., ROFERON-A® (IFN-α-2a), INTRON-A® (IFN-α-2b), REBETRON® (IFN-α-2b), ALFERON-N® (IFN-α-n3), PEG-INTRON® (IFN-α-2b covalently conjugated with monomethoxy polyethylene glycol), INFERGEN® (a non-naturally occurring type 1 interferon with 88% homology to IFN-α-2b), or PEGASYS® (pegylated IFN-α-1a)), and ACTIMMUNE® (IFN-g-1b).

The present invention further provides HSA linker conjugates with binding moieties that antagonize these pro-inflammatory molecules or their specific receptors to treat autoimmunity. Specific application of HSA linker conjugates of the invention for the diagnosis and treatment of MS and RA are described below.

### Multiple Sclerosis

Multiple sclerosis (MS) is a neurological disease characterized by irreversible degeneration of the nerves of the central nervous system (CNS). Although the underlying cause is unclear, the neurodegeneration in MS is the direct result of demyelination, or the stripping of myelin, a protein that normally lines the outer layer and insulates the nerves. T cells play a key role in the development of MS. Inflamed MS lesions, but not normal white matter, can have infiltrating CD4⁺ T cells that respond to self antigens presented by MHC class II-linked molecules such as human HLA-DR2. The infiltrating CD4 T cells (T_{H}1 cells) produce the pro-inflammatory cytokines IL-2, IFN-γ, and TNF-α that activate antigen-presenting cells (APCs) such as macrophages to produce additional pro-inflammatory cytokines (e.g., IL-1β, IL-6, IL-8, and IL-12. IL-12 induces further IFN-γ synthesis. The result is progressive demyelination of neuronal sheaths, leading to human disease.

HSA linker conjugates can be used to aid in the diagnosis of MS. Diagnostic HSA linker conjugates that include binding moieties, as defined herein, that specifically target one or more (e.g., a bispecific HSA linker conjugate) immune cell activation markers (e.g., CD69, CD28, HLA-DR, and CD45). An imbalance of one or more of these pro-inflammatory or immune cell activation mediators relative to other factors or cells may be using measured by an HSA linker conjugate conjoined with a diagnostic agent (e.g., a radioisotope or fluorochrome).

An HSA linker conjugate of the invention can be used to treat a person at risk of developing or suffering from MS or to prevent, ameliorate, or cure the symptoms of the disease. For example, binding moieties, as defined herein, that specifically target and antagonize α4 integrin (e.g., natalizumab), CD52 (e.g., alemtuzumab), CD80, P-selectin, sphingosine-1-phosphate receptor-1 (S1PR1), hyaluronate receptor, leukocyte function antigen-1 (LFA-1), CD11 (e.g., efalizumab), CD18, CD20 (e.g., rituximab), CD85, ICOS ligand, CCR2, CXCR3, or CCR5 can be useful when conjoined to an HSA linker of the invention for therapeutic use in a patient suffering from MS. Similary, binding moieties that neutralize type I interferons (e.g., interferons -alpha and -beta) or that antagonize type I interferon receptors (e.g., IFNαR1) can also be conjoined to an HSA linker of the invention for therapeutic application.

### Rheumatoid arthritis

Rheumatoid arthritis (RA) is a chronic, inflammatory autoimmune disorder that causes the immune system to attack the joints. It is a disabling and painful inflammatory condition, which can lead to substantial loss of mobility due to pain and joint destruction. RA is a systemic disease, often affecting extra-articular tissues throughout the body including the skin, blood vessels, heart, lungs, and muscles.

Patients suffering from RA frequently have an increase in cellular expression of the HLA-DR4/DR1 cluster. HSA linker conjugates specific for one or both of these cell surface molecules are useful for the diagnosis of RA.

An HSA linker conjugate of the invention can be used to treat a person at risk of developing of suffering from RA to prevent, ameliorate, or cure the symptoms of the disease. For example, binding moieties, as defined herein, that specifically target and antagonize TNF-α (e.g., etanercept, infliximab, and adalimumab), IL-1 (e.g., anakinra), or CTLA-4 (e.g., abatacept). Binding moieties that specifically target and deplete B cells (e.g., an anti-CD20 antibody, such as rituximab) can also be conjoined to the HSA linker described herein to treat or prevent RA.

### Uveitis

Uveitis specifically refers to inflammation of the middle layer of the eye, but may refer to any inflammatory process involving the interior of the eye. Uveitis may be autoimmune or idiopathic in origin

An HSA linker conjugate of the invention can be used to treat a person at risk of developing of suffering from autoimmune uveitis to prevent, ameliorate, or cure the symptoms of the disease. For example, alpha-fetoprotein (e.g., human AFP; NCBI Accession No. NM_001134), or biologically-active fragments thereof, can be conjoined to an HSA linker of the invention to reduce or eliminate inflammation associated with autoimmune or idiopathic uveitis.

### Kits

The invention provides kits that include a pharmaceutical composition containing an HSA linker of the invention, and one or more of a binding moiety (e.g., antibodies or antibody fragments), a diagnostic agent (e.g., radionuclide or chelating agents), and a therapeutic agent (e.g., cytotoxic or immunomodulatory agents) with reagents that can be used to conjugate them to the HSA linker, if necessary, and including a pharmaceutically-acceptable carrier, in a therapeutically effective amount for treating a disease or condition (e.g., a cancer, autoimmune disease, or cardiovascular disease).. The kits include instructions to allow a practitioner (e.g., a physician, nurse, or patient) to administer the composition contained therein.

Preferably, the kits include multiple packages of the single-dose pharmaceutical composition(s) containing an effective amount of an HSA linker of the invention, or any binding (e.g., antibodies or antibody fragments (e.g., scFv)), diagnostic (e.g., radionuclide or chelating agents), and/or therapeutic (e.g., cytotoxic or immunomodulatory agents) conjugate thereof: Optionally, instruments or devices necessary for administering the pharmaceutical composition(s) may be included in the kits. For instance, a kit of this invention may provide one or more pre-filled syringes containing an effective amount of an HSA linker of the invention, or any binding, diagnostic, and/or therapeutic agent conjugated thereto. Furthermore, the kits may also include additional components such as instructions or administration schedules for a patient suffering from a disease or condition (e.g., a cancer, autoimmune disease, or cardiovascular disease) to use the pharmaceutical composition(s) containing an HSA linker of the invention, or any binding, diagnostic, and/or therapeutic agent conjugated thereto.

It will be apparent to those skilled in the art that various modifications and variations can be made in the compositions, methods, and kits of the present invention without departing from the spirit or scope of the invention. Thus, it is intended that the present invention cover the modifications and variations of this invention provided they come within the scope of the appended claims and their equivalents.

### EXAMPLES

The following examples arc provided by way of illustration only and not by way of limitation. Those of skill in the art will readily recognize a variety of non-critical parameters that could be changed or modified to yield essentially the same or similar results.

### Example 1: Methods to identity residues in the HSA linker for site-specific conjugation of cytotoxic or cytostatic drugs.

To identify sites for specific conjugation of drug to HSA the crystal structure was studied and surface exposed serine and threonine residues were identified. These particular surface-exposed amino acids can then be mutated to cysteine allowing drug conjugation to the substituted cysteine using a thiol-specific conjugating agent such as maleimide. Mild reduction may be required prior to drug conjugation. The number of drugs conjugated is controlled by the number of surface exposed cysteine residues introduced into HSA. Serine and threonine are selected as the most suitable residues for mutation as they share the most structural identity with cysteine, however, other surface exposed residues may also be mutated to cysteine and successfully conjugated to cytostatic or cytotoxic drugs.

The crystal structure of HSA is deposited in the RSCB Protein Data Bank (1bm0 - Sugio et al., "Crystal structure of human serum albumin at 2.5 A resolution," Protein Eng. 12:439-446 (1999)). This structure was analyzed using the DeepView Swiss PDB Viewer downloaded from the Swiss Institute of Bioinformatics. Serine and threonine residues with 50%, 40%, and 30% surface exposure were identified as the most suitable for mutation to cysteine (Table 2). Mutations can be introduced using standard molecular biology procedures. Conjugation of a thiol reactive drug or chelating agent to introduced cysteines can be tested using standard protein chemistry techniques.

**Table 2**

| % Surface Exposure | Residue |
|---|---|
| 50 | T496 |
| 40 | S58 |
| 30 | T76, T79, T83, T125, T236, S270, S273, S304, S435, T478, T506, T508 |

### Example 2: Methods to identify residues in the HSA linker for introduction of asparagine-linked glycosylation sites.

To identify regions for introduction of asparagine-linked glycosylation sites in HSA, the crystal structure was studied to identify surface exposed (>30%) asparagine, serine and threonine residues that would be suitable for mutation. Glycosylation occurs on asparagine residues when the consensus sequence asparagine - x - serine/threonine is present, where x cannot be a proline. Table 2 lists possible mutation sites in HSA for the introduction of asparagine-linked glycosylation.

**Table 3**

| Residue | Proposed Mutation |
|---|---|
| Gln32 | Asn |
| Val46 | Ser/Thr |
| Asp56 | Asn |
| Asp63 | Ser/Thr* |
| Glu231 | Asn |
| Asp237 | Asn |
| Gln268 | Asn |
| Asp269 | Ser/Thr |
| Glu285 | Asn |
| Ala320 | Ser/Thr* |
| Asp340 | Asn |
| Glu354 | Asn |
| Gln437 | Asn |
| Glu425 | Asn |
| Glu465 | Asn |
| Asp494 | Asn* |

| | |
|---|---|
| *These mutations have also been reported to occur very rarely in HSA (Carlson et al., "Alloalbuminemia in Sweden: Structural study and phenotypic distribution of nine albumin variants," Proc.Nat.Acad.Sci.USA 89:8225- 8229 (1992); Madison et al., "Genetic variants of human serum albumin in Italy: point mutants and a carboxyl-terminal variant," Proc.Nat.Acad.Sci.USA 91:6476-6480 (1994); Hutchinson et al., "The N-terminal sequence of albumin Redhill, a variant of human serum albumin," FEBS Lett. 193:211-212 (1985); Brennan et al., "Albumin Redhill (-1 Arg, 320 Ala-Thr): a glycoprotein variant of human serum albumin whose precursor has an aberrant signal peptidase cleavage site," Proc.Nat.Acad.Sci.USA 87:26-30 (1990); Minchiotti et al., "Structural characterization of four genetic variants of human serum albumin associated with alloalbuminemia in Italy," Eur.J.Biochem. 247:476-482 (1997); Peach et al., "Structural characterization of a glycoprotein variant of human serum albumin: albumin Casebrook (494 Asp-Asn)," Biochim.Biophys.Acta 1097:49-54 (1991)). | |

### Example 3

B2B3-1 is a bispecific scFv antibody fusion molecule consisting of B1D2, a human anti-ErbB2 scFv antibody (SEQ ID NO:27) and H3, a human anti-ErbB3 scFv (SEQ ID NO:26). The two scFvs are joined by a modified human serum albumin (HSA) linker. The anti-ErbB3 scFv, H3, is recombinantly fused to the amino terminus of the HSA linker incorporating a short connector polypeptide and the anti-ErbB2 scFv, B1D2, is recombinantly fused to the carboxy terminus of the modified HSA linker incorporating an additional short connector polypeptide. Each connector polypeptide was selected based on protease resistance properties. The modified HSA linker contains two amino acid substitutions. A cysteine residue at position 34 of native HSA was mutated to serine in order to reduce potential protein heterogeneity due to oxidation at this site. An asparagine residue at amino acid 503 of native HSA, which in native HSA is sensitive to deamidation and can result in decreased pharmacologic half-life, was mutated to glutamine. B2B3-1 selectively binds ErbB2 over-expressing tumors by virtue of its high affinity anti-ErbB2 scFv binding moiety, which has a kD in the range of 10.0 nM to 0.01 nM and more preferably a kD of about 0.3 nM. Subsequent binding of ErbB3 by the anti-ErbB3 scFv, which has a kD in the range of 50 to 1 nM and more preferably about 16 nM, prevents HRG induced phosphorylation of ErbB3. The modified HSA linker confers an extended circulating half-life on the bispecific molecule. B2B3-1 has a molecular weight of 119.6 kDa and is not glycosylated.

B2B3-1 inhibits ligand-induced phosphorylation of ErbB3 with sub-nanomolar potency by exploiting the abundant expression of its dimerization partner, ErbB2, to specifically target cancer cells that express both receptors.

### Example 4

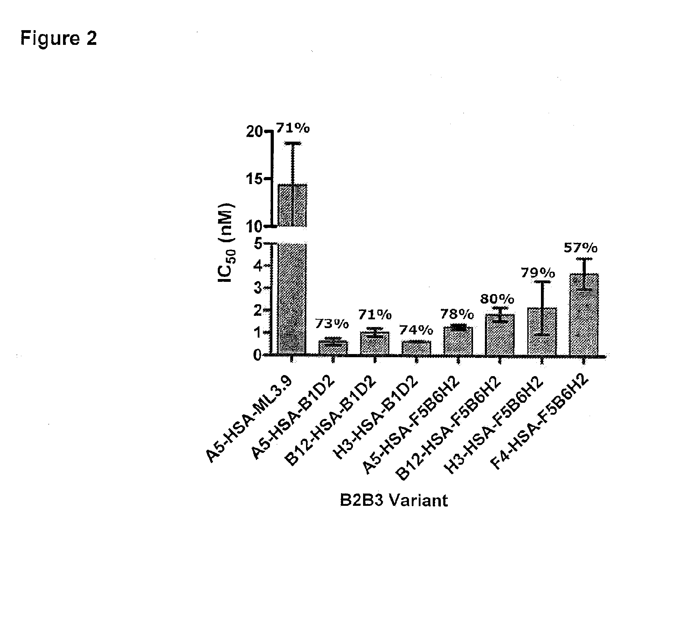

As shown in Figure 2, B2B3-1 variants inhibit HRG-induced pErbB3 in ZR75-1 breast cancer cells. ZR75-1 breast cancer cells were treated with a dose range of B2B3-1 variants for 24 hours followed by HRG stimulation. pErbB3 levels were measured in cell lysates and IC₅₀ values were calculated together with the percent of inhibition. Shown are the mean IC₅₀ values (Y axis) with error bars representing replicate experiments. Percent inhibition values are shown above the corresponding bar.

### Example 5

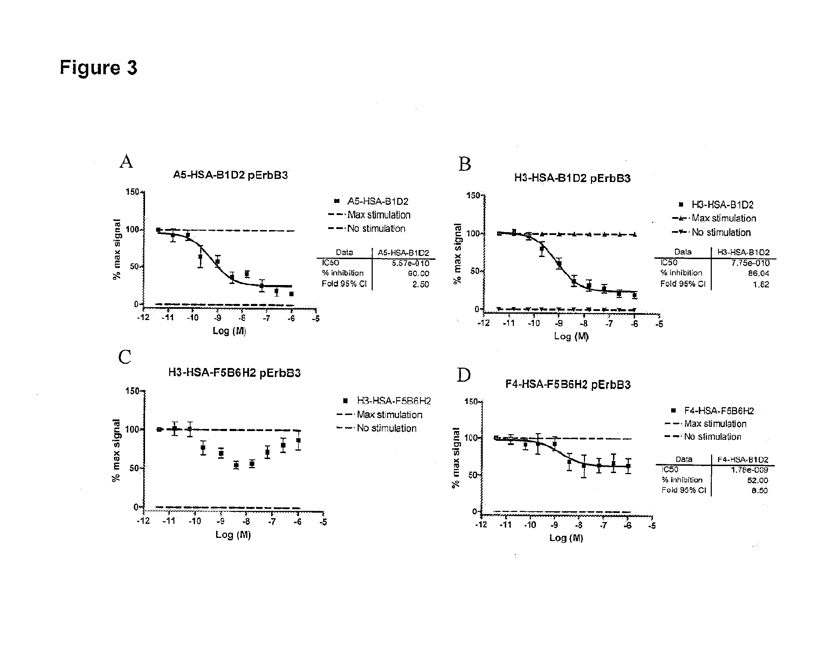

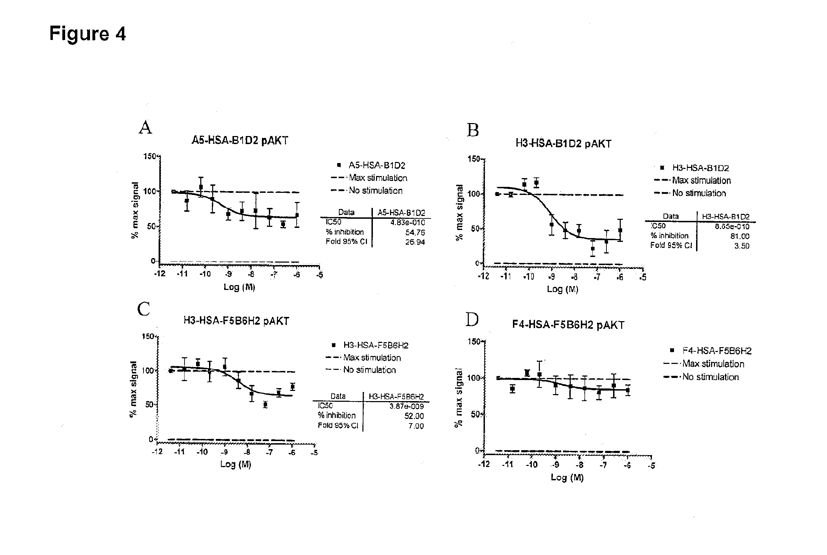

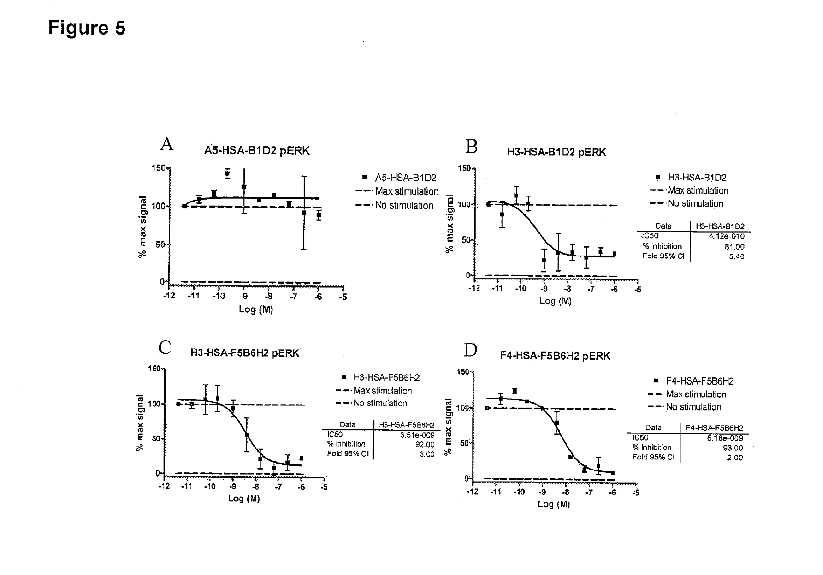

Inhibition of phosphorylated ErbB3 (Figures 3A-D), AKT (Figures 4A-D), and ERK (Figures 5A-D) following 24 hour pre-treatment with B2B3-1 variants A5-HSA-B1D2 (panel A of Figures 3-5), H3-HSA-B1D2 (panel B of Figures 3-5), H3-HSA-F5B6H2 (panel C of Figures 3-5), and F4-HSA-F5B6H2 (panel D of Figures 3-5). BT474 breast cancer cells were treated with a dose range of B2B3-1 variants for 24 hours followed by HRG stimulation. pErbB3, pAKT, and pERK levels were measured in cell lysates and IC₅₀ values were calculated together with the percent of inhibition.

### Example 6

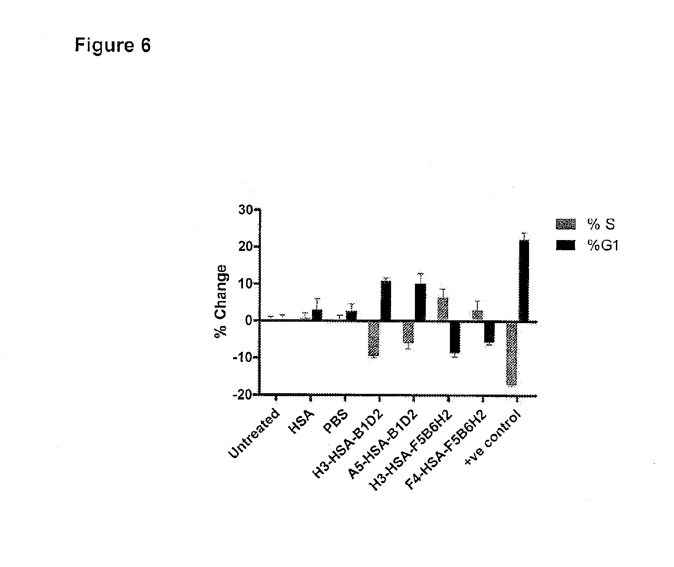

As shown in Figure 6, treatment of BT474 breast tumor cells with B2B3-1 variants causes G1 cell cycle arrest and a decrease in the population of cells in S phase. BT474 cells were treated with 1 µM of B2B3-1 variants and controls for 72 hours. After the end of treatment, cells were trypsinized, gently resuspended in hypotonic solution containing propidium iodide and single cells were analyzed by flow cytometry. Cell cycle distribution in G1 and S phases was measured using curve-fitting algorithms designed for cell cycle analysis (FlowJo software cell cycle platform).

### Example 7

B2B3-1 (SEQ ID NO:16) inhibits ErbB3 activation, utilizing the abundant expression of its dimerization partner, ErbB2, to target tumor cells. A high affinity anti-ErbB2 scFv antibody, B1D2, facilitates targeting of B2B3-1 to tumor cells over-expressing ErbB2. B1D2 is connected by a modified HSA linker to a lower affinity anti-ErbB3 scFv antibody, H3, which blocks binding of ErbB3's ligand, HRG, thereby inhibiting ErbB3 phosphorylation and downstream AKT signaling. The ErbB2 binding scFv, B1D2, is derived from parent scFv C6.5, which possesses neither agonistic nor antagonistic activity. B1D2, therefore, likely functions solely as a targeting agent. The lower affinity binding of the ErbB3 binding scFv prevents strong binding of B2B3-1 to normal, non-cancerous tissues which express ErbB3 but little or no ErbB2, thereby reducing the potential for nonspecific toxicity. In tumor cells expressing both ErbB2 and ErbB3, the avidity effect of bispecific B2B3-1 binding to both receptors overcomes the low affinity of the ErbB3 scFv allowing strong inhibition of HRG interaction.

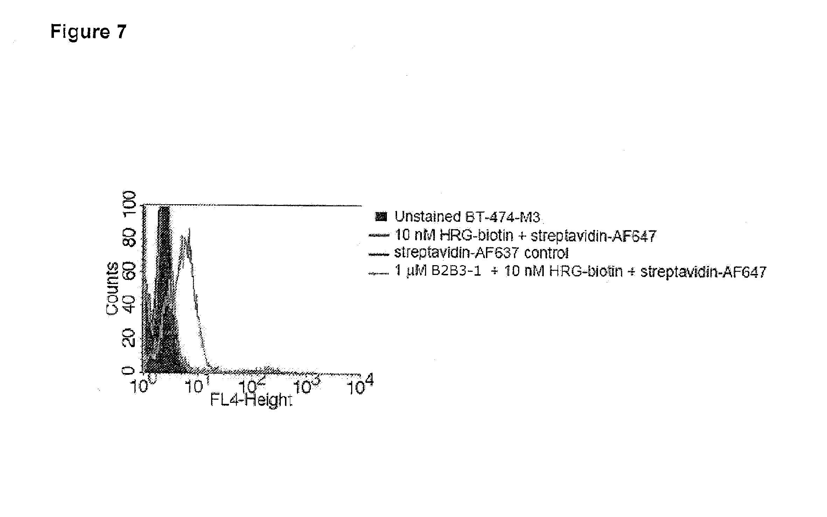

The ability of B2B3-1 to inhibit HRG binding to ErbB3 was investigated using flow cytometry (FACS). Cells of the breast cancer cell line BT-474-M3 (a variant of BT-474 that over-express ErbB2), were pretreated with 1 µM B2B3-1 then incubated with 10 nM biotinylated HRG 1β EGF domain. After extensive washing, binding was assessed using streptavidin-AlexaFluor 647 conjugate. All incubations were performed at 4°C. Figure 7 shows that B2B3-1 is capable of blocking the binding of HRG to ErbB3.

### Example 8

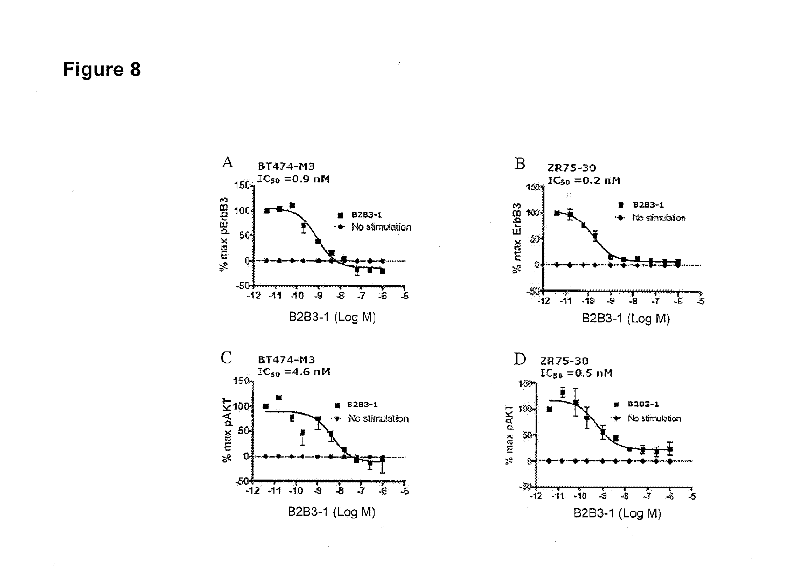

After demonstrating B2B3-1's ability to block HRG binding to ErbB3, we investigated the effect of B2B3-1 on ErbB3 signaling *in vitro* on two cell lines that express ErbB3 and over-express ErbB2. Breast cancer cell lines BT-474-M3 and ZR75-3 0 were serum starved overnight, pre-treated with a dose titration of B2B3-1 for 24 hours and then stimulated for 10 minutes with 5 nM of HRG 1β EGF domain. The phosphorylation status of ErbB3 and AKT was then examined using an ELISA assay. The results show that B2B3-1 inhibits HRG induced activation of both ErbB3 and AKT phosphorylation in a dose-dependent manner and with potent IC₅₀s in both cell lines (Figures 8A-D).

### Example 9

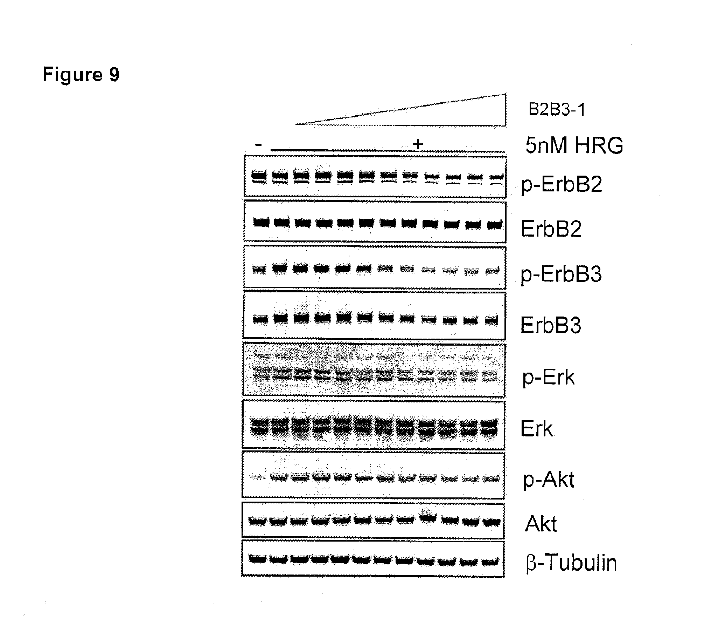

Figure 9 shows the effect of B2B3-1 treatment on signaling proteins in BT474 breast cancer cells. Cells were treated with a dose range of B2B3-1 for 24 hours, followed by heregulin stimulation. Levels of pErbB2, pErbB3, pErk and pAKT and their corresponding total protein levels were determined on cell lysates by Western blot analysis.

### Example 10

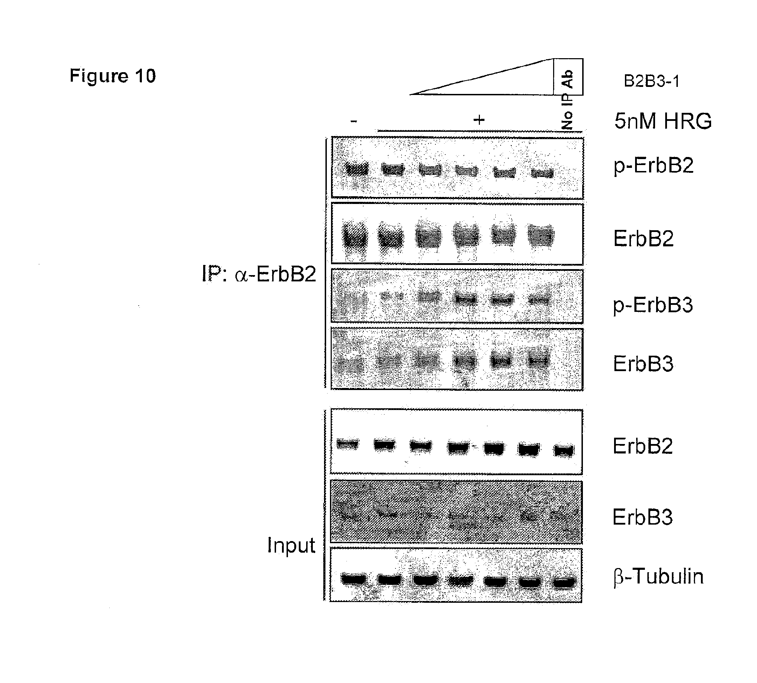

Figure 10 shows the immunoprecipitation-Western blot analysis of B2B3-1 treated BT474 breast cancer cells. Cells were treated with a dose range of B2B3-1 for 24 hours, followed by heregulin stimulation. ErbB2-associated complexes were isolated from cell lysates using an anti-ErbB2 antibody followed by Western blot analysis to detect pErbB2 and pErbB3 and their corresponding total protein levels.

### Example 11

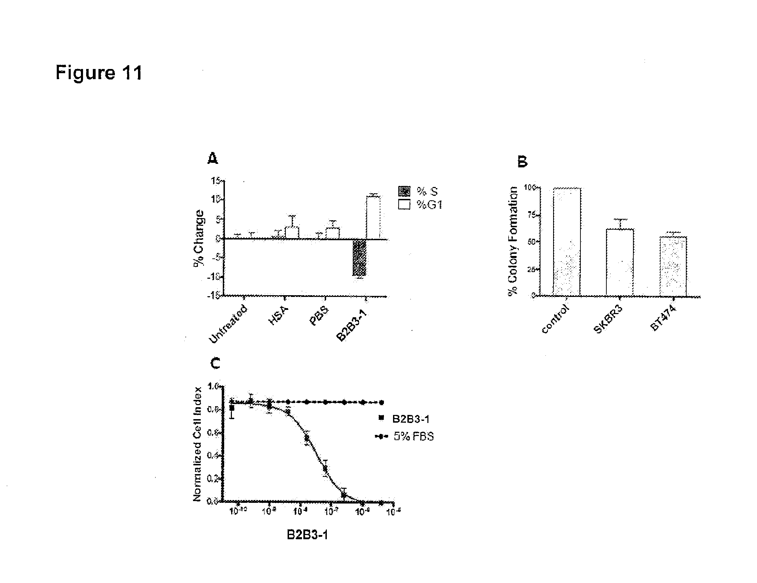

The anti-tumor activity of B2B3-1 was investigated *in vitro* using a number of assays. In the first assay, the effect of B2B3-1 on the accumulation of BT-474 or SKBR3 cells in G1 phase and the concomitant decrease in S phase of the cell cycle was examined. Briefly, cells were treated with 1 µM B2B3-1 or PBS vehicle for 72 hours. After the end of treatment, cells were trypsinized, gently resuspended in hypotonic solution containing propidium iodide and single cells were analyzed by flow cytometry. Cell cycle distribution in G1 and S phases was measured using curve -fitting algorithms designed for cell cycle analysis (FlowJo software cell cycle platform). B2B3-1 was found to modestly decrease the percentage of cells in S phase and increase the population of cells in G1 phase (Figure 11A). In a second experiment, the number of cell colonies formed following treatment with B2B3-1 was studied. BT-474 and SKBR3 breast cancer cells were plated in the presence of 1 µM B2B3-1 and compared to cells plated in media only. Media only or media including treatment was replenished every 3 days. After 14 days the number of colonies were counted and compared to untreated cells. Figure 11B illustrates the 40-45% decrease in the number of colonies formed when cells are treated with B2B3-1 compared to control cells. Finally, the ability of B2B3-1 to inhibit cell proliferation was assessed in a cell impedance assay using a Real-Time Cell Electronic Sensing System (RT-CES: ACEA Biosciences). BT- 474 cells were seeded on plates integrated with microelectronic sensor arrays and treated with a dose titration of B2B3-1 or media only for 72 hours. Data reflecting the generation of cell-electrode impedance response was collected every hour for 72 hours and IC₅₀ values were calculated 68 hours after treatment. Figure 11C illustrates that B2B3-1 was able to inhibit impedance of BT-474 cells with an IC₅₀ of 33 nM.

### Example 12

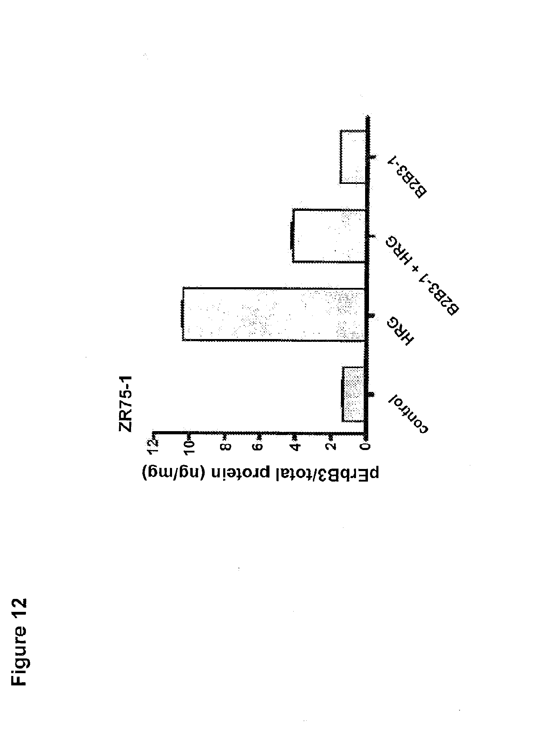

We also investigated whether B2B3-1 exhibits agonistic activity based on its ability to simultaneously bind and cross-link ErbB2 and ErbB3 receptors. Serum starved ZR75-1 breast cancer cells were incubated with 1 µM B2B3-1 or PBS vehicle for 24 hours. Cells were also treated with B2B3-1 or PBS vehicle for 24 hours followed by a 10-minute stimulation with 5 nM HRG 1β EGF domain. Cells were lysed and the pErbB3 content of the lysates was assessed by ELISA. Figure 12 shows that cells treated with B2B3-1 alone contained comparable levels of phosphorylated ErbB3 as untreated cells, indicating that B2B3-1 is non-agonistic.

### Example 13

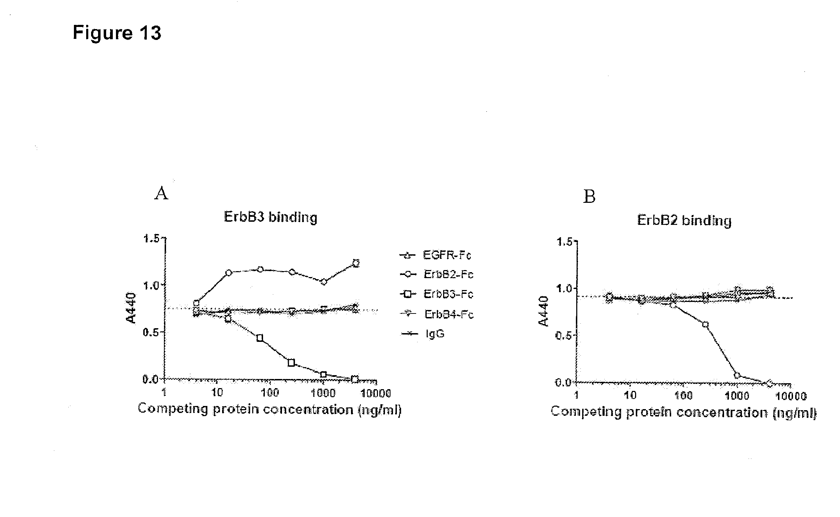

The ability of B2B3-1 to bind with specificity to ErbB2 and ErbB3 and not to related ErbB family members, EGFR and ErbB4, was investigated by ELISA. Plates were coated with the recombinant extracellular domain of either ErbB2 or ErbB3. Plates were blocked and incubated with a half-maximal binding concentration of B2B3-1 in the presence of a dilution series of competing recombinant extracellular domains of EGFR, ErbB2, ErbB3 or ErbB4. The results show only soluble ErbB2 extracellular domain blocked B2B3-1 binding to ErbB2-coated plates (Figure 13A). Likewise, only soluble ErbB3 extracellular domain blocked B2B3-1 binding to ErbB3-coated plates (Figure 13B). These results demonstrate the specificity of the anti-ErbB2 arm B1D2 for ErbB2, and of the anti-ErbB3 arm H3 for ErbB3. The increased signal observed when soluble ErbB2 extracellular domain was incubated with B2B3-1 on the ErbB3 coated plate is assumed to be due to formation of an ErbB2, ErbB3, B2B3-1 complex on the plate.

### Example 14

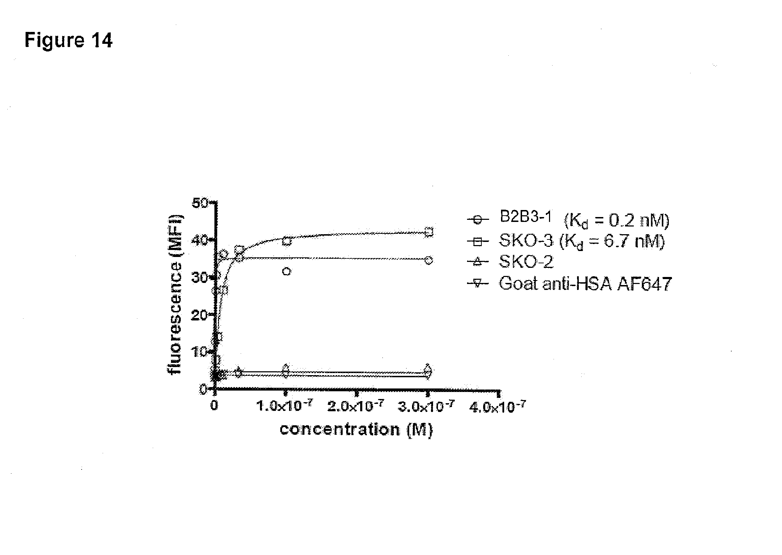

The ability of B2B3-1 to bind avidly to tumor cells expressing both ErbB2 and ErbB3 was studied. SKO2 and SKO3 are variants of B2B3-1 that lack the ability to interact with ErbB2 or ErbB3, respectively. MALME-3M melanoma cells, which express approximately equal numbers of ErbB2 and ErbB3 receptors, were incubated with a dilution series of B2B3-1, SKO2, or SKO3 in the presence of saturating concentrations of a goat anti-HSA Alexafluor-647 conjugated antibody. Cell binding was assessed by flow cytometry and apparent binding affinities were determined for each molecule. Control cells were incubated with secondary antibody alone. No measurable cell binding was observed for SKO2, which retains only low affinity binding to ErbB3 and lacks ErbB2 binding activity. SKO3, which retains a functional, high affinity ErbB2 binding scFv but lacks the ability to bind ErbB3 had a K_{D} of 6.7 nM. B2B3-1 bound cells with a K_{D} of 0.2 nM, demonstrating avidity binding of this bispecific molecule (Figure 14).

### Example 15

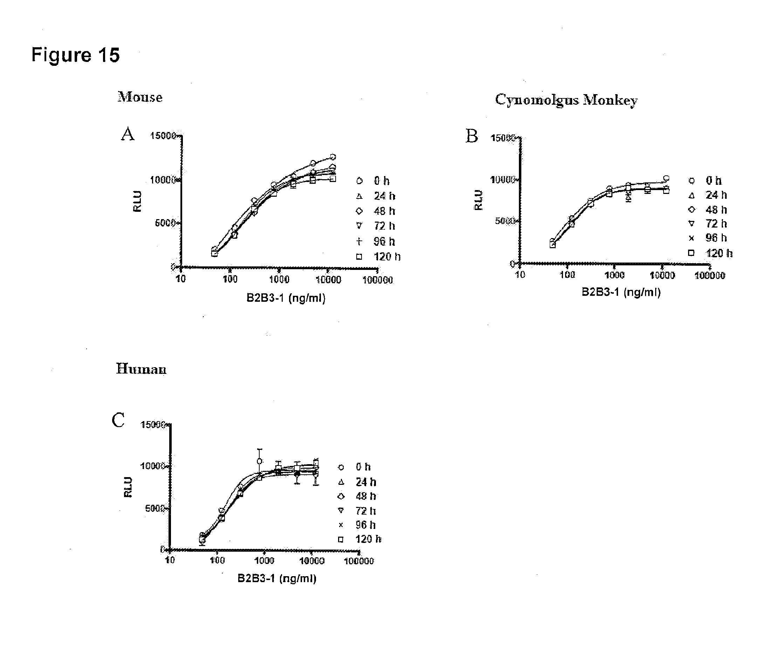

The stability of B2B3-1 under physiological conditions was assessed by incubating 100 nM B2B3-1 in human, Cynomolgus monkey, or mouse serum at 37°C for a period of 120 hours. Samples were removed at 0, 24, 48, 72, 96 and 120 hours and the ability of B2B3-1 to bind both ErbB2 and ErbB3 was measured by ELISA. This ELISA involves coating a 96-well plate with recombinant ErbB2 extracellular domain overnight followed by a blocking step and then incubation with a dilution series of B2B3-1. Plates are then incubated with an Fc-ErbB3 extracellular domain fusion protein followed by a goat antihuman-Fc-HRP conjugate. Plates are developed by addition of supersignal chemiluminesccnse substrate. Figures 15A-C show that B2B3-1 remains stable in serum from all three species under physiological conditions, retaining comparable ability to bind both ErbB2 and ErbB3 at all time points measured.

### Example 16: B2B3-1 dose response in BT-474-M3 breast cancer xenograft model.

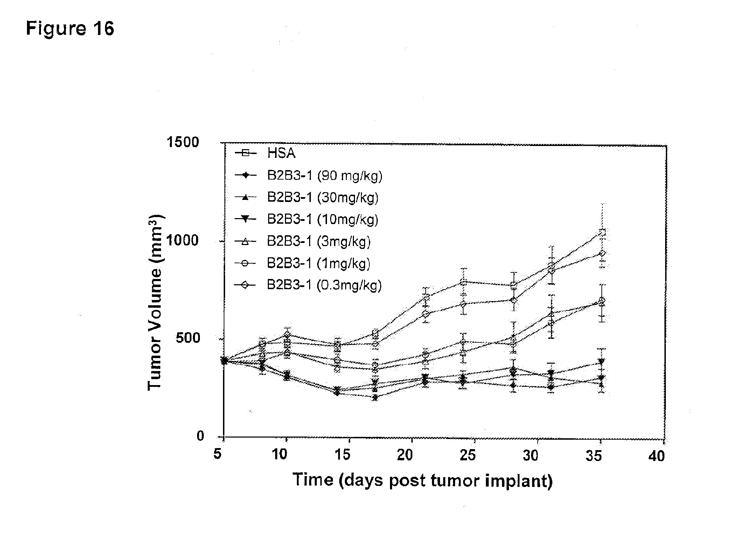

The efficacy of B2B3-1 *in vivo* was assessed using nude mice bearing BT-474-M3 xenografts. Ten mice per group were treated with 12 doses of 0.3, 1, 3, 10, 30 or 90 mg/kg of B2B3-1 every 3 days. Control groups were administered PBS vehicle or HSA at an equimolar dose to the 90 mg/kg B2B3-1 dose. All doses were administered interperitoneally (i.p.). Tumor size was measured twice a week and the corresponding tumor volume was calculated. The results show that B2B3-1 treatment leads to significant reduction in BT-474-M3 tumor size as compared to the control group (Figure 16). Complete regressions were observed in each of the B2B3-1 treatment groups except mice treated with the lowest dose of B2B3-1 (0.1 mg/kg).

### Example 17

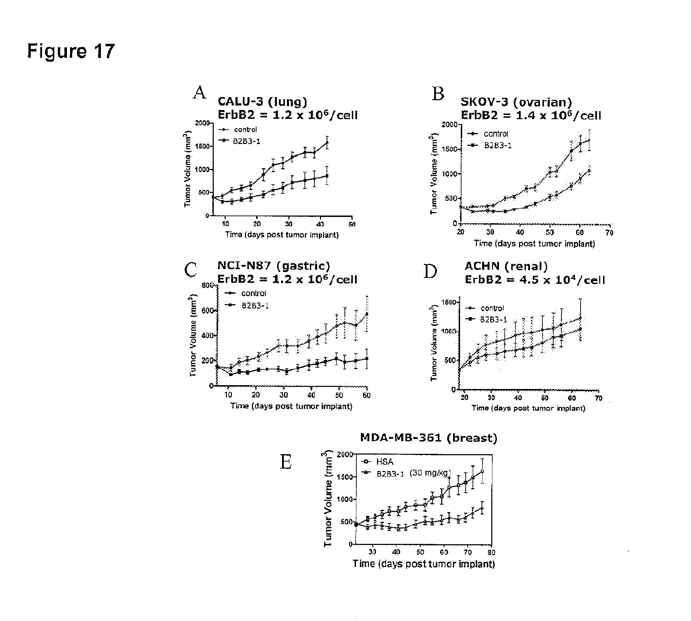

As shown in Figures 17A-E, B2B3-1 is effective in multiple xenograft models in an ErbB2 dependent manner. B2B3-1 was efficacious in the Calu-3 (Figure 17A), SKOV-3 (Figure 17B), NCI-N87 (Figure 17C), and MDA-MB-361 (Figure 17E) xenograft models expressing ErbB2 at > 1 x 10⁵ receptors/cell but worked less well in the ACHN (Figure 17D) xenograft model which expresses 4.5 x 10⁴ ErbB2 receptors/cell. Mice were treated with 30 mg/kg of B2B3-1 every 3 days.

### Example 18

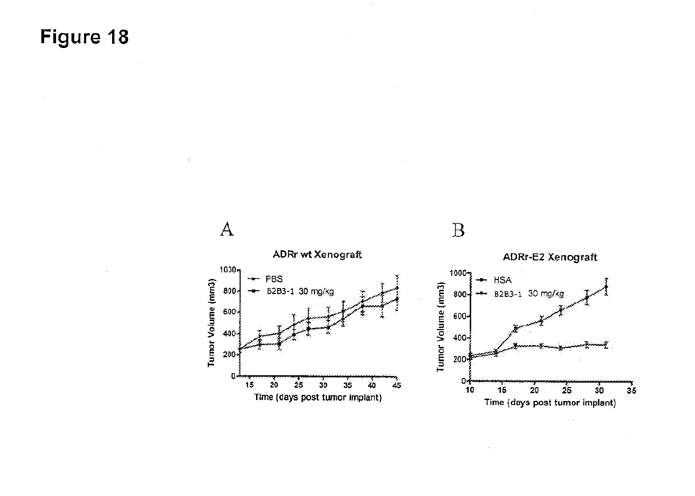

Over-expression of ErbB2 converts B2B3-1 non-responder ADRr breast cancer xenograft model into a responder to B2B3-1 (Figures 18A B). ErbB2 was over-expressed in ADRr breast cancer cells using a retroviral expression system. Transfected cells expressing high levels of ErbB2 (ADRr -E2) were selected using FACS and subsequently injected subcutaneously into nude mice to establish xenograft tumors. Mice were treated with 30 mg/kg of B2B3-1 every 3 days. While no response to B2B3-1 was observed in wild type ADRr xenografts (Figure 18A), ADRr-E2 xenografts (Figure 18B) responded to B2B3-1.

### Example 19

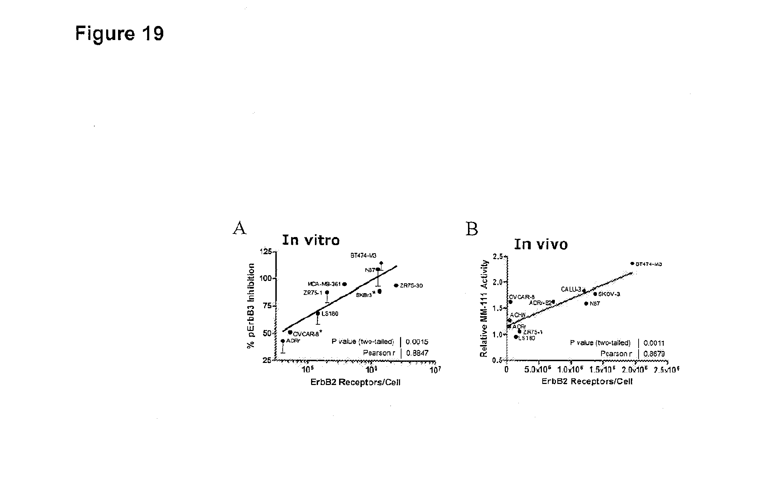

As shown in Figures 19A-B, B2B3-1 activity correlates positively with ErbB2 expression levels *in vitro* (Figure 19A) and *in vivo* (Figure 19B). B2B3-1 inhibition of ErbB3 phosphorylation was determined in 9 tumor cell lines with expression levels of ErbB2 ranging from 5 x 10⁴ receptors/cell to 2.4 x 10⁶ receptors/cell using an ELISA assay. The extent of B2B3-1's ability to inhibit ErbB3 phosphorylation to basal levels (% pErbB3 inhibition) was found to correlate positively with ErbB2 expression levels. Similarly, B2B3-1 activity was assessed in 10 tumor xenograft models expressing low to high levels of ErbB2. Xenograft response also correlated positively with ErbB2 expression levels.

### Example 20

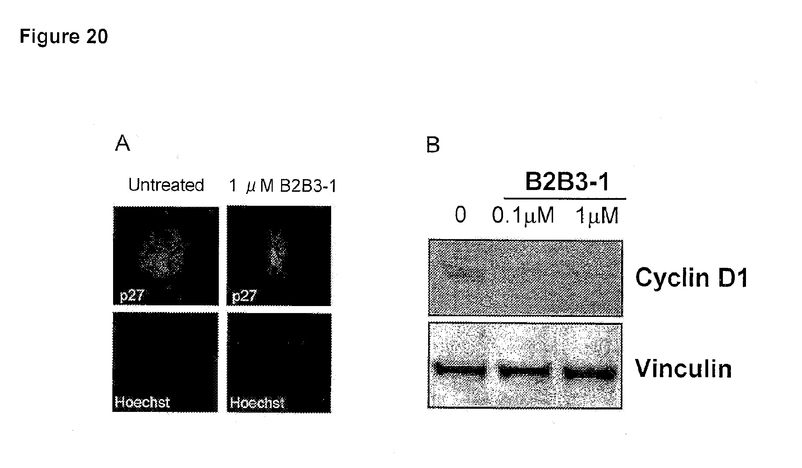

B2B3-1 treatment of BT474-M3 breast tumor cells results in translocation of p27^{kip1} to the nucleus (Figure 20A). BT474-M3 cells were treated with 1 µM of B2B3-1 for 6 hours. The subcellular location of p27^{kip1} was assessed using immunofluorescence techniques. In cells treated with B2B3-1, p27^{kip1} translocated to the nucleus, which has been shown to result in inhibition of cell proliferation. p27^{kip1} remained in the cytoplasm of untreated cells.

To further study the effect of B2B3-1 on the cell cycle, BT-474-M3 cells treated with B2B3-1 for 72 hours were probed for the cell cycle regulator Cyclin D1 using Western blot analysis (Figure 20B). The cytoskeleton protein vinculin was used as a protein loading control in this experiment. B2B3-1 treatment resulted in a decrease in the levels of Cyclin D1 compared to untreated cells.

### Example 21

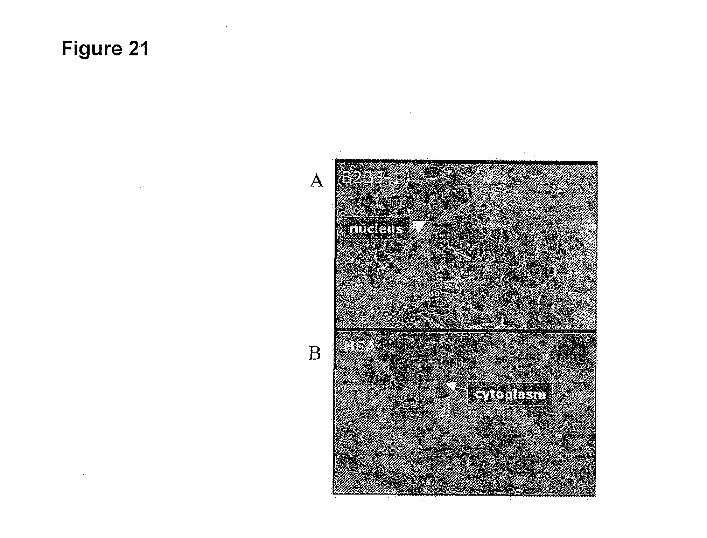

As shown in Figures 21A-B, B2B3-1 treatment of BT474-M3 breast tumor xenografts results in translocation of p27^{kip1} to the nucleus. BT474 breast tumor xenografts were treated with B2B3-1 (Figures 21A) at a dose of 30 mg/kg or an equimolar dose of HSA (Figures 21B) every 3 days for a total of 4 doses. Increased nuclear staining for p27^{kip1} was observed in B2B3-1 treated tumors compared to HSA control tumors indicating an anti-proliferative effect of B2B3-1 *in vivo.*

### Example 22

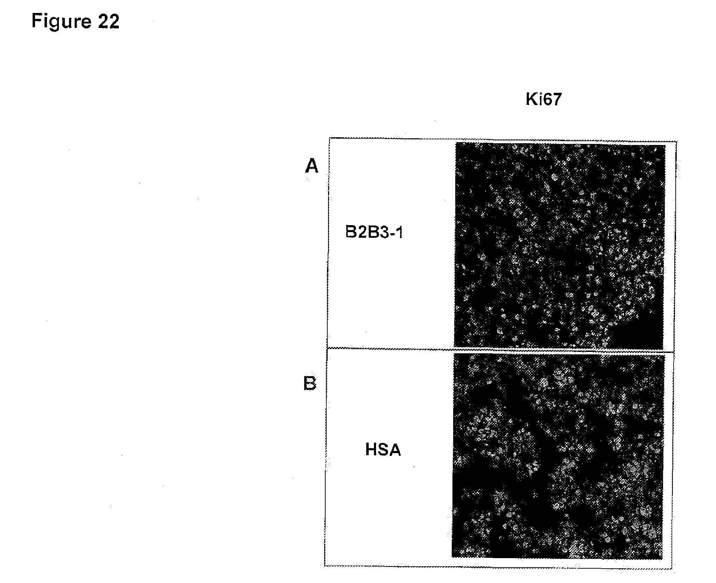

B2B3-1 treatment results in a reduction of the proliferation marker Ki67 in BT474 breast cancer xenograft tumors. BT474-M3 breast tumor xenografts were treated with B2B3-1 (Figure 22A) at a dose of 30 mg/kg or an equimolar dose of HSA (Figure 22B) every 3 days for a total of 4 doses. Subsequent staining of tumor sections for Ki67 demonstrated a reduced expression pattern for B2B3-1 treated tumors compared to HSA treated tumors.

### Example 23

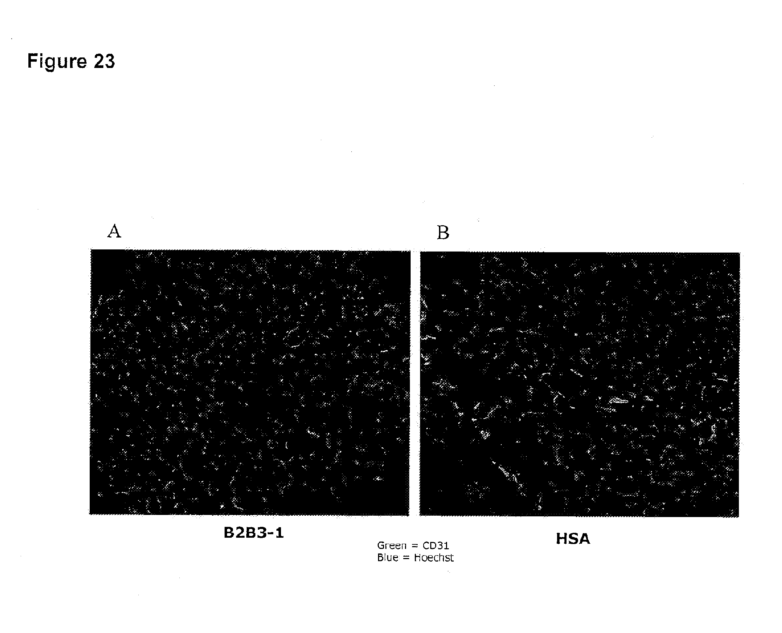

B2B3-1 treatment results in a reduction of vessel density in BT474-M3 breast cancer xenograft tumors, as determined by assaying for CD31 expression (Figures 23A-B). BT474 breast tumor xenografts were treated with B2B3-1 (Figure 23A) at a dose of 30 mg/kg or an equimolar dose of HSA (Figure 23B) every 3 days for a total of 4 doses. Tumors were stained for the presence of vascular marker CD31. Tumors treated with B2B3-1 show a marked decrease in vessel density compared to control tumors treated with HSA.

### Example 24: B2B3-1 inhibits phosphorylation of ErbB3 in vivo.

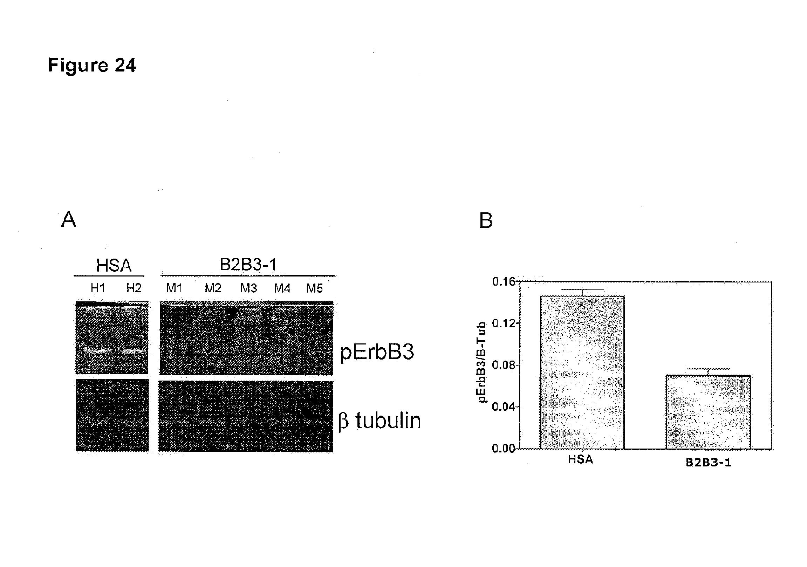

BT-474-M3 xenograft tumors were treated with 30 mg/kg B2B3-1 or 17.5 mg/kg HSA every 3 days for a total of 4 doses and tumors were harvested 24 hours after the final dose. Tumors were lysed and subjected to SDS-PAGE followed by Western analysis to assess relative levels of phosphorylation of B2B3-1's target ErbB3. Equal quantities of protein were loaded in each lane and total protein levels were controlled by probing for beta tubulin. Western blot analysis using antibodies specific for phosphorylated ErbB3 show that B2B3-1 treated tumors contain less pErbB3 than HSA treated tumors (Figure 24A). Densitometry of the western blot analysis followed by normalization of the mean pErbB3 integral band intensity to the mean beta tubulin integral band intensity demonstrated that B2B3-1 treated tumors contained significantly less pErbB3 than control HSA treated tumors (Figure 24B). These data confirmed that B2B3-1 inhibits phosphorylation of its target ErbB3 *in vivo.*

### Example 25: In vivo activity of B2B3-1 in BT-474-M3 xenografts which have reduced PTEN activity.

ShRNA technology was applied to knock out the activity of the tumor suppressor gene phosphatase and tensin homolog (PTEN) in BT-474-M3 breast cancer cells. Briefly, cultured BT-474-M3 cells were transfected with shPTEN or shControlRNA by retroviral transfection. Transfected cells with reduced PTEN were selected using FACS and subsequently injected subcutaneously into the right flank of nude mice to establish xenograft tumors. Cells transfected with a control vector were injected into the left flank of the same mouse. Mice were treated with 30mg/kg B2B3-1 every 3 days or 10mg/kg Herceptin every week. HSA was injected as a control at an equimolar dose to B2B3-1. All injections were done i.p.

B2B3-1 and Herceptin promoted a reduction in the size of tumors formed by control BT-474-M3 breast cancer cells (Figure 25A), whereas only B2B3-1 (and not Herceptin) promoted a reduction in the size of tumors formed by BT-474-M3 breast cancer cells lacking expression of PTEN (Figure 25B).

### Example 26: B2B3-1 inhibits ErbB3 signaling in BT-474-M3 breast cancer cells having reduced PTEN activity.

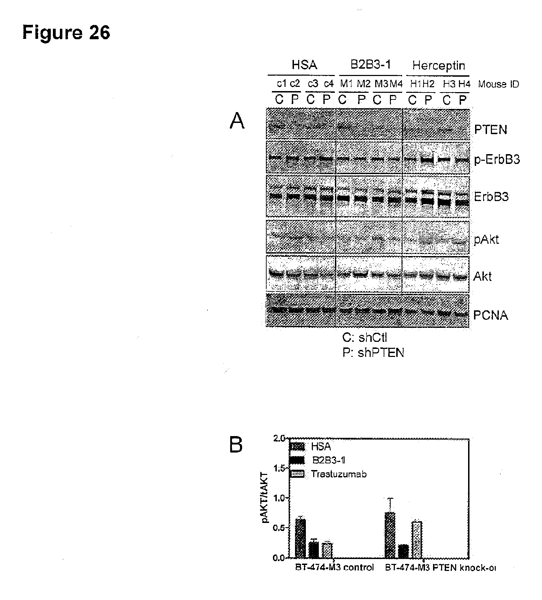

The ability of B2B3-1 to inhibit phosphorylation of ErbB3 signaling in tumor xenografts was studied using the PTEN deficient BT-474-M3 model. Xenograft tumors of the engineered cell line or control cell line were treated with 30 mg/kg B2B3-1, 17.5 mg/kg HSA every 3 days or 10 mg/kg Herceptin weekly and tumors were harvested 24 hours after the final dose. Tumors were lysed and subjected to SDS-PAGE followed by Western analysis to assess relative levels of phosphorylation of B2B3-1's target ErbB3, AKT and total PTEN levels. Equal quantities of protein were loaded in each lane and total protein levels were controlled by probing for PCNA. Western blot analysis using antibodies specific for phosphorylated ErbB3 show that B2B3-1 treated tumors contain less pAKT than HSA treated or Herceptin treated tumors (Figure 26A). Densitometry of the western blot analysis followed by normalization of the mean pAKT integral band intensity to the mean PCNA integral band intensity demonstrated that B2B3-1 treated tumors contained significantly less pAKT than control HSA treated and Herceptin-treated tumors (Figure 26B).

### Example 27

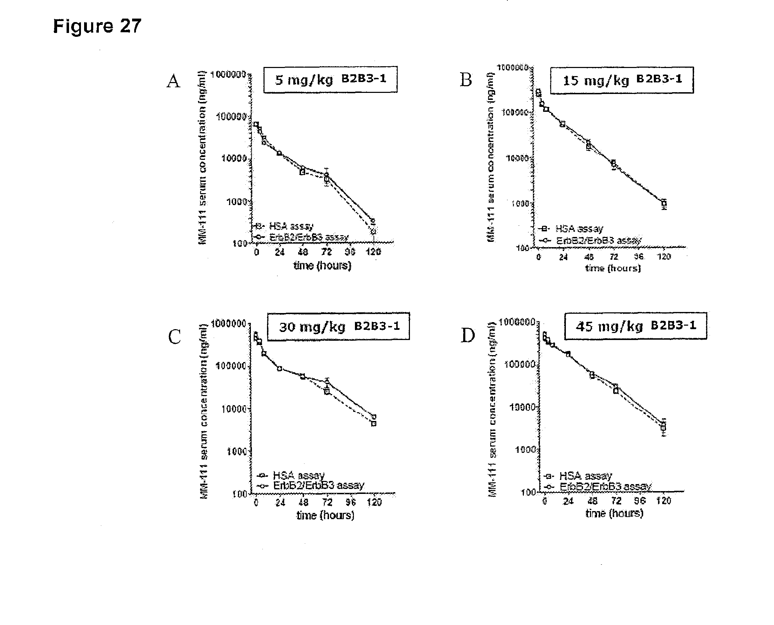

The pharmacokinetic parameters for B2B3-1 were investigated in nu/nu mice. Animals were randomized into groups and administered intravenous (IV) with a single dose of 5, 15, 30, or 45 mg/kg B2B3-1 (Figures 27A-D, respectively). Blood was collected pre-dose and at 0.5, 4, 8, 24, 48, 72, and 120 hours post dose. Three mice were used for each time point. Serum levels of B2B3-1 were measured using two ELISA methods. The first method requires functional binding of B2B3-1 to both ErbB2 and ErbB3 while the second method measures only the HSA component of B2B3-1 in the serum. The HSA ELISA utilizes a polyclonal-anti HSA capture antibody and a HRP-conjugated polyclonal anti-HSA detection antibody. A reduction in B2B3-1 serum concentration measured using the ErbB2/ErbB3 binding method versus the HSA method would indicate a loss in functional B2B3-1. Figures 27A-D show that the pharmacokinetic properties of B2B3-1 are comparable when assessed using either ELISA method, indicating that B2B3-1 is stable in circulation in mice.

### Example 28

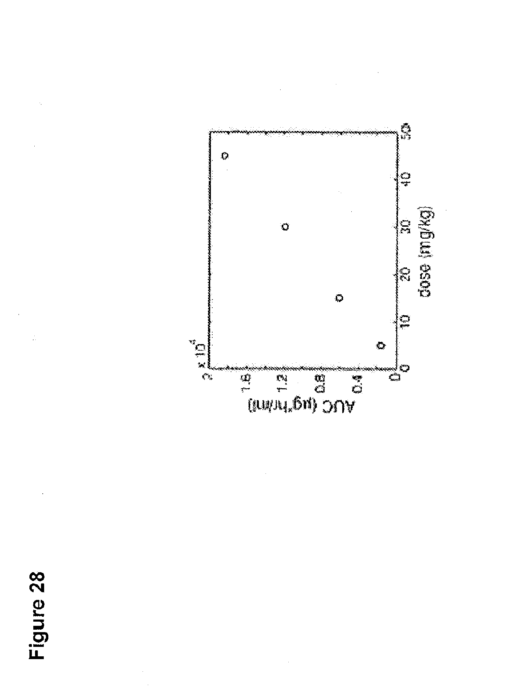

B2B3-1 serum concentrations were fit using a two-compartment, biexponential model and showed biphasic disposition. Terminal half-lives were calculated to be 17, 16, 23, and 18 hrs for the 5, 15, 30, or 45 mg/kg doses, respectively, and are shown in Table 4. Increases in B2B3-1 dose resulted in a linear increase in exposure (Figure 28).

**Table 4: Pharmacokinetic properties of B2B3-1 in mice and Cynomolgus monkeys.**

| **Dose (mg/kg)** | **Species** | **N** | **T½β (hrs)** | **AUC (hr-µg/ml)** | **Clearance (ml/hr/kg)** |
|---|---|---|---|---|---|
| 5 | Mouse (single dose) | 3 | 16.9 | 1.58E+03 | 3.19 |
| 15 | Mouse (single dose) | 3 | 16.2 | 6.10E+03 | 2.47 |
| 30 | Mouse (single dose) | 3 | 22.6 | 1.18E+04 | 2.54 |
| 45 | Mouse (single dose) | 3 | 17.5 | 1.84E+04 | 2.46 |
| 4 | Cynomolgus monkey (1st dose) | 2 | 39.1 | 3.44E-03 | 1.17 |
| 4 | Cynomolgus monkey (4th dose) | 2 | 44.9 | 7.20E+03 | 0.60 |
| 20 | Cynomolgus monkey (1st dose) | 2 | 33.1 | 2.29E+04 | 0.88 |
| 20 | Cynomolgus monkey (4th dose) | 2 | 122.5 | 8.20E+04 | 0.25 |
| 200 | Cynomolgus monkey (1st dose) | 4 | 68.8 | 3.18E+05 | 0.64 |
| 200 | Cynomolgus monkey (4th dose) | 2 | 69.7 | 5.72E+05 | 0.35 |
| 200 | Cynomolgus monkey (4th dose)* | 2 | 66.6 | 5.99E+05 | 0.34 |

| | | | | | |
|---|---|---|---|---|---|
| *recovery animals | | | | | |

### Example 29

Blood samples for pharmacokinetic analysis were also obtained from a dose range-finding toxicology study in female Cynomolgus monkeys. In this study, animals were infused with 4, 20 or 200 mg/kg of B2B3-1 administered every 3 days for 4 doses. Sampling occurred prior to and 5 minutes after dosing on each dosing day (study days 1, 4, 7 and 10) to provide pre-dose and peak/trough concentrations, and at 1, 2, 4, 8, 24 and 48 hours after the end of the first infusion on day 1 and at 1, 2, 4, 8, 24, 48, 72 and 120 hours after the last infusion on day 10. For recovery animals dosed at 200 mg/kg serum samples were also collected at 168, 336 and 456 hours after the last infusion.

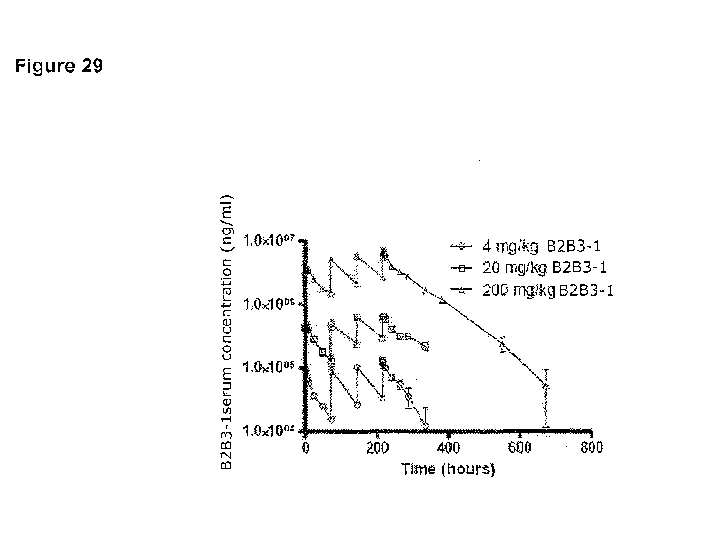

Cynomolgus monkey serum samples were assayed using the ErbB2/ErbB3 ELISA method described previously. Serum concentrations for each dose over the time course are shown in Figure 29. The analysis showed that mean concentration-time profiles for serum B2B3-1 after dosing on days 1 and 10 were qualitatively similar with concentrations generally declining with time from Cmax. Mean half-life estimates ranged from 38.3-67.2 hours on day 1 and 45.0 to 121.0 hours on day 10 (Table 4).

### Example 30

The plasmid encoding the B2B3-1 bispecific scFv antibody fusion protein was created combining gene sequences of a unique human anti-ErbB3 scFv (designated "H3"), a human anti-ErbB2 scFv (designated "B1D2"), and a modified human serum albumin (HSA) linker. The anti-ErbB3 scFv, H3, is recombinantly linked to the amino terminus of the HSA linker via a connecting peptide (Ala-Ala-Ser) and the anti-ErbB2 scFv, B1D2, is genetically linked the carboxy terminus of the HSA linker via a connecting peptide (Ala-Ala- Ala-Leu). The peptide connectors were formed through the introduction of restriction sites during construction of the mammalian expression vector and were synthesized with optimized codon usage for mammalian expression together with the single chain antibody fragments and HSA linker.

The B1D2 scFv was selected from a combinatorial phage display library created by mutagenesis of the ErbB2-binding scFv C6.5, which was selected from a non-immune phage display library. The H3 scFv was selected from a non-immune phage display library originally made by Sheets et al. The gene sequences encoding the B1D2 and H3 single chain antibody fragments were optimized for CHO cell codon preferences and synthesized for subsequent construction of the B2B3-1 encoding plasmid.

The modified HSA linker contains two amino acid substitutions. A cysteine residue at position 34 was mutated to serine in order to reduce potential protein heterogeneity due to oxidation at this site. An asparagine residue at amino acid 503 was mutated to glutamine, which in wild type HSA is sensitive to deamination and can result in decreased pharmacologic half-life.

The gene sequence encoding the modified HSA linker was synthesized with optimized codon usage for mammalian expression for subsequent construction of the B2B3-1 encoding plasmid.

### Example 31

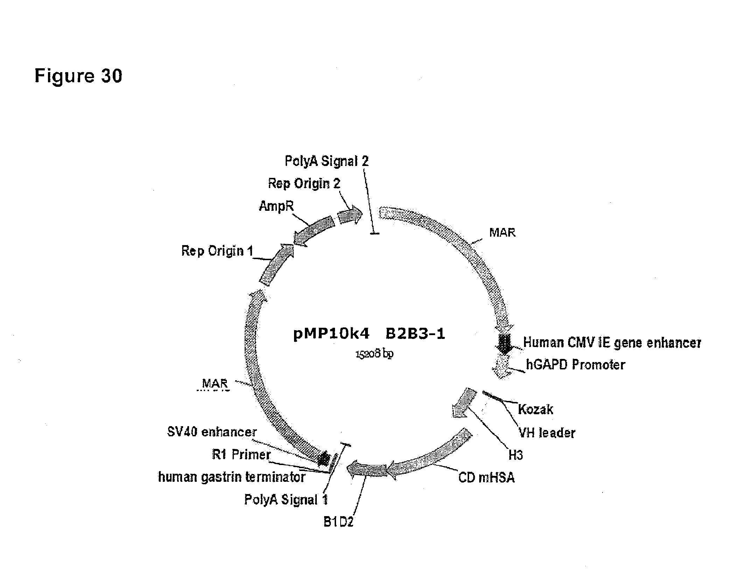

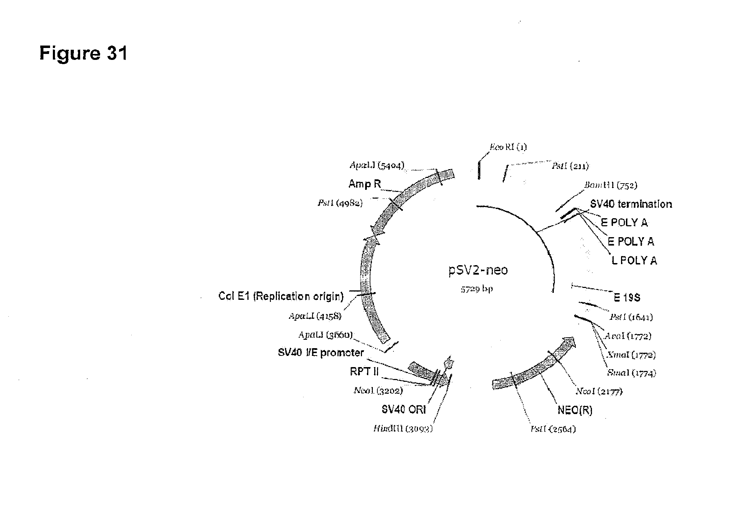

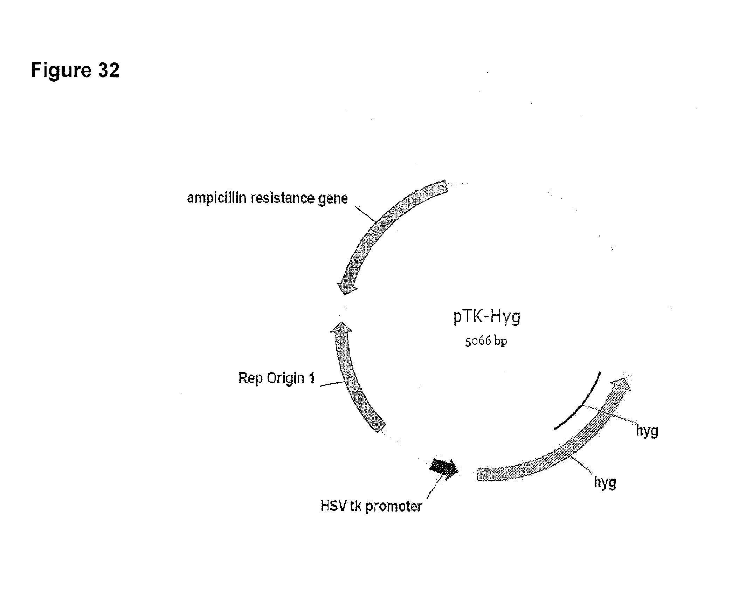

The B2B3-1 coding sequence was cloned into pMP10k base vector using standard molecular biology techniques to create plasmid pMP10k4H3-mHSA-B1D2, shown in Figure 30. For the most part this construct employs commonly used genetic elements. B2B3-1 expression is driven by the human GAPD promoter. This vector utilizes genetic elements referred to as Matrix Attachment Regions or MAR elements. The MAR genetic elements control the dynamic organization of chromatin, and insulate nearby genes from the effect of surrounding chromatin thereby increasing copy number dependent, position-independent, expression of genes. MAR elements have been shown to improve the probability of isolating a clone exhibiting the desired level of expression for the production of a recombinant protein and to increase the stability of production. The MAR elements used in the B2B3-1 constructs are non-coding human MAR elements. In addition to the B2B3-1 plasmid, a neomycin antibiotic resistance plasmid (Figure 31) and a hygromycin resistance plasmid (Figure 32) were also used to select for stable transformants.

### Example 32: First round of Gene Transfection

Chinese Hamster Ovary CHO-K1 cells were purchased from ATCC (ATCC # CCL-61). The CHO-K1 cell line is a serum and proline dependent adherent sub-clone of the parental CHO cell line created by T.T. Puck. The CHO-K1 cells used for B2B3-1 transfection were pre-adapted for suspension growth in serum free media prior to transfection. An iterative transfection procedure was used to develop the B2B3-1 cell line. Twenty-four hours before transfection, CHO-K1 cells were sub passaged to 1.0x10⁶ cells/mL in SFM4CHO (Serum Free) medium (HyClone, Logan, UT) supplemented with 8mM L-glutamine, 0.1 mM sodium hypoxanthine, and 0.016 mM thymidine. On the day of transfection, cells were resuspended in OptiMEM medium (Invitrogen Corp, Carlsbad, CA) and 40,000 cells were placed in each well of a twenty-four well plate. In the first transfection, the B2B3-1 expression plasmid (pMP10k4H3-mHSA-B1D2) and the neomycin resistance plasmid (Figure 30; pSV2-neo (Selexis, Inc., Marlborough, MA) were mixed together using a molar plasmid ratio of 75:1 (B2B3-1:neomycin resistance). The plasmid mixture was subsequently mixed with a cationic lipid transfection reagent (Lipofectamine LTX, Invitrogen Corp, Carlsbad, CA) and lipid/DNA complexes were allowed to form for thirty minutes. The DNA/Lipid complex was than added to the CHO-K1 cells and the 24-well plates were placed in a 37°C, 5% CO₂ incubator.

### Example 33: Selection and Screening for High Producers

The contents of each transfection well were washed with PBS, trypsinized and distributed across two, ninety-six well plates. The growth media used consisted of DMEM/F12 (Invitrogen Corp, Carlsbad, CA) with 10% FBS (Invitrogen Corp, Carlsbad, CA) and 500 mg/L of geneticin (G418; Invitrogen Corp, Carlsbad, CA). Media in the 96-well plates was changed on day 4 to SFM4CHO medium supplemented with 8mM L-glutamine, 0.1 mM sodium hypoxanthine, 0.016 mM thymidine, and 500mg/L geneticin. Following an additional two weeks of culture in selection medium, surviving cells had formed well-defined colonies. The clones were evaluated using quantitative spot blot techniques. The top producing colonies were trypsinized, and expanded to a single well of a 24-well plate.

A seven day productivity assay was used to screen for high B2B3-1 producing colonies. Upon expansion the cells in 24-well plates were allowed to proliferate for seven days in SFM4CHO medium supplemented with 8 mM L-glutamine, 0.1 mM sodium hypoxanthine, and 0.016 mM thymidine. The B2B3-1 concentration in the spent media was determined. Top clones from the 24-well scale were expanded into 125 mL baffled shake flasks. A seven day study in the shake flask in SFM4CHO medium supplemented with 8mM L-glutamine, 0.1 mM sodium hypoxanthine, and 0.016 mM thymidine was used to screen the cell pools for growth and B2B3-1 production.

### Example 34: Second Round of Gene Transfection

The highest producing cell pool determined from the first round of transfection (*supra*) was transfected a second time to increase production. Twenty-four hours before transfection, the cell pool was sub passaged to 1.0x10⁶ cells/mL in SFM4CHO (Serum Free) medium supplemented with 8mM L-glutamine, 0.1 mM sodium hypoxanthine, and 0.016 mM thymidine. On the day of transfection, cells were resuspended in OptiMEM medium (Invitrogen Corp, Carlsbad, CA) and 40,000 cells were placed in each well of a twenty-four well plate. In the first transfection, the B2B3-1 and hygromycin resistance plasmid (Figure 32; pTK-Hyg (Clontech, Mountain View, CA)) were mixed together using a molar plasmid ratio of 50:1 (B2B3-1:hygromycin resistance). The plasmid mixture was subsequently mixed with a cationic lipid transfection reagent (Lipofectamine LTX, Invitrogen Corp) and lipid/DNA complexes were allowed to form for thirty minutes. The DNA/Lipid complex was than added to the cell pool and the 24-well plates were placed in a 37°C, 5% CO₂ incubator.

### Example 35: Selection and Screening for High Producers From Second Transfection

The contents of each transfection well were washed with PBS, trypsinized and distributed across two, 96-well plates. The growth media used consisted of DMEM/F12 supplemented with 10% FBS and 400 mg/L of hygromycin B (Invitrogen Corp). Media in the 96-well plates was changed on day 4 to Hyclone SFM4CHO medium supplemented with 8mM L-glutamine, 0.1 mM sodium hypoxanthine, 0.016 mM thymidine , and 400 mg/L of hygromycin B. After an additional two weeks of selection, surviving cells had formed well-defined colonies. The clones were evaluated using quantitative spot blot techniques. The top producing colonies were trypsinized, and expanded to a single well of a 24-well plate.

A seven day productivity assay was used to screen for high B2B3-1 producing colonies. Upon expansion the cells were allowed to proliferate for seven days, and the B2B3-1 concentration in the spent media was determined.

Top clones from the 24-well plates were expanded into 125 mL baffled shaker flasks in the Hyclone SFM4CHO medium supplemented with 8mM L-glutamine, 0.1 mM sodium hypoxanthine, and 0.016 mM thymidine. A seven day study in shake flask was used to screen the cell pools for growth and B2B3-1 production. The spent media was quantitated using Protein Aresin and an HPLC instrument.

### Example 36: Limiting Dilution Cloning

The best growing and highest B2B3-1-producing colony identified by the productivity assay was transferred from the 125 mL shaker flask and plated in five 96-well plates at a cell concentration calculated to give one cell/well. The 96-well plates were placed in an incubator at 37°C and 5% CO₂. The wells were examined bi-weekly to track formation of colonies. Colonies arising from a single cell were identified based on the symmetrical shape of the colony. Wells containing such colonies were marked for further screening by 24-well 7-day assessment, and 125mL shaker flask 7-day assessment.

The second round of limiting dilution cloning was performed in a similar manner to the first round. An additional 100 mL fed batch evaluation was performed to confirm clone choice. A pre-seed bank was cryopreserved.

### Example 37: Formulation of B2B3-1

B2B3-1 can be administered once a week via intravenous infusion over a period of 60 or 90 minutes, depending on patient tolerability. B2B3-1 can be formulated in a sterile 20 mM L-histidine hydrochloride, 150 mM sodium chloride, pH 6.5 solution at a concentration of 25mg/mL for administration to a patient (e.g., a human).

### Example 38: Treatment of Breast Cancer

An HSA linker of the invention (e.g., B2B3-1; SEQ ID NO:16) can be administered to a patient diagnosed with breast cancer. For example, a patient whose cancer is diagnosed as expressing high levels of epidermal growth factor receptors, such as ErbB2 (HER2/neu) can be treated with an HSA linker conjoined to an ErbB2 biding moiety. A physician providing care to a patient diagnosed with breast cancer can administer an HSA linker of the invention suitable and capable of providing the best therapeutic benefit to the patient. Selection of, for example, the binding moieties, conjoined to an HSA linker of the invention can be based on clinical determinants of the patient and specific cancer variant. For example, genotypic or histologic screens of cancer biopsies can reveal increased expression of ErbB2, indicating that an HSA linker of the invention that incorporates and anti-ErbB2 binding moiety (e.g., B2B3-1) can be used therapeutically.

In one embodiment of the invention, B2B3-1 can be administered to a patient diagnosed with breast cancer once a week via intravenous infusion over a period of, e.g., 60 or 90 minutes, depending on patient tolerability. B2B3-1 can be formulated in a sterile 20 mM L-histidine hydrochloride, 150 mM sodium chloride, pH 6.5 solution at a concentration of 25mg/mL for administration to a patient (e.g., a human). A clinician supervising the administration of B2B3-1 or any other HSA linker of the invention can follow common formulation and dosing practices to determine the proper course of therapy for any given patient.

The invention further provides for the co-administration of one or more therapeutic drugs or compounds in combination with the HSA linker of the invention (e.g., B2B3-1; SEQ ID NO:16), such as the common chemothcrapeutic regimen for the treatment of breast cancer includes doxorubicin, cyclophosphamide, and paclitaxel. Alternatively, a clinician can administer the HSA linker of invention (e.g., B2B3-1) in combination with surgical or radiation therapy to treat breast cancer in a patient in need thereof.

### Example 39: Treatment of Ovarian Cancer

An HSA linker of the invention can be administered to a patient diagnosed with ovarian cancer (ovarian carcinoma), for example, a patient whose cancer is diagnosed as expressing high levels of epidermal growth factor receptors, such as ErbB2 (HER2/neu) can be treated with an HSA linker conjoined to an ErbB2 biding moiety. A physician providing care to a patient diagnosed with ovarian cancer can administer an HSA linker of the invention suitable and capable of providing the best therapeutic benefit to the patient. Selection of, for example, the binding moieties, conjoined to an HSA linker of the invention can be based on clinical determinants of the patient and specific cancer variant. For example, genotypic or histologic screens of cancer biopsies can reveal increased expression of ErbB2 or other targeting molecules (e.g., overexpressed receptors or ligands specific to the cancer), which can be targeted by an HSA linker of the invention that incorporates an anti-ErbB2 binding moiety (e.g., B2B3-1) or other target specific binding moiety.

In one embodiment of the invention, B2B3-1 can be administered to a patient diagnosed with ovarian cancer once a week via intravenous infusion over a period of, e.g., 60 or 90 minutes, depending on patient tolerability. B2B3-1 can be formulated in a sterile 20 mM L-histidine hydrochloride, 150 mM sodium chloride, pH 6.5 solution at a concentration of 25mg/mL for administration to a patient (e.g., a human). A clinician supervising the administration of B2B3-1 or any other HSA linker of the invention for the treatment of ovarian cancer can follow common formulation and dosing practices (e.g., intraperitoneal injection) to determine the proper course of therapy for any given patient.

The invention further provides for the co-administration of one or more therapeutic drugs or compounds in combination with the HSA linker of the invention (e.g., B2B3-1; SEQ ID NO:16). Alternatively, a clinician can administer an HSA linker of invention in combination with surgical or radiation therapy to treat ovarian cancer in a patient in need thereof.

### Example 40

HSA linker conjugates of the invention can be constructed using one or more of the elements (groups A-E) listed in Table 5 below. In particular, an HSA linker of the invention, which is shown as Group C in Table 5 below, can incorporate one or more binding moieties selected from groups A and E shown in Table 5. In addition, the HSA linker conjugates can also include one or more peptide connectors, which are selected from groups B and D in Table 5, at each of the amino and carboxy terminal ends of the HSA linker. Peptide connectors can be repeated or truncated to increase or decrease the length of the connector sequence.

The invention provides specific embodiments of the HSA linkers, polypeptide connectors, and binding moieties discussed above. Table 6 lists ten HSA linker conjugates with varying ErbB2-specific or ErbB3-specific binding moieties, as well as polypeptide connectors, at the amino and carboxy termini of an HSA linker of the invention.

**Table 6**

| **Agent of the Invention** | **Amino Terminal Binding Moiety** | **N-Terminal Connector** | **HSA** | **C-Terminal Connector** | **Carboxy Terminal Binding Moiety** |
|---|---|---|---|---|---|
| B2B3-1 (SEQ ID NO:16) | H3 (SEQ ID NO:26) | AAS | mHSA (SEQ ID NO:1) | AAAL(SEQ ID NO:5) | B1D2 (SEQ ID NO:27) |
| B2B3-2 (SEQ ID NO:17) | A5 (SEQ ID NO:28) | AAS | mHSA (SEQ ID NO:1) | AAAL (SEQ ID NO:5) | B1D2 (SEQ) ID NO:27) |
| B2B3-3 (SEQ ID NO:18) | A5 (SEQ ID NO:28) | AAS | mHSA (SEQ ID NO:1) | AAAL (SEQ ID NO:5) | F5B6H2 (SEQ ID NO:32) |
| B2B3-4 (SEQ ID NO:19) | A5 (SEQ ID NO:28) | AAS | mHSA (SEQ ID NO:1) | AAAL (SEQ ID NO:5) | ML3.9 (SEQ ID NO:29) |
| B2B3-5 (SEQ ID NO:20) | B12 (SEQ ID NO:30) | AAS | mHSA (SEQ ID NO:1) | AAAL (SEQ ID NO:5) | B1D2 (SEQ ID NO:27) |
| B2B3-6 (SEQ ID NO:21) | B12 (SEQ ID NO:30) | AAS | mHSA (SEQ ID NO:1) | AAAL (SEQ ID NO:5) | F5B6H2 (SEQ ID NO:32) |
| B2B3-7 (SEQ ID NO:22) | F4 (SEQ ID NO:31) | AAS | mHSA (SEQ ID NO:1) | AAAL(SEQ ID NO:5) | B1D2 (SEQ ID NO:27) |
| B2B3-8 (SEQ ID NO:23) | F4 (SEQ ID NO:31) | AAS | mHSA (SEQ ID NO:1) | AAAL (SEQ ID NO:5) | F5B6H2 (SEQ ID NO:32) |
| B2B3-9 (SEQ ID NO:24) | H3 (SEQ ID NO:26) | AAS | HSA (SEQ ID NO:3) | AAAL (SEQ ID NO:5) | B1D2 (SEQ ID NO:27) |
| B2B3-10 (SEQ ID NO:25) | H3 (SEQ ID NO:26) | AAS | mHSA (SEQ ID NO:1) | AAAL (SEQ ID NO:5) | F5B6H2 (SEQ ID NO:32) |

APPENDIX A
SEQUENCE LISTINGS
SEQ ID NO:1
Human Serum Albumin (HSA) Linker with C34S and N503Q substitutions ("mHSA")
Amino Acid Sequence

DAHKSEVAHRFKDLGEENFKALVLIAFAQYLQQSPFEDHVKLVNEVTEFAKTCVADESAE
NCDKSLHTLFGDKLCTVATLRETYGEMADCCAKQEPERNECFLQHKDDNPNLPRLVRPEV
DVMCTAFHDNEETFLKKYLYEIARRHPYFYAPELLFFAKRYKAAFTECCQAADKAACLLP
KLDELRDEGKASSAKQRLKCASLQKFGERAFKAWAVARLSQRFPKAEFAEVSKLVTDLTK
VHTECCHGDLLECADDRADLAKYICENQDSISSKLKECCEKPLLEKSHCIAEVENDEMPAD
LPSLAADFVESKDVCKNYAEAKDVFLGMFLYEYARRHPDYSVVLLLRLAKTYETTLEKCC
AAADPHECYAKVFDEFKPLVEEPQNLIKQNCELFEQLGEYKFQNALLVRYTKKVPQVSTPT
LVEVSRNLGKVGSKCCKHPEAKRMPCAEDYLSVVLNQLCVLHEKTPVSDRVTKCCTESLV
NRRPCFSALEVDETYVPKEFQAETFTFHADICTLSEKERQIKKQTALVELVKHKPKATKEQL
KAVMDDFAAFVEKCCKADDKETCFAEEGKKLVAASQAALGL

SEQ ID NO:2
Human Serum Albumin (HSA) Linker w/C34S and N503Q substitutions ("mHSA")
Nucleotide Sequence

GACGCTCACAAGAGCGAAGTGGCACATAGGTTCAAAGATCTGGGCGAAGAGAACTTTA
AGGCCCTCGTCCTGATCGCTTTCGCACAGTACCTCCAGCAGTCTCCCTTTGAAGATCAC
GTGAAACTGGTCAATGAGGTGACCGAATTTGCCAAGACATGCGTGGCTGATGAGAGTG
CAGAAAACTGTGACAAATCACTGCATACTCTCTTTGGAGATAAGCTGTGCACCGTCGCC
ACACTCAGAGAGACTTATGGGGAAATGGCTGACTGTTGCGCAAAACAGGAGCCTGAAC
GGAATGAGTGTTTCCTCCAGCACAAGGATGACAACCCAAATCTGCCCCGCCTCGTGCGA
CCTGAGGTCGATGTGATGTGCACCGCCTTTCATGACAACGAAGAGACATTCCTGAAGA
AATACCTGTATGAAATTGCTCGTAGGCACCCATACTTTTATGCCCCCGAGCTCCTGTTCT
TTGCAAAGAGATACAAAGCTGCCTTCACTGAATGTTGCCAGGCAGCTGATAAGGCCGC
ATGTCTCCTGCCTAAACTGGACGAGCTCCGGGATGAAGGTAAGGCTTCCAGCGCCAAA
CAGCGCCTGAAGTGCGCTTCTCTCCAGAAGTTTGGCGAGCGAGCATTCAAAGCCTGGGC
TGTGGCCCGTCTCAGTCAGAGGTTTCCAAAGGCAGAATTTGCTGAGGTCTCAAAACTGG
TGACCGACCTCACAAAGGTCCATACTGAGTGTTGCCACGGAGATCTGCTGGAATGTGCC
GACGATAGAGCAGACCTCGCTAAATATATCTGCGAGAATCAGGATTCCATTAGCTCTAA
GCTGAAAGAATGTTGCGAGAAGCCCCTCCTGGAAAAGAGTCATTGTATCGCCGAGGTG
GAAAACGACGAGATGCCAGCAGATCTGCCATCACTCGCTGCCGACTTTGTGGAATCCA
AAGATGTCTGCAAGAATTACGCAGAGGCTAAAGACGTGTTCCTGGGGATGTTTCTGTAT
GAGTACGCCCGGCGTCACCCCGATTATAGCGTCGTGCTCCTGCTCCGACTGGCAAAGAC
CTACGAAACAACTCTGGAGAAATGTTGCGCTGCCGCAGACCCTCATGAATGTTATGCTA
AGGTGTTCGATGAGTTTAAGCCACTCGTCGAAGAGCCCCAGAACCTGATTAAACAGAA
TTGCGAACTGTTCGAGCAGCTCGGTGAATACAAGTTTCAGAACGCCCTGCTCGTGCGTT
ATACCAAAAAGGTCCCTCAGGTGTCTACACCAACTCTGGTGGAGGTCAGTAGGAATCT
GGGCAAAGTGGGATCAAAGTGTTGCAAACACCCCGAGGCAAAGAGAATGCCTTGTGCT

GAAGATTACCTCTCCGTCGTGCTGAACCAGCTCTGCGTGCTGCATGAAAAGACCCCAGT
CAGCGATCGGGTGACAAAATGTTGCACCGAATCTCTGGTCAATCGCCGACCCTGTTTCA
GTGCCCTCGAAGTGGACGAAACTTATGTGCCTAAGGAGTTTCAGGCTGAAACATTCACC
TTTCACGCCGATATCTGCACTCTGTCCGAGAAAGAAAGGCAGATTAAGAAACAGACAG
CACTGGTCGAGCTCGTGAAGCATAAACCAAAGGCTACCAAGGAGCAGCTGAAAGCCGT
CATGGACGATTTCGCAGCTTTTGTGGAAAAGTGTTGCAAAGCCGACGATAAGGAGACT
TGTTTCGCAGAAGAGGGGAAAAAGCTCGTGGCTGCCAGCCAGGCAGCTCTGGGTCTG

SEQ ID NO:3
Human Serum Albumin (HSA)
Amino Acid Sequence












SEQ ID NO:4
Human Serum Albumin (HSA)
Nucleotide Sequence

GATGCACACAAGAGTGAGGTTGCTCATCGGTTTAAAGATTTGGGAGAAGAAAATTTCA
AAGCCTTGGTGTTGATTGCCTTTGCTCAGTATCTTCAGCAGTGTCCATTTGAAGATCATG
TAAAATTAGTGAATGAAGTAACTGAATTTGCAAAAACATGTGTAGCTGATGAGTCAGC
TGAAAATTGTGACAAATCACTTCATACCCTTTTTGGAGACAAATTATGCACAGTTGCAA
CTCTTCGTGAAACCTATGGTGAAATGGCTGACTGCTGTGCAAAACAAGAACCTGAGAG
AAATGAATGCTTCTTGCAACACAAAGATGACAACCCAAACCTCCCCCGATTGGTGAGA
CCAGAGGTTGATGTGATGTGCACTGCTTTTCATGACAATGAAGAGACATTTTTGAAAAA
ATACTTATATGAAATTGCCAGAAGACATCCTTACTTTTATGCCCCGGAACTCCTTTTCTT
TGCTAAAAGGTATAAAGCTGCTTTTACAGAATGTTGCCAAGCTGCTGATAAAGCTGCCT
GCCTGTTGCCAAAGCTCGATGAACTTCGGGATGAAGGGAAGGCTTCGTCTGCCAAACA
GAGACTCAAATGTGCCAGTCTCCAAAAATTTGGAGAAAGAGCTTTCAAAGCATGGGCA
GTGGCTCGCCTGAGCCAGAGATTTCCCAAAGCTGAGTTTGCAGAAGTTTCCAAGTTAGT
GACAGATCTTACCAAAGTCCACACGGAATGCTGCCATGGAGATCTGCTTGAATGTGCTG
ATGACAGGGCGGACCTTGCCAAGTATATCTGTGAAAATCAGGATTCGATCTCCAGTAA
ACTGAAGGAATGCTGTGAAAAACCTCTGTTGGAAAAATCCCACTGCATTGCCGAAGTG
GAAAATGATGAGATGCCTGCTGACTTGCCTTCATTAGCTGCTGATTTTGTTGAAAGTAA

GGATGTTTGCAAAAACTATGCTGAGGCAAAGGATGTCTTCCTGGGCATGTTTTTGTATG
AATATGCAAGAAGGCATCCTGATTACTCTGTCGTGCTGCTGCTGAGACTTGCCAAGACA
TATGAAACCACTCTAGAGAAGTGCTGTGCCGCTGCAGATCCTCATGAATGCTATGCCAA
AGTGTTCGATGAATTTAAACCTCTTGTGGAAGAGCCTCAGAATTTAATCAAACAAAACT
GTGAGCTTTTTAAGCAGCTTGGAGAGTACAAATTCCAGAATGCGCTATTAGTTCGTTAC
ACCAAGAAAGTACCCCAAGTGTCAACTCCAACTCTTGTAGAGGTCTCAAGAAACCTAG
GAAAAGTGGGCAGCAAATGTTGTAAACATCCTGAAGCAAAAAGAATGCCCTGTGCAGA
AGACTATCTATCCGTGGTCCTGAACCAGTTATGTGTGTTGCATGAGAAAACGCCAGTAA
GTGACAGAGTCACAAAATGCTGCACAGAGTCCTTGGTGAACAGGCGACCATGCTTTTC
AGCTCTGGAAGTCGATGAAACATACGTTCCCAAAGAGTTTAATGCTGAAACATTCACCT
TCCATGCAGATATATGCACACTTTCTGAGAAGGAGAGACAAATCAAGAAACAAACTGC
ACTTGTTGAGCTTGTGAAACACAAGCCCAAGGCAACAAAAGAGCAACTGAAAGCTGTT
ATGGATGATTTCGCAGCTTTTGTAGAGAAGTGCTGCAAGGCTGACGATAAGGAGACCT
GCTTTGCCGAGGAGGGTAAAAAACTTGTTGCTGCAAGTCAAGCTGCCTTAGGCTTA

SEQ ID NO:5
Polypeptide Connector
Amino Acid Sequence

AAAL

SEQ ID NO:6
Human Serum Albumin (HSA) Linker with C34S and N503Q substitutions ("mHSA") and
Polypeptide Connector
Amino Acid Sequence

AASDAHKSEVAHRFKDLGEENFKALVLIAFAQYLQQSPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFQAETFTFHADICTLSEKERQIK
KQTALVELVKHKPKATKEQLKAVMDDFAAFVEKCCKADDKETCFAEEGKKLVAASQA
ALGL

SEQ ID NO:7
Human Serum Albumin (HSA) Linker with C34S and N503Q substitutions ("mHSA") and
Polypeptide Connector
Amino Acid Sequence

AAQDAHKSEVAHRFKDLGEENFKALVLIAFAQYLQQSPFEDHVKLVNEVTEFAKTCVA
DESAENCDKSLHTLFGDKLCTVATLRETYGEMADCCAKQEPERNECFLQHKDDNPNLP

RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFQAETFTFHADICTLSEKERQIK
KQTALVELVKHKPKATKEQLKAVMDDFAAFVEKCCKADDKETCFAEEGKKLVAASQA
ALGL

SEQ ID NO:8
Human Serum Albumin (HSA) Linker with C34S and N503Qsubstitutions ("mHSA") and
Polypeptide Connector
Amino Acid Sequence

DAHKSEVAHRFKDLGEENFKALVLIAFAQYLQQSPFEDHVKLVNEVTEFAKTCVADES
AENCDKSLHTLFGDKLCTVATLRETYGEMADCCAKQEPERNECFLQHKDDNPNLPRLV
RPEVDVMCTAFHDNEETFLKKYLYEIARRHPYFYAPELLFFAKRYKAAFTECCQAADK
AACLLPKLDELRDEGKASSAKQRLKCASLQKFGERAFKAWAVARLSQRFPKAEFAEVS
KLVTDLTKVHTECCHGDLLECADDRADLAKYICENQDSISSKLKECCEKPLLEKSHCIAE
VENDEMPADLPSLAADFVESKDVCKNYAEAKDVFLGMFLYEYARRHPDYSVVLLLRL
AKTYETTLEKCCAAADPHECYAKVFDEFKPLVEEPQNLIKQNCELFEQLGEYKFQNALL
VRYTKKVPQVSTPTLVEVSRNLGKVGSKCCKHPEAKRMPCAEDYLSVVLNQLCVLHEK
TPVSDRVTKCCTESLVNRRPCFSALEVDETYVPKEFQAETFTFHADICTLSEKERQIKKQ
TALVELVKHKPKATKEQLKAVMDDFAAFVEKCCKADDKETCFAEEGKKLVAASQAAL
GLAAAL

SEQ ID NO:9
Human Serum Albumin (HSA) Linker with C34S and N503Q substitutions ("mHSA") and
Polypeptide Connector
Amino Acid Sequence

AASDAHKSEVAHRFKDLGEENFKALVLIAFAQYLQQSPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFQAETFTFHADICTLSEKERQIK
KQTALVELVKHKPKATKEQLKAVMDDFAAFVEKCCKADDKETCFAEEGKKLVAASQA
ALGLAAAL

SEQ ID NO:10
Human Serum Albumin (HSA) Linker with C34S and N503Q substitutions ("mHSA") and
Polypeptide Connector
Amino Acid Sequence

AAQDAHKSEVAHRFKDLGEENFKALVLIAFAQYLQQSPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFQAETFTFHADICTLSEKERQIK
KQTALVELVKHKPKATKEQLKAVMDDFAAFVEKCCKADDKETCFAEEGKKLVAASQA
ALGLAAAL

SEQ ID NO:11
Human Serum Albumin (HSA) Linker and Polypeptide Connector
Amino Acid Sequence

AASDAHKSEVAHRFKDLGEENFKALVLIAFAQYLQQCPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFNAETFTFHADICTLSEKERQIK
KQTALVELVKHKPKATKEQLKAVMDDFAAFVEKCCKADDKETCFAEEGKKLVAASQA
ALGL

SEQ ID NO:12
Human Serum Albumin (HSA) Linker and Polypeptide Connector
Amino Acid Sequence

AAQDAHKSEVAHRFKDLGEENFKALVLIAFAQYLQQCPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH

EKTPVSDRVTKCCTESLVNRRPCFSALEVDETYVPKEFNAETFTFHADICTLSEKERQIK
KQTALVELVKHKPKATKEQLKAVMDDFAAFVEKCCKADDKETCFAEEGKKLVAASQA
ALGL

SEQ ID NO:13
Human Serum Albumin (HSA) Linker and Polypeptide Connector
Amino Acid Sequence

DAHKSEVAHRFKDLGEENFKALVLIAFAQYLQQCPFEDHVKLVNEVTEFAKTCVADES
AENCDKSLHTLFGDKLCTVATLRETYGEMADCCAKQEPERNECFLQHKDDNPNLPRLV
RPEVDVMCTAFHDNEETFLKKYLYEIARRHPYFYAPELLFFAKRYKAAFTECCQAADK
AACLLPKLDELRDEGKASSAKQRLKCASLQKFGERAFKAWAVARLSQRFPKAEFAEVS
KLVTDLTKVHTECCHGDLLECADDRADLAKYICENQDSISSKLKECCEKPLLEKSHCIAE
VENDEMPADLPSLAADFVESKDVCKNYAEAKDVFLGMFLYEYARRHPDYSVVLLLRL
AKTYETTLEKCCAAADPHECYAKVFDEFKPLVEEPQNLIKQNCELFEQLGEYKFQNALL
VRYTKKVPQVSTPTLVEVSRNLGKVGSKCCKHPEAKRMPCAEDYLSVVLNQLCVLHEK
TPVSDRVTKCCTESLVNRRPCFSALEVDETYVPKEFNAETFTFHADICTLSEKERQIKKQ
TALVELVKHKPKATKEQLKAVMDDFAAFVEKCCKADDKETCFAEEGKKLVAASQAAL
GLAAAL

SEQ ID NO:14
Human Serum Albumin (HSA) Linker and Polypeptide Connector
Amino Acid Sequence

AASDAHKSEVAHRFKDLGEENFKALVLIAFAQYLQQCPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL
RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFNAETFTFHADICTLSEKERQIK
KQTALVELVKHKPKATKEQLKAVMDDFAAFVEKCCKADDKETCFAEEGKKLVAASQA
ALGLAAAL

SEQ ID NO:15
Human Serum Albumin (HSA) Linker and Polypeptide Connector
Amino Acid Sequence

AAQDAHKSEVAHRFKDLGEENFKALVLIAFAQYLQQCPFEDHVKLVNEVTEFAKTCVA
DESAENCDKSLHTLFGDKLCTVATLRETYGEMADCCAKQEPERNECFLQHKDDNPNLP
RLVRPEVDVMCTAFHDNEETFLKKYLYEIARRHPYFYAPELLFFAKRYKAAFTECCQAA
DKAACLLPKLDELRDEGKASSAKQRLKCASLQKFGERAFKAWAVARLSQRFPKAEFAE
VSKLVTDLTKVHTECCHGDLLECADDRADLAKYICENQDSISSKLKECCEKPLLEKSHCI
AEVENDEMPADLPSLAADFVESKDVCKNYAEAKDVFLGMFLYEYARRHPDYSVVLLL

RLAKTYETTLEKCCAAADPHECYAKVFDEFKPLVEEPQNLIKQNCELFEQLGEYKFQNA
LLVRYTKKVPQVSTPTLVEVSRNLGKVGSKCCKHPEAKRMPCAEDYLSVVLNQLCVLH
EKTPVSDRVTKCCTESLVNRRPCFSALEVDETYVPKEFNAETFTFHADICTLSEKERQIK
KQTALVELVKHKPKATKEQLKAVMDDFAAFVEKCCKADDKETCFAEEGKKLVAASQA
ALGLAAAL

SEQ ID NO:16

mHSA Linker, Polypeptide Connectors, H3 (anti-ErbB3) scFv, and B1D2 (anti-ErbB2) scFv
("B2B3-1")
Amino Acid Sequence

QVQLQESGGGLVKPGGSLRLSCAASGFTFSSYWMSWVRQAPGKGLEWVANINRDGSA
SYYVDSVKGRFTISRDDAKNSLYLQMNSLRAEDTAVYYCARDRGVGYFDLWGRGTLV
TVSSASTGGGGSGGGGSGGGGSQSALTQPASVSGSPGQSITISCTGTSSDVGGYNFVSWY
QQHPGKAPKLMIYDVSDRPSGVSDRFSGSKSGNTASLIISGLQADDEADYYCSSYGSSST
HVIFGGGTKVTVLGAASDAHKSEVAHRFKDLGEENFKALVLIAFAQYLQQSPFEDHVKL
VNEVTEFAKTCVADESAENCDKSLHTLFGDKLCTVATLRETYGEMADCCAKQEPERNE
CFLQHKDDNPNLPRLVRPEVDVMCTAFHDNEETFLKKYLYEIARRHPYFYAPELLFFAK
RYKAAFTECCQAADKAACLLPKLDELRDEGKASSAKQRLKCASLQKFGERAFKAWAV
ARLSQRFPKAEFAEVSKLVTDLTKVHTECCHGDLLECADDRADLAKYICENQDSISSKL
KECCEKPLLEKSHCIAEVENDEMPADLPSLAADFVESKDVCKNYAEAKDVFLGMFLYE
YARRHPDYSVVLLLRLAKTYETTLEKCCAAADPHECYAKVFDEFKPLVEEPQNLIKQNC
ELFEQLGEYKFQNALLVRYTKKVPQVSTPTLVEVSRNLGKVGSKCCKHPEAKRMPCAE
DYLSVVLNQLCVLHEKTPVSDRVTKCCTESLVNRRPCFSALEVDETYVPKEFQAETFTF
HADICTLSEKERQIKKQTALVELVKHKPKATKEQLKAVMDDFAAFVEKCCKADDKETC
FAEEGKKLVAASQAALGLAAALQVQLVQSGAEVKKPGESLKISCKGSGYSFTSYWIAW
VRQMPGKGLEYMGLIYPGDSDTKYSPSFQGQVTISVDKSVSTAYLQWSSLKPSDSAVYF
CARHDVGYCTDRTCAKWPEWLGVWGQGTLVTVSSGGGGSSGGGSGGGGSQSVLTQPP
SVSAAPGQKVTISCSGSSSNIGNNYVSWYQQLPGTAPKLLIYDHTNRPAGVPDRFSGSKS
GTSASLAISGFRSEDEADYYCASWDYTLSGWVFGGGTKLTVLG

SEQ ID NO:17
B2B3-2 (A5-mHSA-B1D2)

QVQLVQSGGGLVKPGGSLRLSCAASGFSFNTYDMNWVRQAPGKGLEWVSSISSSSSYIY
YADSVKGRFTISRDNAKNSLYLQMNSLRAEDTAVYYCARDGVATTPFDYWGQGTLVT
VSSGGGGSGGGGSGGGGSQSVLTQPPSVSGAPGQRVTISCTGSSSNIGAGYDVHWYQQL
PGTAPKLLIYGNSNRPSGVPDRFSGSKSGTSASLAITGLQAEDEADYYCQSYDSSLSALF
GGGTKLTVLGAASDAHKSEVAHRFKDLGEENFKALVLIAFAQYLQQSPFEDHVKLVNE
VTEFAKTCVADESAENCDKSLHTLFGDKLCTVATLRETYGEMADCCAKQEPERNECFL
QHKDDNPNLPRLVRPEVDVMCTAFHDNEETFLKKYLYEIARRHPYFYAPELLFFAKRYK
AAFTECCQAADKAACLLPKLDELRDEGKASSAKQRLKCASLQKFGERAFKAWAVARL
SQRFPKAEFAEVSKLVTDLTKVHTECCHGDLLECADDRADLAKYICENQDSISSKLKEC
CEKPLLEKSHCIAEVENDEMPADLPSLAADFVESKDVCKNYAEAKDVFLGMFLYEYAR
RHPDYSVVLLLRLAKTYETTLEKCCAAADPHECYAKVFDEFKPLVEEPQNLIKQNCELF
EQLGEYKFQNALLVRYTKKVPQVSTPTLVEVSRNLGKVGSKCCKHPEAKRMPCAEDYL

SVVLNQLCVLHEKTPVSDRVTKCCTESLVNRRPCFSALEVDETYVPKEFQAETFTFHADI
CTLSEKERQIKKQTALVELVKHKPKATKEQLKAVMDDFAAFVEKCCKADDKETCFAEE
GKKLVAASQAALGLAAALQVQLVQSGAEVKKPGESLKISCKGSGYSFTSYWIAWVRQ
MPGKGLEYMGLIYPGDSDTKYSPSFQGQVTISVDKSVSTAYLQWSSLKPSDSAVYFCAR
HDVGYCTDRTCAKWPEWLGVWGQGTLVTVSSGGGGSSGGGSGGGGSQSVLTQPPSVS
AAPGQKVTISCSGSSSNIGNNYVSWYQQLPGTAPKLLIYDHTNRPAGVPDRFSGSKSGTS
ASLAISGFRSEDEADYYCASWDYTLSGWVFGGGTKLTVLG

SEQ ID NO:18
B2B3-3 (A5-mHSA-F5B6H2)

QVQLVQSGGGLVKPGGSLRLSCAASGFSFNTYDMNWVRQAPGKGLEWVSSISSSSSYIY
YADSVKGRFTISRDNAKNSLYLQMNSLRAEDTAVYYCARDGVATTPFDYWGQGTLVT
VSSGGGGSGGGGSGGGGSQSVLTQPPSVSGAPGQRVTISCTGSSSNIGAGYDVHWYQQL
PGTAPKLLIYGNSNRPSGVPDRFSGSKSGTSASLAITGLQAEDEADYYCQSYDSSLSALF
GGGTKLTVLGAASDAHKSEVAHRFKDLGEENFKALVLIAFAQYLQQSPFEDHVKLVNE
VTEFAKTCVADESAENCDKSLHTLFGDKLCTVATLRETYGEMADCCAKQEPERNECFL
QHKDDNPNLPRLVRPEVDVMCTAFHDNEETFLKKYLYEIARRHPYFYAPELLFFAKRYK
AAFTECCQAADKAACLLPKLDELRDEGKASSAKQRLKCASLQKFGERAFKAWAVARL
SQRFPKAEFAEVSKLVTDLTKVHTECCHGDLLECADDRADLAKYICENQDSISSKLKEC
CEKPLLEKSHCIAEVENDEMPADLPSLAADFVESKDVCKNYAEAKDVFLGMFLYEYAR
RHPDYSVVLLLRLAKTYETTLEKCCAAADPHECYAKVFDEFKPLVEEPQNLIKQNCELF
EQLGEYKFQNALLVRYTKKVPQVSTPTLVEVSRNLGKVGSKCCKHPEAKRMPCAEDYL
SVVLNQLCVLHEKTPVSDRVTKCCTESLVNRRPCFSALEVDETYVPKEFQAETFTFHADI
CTLSEKERQIKKQTALVELVKHKPKATKEQLKAVMDDFAAFVEKCCKADDKETCFAEE
GKKLVAASQAALGLAAALQVQLVESGGGLVQPGGSLRLSCAASGFTFRSYAMSWVRQ
APGKGLEWVSAISGRGDNTYYADSVKGRFTISRDNSKNTLYLQMNSLRAEDTAVYYCA
KMTSNAVGFDYWGQGTLVTVSSGGGGSGGGSGGGGSGQSVLTQPPSVSGAPGQRVTIS
CTGRHSNIGLGYGVHWYQQLPGTAPKLLIYGNTNRPSGVPDRFSGFKSGTSASLAITGLQ
AEDEADYYCQSYDRRTPGWVFGGGTKLTVLG

SEQ ID NO:19
B2B3-4 (A5-mHSA-ML3.9)






















SEQ ID NO:20
B2B3-5 (B12-mHSA-B1D2)





















SEQ ID NO:21
B2B3-6 (B12-mHSA-F5B6H2)

QVQLVQSGGGLVQPGRSLRLSCAASGFTFDDYAMHWVRQAPGKGLEWVSGISWNSGSI
GYADSVKGRFTISRDNAKNSLYLQMNSLRPEDTAVYYCARDLGAKQWLEGFDYWGQG
TLVTVSSASTGGGGSGGGGSGGGGSSYELTQDPAVSVALGQTVRITCQGDSLRSYYAS
WYQQKPGQAPVLVIYGKNNRPSGIPDRFSGSTSGNSASLTITGAQAEDEADYYCNSRDS
SGNHWVFGGGTKVTVLGAASDAHKSEVAHRFKDLGEENFKALVLIAFAQYLQQSPFED
HVKLVNEVTEFAKTCVADESAENCDKSLHTLFGDKLCTVATLRETYGEMADCCAKQEP
ERNECFLQHKDDNPNLPRLVRPEVDVMCTAFHDNEETFKKYLYEIARRHPYFYAPELLF
FAKRYKAAFTECCQAADKAACLLPKLDELRDEGKASSAKQRLKCASLQKFGERAFKA
WAVARLSQRFPKAEFAEVSKLVTDLTKVHTECCHGDLLECADDRADLAKYICENQDSIS
SKLKECCEKPLLEKSHCIAEVENDEMPADLPSLAADFVESKDVCKNYAEAKDVFLGMF
LYEYARRHPDYSVVLLLRLAKTYETTLEKCCAAADPHECYAKVFDEFKPLVEEPQNLIK
QNCELFEQLGEYKFQNALLVRYTKKVPQVSTPTLVEVSRNLGKVGSKCCKHPEAKRMP

CAEDYLSVVLNQLCVLHEKTPVSDRVTKCCTESLVNRRPCFSALEVDETYVPKEFQAET
FTFHADICTLSEKERQIKKQTALVELVKHKPKATKEQLKAVMDDFAAFVEKCCKADDK
ETCFAEEGKKLVAASQAALGLAAALQVQLVESGGGLVQPGGSLRLSCAASGFTFRSYA
MSWVRQAPGKGLEWVSAISGRGDNTYYADSVKGRFTISRDNSKNTLYLQMNSLRAED
TAVYYCAKMTSNAVGFDYWGQGTLVTVSSGGGGSGGGSGGGGSGQSVLTQPPSVSGA
PGQRVTISCTGRHSNIGLGYGVHWYQQLPGTAPKLLIYGNTNRPSGVPDRFSGFKSGTSA
SLAITGLQAEDEADYYCQSYDRRTPGWVFGGGTKLTVLG

SEQ ID NO:22
B2B3-7 (F4-mHSA-B1D2)





















SEQ ID NO:23
B2B3-8 (F4-mHSA-F5B6H2)






















SEQ ID NO:24
B2B3-9 (H3-HSA-B1D2)

QVQLQESGGGLVKPGGSLRLSCAASGFTFSSYWMSWVRQAPGKGLEWVANINRDGSA
SYYVDSVKGRFTISRDDAKNSLYLQMNSLRAEDTAVYYCARDRGVGYFDLWGRGTLV
TVSSASTGGGGSGGGGSGGGGSQSALTQPASVSGSPGQSITISCTGTSSDVGGYNFVSWY
QQHPGKAPKLMIYDVSDRPSGVSDRFSGSKSGNTASLIISGLQADDEADYYCSSYGSSST
HVIFGGGTKVTVLGAASDAHKSEVAHRFKDLGEENFKALVLIAFAQYLQQCPFEDHVK
LVNEVTEFAKTCVADESAENCDKSLHTLFGDKLCTVATLRETYGEMADCCAKQEPERN
ECFLQHKDDNPNLPRLVRPEVDVMCTAFHDNEETFLKKYLYEIARRHPYFYAPELLFFA
KRYKAAFTECCQAADKAACLLPKLDELRDEGKASSAKQRLKCASLQKFGERAFKAWA
VARLSQRFPKAEFAEVSKLVTDLTKVHTECCHGDLLECADDRADLAKYICENQDSISSK
LKECCEKPLLEKSHCIAEVENDEMPADLPSLAADFVESKDVCKNYAEAKDVFLGMFLY
EYARRHPDYSVVLLLRLAKTYETTLEKCCAAADPHECYAKVFDEFKPLVEEPQNLIKQN
CELFEQLGEYKFQNALLVRYTKKVPQVSTPTLVEVSRNLGKVGSKCCKHPEAKRMPCA
EDYLSVVLNQLCVLHEKTPVSDRVTKCCTESLVNRRPCFSALEVDETYVPKEFNAETFT
FHADICTLSEKERQIKKQTALVELVKHKPKATKEQLKAVMDDFAAFVEKCCKADDKET
CFAEEGKKLVAASQAALGLAAALQVQLVQSGAEVKKPGESLKISCKGSGYSFTSYWIA
WVRQMPGKGLEYMGLIYPGDSDTKYSPSFQGQVTISVDKSVSTAYLQWSSLKPSDSAV
YFCARHDVGYCTDRTCAKWPEWLGVWGQGTLVTVSSGGGGSSGGGSGGGGSQSVLT
QPPSVSAAPGQKVTISCSGSSSNIGNNYVSWYQQLPGTAPKLLIYDHTNRPAGVPDRFSG
SKSGTSASLAISGFRSEDEADYYCASWDYTLSGWVFGGGTKLTVLG

SEQ ID NO:25
B2B3-10 (H3-mHSA-F5B6H2)

QVQLQESGGGLVKPGGSLRLSCAASGFTFSSYWMSWVRQAPGKGLEWVANINRDGSA
SYYVDSVKGRFTISRDDAKNSLYLQMNSLRAEDTAVYYCARDRGVGYFDLWGRGTLV
TVSSASTGGGGSGGGGSGGGGSQSALTQPASVSGSPGQSITISCTGTSSDVGGYNFVSWY
QQHPGKAPKLMIYDVSDRPSGVSDRFSGSKSGNTASLIISGLQADDEADYYCSSYGSSST
HVIFGGGTKVTVLGAASDAHKSEVAHRFKDLGEENFKALVLIAFAQYLQQSPFEDHVKL
VNEVTEFAKTCVADESAENCDKSLHTLFGDKLCTVATLRETYGEMADCCAKQEPERNE
CFLQHKDDNPNLPRLVRPEVDVMCTAFHDNEETFLKKYLYEIARRHPYFYAPELLFFAK
RYKAAFTECCQAADKAACLLPKLDELRDEGKASSAKQRLKCASLQKFGERAFKAWAV
ARLSQRFPKAEFAEVSKLVTDLTKVHTECCHGDLLECADDRADLAKYICENQDSISSKL
KECCEKPLLEKSHCIAEVENDEMPADLPSLAADFVESKDVCKNYAEAKDVFLGMFLYE
YARRHPDYSVVLLLRLAKTYETTLEKCCAAADPHECYAKVFDEFKPLVEEPQNLIKQNC
ELFEQLGEYKFQNALLVRYTKKVPQVSTPTLVEVSRNLGKVGSKCCKHPEAKRMPCAE

DYLSVVLNQLCVLHEKTPVSDRVTKCCTESLVNRRPCFSALEVDETYVPKEFQAETFTF
HADICTLSEKERQIKKQTALVELVKHKPKATKEQLKAVMDDFAAFVEKCCKADDKETC
FAEEGKKLVAASQAALGLAAALQVQLVESGGGLVQPGGSLRLSCAASGFTFRSYAMSW
VRQAPGKGLEWVSAISGRGDNTYYADSVKGRFTISRDNSKNTLYLQMNSLRAEDTAVY
YCAKMTSNAVGFDYWGQGTLVTVSSGGGGSGGGSGGGGSGQSVLTQPPSVSGAPGQR
VTISCTGRHSNIGLGYGVHWYQQLPGTAPKLLIYGNTNRPSGVPDRFSGFKSGTSASLAI
TGLQAEDEADYYCQSYDRRTPGWVFGGGTKLTVLG

SEQ ID NO:26
H3 (anti-ErbB3) scFv

QVQLQESGGGLVKPGGSLRLSCAASGFTFSSYWMSWVRQAPGKGLEWVANINRDGSA
SYYVDSVKGRFTISRDDAKNSLYLQMNSLRAEDTAVYYCARDRGVGYFDLWGRGTLV
TVSSASTGGGGSGGGGSGGGGSQSALTQPASVSGSPGQSITISCTGTSSDVGGYNFVSWY
QQHPGKAPKLMIYDVSDRPSGVSDRFSGSKSGNTASLIISGLQADDEADYYCSSYGSSST
HVIFGGGTKVTVLG

SEQ ID NO:27
B1D2 (anti-ErbB2)

QVQLVQSGAEVKKPGESLKISCKGSGYSFTSYWIAWVRQMPGKGLEYMGLIYPGDSDT
KYSPSFQGQVTISVDKSVSTAYLQWSSLKPSDSAVYFCARHDVGYCTDRTCAKWPEWL
GVWGQGTLVTVSSGGGGSSGGGSGGGGSQSVLTQPPSVSAAPGQKVTISCSGSSSNIGN
NYVSWYQQLPGTAPKLLIYDHTNRPAGVPDRFSGSKSGTSASLAISGFRSEDEADYYCA
SWDYTLSGWVFGGGTKLTVLG

SEQ ID NO:28
A5 (anti-ErbB3) scFv

QVQLVQSGGGLVKPGGSLRLSCAASGFSFNTYDMNWVRQAPGKGLEWVSSISSSSSYIY
YADSVKGRFTISRDNAKNSLYLQMNSLRAEDTAVYYCARDGVATTPFDYWGQGTLVT
VSSGGGGSGGGGSGGGGSQSVLTQPPSVSGAPGQRVTISCTGSSSNIGAGYDVHWYQQL
PGTAPKLLIYGNSNRPSGVPDRFSGSKSGTSASLAITGLQAEDEADYYCQSYDSSLSALF
GGGTKLTVLG

SEQ ID NO:29
ML3.9 (anti-ErbB2) scFv

QVQLVQSGAEVKKPGESLKISCKGSGYSFTSYWIAWVRQMPGKGLEYMGLIYPGDSDT
KYSPSFQGQVTISVDKSVSTAYLQWSSLKPSDSAVYFCARHDVGYCSSSNCAKWPEYFQ
HWGQGTLVTVSSGGGGSSGGGSGGGGSQSVLTQPPSVSAAPGQKVTISCSGSSSNIGNN
YVSWYQQLPGTAPKLLIYDHTNRPAGVPDRFSGSKSGTSASLAISGFRSEDEADYYCAS
WDYTLSGWVFGGGTKLTVLG

SEQ ID NO:30
B12 (anti-ErbB3) scFv

QVQLVQSGGGLVQPGRSLRLSCAASGFTFDDYAMHWVRQAPGKGLEWVSGISWNSGSI
GYADSVKGRFTISRDNAKNSLYLQMNSLRPEDTAVYYCARDLGAKQWLEGFDYWGQG
TLVTVSSASTGGGGSGGGGSGGGGSSYELTQDPAVSVALGQTVRITCQGDSLRSYYAS
WYQQKPGQAPVLVIYGKNNRPSGIPDRFSGSTSGNSASLTITGAQAEDEADYYCNSRDS
SGNHWVFGGGTKVTVLG

SEQ ID NO:31
F4 (anti-ErbB3) scFv

QVQLQESGGGLVKPGGSLRLSCAASGFTFSSYAMSWVRQAPGKGLEWVSTISGSGGST
YYADSVKGRFTISRDNSKNTLYLQMNSLRAEDTAVYYCAKGYSSSWSEVASGYWGQG
TLVTVSSASTGGGGSGGGGSGGGGSAIVMTQSPSSLSASVGDRVTITCRASQGIRNDLG
WYQQKAGKAPKLLIYAASSLQSGVPSRFSGSGSGTDFTLTISSLQPDDFATYFCQQAHSF
PPTFGGGTKVEIKRG

SEQ ID NO:32
F5B6H2 (anti-ErbB2) scFv

QVQLVESGGGLVQPGGSLRLSCAASGFTFRSYAMSWVRQAPGKGLEWVSAISGRGDNT
YYADSVKGRFTISRDNSKNTLYLQMNSLRAEDTAVYYCAKMTSNAVGFDYWGQGTLV
TVSSGGGGSGGGSGGGGSGQSVLTQPPSVSGAPGQRVTISCTGRHSNIGLGYGVHWYQ
QLPGTAPKLLIYGNTNRPSGVPDRFSGFKSGTSASLAITGLQAEDEADYYCQSYDRRTPG
WVFGGGTKLTVLG

SEQ ID NO:33
c-myc
EQKLISEEDL

SEQ ID NO:34
hemagglutinin
YPYDVPDYA

SEQ ID NO:35
Histidine tag (HIS6)
HHHHHH

SEQ ID NO:36
Caspase 3
Homo sapiens
CAC88866








SEQ ID NO:37
Caspase 8
Homo sapiens
AAD24962










SEQ ID NO:38
Granzyme B
Homo sapiens
AAA75490







SEQ ID NO:39
Cytochrome c
Homo sapiens
NP_061820

MGDVEKGKKIFIMKCSQCHTVEKGGKHKTGPNLHGLFGRKTGQAPGYSYTAANKNKGI
IWGEDTLMEYLENPKKYIPGTKMIFVGIKKKEERADLIAYLKKATNE

SEQ ID NO:40
Tumor Necrosis Factor Alpha (TNFα)
Homo sapiens
CAA26669

MSTESMIRDVELAEEALPKKTGGPQGSRRCLFLSLFSFLIVAGATTLFCLLHFGVIGPQRE
EFPRDLSLISPLAQAVRSSSRTPSDKPVAHVVANPQAEGQLQWLNRRANALLANGVELR
DNQLVVPSEGLYLIYSQVLFKGQGCPSTHVLLTHTISRIAVSYQTKVNLLSAIKSPCQRET
PEGAEAKPWYEPIYLGGVFQLEKGDRLSAEINRPDYLDFAESGQVYFGIIAL

SEQ ID NO:41
Fas
Homo sapiens
CAI13871

MLGIWTLLPLVLTSVARLSSKSVNAQVTDINSKGLELRKTVTTVETQNLEGLHHDGQFC
HKPCPPGERKARDCTVNGDEPDCVPCQEGKEYTDKAHFSSKCRRCRLCDEGHGLEVEI
NCTRTQNTKCRCKPNFFCNSTVCEHCDPCTKCEHGIIKECTLTSNTKCKEEVKRKEVQK
TCRKHRKENQGSHESPTLNPETVAINLSDVDLSKYITTIAGVMTLSQVKGFVRKNGVNE
AKIDEIKNDNVQDTAEQKVQLLRNWHQLHGKKEAYDTLIKDLKKANLCTLAEKIQTIIL
KDITSDSENSNFRNEIQSLV

SEQ ID NO:42
Bad (Bcl2 antagonist of cell death)
Homo sapiens
CAG46757

MFQIPEFEPSEQEDSSSAERGLGPSPAGDGPSGSGKHHRQAPGLLWDASHQQEQPTSSSH
HGGAGAVEIRSRHSSYPAGTEDDEGMGEEPSPFRGRSRSAPPNLWAAQRYGRELRRMS
DEFVDSFKKGLPRPKSAGTATQMRQSSSWTRVFQSWWDRNLGRGSSAPSQ

SEQ ID NO:43
Apoptosis Inducing Protein (AIP)
Homo sapiens
AAK67626

MVDHLANTEINSQRIAAVESCFGASGQPLALPGRVLLGEGVLTKECRKKAKPRIFFLFND
ILVYGSIVLNKRKYRSQHIIPLEEVTLELLPETLQAKNRWMIKTAKKSFVVSAASATERQ
EWISHIEECVRRQLKATGRPPSTEHAAPWIPDKATDICMRCTQTRFSALTRRHHCRKCGF
VVCAECSRQRFLLPRLSPKPVRVCSLCYRELAAQQRQEEAEEQGAGSPRQPAHLARPIC
GASSGDDDDSDEDKEGSRDGDWPSSVEFYASGVAWSAFHS

SEQ ID NO:44
Pierisin-1
Pieris rapae
Q9U8Q4

MADRQPYMTNGIQAAVVEWIRALDLEIISLLLSRAWPMALLATSELRWRPTVLTDTDN
VVRLDRRQRLVRWDRRPPNEIFLDGFVPIVTRENPDWEETDLYGFAKNNHPSIFVSTTKT
QRNKKKYVWTPRNANRGIVYQYEIYAPGGVDVNDSFSDASPWPNQMEVAFPGGIQNIY
IRSARELHNGRIQRIWINPNFLDPGDLEPIVSSSRTPQVIWRMNHPDGGHRDQRSERSASS
YDDLMYGGTGNVQEDTFGDEPNNPKPIAAGEFMIESIKDKNSFLDLSKNVNGGVIHSNL
YSGGDNQIWVFSYDDNKKAYRIQSYQNSYLYLSWDSNASSKEMILRGYTNSGSNNQY
WQIEQTGKNYRLRNLLNLDMIITAQDKPSAFGGKEVIVNTEISNSNTKISQEWKMIPFDF
RPIIDGDYNIFNVDLSNQVVDFSNQPDLLVHGHIFCDNENQTWHFTYNSTYHAYKIWSG
RKSNLLLTWDSNAASKEMVVRAYTESRSKNQYWRIEQTGSKSYKVRNLENSSMILGLT

RVSTPYGGLNLMVEDDSDGHSDLHSDWDIKPIFYQDIPDGDYNIFNDNFPNIAIDFTNQE
GSLIHGHNFCSNNNQKWSFVFDGKRKAYRIKSGVRSNLWLSWDSNASSKEMVLRAYTE
SGSSNQYWRLDEANDGSYRIRNLQDYYKLIALTNKNTPYGGKELIVSDNKESGNTWYL
KKLGEVPLPNRKFRIATKLNYKKVIDSSTSYNLIITHDLNFASSIWELVYDSSKKAYNIYS
SDINNLGWIYQNKNFFVKLGNIDGPDHGDLRYFWTIEYSMQTGCYLIRSLHDPANAVGY
TDSESVITDTSTYSDNQLFHFILM

SEQ ID NO:45
TRAIL (TNF-related apoptosis-inducing ligand)
Homo sapiens
P50591







SEQ ID NO:46
Bax
Homo sapiens
Q07812

MDGSGEQPRGGGPTSSEQIMKTGALLLQGFIQDRAGRMGGEAPELALDPVPQDASTKK
LSECLKRIGDELDSNMELQRMIAAVDTDSPREVFFRVAADMFSDGNFNWGRVVALFYF
ASKLVLKALCTKVPELIRTIMGWTLDFLRERLLGWIQDQGGWDGLLSYFGTPTWQTVTI
FVAGVLTASLTIWKKMG

SEQ ID NO:47
Green Fluorescent Protein (GFP)
Aequorea victoria
P42212

MSKGEELFTGVVPILVELDGDVNGHKFSVSGEGEGDATYGKLTLKFICTTGKLPVPWPT
LVTTFSYGVQCFSRYPDHMKQHDFFKSAMPEGYVQERTIFFKDDGNYKTRAEVKFEGD
TLVNRIELKGIDFKEDGNILGHKLEYNYNSHNVYIMADKQKNGIKVNFKIRHNIEDGSVQ
LADHYQQNTPIGDGPVLLPDNHYLSTQSALSKDPNEKRDHMVLLEFVTAAGITHGMDE
LYK

SEQ ID NO:48
Yellow Fluorescent Protein (YFP)
Aliivibrio fischeri
P21578

MFKGIVEGIGIIEKIDIYTDLDKYAIRFPENMLNGIKKESSIMFNGCFLTVTSVNSNIVWFD
IFEKEARKLDTFREYKVGDRVNLGTFPKFGAASGGHILSARISCVASIIEIIENEDYQQMW
IQIPENFTEFLIDKDYIAVDGISLTIDTIKNNQFFISLPLKIAQNTNMKWRKKGDKVNVELS
NKINANQCW

SEQ ID NO:49
Cyan Fluorescent Protein (CFP)
Montastraea cavernosa
AAL17905

MSVIKSVMKIKLRMDGIVNGHKFMITGEGEGKPFEGTHTIILKVKEGGPLPFAYDILTTAF
QYGNRVFTKYPKDIPDYFKQSFPEGYSWERSMTFEDQGVCTVTSDIKLEGDCFFYEIRFY
GVNFPSSGPVMQKKTLKWEPSTENMYVRDGVLLGDVSRTLLLEGDKHHRCNFRSTYG
AKKGVVLPEYHFVDHRIEILSHDKDYNTVEVYENAVARPSMLPVKAK

SEQ ID NO:50
Red Fluorescent Protein (RFP)
Discosoma sp. SSAL-2000
AAG16224

MSCSKNVIKEFMRFKVRMEGTVNGHEFEIKGEGEGRPYEGHCSVKLMVTKGGPLPFAF
DILSPQFQYGSKVYVKHPADIPDYKKLSFPEGFKWERVMNFEDGGVVTVSQDSSLKDG
CFIYEVKFIGVNFPSDGPVMQRRTRGWEASSERLYPRDGVLKGDIHMALRLEGGGHYL
VEFKSIYMVKKPSVQLPGYYYVDSKLDMTSHNEDYTVVEQYEKTQGRHHPFIKPLQ

SEQ ID NO:51
Luciferase
Photinus pyralis
CAA59282

MEDAKNIKKGPAPFYPLEDGTAGEQLHKAMKRYALVPGTIAFTDAHIEVNITYAEYFEM
SVRLAEAMKRYGLNTNHRIVVCSENSLQFFMPVLGALFIGVAVAPANDIYNERELLNSM
NISQPTVVFVSKKGLQKILNVQKKLPIIQKIIIMDSKTDYQGFQSMYTFVTSHLPPGFNEY
DFVPESFDRDKTIALIMNSSGSTGSPKGVALPHRTACVRFSHARDPIFGNQIIPDTAILSVV
PFHHGFGMFTTLGYLICGFRVVLMYRFEEELFLRSLQDYKIQSALLVPTLFSFFAKSTLID
KYDLSNLHEIASGGAPLSKEVGEAVAKRFHLPGIRQGYGLTETTSAILITPEGDDKPGAV
GKVVPFFEAKVVDLDTGKTLGVNQRGELCVRGPMIMSGYVNDPEATNALIDKDGWLH
SGDIAYWDEDEHFFIVDRLKSLIKYKGCQVAPAELESILLQHPNIFDAGVAGLPGDDAGE
LPAAVVVLEHGKTMTEKEIVDYVASQVTTAKKLRGGVVFVDEVPKGLTGKLDARKIRE
ILIKAKKGGKSKL

SEQ ID NO:52
Luciferase
Renilla reniformis
AAA29804

MTSKVYDPEQRKRMITGPQWWARCKQMNVLDSFINYYDSEKHAENAVIFLHGNAASS
YLWRHVVPHIEPVARCIIPDLIGMGKSGKSGNGSYRLLDHYKYLTAWFELLNLPKKIIFV
GHDWGACLAFHYSYEHQDKIKAIVHAESVVDVIESWDEWPDIEEDIALIKSEEGEKMVL
ENNFFVETMLPSKIMRKLEPEEFAAYLEPFKEKGEVRRPTLSWPREIPLVKGGKPDVVQI
VRNYNAYLRASDDLPKMFIESDPGFFSNAIVEGAKKFPNTEFVKVKGLHFSQEDAPDEM
GKYIKSFVERVLKNEQ

SEQ ID NO:53
Human Serum Albumin (HSA) Linker with C34S substitution, Domain I
Amino Acid Sequence

DAHKSEVAHRFKDLGEENFKALVLIAFAQYLQQSPFEDHVKLVNEVTEFAKTCVADESAE
NCDKSLHTLFGDKLCTVATLRETYGEMADCCAKQEPERNECFLQHKDDNPNLPRLVRPEV
DVMCTAFHDNEETFLKKYLYEIARRHPYFYAPELLFFAKRYKAAFTECCQAADKAACLLP
KLDELRDEGKASSAKQR

SEQ ID NO:54
Human Serum Albumin (HSA) Linker, Domain II
Amino Acid Sequence

GKASSAKQRLKCASLQKFGERAFKAWAVARLSQRFPKAEFAEVSKLVTDLTKVHTECC
HGDLLECADDRADLAKYICENQDSISSKLKECCEKPLLEKSHCIAEVENDEMPADLPSLA
ADFVESKDVCKNYAEAKDVFLGMFLYEYARRHPDYSVVLLLRLAKTYETTLEKCCAA
ADPHECYAKVFDEFKPLVEEPQ

SEQ ID NO:55
Human Serum Albumin (HSA) Linker with N503Q substitution, Domain III
Amino Acid Sequence

VEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETY
VPKEFQAETFTFHADICTLSEKERQIKKQTALVELVKHKPKATKEQLKAVMDDFAAFVE
KCCKADDKETCFAEEGKKLVAASQAALGL

SEQ ID NO:56
Human Serum Albumin (HSA) Linker, Domain I
Amino Acid Sequence

DAHKSEVAHRFKDLGEENFKALVLIAFAQYLQQCPFEDHVKLVNEVTEFAKTCVADES
AENCDKSLHTLFGDKLCTVATLRETYGEMADCCAKQEPERNECFLQHKDDNPNLPRLV

RPEVDVMCTAFHDNEETFLKKYLYEIARRHPYFYAPELLFFAKRYKAAFTECCQAADK
AACLLPKLDELRDEGKASSAKQR

SEQ ID NO:57
Human Serum Albumin (HSA) Linker, Domain III
Amino Acid Sequence






SEQ ID NO:58
Human Alpha-Fetoprotein
Amino Acid Sequence













APPENDIX B
SEQUENCE ALIGNMENTS

B2B3-1 (H3-mHSA-B1D2)

1 QVQLQESGGG LVKPGGSLRL SCAASGFTFS SYWMSWVRQA PGKGLEWVAN
51 INRDGSASYY VDSVKGRFTI SRDDAKNSLY LQMNSLRAED TAVYYCARDR
101 GVGYFDLWGR GTLVTVSSAS TGGGGSGGGG SGGGGSQSAL TQPASVSGSP
Anti-ErbB3 scFv (H3)
151 GQSITISCTG TSSDVGGYNF VSWYQQHPGK APKLMIYDVS DRPSGVSDRF
201 SGSKSGNTAS LIISGLQADD EADYYCSSYG SSSTHVIFGG GTKVTVLGAA
251 SDAHKSEVAH RFKDLGEENF KALVLIAFAQ YLQQSPFEDH VKLVNEVTEF
301 AKTCVADESA ENCDKSLHTL FGDKLCTVAT LRETYGEMAD CCAKQEPERN
351 ECFLQHKDDN PNLPRLVRPE VDVMCTAFHD NEETFLKKYL YEIARRHPYF
401 YAPELLFFAK RYKAAFTECC QAADKAACLL PKLDELRDEG KASSAKQRLK
451 CASLQKFGER AFKAWAVARL SQRFPKAEFA EVSKLVTDLT KVHTECCHGD
501 LLECADDRAD LAKYICENQD SISSKLKECC EKPLLEKSHC IAEVENDEMP
Modified Human Serum Albumin
551 ADLPSLAADF VESKDVCKNY AEAKDVFLGM FLYEYARRHP DYSVVLLLRL
601 AKTYETTLEK CCAAADPHEC YAKVFDEFKP LVEEPQNLIK QNCELFEQLG
651 EYKFQNALLV RYTKKVPQVS TPTLVEVSRN LGKVGSKCCK HPEAKRMPCA
701 EDYLSVVLNQ LCVLHEKTPV SDRVTKCCTE SLVNRRPCFS ALEVDETYVP
751 KEFQAETFTF HADICTLSEK ERQIKKQTAL VELVKHKPKA TKEQLKAVMD
801 DFAAFVEKCC KADDKETCFA EEGKKLVAAS QAALGLAAAL QVQLVQSGAE
851 VKKPGESLKI SCKGSGYSFT SYWIAWVRQM PGKGLEYMGL IYPGDSDTKY
901 SPSFQGQVTI SVDKSVSTAY LQWSSLKPSD SAVYFCARHD VGYCTDRTCA
951 KWPEWLGVWG QGTLVTVSSG GGGSSGGGSG GGGSQSVLTQ PPSVSAAPGQ
Anti-ErbB2 scFv (B1D2)
1001 KVTISCSGSS SNIGNNYVSW YQQLPGTAPK LLIYDHTNRP AGVPDRFSGS
1051 KSGTSASLAI SGFRSEDEAD YYCASWDYTL SGWVFGGGTK LTVLG

CDR loops are highlighted within H3 (blue with pink CDRs) and B1D2 (pink with cyan CDRs).
Connectors to modified HSA are shown in red.

Sequence alignments for B2B3/mHSA variants

1 45
A5-mHSA-ML3.9 (1) QVQLVQSGGGLVKPGGSLRLSCAASGFSFNTYDMNWVRQAPGKGL
A5-mHSA-B1D2 (1) QVQLVQSGGGLVKPGGSLRLSCAASGFSFNTYDMNWVRQAPGKGL
A5-mHSA-F5B6H2 (1) QVQLVQSGGGLVKPGGSLRLSCAASGFSFNTYDMNWVRQAPGKGL
B12-mHSA-B1D2 (1) QVQLVQSGGGLVQPGRSLRLSCAASGFTFDDYAMHWVRQAPGKGL
B12-mHSA-F5B6H2 (1) QVQLVQSGGGLVQPGRSLRLSCAASGFTFDDYAMHWVRQAPGKGL
F4-mHSA-B1D2 (1) QVQLQESGGGLVKPGGSLRLSCAASGFTFSSYAMSWVRQAPGKGL
F4-mHSA-F5B6H2 (1) QVQLQESGGGLVKPGGSLRLSCAASGFTFSSYAMSWVRQAPGKGL
H3-mHSA-B1D2 (1) QVQLQESGGGLVKPGGSLRLSCAASGFTFSSYWMSWVRQAPGKGL
H3-mHSA-F5B6H2 (1) QVQLQESGGGLVKPGGSLRLSCAASGFTFSSYWMSWVRQAPGKGL

46 90
A5-mHSA-ML3.9 (46) EWVSSISSSSSYIYYADSVKGRFTISRDNAKNSLYLQMNSLRAED
A5-mHSA-B1D2 (46) EWVSSISSSSSYIYYADSVKGRFTISRDNAKNSLYLQMNSLRAED
A5-mHSA-F5B6H2 (46) EWVSSISSSSSYIYYADSVKGRFTISRDNAKNSLYLQMNSLRAED
B12-mHSA-B1D2 (46) EWVSGISWNSGSIGYADSVKGRFTISRDNAKNSLYLQMNSLRPED
B12-mHSA-F5B6H2 (46) EWVSGISWNSGSIGYADSVKGRFTISRDNAKNSLYLQMNSLRPED
F4-mHSA-B1D2 (46) EWVSTISGSGGSTYYADSVKGRFTISRDNSKNTLYLQMNSLRAED
F4-mHSA-F5B6H2 (46) EWVSTISGSGGSTYYADSVKGRFTISRDNSKNTLYLQMNSLRAED
H3-mHSA-B1D2 (46) EWVANINRDGSASYYVDSVKGRFTISRDDAKNSLYLQMNSLRAED
H3-mHSA-F5B6H2 (46) EWVANINRDGSASYYVDSVKGRFTISRDDAKNSLYLQMNSLRAED

91 135
A5-mHSA-ML3.9 (91) TAVYYCARDG---VATTPFDYWGQGTLVTVS---SGGGGSGGGGS
A5-mHSA-B1D2 (91) TAVYYCARDG---VATTPFDYWGQGTLVTVS---SGGGGSGGGGS
A5-mHSA-F5B6H2 (91) TAVYYCARDG---VATTPFDYWGQGTLVTVS---SGGGGSGGGGS
B12-mHSA-B1D2 (91) TAVYYCARDLGAKQWLEGFDYWGQGTLVTVSSASTGGGGSGGGGS
B12-mHSA-F5B6H2 (91) TAVYYCARDLGAKQWLEGFDYWGQGTLVTVSSASTGGGGSGGGGS
F4-mHSA-B1D2 (91) TAVYYCAKGYSSSWSEVASGYWGQGTLVTVSSASTGGGGSGGGGS
F4-mHSA-F5B6H2 (91) TAVYYCAKGYSSSWSEVASGYWGQGTLVTVSSASTGGGGSGGGGS
H3-mHSA-B1D2 (91) TAVYYCARDR----GVGYFDLWGRGTLVTVSSASTGGGGSGGGGS
H3-mHSA-F5B6H2 (91) TAVYYCARDR----GVGYFDLWGRGTLVTVSSASTGGGGSGGGGS

136 180
A5-mHSA-ML3.9 (130) GGGGSQSVLTQPPS-VSGAPGQRVTISCTGSSSNIGAGYDVHWYQ
A5-mHSA-B1D2 (130) GGGGSQSVLTQPPS-VSGAPGQRVTISCTGSSSNIGAGYDVHWYQ
A5-mHSA-F5B6H2 (130) GGGGSQSVLTQPPS-VSGAPGQRVTISCTGSSSNIGAGYDVHWYQ
B12-mHSA-B1D2 (136) GGGGSSYELTQDPA-VSVALGQTVRITCQGDSLRS---YYASWYQ
B12-mHSA-F5B6H2 (136) GGGGSSYELTQDPA-VSVALGQTVRITCQGDSLRS---YYASWYQ
F4-mHSA-B1D2 (136) GGGGSAIVMTQSPSSLSASVGDRVTITCRASQGIR---NDLGWYQ
F4-mHSA-F5B6H2 (136) GGGGSAIVMTQSPSSLSASVGDRVTITCRASQGIR---NDLGWYQ
H3-mHSA-B1D2 (132) GGGGSQSALTQPAS-VSGSPGQSITISCTGTSSDVGGYNFVSWYQ
H3-mHSA-F5B6H2 (132) GGGGSQSALTQPAS-VSGSPGQSITISCTGTSSDVGGYNFVSWYQ

181 225
A5-mHSA-ML3.9 (174) QLPGTAPKLLIYGNSNRPSGVPDRFSGSKSGTSASLAITGLQAED
A5-mHSA-B1D2 (174) QLPGTAPKLLIYGNSNRPSGVPDRFSGSKSGTSASLAITGLQAED
A5-mHSA-F5B6H2 (174) QLPGTAPKLLIYGNSNRPSGVPDRFSGSKSGTSASLAITGLQAED
B12-mHSA-B1D2 (177) QKPGQAPVLVIYGKNNRPSGIPDRFSGSTSGNSASLTITGAQAED
B12-mHSA-F5B6H2 (177) QKPGQAPVLVIYGKNNRPSGIPDRFSGSTSGNSASLTITGAQAED
F4-mHSA-B1D2 (178) QKAGKAPKLLIYAASSLQSGVPSRFSGSGSGTDFTLTISSLQPDD
F4-mHSA-F5B6H2 (178) QKAGKAPKLLIYAASSLQSGVPSRFSGSGSGTDFTLTISSLQPDD
H3-mHSA-B1D2 (176) QHPGKAPKLMIYDVSDRPSGVSDRFSGSKSGNTASLIISGLQADD
H3-mHSA-F5B6H2 (176) QHPGKAPKLMIYDVSDRPSGVSDRFSGSKSGNTASLIISGLQADD

226 270
A5-mHSA-ML3.9 (219) EADYYCQSYDSS-LSALFGGGTKLTVLG-AASDAHKSEVAHRFKD
A5-mHSA-B1D2 (219) EADYYCQSYDSS-LSALFGGGTKLTVLG-AASDAHKSEVAHRFKD
A5-mHSA-F5B6H2 (219) EADYYCQSYDSS-LSALFGGGTKLTVLG-AASDAHKSEVAHRFKD
B12-mHSA-B1D2 (222) EADYYCNSRDSSGNHWVFGGGTKVTVLG-AASDAHKSEVAHRFKD
B12-mHSA-F5B6H2 (222) EADYYCNSRDSSGNHWVFGGGTKVTVLG-AASDAHKSEVAHRFKD
F4-mHSA-B1D2 (223) FATYFCQQAHSF--PPTFGGGTKVEIKRGAASDAHKSEVAHRFKD
F4-mHSA-F5B6H2 (223) FATYFCQQAHSF--PPTFGGGTKVEIKRGAASDAHKSEVAHRFKD
H3-mHSA-B1D2 (221) EADYYCSSYGSSSTHVIFGGGTKVTVLG-AASDAHKSEVAHRFKD
H3-mHSA-F5B6H2 (221) EADYYCSSYGSSSTHVIFGGGTKVTVLG-AASDAHKSEVAHRFKD

271 315
A5-mHSA-ML3.9 (262) LGEENFKALVLIAFAQYLQQSPFEDHVKLVNEVTEFAKTCVADES
A5-mHSA-B1D2 (262) LGEENFKALVLIAFAQYLQQSPFEDHVKLVNEVTEFAKTCVADES
A5-mHSA-F5B6H2 (262) LGEENFKALVLIAFAQYLQQSPFEDHVKLVNEVTEFAKTCVADES
B12-mHSA-B1D2 (266) LGEENFKALVLIAFAQYLQQSPFEDHVKLVNEVTEFAKTCVADES
B12-mHSA-F5B6H2 (266) LGEENFKALVLIAFAQYLQQSPFEDHVKLVNEVTEFAKTCVADES
F4-mHSA-B1D2 (266) LGEENFKALVLIAFAQYLQQSPFEDHVKLVNEVTEFAKTCVADES
F4-mHSA-F5B6H2 (266) LGEENFKALVLIAFAQYLQQSPFEDHVKLVNEVTEFAKTCVADES
H3-mHSA-B1D2 (265) LGEENFKALVLIAFAQYLQQSPFEDHVKLVNEVTEFAKTCVADES
H3-mHSA-F5B6H2 (265) LGEENFKALVLIAFAQYLQQSPFEDHVKLVNEVTEFAKTCVADES

316 360
A5-mHSA-ML3.9 (307) AENCDKSLHTLFGDKLCTVATLRETYGEMADCCAKQEPERNECFL
A5-mHSA-B1D2 (307) AENCDKSLHTLFGDKLCTVATLRETYGEMADCCAKQEPERNECFL
A5-mHSA-F5B6H2 (307) AENCDKSLHTLFGDKLCTVATLRETYGEMADCCAKQEPERNECFL
B12-mHSA-B1D2 (311) AENCDKSLHTLFGDKLCTVATLRETYGEMADCCAKQEPERNECFL
B12-mHSA-F5B6H2 (311) AENCDKSLHTLFGDKLCTVATLRETYGEMADCCAKQEPERNECFL
F4-mHSA-B1D2 (311) AENCDKSLHTLFGDKLCTVATLRETYGEMADCCAKQEPERNECFL
F4-mHSA-F5B6H2 (311) AENCDKSLHTLFGDKLCTVATLRETYGEMADCCAKQEPERNECFL
H3-mHSA-B1D2 (310) AENCDKSLHTLFGDKLCTVATLRETYGEMADCCAKQEPERNECFL
H3-mHSA-F5B6H2 (310) AENCDKSLHTLFGDKLCTVATLRETYGEMADCCAKQEPERNECFL

361 405
A5-mHSA-ML3.9 (352) QHKDDNPNLPRLVRPEVDVMCTAFHDNEETFLKKYLYEIARRHPY
A5-mHSA-B1D2 (352) QHKDDNPNLPRLVRPEVDVMCTAFHDNEETFLKKYLYEIARRHPY
A5-mHSA-F5B6H2 (352) QHKDDNPNLPRLVRPEVDVMCTAFHDNEETFLKKYLYEIARRHPY
B12-mHSA-B1D2 (356) QHKDDNPNLPRLVRPEVDVMCTAFHDNEETFLKKYLYEIARRHPY
B12-mHSA-F5B6H2 (356) QHKDDNPNLPRLVRPEVDVMCTAFHDNEETFLKKYLYEIARRHPY
F4-mHSA-B1D2 (356) QHKDDNPNLPRLVRPEVDVMCTAFHDNEETFLKKYLYEIARRHPY
F4-mHSA-F5B6H2 (356) QHKDDNPNLPRLVRPEVDVMCTAFHDNEETFLKKYLYEIARRHPY
H3-mHSA-B1D2 (355) QHKDDNPNLPRLVRPEVDVMCTAFHDNEETFLKKYLYEIARRHPY
H3-mHSA-F5B6H2 (355) QHKDDNPNLPRLVRPEVDVMCTAFHDNEETFLKKYLYEIARRHPY

406 450
A5-mHSA-ML3.9 (397) FYAPELLFFAKRYKAAFTECCQAADKAACLLPKLDELRDEGKASS
A5-mHSA-B1D2 (397) FYAPELLFFAKRYKAAFTECCQAADKAACLLPKLDELRDEGKASS
A5-mHSA-F5B6H2 (397) FYAPELLFFAKRYKAAFTECCQAADKAACLLPKLDELRDEGKASS
B12-mHSA-B1D2 (401) FYAPELLFFAKRYKAAFTECCQAADKAACLLPKLDELRDEGKASS
B12-mHSA-F5B6H2 (401) FYAPELLFFAKRYKAAFTECCQAADKAACLLPKLDELRDEGKASS
F4-mHSA-B1D2 (401) FYAPELLFFAKRYKAAFTECCQAADKAACLLPKLDELRDEGKASS
F4-mHSA-F5B6H2 (401) FYAPELLFFAKRYKAAFTECCQAADKAACLLPKLDELRDEGKASS
H3-mHSA-B1D2 (400) FYAPELLFFAKRYKAAFTECCQAADKAACLLPKLDELRDEGKASS
H3-mHSA-F5B6H2 (400) FYAPELLFFAKRYKAAFTECCQAADKAACLLPKLDELRDEGKASS

451 495
A5-mHSA-ML3.9 (442) AKQRLKCASLQKFGERAFKAWAVARLSQRFPKAEFAEVSKLVTDL

A5-mHSA-B1D2 (442) AKQRLKCASLQKFGERAFKAWAVARLSQRFPKAEFAEVSKLVTDL
A5-mHSA-F5B6H2 (442) AKQRLKCASLQKFGERAFKAWAVARLSQRFPKAEFAEVSKLVTDL
B12-mHSA-B1D2 (446) AKQRLKCASLQKFGERAFKAWAVARLSQRFPKAEFAEVSKLVTDL
B12-mHSA-F5B6H2 (446) AKQRLKCASLQKFGERAFKAWAVARLSQRFPKAEFAEVSKLVTDL
F4-mHSA-B1D2 (446) AKQRLKCASLQKFGERAFKAWAVARLSQRFPKAEFAEVSKLVTDL
F4-mHSA-F5B6H2 (446) AKQRLKCASLQKFGERAFKAWAVARLSQRFPKAEFAEVSKLVTDL
H3-mHSA-B1D2 (445) AKQRLKCASLQKFGERAFKAWAVARLSQRFPKAEFAEVSKLVTDL
H3-mHSA-F5B6H2 (445) AKQRLKCASLQKFGERAFKAWAVARLSQRFPKAEFAEVSKLVTDL

496 540
A5-mHSA-ML3.9 (487) TKVHTECCHGDLLECADDRADLAKYICENQDSISSKLKECCEKPL
A5-mHSA-B1D2 (487) TKVHTECCHGDLLECADDRADLAKYICENQDSISSKLKECCEKPL
A5-mHSA-F5B6H2 (487) TKVHTECCHGDLLECADDRADLAKYICENQDSISSKLKECCEKPL
B12-mHSA-B1D2 (491) TKVHTECCHGDLLECADDRADLAKYICENQDSISSKLKECCEKPL
B12-mHSA-F5B6H2 (491) TKVHTECCHGDLLECADDRADLAKYICENQDSISSKLKECCEKPL
F4-mHSA-B1D2 (491) TKVHTECCHGDLLECADDRADLAKYICENQDSISSKLKECCEKPL
F4-mHSA-F5B6H2 (491) TKVHTECCHGDLLECADDRADLAKYICENQDSISSKLKECCEKPL
H3-mHSA-B1D2 (490) TKVHTECCHGDLLECADDRADLAKYICENQDSISSKLKECCEKPL
H3-mHSA-F5B6H2 (490) TKVHTECCHGDLLECADDRADLAKYICENQDSISSKLKECCEKPL

541 585
A5-mHSA-ML3.9 (532) LEKSHCIAEVENDEMPADLPSLAADFVESKDVCKNYAEAKDVFLG
A5-mHSA-B1D2 (532) LEKSHCIAEVENDEMPADLPSLAADFVESKDVCKNYAEAKDVFLG
A5-mHSA-F5B6H2 (532) LEKSHCIAEVENDEMPADLPSLAADFVESKDVCKNYAEAKDVFLG
B12-mHSA-B1D2 (536) LEKSHCIAEVENDEMPADLPSLAADFVESKDVCKNYAEAKDVFLG
B12-mHSA-F5B6H2 (536) LEKSHCIAEVENDEMPADLPSLAADFVESKDVCKNYAEAKDVFLG
F4-mHSA-B1D2 (536) LEKSHCIAEVENDEMPADLPSLAADFVESKDVCKNYAEAKDVFLG
F4-mHSA-F5B6H2 (536) LEKSHCIAEVENDEMPADLPSLAADFVESKDVCKNYAEAKDVFLG
H3-mHSA-B1D2 (535) LEKSHCIAEVENDEMPADLPSLAADFVESKDVCKNYAEAKDVFLG
H3-mHSA-F5B6H2 (535) LEKSHCIAEVENDEMPADLPSLAADFVESKDVCKNYAEAKDVFLG

586 630
A5-mHSA-ML3.9 (577) MFLYEYARRHPDYSVVLLLRLAKTYETTLEKCCAAADPHECYAKV
A5-mHSA-B1D2 (577) MFLYEYARRHPDYSVVLLLRLAKTYETTLEKCCAAADPHECYAKV
A5-mHSA-F5B6H2 (577) MFLYEYARRHPDYSVVLLLRLAKTYETTLEKCCAAADPHECYAKV
B12-mHSA-B1D2 (581) MFLYEYARRHPDYSVVLLLRLAKTYETTLEKCCAAADPHECYAKV
B12-mHSA-F5B6H2 (581) MFLYEYARRHPDYSVVLLLRLAKTYETTLEKCCAAADPHECYAKV
F4-mHSA-B1D2 (581) MFLYEYARRHPDYSVVLLLRLAKTYETTLEKCCAAADPHECYAKV
F4-mHSA-F5B6H2 (581) MFLYEYARRHPDYSVVLLLRLAKTYETTLEKCCAAADPHECYAKV
H3-mHSA-B1D2 (580) MFLYEYARRHPDYSVVLLLRLAKTYETTLEKCCAAADPHECYAKV
H3-mHSA-F5B6H2 (580) MFLYEYARRHPDYSVVLLLRLAKTYETTLEKCCAAADPHECYAKV

631 675
A5-mHSA-ML3.9 (622) FDEFKPLVEEPQNLIKQNCELFEQLGEYKFQNALLVRYTKKVPQV
A5-mHSA-B1D2 (622) FDEFKPLVEEPQNLIKQNCELFEQLGEYKFQNALLVRYTKKVPQV
A5-mHSA-F5B6H2 (622) FDEFKPLVEEPQNLIKQNCELFEQLGEYKFQNALLVRYTKKVPQV
B12-mHSA-B1D2 (626) FDEFKPLVEEPQNLIKQNCELFEQLGEYKFQNALLVRYTKKVPQV
B12-mHSA-F5B6H2 (626) FDEFKPLVEEPQNLIKQNCELFEQLGEYKFQNALLVRYTKKVPQV
F4-mHSA-B1D2 (626) FDEFKPLVEEPQNLIKQNCELFEQLGEYKFQNALLVRYTKKVPQV
F4-mHSA-F5B6H2 (626) FDEFKPLVEEPQNLIKQNCELFEQLGEYKFQNALLVRYTKKVPQV
H3-mHSA-B1D2 (625) FDEFKPLVEEPQNLIKQNCELFEQLGEYKFQNALLVRYTKKVPQV
H3-mHSA-F5B6H2 (625) FDEFKPLVEEPQNLIKQNCELFEQLGEYKFQNALLVRYTKKVPQV

676 720
A5-mHSA-ML3.9 (667) STPTLVEVSRNLGKVGSKCCKHPEAKRMPCAEDYLSVVLNQLCVL
A5-mHSA-B1D2 (667) STPTLVEVSRNLGKVGSKCCKHPEAKRMPCAEDYLSVVLNQLCVL
A5-mHSA-F5B6H2 (667) STPTLVEVSRNLGKVGSKCCKHPEAKRMPCAEDYLSVVLNQLCVL

B12-mHSA-B1D2 (671) STPTLVEVSRNLGKVGSKCCKHPEAKRMPCAEDYLSVVLNQLCVL
B12-mHSA-F5B6H2 (671) STPTLVEVSRNLGKVGSKCCKHPEAKRMPCAEDYLSVVLNQLCVL
F4-mHSA-B1D2 (671) STPTLVEVSRNLGKVGSKCCKHPEAKRMPCAEDYLSVVLNQLCVL
F4-mHSA-F5B6H2 (671) STPTLVEVSRNLGKVGSKCCKHPEAKRMPCAEDYLSVVLNQLCVL
H3-mHSA-B1D2 (670) STPTLVEVSRNLGKVGSKCCKHPEAKRMPCAEDYLSVVLNQLCVL
H3-mHSA-F5B6H2 (670) STPTLVEVSRNLGKVGSKCCKHPEAKRMPCAEDYLSVVLNQLCVL

721 765
A5-mHSA-ML3.9 (712) HEKTPVSDRVTKCCTESLVNRRPCFSALEVDETYVPKEFQAETFT
A5-mHSA-B1D2 (712) HEKTPVSDRVTKCCTESLVNRRPCFSALEVDETYVPKEFQAETFT
A5-mHSA-F5B6H2 (712) HEKTPVSDRVTKCCTESLVNRRPCFSALEVDETYVPKEFQAETFT
B12-mHSA-B1D2 (716) HEKTPVSDRVTKCCTESLVNRRPCFSALEVDETYVPKEFQAETFT
B12-mHSA-F5B6H2 (716) HEKTPVSDRVTKCCTESLVNRRPCFSALEVDETYVPKEFQAETFT
F4-mHSA-B1D2 (716) HEKTPVSDRVTKCCTESLVNRRPCFSALEVDETYVPKEFQAETFT
F4-mHSA-F5B6H2 (716) HEKTPVSDRVTKCCTESLVNRRPCFSALEVDETYVPKEFQAETFT
H3-mHSA-B1D2 (715) HEKTPVSDRVTKCCTESLVNRRPCFSALEVDETYVPKEFQAETFT
H3-mHSA-F5B6H2 (715) HEKTPVSDRVTKCCTESLVNRRPCFSALEVDETYVPKEFQAETFT

766 810
A5-mHSA-ML3.9 (757) FHADICTLSEKERQIKKQTALVELVKHKPKATKEQLKAVMDDFAA
A5-mHSA-B1D2 (757) FHADICTLSEKERQIKKQTALVELVKHKPKATKEQLKAVMDDFAA
A5-mHSA-F5B6H2 (757) FHADICTLSEKERQIKKQTALVELVKHKPKATKEQLKAVMDDFAA
B12-mHSA-B1D2 (761) FHADICTLSEKERQIKKQTALVELVKHKPKATKEQLKAVMDDFAA
B12-mHSA-F5B6H2 (761) FHADICTLSEKERQIKKQTALVELVKHKPKATKEQLKAVMDDFAA
F4-mHSA-B1D2 (761) FHADICTLSEKERQIKKQTALVELVKHKPKATKEQLKAVMDDFAA
F4-mHSA-F5B6H2 (761) FHADICTLSEKERQIKKQTALVELVKHKPKATKEQLKAVMDDFAA
H3-mHSA-B1D2 (760) FHADICTLSEKERQIKKQTALVELVKHKPKATKEQLKAVMDDFAA
H3-mHSA-F5B6H2 (760) FHADICTLSEKERQIKKQTALVELVKHKPKATKEQLKAVMDDFAA

811 855
A5-mHSA-ML3.9 (802) FVEKCCKADDKETCFAEEGKKLVAASQAALGLAAALQVQLVQSGA
A5-mHSA-B1D2 (802) FVEKCCKADDKETCFAEEGKKLVAASQAALGLAAALQVQLVQSGA
A5-mHSA-F5B6H2 (802) FVEKCCKADDKETCFAEEGKKLVAASQAALGLAAALQVQLVESGG
B12-mHSA-B1D2 (806) FVEKCCKADDKETCFAEEGKKLVAASQAALGLAAALQVQLVQSGA
B12-mHSA-F5B6H2 (806) FVEKCCKADDKETCFAEEGKKLVAASQAALGLAAALQVQLVESGG
F4-mHSA-B1D2 (806) FVEKCCKADDKETCFAEEGKKLVAASQAALGLAAALQVQLVQSGA
F4-mHSA-F5B6H2 (806) FVEKCCKADDKETCFAEEGKKLVAASQAALGLAAALQVQLVESGG
H3-mHSA-B1D2 (805) FVEKCCKADDKETCFAEEGKKLVAASQAALGLAAALQVQLVQSGA
H3-mHSA-F5B6H2 (805) FVEKCCKADDKETCFAEEGKKLVAASQAALGLAAALQVQLVESGG

856 900
A5-mHSA-ML3.9 (847) EVKKPGESLKISCKGSGYSFTSYWIAWVRQMPGKGLEYMGLIYPG
A5-mHSA-B1D2 (847) EVKKPGESLKISCKGSGYSFTSYWIAWVRQMPGKGLEYMGLIYPG
A5-mHSA-F5B6H2 (847) GLVQPGGSLRLSCAASGFTFRSYAMSWVRQAPGKGLEWVSAISGR
B12-mHSA-B1D2 (851) EVKKPGESLKISCKGSGYSFTSYWIAWVRQMPGKGLEYMGLIYPG
B12-mHSA-F5B6H2 (851) GLVQPGGSLRLSCAASGFTFRSYAMSWVRQAPGKGLEWVSAISGR
F4-mHSA-B1D2 (851) EVKKPGESLKISCKGSGYSFTSYWIAWVRQMPGKGLEYMGLIYPG
F4-mHSA-F5B6H2 (851) GLVQPGGSLRLSCAASGFTFRSYAMSWVRQAPGKGLEWVSAISGR
H3-mHSA-B1D2 (850) EVKKPGESLKISCKGSGYSFTSYWIAWVRQMPGKGLEYMGLIYPG
H3-mHSA-F5B6H2 (850) GLVQPGGSLRLSCAASGFTFRSYAMSWVRQAPGKGLEWVSAISGR

901 945
A5-mHSA-ML3.9 (892) DSDTKYSPSFQGQVTISVDKSVSTAYLQWSSLKPSDSAVYFCARH
A5-mHSA-B1D2 (892) DSDTKYSPSFQGQVTISVDKSVSTAYLQWSSLKPSDSAVYFCARH
A5-mHSA-F5B6H2 (892) GDNTYYADSVKGRFTISRDNSKNTLYLQMNSLRAEDTAVYYCAKM
B12-mHSA-B1D2 (896) DSDTKYSPSFQGQVTISVDKSVSTAYLQWSSLKPSDSAVYFCARH
B12-mHSA-F5B6H2 (896) GDNTYYADSVKGRFTISRDNSKNTLYLQMNSLRAEDTAVYYCAKM

F4-mHSA-B1D2 (896) DSDTKYSPSFQGQVTISVDKSVSTAYLQWSSLKPSDSAVYFCARH
F4-mHSA-F5B6H2 (896) GDNTYYADSVKGRFTISRDNSKNTLYLQMNSLRAEDTAVYYCAKM
H3-mHSA-B1D2 (895) DSDTKYSPSFQGQVTISVDKSVSTAYLQWSSLKPSDSAVYFCARH
H3-mHSA-F5B6H2 (895) GDNTYYADSVKGRFTISRDNSKNTLYLQMNSLRAEDTAVYYCAKM

946 990
A5-mHSA-ML3.9 (937) DVGYCSSSNCAKWPEYFQHWGQGTLVTVSSGGGGSSGGGSGGGGS
A5-mHSA-B1D2 (937) DVGYCTDRTCAKWPEWLGVWGQGTLVTVSSGGGGSSGGGSGGGGS
A5-mHSA-F5B6H2 (937) TSNAVG-----------FDYWGQGTLVTVSSGGGGSGGGSGGGGSG
B12-mHSA-B1D2 (941) DVGYCTDRTCAKWPEWLGVWGQGTLVTVSSGGGGSSGGGSGGGGS
B12-mHSA-F5B6H2 (941) TSNAVG-----------FDYWGQGTLVTVSSGGGGSGGGSGGGGSG
F4-mHSA-B1D2 (941) DVGYCTDRTCAKWPEWLGVWGQGTLVTVSSGGGGSSGGGSGGGGS
F4-mHSA-F5B6H2 (941) TS-----------NAVGFDYWGQGTLVTVSSGGGGSGGGSGGGGSG
H3-mHSA-B1D2 (940) DVGYCTDRTCAKWPEWLGVWGQGTLVTVSSGGGGSSGGGSGGGGS
H3-mHSA-F5B6H2 (940) TSNAVG-----------FDYWGQGTLVTVSSGGGGSGGGSGGGGSG

991 1035
A5-mHSA-ML3.9 (982) QSVLTQPPSVSAAPGQKVTISCSGSSSNIGNNY-VSWYQQLPGTA
A5-mHSA-B1D2 (982) QSVLTQPPSVSAAPGQKVTISCSGSSSNIGNNY-VSWYQQLPGTA
A5-mHSA-F5B6H2 (972) QSVLTQPPSVSGAPGQRVTISCTGRHSNIGLGYGVHWYQQLPGTA
B12-mHSA-B1D2 (986) QSVLTQPPSVSAAPGQKVTISCSGSSSNIGNNY-VSWYQQLPGTA
B12-mHSA-F5B6H2 (976) QSVLTQPPSVSGAPGQRVTISCTGRHSNIGLGYGVHWYQQLPGTA
F4-mHSA-B1D2 (986) QSVLTQPPSVSAAPGQKVTISCSGSSSNIGNNY-VSWYQQLPGTA
F4-mHSA-F5B6H2 (976) QSVLTQPPSVSGAPGQRVTISCTGRHSNIGLGYGVHWYQQLPGTA
H3-mHSA-B1D2 (985) QSVLTQPPSVSAAPGQKVTISCSGSSSNIGNNY-VSWYQQLPGTA
H3-mHSA-F5B6H2 (975) QSVLTQPPSVSGAPGQRVTISCTGRHSNIGLGYGVHWYQQLPGTA

1036 1080
A5-mHSA-ML3.9 (1026) PKLLIYDHTNRPAGVPDRFSGSKSGTSASLAISGFRSEDEADYYC
A5-mHSA-B1D2 (1026) PKLLIYDHTNRPAGVPDRFSGSKSGTSASLAISGFRSEDEADYYC
A5-mHSA-F5B6H2 (1017) PKLLIYGNTNRPSGVPDRFSGFKSGTSASLAITGLQAEDEADYYC
B12-mHSA-B1D2 (1030) PKLLIYDHTNRPAGVPDRFSGSKSGTSASLAISGFRSEDEADYYC
B12-mHSA-F5B6H2 (1021) PKLLIYGNTNRPSGVPDRFSGFKSGTSASLAITGLQAEDEADYYC
F4-mHSA-B1D2 (1030) PKLLIYDHTNRPAGVPDRFSGSKSGTSASLAISGFRSEDEADYYC
F4-mHSA-F5B6H2 (1021) PKLLIYGNTNRPSGVPDRFSGFKSGTSASLAITGLQAEDEADYYC
H3-mHSA-B1D2 (1029) PKLLIYDHTNRPAGVPDRFSGSKSGTSASLAISGFRSEDEADYYC
H3-mHSA-F5B6H2 (1020) PKLLIYGNTNRPSGVPDRFSGFKSGTSASLAITGLQAEDEADYYC

1081 1104
A5-mHSA-ML3.9 (1071) ASWDYTLSGWVFGGGTKLTVLG--
A5-mHSA-B1D2 (1071) ASWDYTLSGWVFGGGTKLTVLG--
A5-mHSA-F5B6H2 (1062) QSYDRRTPGWVFGGGTKLTVLG--
B12-mHSA-B1D2 (1075) ASWDYTLSGWVFGGGTKLTVLG--

APPENDIX C
ANTICANCER AGENTS

Anticancer agents for combination with B2B3-1 | Brand Name(s) | Manufacturer/Proprietor
Anti-IGF1R Antibodies | |
AMG 479 (fully humanized mAb) | | Amgen
IMCA12 (fully humanized mAb) | | ImClone
NSC-742460 | | Dyax
19D12 (fully humanized mAb) | |
CP751-871 (fully humanized mAb) | | Pfizer
H7C10 (humanized mAb) | |
alphaIR3 (mouse) | |
scFV/FC (mouse/human chimera) | |
EM/164 (mousc) | |
MK-0646, F50035 | | Pierre Fabre Medicament, Merck
Small Molecules Targeting IGF1R | |
NVP-AEW541 | | Novartis
BMS-536,924 (1H-benzoimidazol-2-yl)-1H-pyridin-2-one) | | Bristol-Myers Squibb
BMS-554,417 | | Bristol-Myers Squibb
Cycloligan | |
TAE226 | |
PQ401 | |
Anti-EGFR Monoclonal Antibodies | |
INCB7839 | | Incyte
Bevacizumab | Avastin | Genentech
Cetuximab | Erbitux® | IMCLONE
mAb 806 | |
Matuzumab (EMD72000) | |
Nimotuzumab (TheraCIM) | |
Panitumumab | Vectibix® | Amgen
Anti-ErbB3 Therapeutics | |
U3-1287 / AMG888 | | U3 Pharma/Amgen
MM-121 | | Merrimack Pharmaceuticals
Anti-ErbB2 Therapeutics | |
Trastuzumab | Herceptin | Genentech
HKI-272 | Neratinib | Wyeth

KOS-953 | Tanespimycin | Kosan Biosciences
Her/ErbB Dimerization Inhibitors | |
2C4, R1273 | Pertuzumab, Omintarg | Genentech, Roche
Small Molecules Targeting EGFR | |
CI-1033 (PD 183805) | | Pzifer, Inc.
EKB-569 | |
Gefitinib | IRessa® | AstraZeneca
Lapatinib (GW572016) | | GlaxoSmithKline
Lapatinib Ditosylate | Tykerb® | SmithKline Beecham
Erlotinib HCl (OSI-774) | Tarceva® | OSI Pharms
PD158780 | |
PKI-166 | | Novartis
Tyrphostin AG 1478 (4-(3-Chloroanillino)-
6,7-dimethoxyquinazoline) | |
Anti-cmet Antibody Therapies | |
AVEO (AV299) | | AVEO
AMG102 | | Amgen
5D5 (OA-5D5) | | Genentech
Small Molecules Targeting cmet | |
PHA665752 | |
ARQ-650RP | | ArQule
ARQ 197 | | ArQule
Alkylating Agents | |
BCNU→ 1 ,3-bis t2-chloroethyl)-
nitrosourea | |
Bendamustine | |
Busulfan | Myleran | GlaxoSmithKline
Carboplatin | Paraplatin | Bristol-Myers Squibb
Carboquone | |
Carmustine | |
CCNU→ 1,-(2-chloroethyl)-3-cyclohexyl-
1-nitrosourea (methyl CCNU) | |
Chlorambucil | Leukeran® | Smithkline Beecham
Chlormethine | |
Cisplatin (Cisplatinum, CDDP) | Platinol | Bristol-Myers
Cyclophosphamide | Cytoxan
Neosar | Bristol-Myers Squibb
Teva Parenteral
Dacarbazine (DTIC) | |

Fotemustine | |
Hexamethylmelamine (Altretamine, HMM) | Hexalen® | MGI Pharma, Inc.
Ifosfamide | Mitoxana® | ASTA Medica
Lomustine | |
Mannosulfan | |
Melphalan | Alkeran® | GlaxoSmithKline
Nedaplatin | |
Nimustine | |
Oxaliplatin | Eloxatin® | Sanofi-Aventis US
Prednimustine, | |
Procarbazine HCL | Matulane | Sigma-Tau Pharmaceuticals, Inc.
Ribonucleotide Reductase Inhibitor (RNR) | |
Ranimustine | |
Satraplatin | |
Semustine | |
Streptozocin | |
Temozolomide | |
Treosulfan | |
Triaziquone | |
Triethylene Melamine | |
ThioTEPA | | Bedford, Abraxis, Teva
Triplatin tetranitrate | |
Trofosfamide | |
Uramustine | |
Antimetabolites | |
5-azacytidine | |
Flourouracil (5-FU)/Capecitabine | |
6-mercaptopurine
(Mercaptopurine, 6-MP) | |
6-Thioguanine (6-TG) | Purinethol® | Teva
Cytosine Arabinoside (Cytarabine,
Ara-C) | Thioguanine® | GlaxoSmithKline
Azathioprine | Azasan® | AAIPHARMA LLC
Capecitabine | XELODA® | HLR (Roche)
Cladribine (2-CdA, 2-
chlorodeoxyadenosine) | Leustatin® | Ortho Biotech
5-Trifluoromethyl-2'-deoxyuridine | |
Fludarabine phosphate | Fludara® | Bayer Health Care
Floxuridine (5-fluoro-2) | FUDR® | Hospira, Inc.
Methotrexate sodium | Trexall | Barr

Pemetrexed | Alimta® | Lilly
Pentostatin | Nipent® | Hospira, Inc.
Raltitrexed | Tomudex® | AstraZeneca
Tegafur | |
| |
Aromatose Inhibitor | |
Ketoconazole | |
| |
Glucocorticoids | |
Dexamethasone | Decadron® Dexasone, Diodex, Hexadrol, Maxidex | Wyeth, Inc.
Prednisolone | |
Prednisone | Deltasone, Orasone, Liquid Pred, Sterapred® |
| |
Immunotherapeutics | |
Alpha interferon | |
Angiogenesis Inhibitor | Avastin® | Genentech
IL-12→ Interleukin 12 | |
IL-2→ Interleukin 2 (Aldesleukin) | Proleukin ® | Chiron
| |
Kinase Inhibitors | |
AMG 386 | | Amgen
Axitinib ((AG-013736) | | Pfizer, Inc
Bosutinib (SKI-606) | | Wyeth
Brivanib alalinate (BMS-582664) | | BMS
Cediranib ( AZD2171) | Recentin | AstraVeneca
Dasatinib (BMS-354825) | Sprycel® | Bristol-Myers Squibb
Imatinib mesylate | Gleevec | Novartis
Lestaurtinib (CEP-701) | | Cephalon
Motesanib diphosphate (AMG-706) | | Amgen/Takeda
Nilotinib hydrochloride monohydrate | Tasigna® | Novartis
Pazopanib HCL (GW786034) | Armala | GSK
Semaxanib (SU5416) | | Pharmacia,
Sorafenib tosylate | Nexavar® | Bayer
Sunitinib malate | Sutent® | Pfizer, Inc.
Vandetanib (AZD647) | Zactima | AstraZeneca
Vatalanib; PTK-787 | | Novartis; Bayer Schering Pharma
XL184, NSC718781 | | Exelixis, GSK
| |
Microtubule-Targeting Agents | |
Docetaxel | Taxotere® | Sanofi-Aventis US
Ixabepilone | IXEMPRA™ | Bristol-Myers Squibb

Larotaxel | |
Ortataxel | |
Nanoparticle paclitaxel (ABI-007) | Abraxane® | Abraxis BioScience, Inc.
Paclitaxel | Taxol® | Bristol-Myers Squibb
Tesetaxel | |
Vinblastine sulfate | Velban® | Lilly
Vincristine | Oncovin® | Lilly
Vindesine sulphate | Eldisine® | Lilly
Vinflunine | |
Vinorelbine tartrate | Navelbine® | Pierre Fabre
| |
mTOR Inhibitors | |
Deforolimus (AP23573, MK 8669) | | ARIAD Pharmaceuticals, Inc
Everolimus (RAD001, RAD001C) | Certican®, Afinitor | Novartis
Sirolimus (Rapamycin) | Rapamune® | Wyeth Pharama
Temsirolimus (CCI-779) | Torisel® | Wyeth Pharama
| |
Protein Synthesis Inhibitor | |
L-asparaginase | Elspar® | Merck & Co.
| |
Somatostatin Analogue | |
Octreotide acetate | Sandostatin® | Novartis
| |
Topoisomerase Inhibitors | |
Actinomycin D | |
Camptothecin (CPT) | |
Belotecan | |
Daunorubicin citrate | Daunoxome® | Gilead
Doxorubicin hydrochloride | Doxil® | Alza
Etoposide | Vepesid® | Bristol-Myers Squibb
| Etopophos | Hospira, Bedford, Teva Parenteral, Etc.
Irinotecan HCL (CPT-11) | Camptosar® | Pharmacia & Upjohn
Mitoxantrone HCL | Novantrone | EMD Serono
Rubitecan | |
Teniposide (VM-26) | Vumon® | Bristol-Myers Squibb
Topotecan HCL | Hycamtin® | GlaxoSmithKline
| |

Chemotherapeutic Agents

Adriamycin, 5-Fluorouracil, Cytoxin,
Bleomycin, Mitomycin C, Daunomycin,
Carminomycin, Aminopterin,
Dactinomycin, Mitomycins, Esperamicins
Clofarabine, Mercaptopurine, Pentostatin,
Thioguanine, Cytarabine, Decitabine,
Floxuridine, Gemcitabine (Gemzar),
Enocitabine, Sapacitabine

Hormonal Therapy | |
Abarelix | Plenaxis™ | Amgen
Abiraterone acetate | CB7630 | BTG plc
Afimoxifene | TamoGel | Ascend Therapeutics, Inc.
Anastrazole | Arimidex® | AstraZeneca
Aromatase inhibitor | Atamestane plus toremifene | Intarcia Therapeutics, Inc.
| Arzoxifene | Eli Lilly & Co.
Asentar; DN-101 | | Novartis; Oregon Health & Science Univ.
Bicalutamide | Casodex® | AstraZeneca
Buserelin | Suprefact® | Sanofi Aventis
Cetrorelix | Cetrotide® | EMD Serono
Exemestane | Aromasin® | Pfizer
Exemestane | Xtane | Natco Pharma, Ltd.
Fadrozole (CGS 16949A ) | |
Flutamide | Eulexin® | Schering
Flutamide | Prostacur | Laboratorios Almirall, S.A.
Fulvestrant | Faslodex® | AstraZeneca
Goserelin acetate | Zoladex® | AstraZeneca
Letrozole | Femara® | Novartis
Letrozole (CGS20267) | Femara | Chugai Pharmaceutical Co., Ltd.
Letrozole | Estrochek | Jagsonpal Pharmaceuticals, Ltd.
Letrozole | Letrozole | Indchemie Health Specialities
Leuprolide acetate | Eligard® | Sanofi Aventis
Leuprolide acetate | Leopril | VHB Life Sciences, Inc.
Leuprolide acetate | Lupron®/Lupron Depot | TAP Pharma
Leuprolide acetate | Viador | Bayer AG
Megestrol acetate | Megace® | Bristol-Myers Squibb
Magestrol acetate | Estradiol Valerate (Delestrogen) | Jagsonpal Pharmaceuticals, Ltd.
Medroxyprogesterone acetate | Veraplex | Combiphar

MT206 | | Medisyn Technologies, Inc.
Nafarelin | |
Nandrolone decanoate | Zestabolin | Mankind Pharma, Ltd.
Nilutamide | Nilandron® | Aventis Pharmaceuticals
Raloxifene HCL | Evista® | Lilly
Tamoxifen | Taxifen | Yung Shin Pharmaceutical
Tamoxifen | Tomifen | Alkem Laboratories, Ltd.
Tamoxifen citrate | Nolvadex | AstraZeneca
Tamoxifen citrate | Soltamox | EUSA Pharma, Inc.
Tamoxifen citrate | Tamoxifen citrate SOPHARMA | Sopharma JSCo.
Toremifene citrate | Fareston® | GTX, Inc.
Triptorelin pamoate | Trelstar® | Watson Labs
Triptorelin pamoate | Trelstar Depot | Paladin Labs, Inc.
| |
Protein Kinase B (PKB) Inhibitor | |
Akt Inhibitor ASTEX | | Astex Therapeutics
Akt Inhibitors NERVIANO | | Nerviano Medical Sciences
AKT Kinase Inhibitor TELIK | | Telik, Inc.
AKT DECIPHERA | | Deciphera Pharmaceuticals, LLC
Perifosine (KRX0401, D-21266) | | Keryx Biopharmaceuticals, Inc., AEterna Zentaris, Inc.
Perifosine with Docetaxel | | Keryx Biopharmaceuticals, Inc., AEterna Zentaris, Inc.
Perifosine with Gemcitabine | | AEterna Zentaris, Inc.
Perifosine with Paclitaxel | | Keryx Biopharmaceuticals, Inc, AEterna Zentaris, Inc.
Protein Kinase-B inhibitor DEVELOGEN | | DeveloGen AG
PX316 | | Oncothyreon, Inc.
RX0183 | | Rexahn Pharmaceuticals, Inc.
RX0201 | | Rexahn Pharmaceuticals, Inc.
VQD002 | | VioQuest Pharmaceuticals, Inc.
XL418 | | Exelixis, Inc.
ZEN027 | | AEterna Zentaris, Inc.
| |
Phosphatidylinositol 3-Kinase (PI3K) Inhibitor | |
BEZ235 | | Novartis AG
BGT226 | | Novartis AG
CAL101 | | Calistoga Pharmaceuticals, Inc.
CHR4432 | | Chroma Therapeutics, Ltd.
Erk/PI3K Inhibitors ETERNA | | AEterna Zentaris, Inc.

GDC0941 | | Genentech Inc./Piramed Limited/Roche Holdings, Ltd.
Enzastaurin HCL (LY317615) | Enzastaurin | Eli Lilly
LY294002/Wortmannin | |
PI3K Inhibitors SEMAFORE | | Semafore Pharmaceuticals
PX866 | | Oncothyreon, Inc.
SF1126 | | Semafore Pharmaceuticals
VMD-8000 | | VM Discovery, Inc.
XL147 | | Exelixis, Inc.
XL147 with XL647 | | Exelixis, Inc.
XL765 | | Exelixis, Inc.
PI-103 | | Roche/Piramed

Cyclin-dependent kinase inhibitors | |
CYC200, r-roscovitine | Seliciclib | Cyclacel Pharma
NSC-649890, L86-8275, HMR-1275 | Alvocidib | NCI

TLr9, CD289 | |
IMOxine | | Merck KGaA
HYB2055 | | Idera
IMO-2055 | | Isis Pharma
1018 ISS | | Dynavax Technologies/UCSF
PF-3512676 | | Pfizer

Enzyme Inhibitor | |
Lonafarnib (SCH66336) | Sarasar | SuperGen, U Arizona

Anti-TRAIL | |
AMG-655 | | Aeterna Zentaris, Keryx Biopharma
Apo2L/TRAIL, AMG951 | | Genentech, Amgen
Apomab (fully humanized mAb | | Genentech

Anticancer agents for combination with B2B3-1 | Target | Manufacturer/Proprietor
Other | |
Imprime PGG | | Biothera
CHR-2797 | AminopeptidaseM1 | Chroma Therapeutics
E7820, NSC 719239 | Integrin-alpha2 | Eisai
INCB007839 | ADAM 17, TACE | Incyte
CNF2024, BIIB021 | Hsp90 | Biogen Idec
MP470, HPK-56 | Kit/Met/Ret | Shering-Plough

SNDX-275/MS-275 | HDAC | Syndax
Zarnestra, Tipifarnib, R115777 | Ras | Janssen Pharma
Volociximab; Eos 200-4, M200 | alpha581 integrin | Biogen Idec; Eli Lilly/UCSF/PDL BioPharma
Apricoxib (TP2001) | COX-2 Inhibitor | Daiichi Sankyo; Tragara Pharma

Other Embodiments
While the invention has been described in connection with specific embodiments thereof, it
will be understood that it is capable of further modifications and this application is intended to cover
any variations, uses, or adaptations of the invention following, in general, the principles of the
invention and including such departures from the present disclosure that come within known or
customary practice within the art to which the invention pertains and may be applied to the essential
features hereinbefore set forth.
All publications and patent applications mentioned in this specification are herein incorporated
by reference to the same extent as if each independent publication or patent application was
specifically and individually indicated to be incorporated by reference in their entirety.

The invention further includes the subject matter of the claims of WO2009/126920 from which this application is derived, the content of which is reproduced below as numbered paragraphs.
Paragraph 1. An agent comprising:
   i) a human serum albumin (HSA) linker comprising an amino acid sequence set forth in any one of SEQ ID NOS:6-10; and
   ii) first and second binding moieties selected from the group consisting of antibodies, single-chain Fv molecules, bispecific single chain Fv ((scFv')2) molecules, domain antibodies, diabodies, triabodies, hormones, Fab fragments, F(ab')₂ molecules, tandem scFv (taFv) fragments, receptors, ligands, aptamers, and biologically-active fragments thereof, wherein said first binding moiety is bonded to the amino terminus of said HSA linker and said second binding moiety is bonded to the carboxy terminus of said HSA linker.
Paragraph 2. An agent comprising:
   i) a human serum albumin (HSA) linker comprising an amino acid sequence set forth in any one SEQ ID NOS:11-15; and
   ii) first and second binding moieties selected from the group consisting of antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, receptors, ligands, aptamers, and biologically-active fragments thereof, wherein said first binding moiety is bonded to the amino terminus and said second binding moiety is bonded to the carboxy terminus of said sequence.
Paragraph 3. The agent of paragraph 1 or 2, wherein said first binding moiety specifically binds ErbB3 and said second binding moiety specifically binds ErbB2.
Paragraph 4. The agent of paragraph 1 comprising the amino acid sequence set forth in SEQ ID NO:9.
Paragraph 5. The agent of paragraph 1 comprising the amino acid sequence set forth in SEQ ID NO:10.
Paragraph 6. The agent of paragraph 2 comprising the amino acid sequence set forth in SEQ ID NO:14.
Paragraph 7. The agent of paragraph 2 comprising the amino acid sequence set forth in SEQ ID NO:15.
Paragraph 8. A human serum albumin (HSA) linker comprising an amino acid sequence having at least 90% sequence identity to the sequence set forth in SEQ ID NO:1 and comprising a serine residue at position 34 of SEQ ID NO: 1 and a glutamine residue at position 503 of SEQ ID NO: 1.
Paragraph 9. The HSA linker of paragraph 8, comprising an amino acid sequence having at least 95% sequence identity to the sequence set forth in SEQ ID NO:1.
Paragraph 10. The HSA linker of paragraph 8, comprising the amino acid sequence set forth in SEQ ID NO:1.
Paragraph 11. An agent comprising the HSA linker of paragraph 8 and at least a first binding moiety.
Paragraph 12. The agent of paragraph 11 further comprising a first polypeptide connector that binds said first binding moiety to said HSA linker.
Paragraph 13. The agent of paragraph 12, wherein said connector binds said first binding moiety to the amino terminus of said HSA linker.
Paragraph 14. The agent of paragraph 12, wherein said connector binds said first binding moiety to the carboxy terminus of said HSA linker.
Paragraph 15. The agent of paragraph 12, wherein said connector covalently binds said first binding moiety to said HSA linker.
Paragraph 16. The agent of paragraph 11 further comprising a second binding moiety.
Paragraph 17. The agent of paragraph 16 further comprising a second polypeptide connector that binds said second binding moiety to said HSA linker.
Paragraph 18. The agent of paragraph 17, wherein said connector binds said second binding moiety to the amino terminus of said HSA linker.
Paragraph 19. The agent of paragraph 17, wherein said connector binds said second binding moiety to the carboxy terminus of said HSA linker.
Paragraph 20. The agent of paragraph 17, wherein said connector covalently binds said second binding moiety to said HSA linker.
Paragraph 21. The agent of paragraph 16 further comprising a first connector that covalently binds said first binding moiety to the amino terminus of said HSA linker and a second connector that covalently binds a second binding moiety to the carboxy terminus of said HSA linker.
Paragraph 22. The agent of paragraph 21, wherein said first connector comprises the amino acid sequence AAS or AAQ and said second connector comprises the amino acid sequence set forth in SEQ ID NO:5.
Paragraph 23. The agent of paragraph 16, wherein said first binding moiety or second binding moiety is selected from the group consisting of antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, cell surface receptors or ligands, aptamers, and biologically-active fragments thereof,.
Paragraph 24. The agent of paragraph 16, wherein said first or second binding moiety specifically binds a protein selected from the group consisting of insulin-like growth factor 1 receptor (IGF1R), IGF2R, insulin-like growth factor (IGF), mesenchymal epithelial transition factor receptor (c-met), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, α4 integrin, P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, receptors alpha and beta, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof.
Paragraph 25. The agent of paragraph 24, wherein said EGFR is erythroblastic leukemia viral oncogene homolog (ErbB) receptor.
Paragraph 26. The agent of paragraph 16, wherein said first or second binding moiety is alpha-fetoprotein (AFP) or an interferon, or a biologically-active fragment thereof.
Paragraph 27. The agent of paragraph 16, wherein said first or second binding moiety is selected from the group consisting of natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, and anakinra.
Paragraph 28. The agent of paragraph 16, wherein said first or second binding moiety is a single-chain Fv molecule.
Paragraph 29. The agent of paragraph 28, wherein said single-chain Fv molecule is human or humanized.
Paragraph 30. The agent of paragraph 11 further comprising a diagnostic or therapeutic agent.
Paragraph 31. The agent of paragraph 30, wherein said diagnostic agent is a detectable label.
Paragraph 32. The agent of paragraph 31, wherein said detectable label is a radioactive, fluorescent, or heavy metal label.
Paragraph 33. The agent of paragraph 30, wherein said therapeutic agent is a cytotoxic, cytostatic, or immunomodulatory agent.
Paragraph 34. The agent of paragraph 33, wherein said cytotoxic agent is an alkylating agent, an antibiotic, an antineoplastic agent, an antiproliferative agent, an antimetabolite, a tubulin inhibitor, a topoisomerase I or II inhibitor, a hormonal agonist or antagonist, an immunomodulator, a DNA minor groove binder, or a radioactive agent.
Paragraph 35. The agent of paragraph 34, wherein said antineoplastic agent is selected from the group consisting of cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and derivatives thereof.
Paragraph 36. The agent of paragraph 33, wherein said agent is capable of binding to and killing a tumor cell or inhibiting tumor cell proliferation.
Paragraph 37. The agent of paragraph 11 admixed with a pharmaceutically acceptable carrier, excipient, or diluent.
Paragraph 38. The agent of paragraph 11, wherein said agent exhibits an *in vivo* half-life of between 6 hours and 7 days.
Paragraph 39. The agent of paragraph 11, wherein said agent exhibits an *in vivo* half-life greater than 8 hours.
Paragraph 40. An agent comprising:
   i) a human serum albumin (HSA) linker comprising the amino acid sequence set forth in any one of SEQ ID NOS:11-15, or a fragment or variant thereof; and
   ii) at least a first binding moiety.
Paragraph 41. The agent of paragraph 40, wherein said first binding moiety is bonded to the amino terminus of said HSA linker.
Paragraph 42. The agent of paragraph 40, wherein said first binding moiety is bonded to the carboxy terminus of said HSA linker.
Paragraph 43. The agent of paragraph 40, wherein said first binding moiety is covalently bonded to said HSA linker.
Paragraph 44. The agent of paragraph 40 further comprising a second binding moiety.
Paragraph 45. The agent of paragraph 44, wherein said second binding moiety is bonded to the amino terminus of said HSA linker.
Paragraph 46. The agent of paragraph 44, wherein said second binding moiety is bonded to the carboxy terminus of said HSA linker.
Paragraph 47. The agent of paragraph 44, wherein said second binding moiety is covalently bonded to said HSA linker.
Paragraph 48. The agent of paragraph 44, wherein said first binding moiety or second binding moiety is selected from the group consisting of antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, cell surface receptors or ligands, aptamers, and biologically-active fragments thereof.
Paragraph 49. The agent of paragraph 44, wherein said first or second binding moiety specifically binds a protein selected from the group consisting of insulin-like growth factor 1 receptor (IGF1R), IGF2R, insulin-like growth factor (IGF), mesenchymal epithelial transition factor receptor (c-met), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, α4 integrin, P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, receptors alpha and beta, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof.
Paragraph 50. The agent of paragraph 49, wherein said EGFR is erythroblastic leukemia viral oncogene homolog (ErbB) receptor.
Paragraph 51. The agent of paragraph 44, wherein said first or second binding moiety is alpha-fetoprotein (AFP) or an interferon, or a biologically-active fragment thereof.
Paragraph 52. The agent of paragraph 44, wherein said first or second binding moiety is selected from the group consisting of natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, and anakinra.
Paragraph 53. The agent of paragraph 44, wherein said first or second binding moiety is a single-chain Fv molecule.
Paragraph 54. The agent of paragraph 53, wherein said single-chain Fv molecule is human or humanized.
Paragraph 55. The agent of paragraph 40 further comprising a diagnostic or therapeutic agent.
Paragraph 56. The agent of paragraph 55, wherein said diagnostic agent is a detectable label.
Paragraph 57. The agent of paragraph 56, wherein said detectable label is a radioactive, fluorescent, or heavy metal label.
Paragraph 58. The agent of paragraph 55, wherein said therapeutic agent is a cytotoxic, cytostatic, or immunomodulatory agent.
Paragraph 59. The agent of paragraph 58, wherein said cytotoxic agent is an alkylating agent, an antibiotic, an antineoplastic agent, an antiproliferative agent, an antimetabolite, a tubulin inhibitor, a topoisomerase I or II inhibitor, a hormonal agonist or antagonist, an immunomodulator, a DNA minor groove binder, or a radioactive agent.
Paragraph 60. The agent of paragraph 59, wherein said antineoplastic agent is selected from the group consisting of cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and derivatives thereof.
Paragraph 61. The agent of paragraph 58, wherein said agent is capable of binding to and killing a tumor cell or inhibiting tumor cell proliferation.
Paragraph 62. The agent of paragraph 40 admixed with a pharmaceutically acceptable carrier, excipient, or diluent.
Paragraph 63. The agent of paragraph 40, wherein said agent exhibits an *in vivo* half-life of between 6 hours and 7 days.
Paragraph 64. The agent of paragraph 40, wherein said agent exhibits an *in vivo* half-life greater than 8 hours.
Paragraph 65. A method for treating a mammal having a disease or disorder, wherein said method comprises administering to said mammal the agent of paragraph 11 or 40.
Paragraph 66. The method of paragraph 65, wherein said disease or disorder is associated with cellular signaling through a cell surface receptor.
Paragraph 67. The method of paragraph 65, wherein said mammal is a human.
Paragraph 68. The method of paragraph 65, wherein said disease or disorder is a proliferative or autoimmune disease.
Paragraph 69. The method of paragraph 68, wherein said proliferative disease is melanoma, clear cell sarcoma, head and neck cancer, bladder cancer, breast cancer, colon cancer, ovarian cancer, endometrial cancer, gastric cancer, pancreatic cancer, renal cancer, prostate cancer, salivary gland cancer, lung cancer, liver cancer, skin cancer, or brain cancer.
Paragraph 70. The method of paragraph 68, wherein said autoimmune disease is multiple sclerosis, psoriasis, myasthenia gravis, uveitis, systemic lupus erythematosus, or rheumatoid arthritis.
Paragraph 71. A method for making a human serum albumin (HSA) agent comprising bonding a first binding moiety to the amino terminus and a second binding moiety to the carboxy terminus of the amino acid sequence set forth in any one of SEQ ID NOS:1, 3, or 6-15.
Paragraph 72. The method of paragraph 71, wherein said joining comprises forming a covalent bond between said first and said second binding moiety and said amino and carboxy termini, respectively.
Paragraph 73. The method of paragraph 71, wherein said first binding moiety or second binding moiety is selected from the group consisting of antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, cell surface receptors or ligands, aptamers, and biologically-active fragments thereof,.
Paragraph 74. The method of paragraph 71, wherein said first or second binding moiety specifically binds a protein selected from the group consisting of insulin-like growth factor 1 receptor (IGF1R), IGF2R, insulin-like growth factor (IGF), mesenchymal epithelial transition factor receptor (c-met), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, α4 integrin, P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, receptors alpha and beta, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof.
Paragraph 75. The method of paragraph 74, wherein said EGFR is erythroblastic leukemia viral oncogene homolog (ErbB) receptor.
Paragraph 76. The method of paragraph 71, wherein said first or second binding moiety is alpha-fetoprotein (AFP) or an interferon, or a biologically-active fragment thereof.
Paragraph 77. The method of paragraph 71, wherein said first or second binding moiety is selected from the group consisting of natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, and anakinra.
Paragraph 78. The method of paragraph 71, wherein said first or second binding moiety is a single-chain Fv molecule.
Paragraph 79. The method of paragraph 71, wherein said single-chain Fv molecule is human or humanized.
Paragraph 80. The method of paragraph 71, wherein said method further comprises conjugating one or more detectable labels to said agent.
Paragraph 81. The method of paragraph 80, wherein said detectable label is a radioactive, fluorescent, or heavy metal label.
Paragraph 82. The method of paragraph 71, wherein said method further comprises conjugating one ore more cytotoxic, cytostatic, or immunomodulatory agents to said agent.
Paragraph 83. The method of paragraph 82, wherein said cytotoxic agent is an alkylating agent, an antibiotic, an antineoplastic agent, an antiproliferative agent, an antimetabolite, a tubulin inhibitor, a topoisomerase I or II inhibitor, a hormonal agonist or antagonist, an immunomodulator, or a radioactive agent.
Paragraph 84. The method of paragraph 83, wherein said antineoplastic agent is selected from the group consisting of cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and derivatives thereof.
Paragraph 85. The method of any one of paragraphs 71-84, wherein said method further comprises admixing said agent with a pharmaceutically acceptable carrier.
Paragraph 86. A method for making a human serum albumin (HSA) agent comprising substituting one or more surface-exposed amino acid residues in the amino acid sequences set forth in any one of SEQ ID NOS:1, 3, and 6-15 with a substitute amino acid capable of chemical modification, wherein said modification allows conjugation of a diagnostic or therapeutic agent.
Paragraph 87. The method of paragraph 86, wherein said substitute amino acid is cysteine and wherein said surface exposed amino acid residues are serine or threonine.
Paragraph 88. The method of paragraph 86, wherein said chemical modification results in a covalent bond between said substitute amino acid and said diagnostic or therapeutic agent.
Paragraph 89. The method of paragraph 86, wherein said surface-exposed amino acid residues are selected from the group consisting of threonine at position 496, serine at position 58, threonine at position 76, threonine at position 79, threonine at position 83, threonine at position 125, threonine at position 236, serine at position 270, serine at position 273, serine at position 304, serine at position 435, threonine at position 478, threonine at position 506, and threonine at position 508.
Paragraph 90. A method for making a human serum albumin (HSA) agent comprising substituting one or more of the amino acid residues in the amino acid sequences set forth in any one of SEQ ID NOS:1, 3, and 6-15 with an asparagine, serine, or threonine, wherein said substituting incorporates a glycosylation site on said HSA agent.
Paragraph 91. A method for making a human serum albumin (HSA) agent comprising substituting one or more of the asparagine, serine, or threonine residues in the amino acid sequences set forth in any one of SEQ ID NOS:1, 3, and 6-15 with any amino acid other than asparagine, serine, or threonine, wherein said substituting removes a glycosylation site from said HSA agent.
Paragraph 92. A kit comprising the HSA linker of paragraphs 8-10.
Paragraph 93. A kit comprising the agent of paragraphs 1, 2, 11, or 40.
Paragraph 94. An agent comprising an amino acid sequence having at least 90% sequence identity to any one of the amino acid sequences set forth in SEQ ID NOS:16-25.
Paragraph 95. The agent of paragraph 94, comprising an amino acid sequence having at least 95% sequence identity to any one of the amino acid sequences set forth in SEQ ID NOS:16-25.
Paragraph 96. The agent of paragraph 94, comprising any one of the amino acid sequence set forth in SEQ ID NOS:16-25.
Paragraph 97. The agent of paragraph 94, further comprising a detectable label or therapeutic agent.
Paragraph 98. The agent of paragraph 97, wherein said detectable label is a radioactive, fluorescent, bioluminescent, or heavy metal molecule, or epitope tag.
Paragraph 99. The agent of paragraph 98, wherein said fluorescent molecule is green fluorescent protein (GFP), enhanced GFP (eGFP), yellow fluorescent protein (YFP), cyan fluorescent protein (CFP), red fluorescent protein (RFP), or dsRed.
Paragraph 100. The agent of paragraph 98, wherein said bioluminescent molecule is luciferase.
Paragraph 101. The agent of paragraph 98, wherein said epitope tag is c-myc, hemagglutinin, or a histidine tag.
Paragraph 102. The agent of paragraph 97, wherein said therapeutic agent is a cytotoxic polypeptide selected from the group consisting of cytochrome c, caspase 1-10, granzyme A or B, tumor necrosis factor-alpha (TNF-α), TNF-β, Fas, Fas ligand, Fas-associated death doman-like IL-1β converting enzyme (FLICE), TRAIL/APO2L, TWEAK/APO3L, Bax, Bid, Bik, Bad, Bak, RICK, vascular apoptosis inducing proteins 1 and 2 (VAP1 and VAP2), pierisin, apoptosis-inducing protein (AIP), IL-1α propiece polypeptide, apoptin, apoptin-associated protein 1 (AAP-1), endostatin, angiostatin, and biologically-active fragments thereof.
Paragraph 103. The agent of paragraph 94, in combination with one or more therapeutic agents selected from the group consisting of cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and derivatives thereof.
Paragraph 104. The agent of paragraph 1, 2, 16, or 44, wherein said first and second binding moieties specifically bind the same target molecule.
Paragraph 105. The agent of paragraph 1, 2, 16, or 44, wherein said first and second binding moieties specifically bind different target molecules.
Paragraph 106. The agent of paragraph 1, 2, 16, or 44, wherein said first and second binding moieties specifically bind different epitopes on the same target molecule.
Paragraph 107. The method of paragraph 65, wherein said first and second binding moieties specifically bind the same target molecule.
Paragraph 108. The method of paragraph 65, wherein said first and second binding moieties specifically bind different target molecules.
Paragraph 109. The method of paragraph 65, wherein said first and second binding moieties specifically bind different epitopes on the same target molecule.
Paragraph 110. The method of paragraph 71, wherein said first and second binding moieties specifically bind the same target molecule.
Paragraph 111. The method of paragraph 71, wherein said first and second binding moieties specifically bind different target molecules.
Paragraph 112. The method of paragraph 71, wherein said first and second binding moieties specifically bind different epitopes on the same target molecule.
Paragraph 113. A polypeptide linker comprising amino acid residues 25-44 and amino acid residues 494-513 of SEQ ID NO: 1.
Paragraph 114. The polypeptide linker of paragraph 113, comprising amino acid residues 25-70 and amino acid residues 450-513 of SEQ ID NO: 1.
Paragraph 115. The polypeptide linker of paragraph 114, comprising amino acid residues 15-100 and amino acid residues 400-520 of SEQ ID NO: 1.
Paragraph 116. The polypeptide linker of paragraph 115, comprising amino acid residues 10-200 and amino acid residues 300-575 of SEQ ID NO: 1.
Paragraph 117. The polypeptide linker of paragraph 116, comprising amino acid residues 5-250 and 275-580 of SEQ ID NO: 1.
Paragraph 118. The polypeptide linker of paragraph 113, further comprising at least a first binding moiety.
Paragraph 119. The polypeptide linker of paragraph 118 further comprising a first polypeptide connector that binds said first binding moiety to said polypeptide linker.
Paragraph 120. The polypeptide linker of paragraph 119, wherein said connector binds said first binding moiety to the amino-terminus of said polypeptide linker.
Paragraph 121. The polypeptide linker of paragraph 119, wherein said connector binds said first binding moiety to the carboxy terminus of said polypeptide linker.
Paragraph 122. The polypeptide linker of paragraph 119, wherein said connector covalently binds said first binding moiety to said polypeptide linker.
Paragraph 123. The polypeptide linker of paragraph 118 further comprising a second binding moiety.
Paragraph 124. The polypeptide linker of paragraph 123 further comprising a second polypeptide connector that binds said second binding moiety to said polypeptide linker.
Paragraph 125. The polypeptide linker of paragraph 124, wherein said connector binds said second binding moiety to the amino terminus of said polypeptide linker.
Paragraph 126. The polypeptide linker of paragraph 124, wherein said connector binds said second binding moiety to the carboxy terminus of said polypeptide linker.
Paragraph 127. The polypeptide linker of paragraph 124, wherein said connector covalently binds said second binding moiety to said polypeptide linker.
Paragraph 128. The polypeptide linker of paragraph 123 further comprising a first connector that covalently binds said first binding moiety to the amino terminus of said polypeptide linker and a second connector that covalently binds a second binding moiety to the carboxy terminus of said polypeptide linker.
Paragraph 129. The polypeptide linker of paragraph 128, wherein said first connector comprises the amino acid sequence AAS or AAQ and said second connector comprises the amino acid sequence set forth in SEQ ID NO:5.
Paragraph 130. The polypeptide linker of paragraph 123, wherein said first binding moiety or second binding moiety is selected from the group consisting of antibodies, single-chain Fv molecules, bispecific single chain Fv ((scFv')₂) molecules, domain antibodies, diabodies, triabodies, hormones, Fab fragments, F(ab')₂ molecules, tandem scFv (taFv) fragments, cell surface receptors or ligands, aptamers, and biologically-active fragments thereof,.
Paragraph 131. The polypeptide linker of paragraph 123, wherein said first or second binding moiety specifically binds a protein selected from the group consisting of insulin-like growth factor 1 receptor (IGF1R), IGF2R, insulin-like growth factor (IGF), mesenchymal epithelial transition factor receptor (c-met), hepatocyte growth factor (HGF), epidermal growth factor receptor (EGFR), epidermal growth factor (EGF), heregulin, fibroblast growth factor receptor (FGFR), platelet-derived growth factor receptor (PDGFR), platelet-derived growth factor (PDGF), vascular endothelial growth factor receptor (VEGFR), vascular endothelial growth factor (VEGF), tumor necrosis factor receptor (TNFR), tumor necrosis factor alpha (TNF-α), TNF-β, folate receptor (FOLR), folate, transferrin receptor (TfR), mesothelin, Fc receptor, c-kit receptor, c-kit, α4 integrin, P-selectin, sphingosine-1-phosphate receptor-1 (S1PR), hyaluronate receptor, leukocyte function antigen-1 (LFA-1), CD4, CD11, CD18, CD20, CD25, CD27, CD52, CD70, CD80, CD85, CD95 (Fas receptor), CD106 (vascular cell adhesion molecule 1 (VCAM1), CD166 (activated leukocyte cell adhesion molecule (ALCAM)), CD178 (Fas ligand), CD253 (TNF-related apoptosis-inducing ligand (TRAIL)), ICOS ligand, CCR2, CXCR3, CCR5, CXCL12 (stromal cell-derived factor 1 (SDF-1)), interleukin 1 (IL-1), CTLA-4, receptors alpha and beta, MART-1, gp100, MAGE-1, ephrin (Eph) receptor, mucosal addressin cell adhesion molecule 1 (MAdCAM-1), carcinoembryonic antigen (CEA), Lewis^{Y}, MUC-1, epithelial cell adhesion molecule (EpCAM), cancer antigen 125 (CA125), prostate specific membrane antigen (PSMA), TAG-72 antigen, and fragments thereof.
Paragraph 132. The polypeptide linker of paragraph 131, wherein said EGFR is erythroblastic leukemia viral oncogene homolog (ErbB) receptor.
Paragraph 133. The polypeptide linker of paragraph 123, wherein said first or second binding moiety is alpha-fetoprotein (AFP) or an interferon, or a biologically-active fragment thereof.
Paragraph 134. The polypeptide linker of paragraph 123, wherein said first or second binding moiety is selected from the group consisting of natalizumab, infliximab, adalimumab, rituximab, alemtuzumab, bevacizumab, daclizumab, efalizumab, golimumab, certolizumab, trastuzumab, abatacept, etanercept, pertuzumab, cetuximab, panitumumab, and anakinra.
Paragraph 135. The polypeptide linker of paragraph 123, wherein said first or second binding moiety is a single-chain Fv molecule.
Paragraph 136. The polypeptide linker of paragraph 135, wherein said single-chain Fv molecule is human or humanized.
Paragraph 137. The polypeptide linker of paragraph 118 further comprising a diagnostic or therapeutic agent.
Paragraph 138. The polypeptide linker of paragraph 137, wherein said diagnostic agent is a detectable label.
Paragraph 139. The polypeptide linker of paragraph 138, wherein said detectable label is a radioactive, fluorescent, or heavy metal label.
Paragraph 140. The polypeptide linker of paragraph 137, wherein said therapeutic agent is a cytotoxic, cytostatic, or immunomodulatory agent.
Paragraph 141. The polypeptide linker of paragraph 140, wherein said cytotoxic agent is an alkylating agent, an antibiotic, an antineoplastic agent, an antiproliferative agent, an antimetabolite, a tubulin inhibitor, a topoisomerase I or II inhibitor, a hormonal agonist or antagonist, an immunomodulator, a DNA minor groove binder, or a radioactive agent.
Paragraph 142. The polypeptide linker of paragraph 141, wherein said antineoplastic agent is selected from the group consisting of cyclophosphamide, camptothecin, homocamptothecin, colchicine, combrestatin, combrestatin, rhizoxin, dolistatin, ansamitocin p3, maytansinoid, auristatin, caleachimicin, methotrexate, 5-fluorouracil (5-FU), doxorubicin, paclitaxel, docetaxel, cisplatin, carboplatin, tamoxifen, raloxifene, letrozole, epirubicin, bevacizumab, pertuzumab, trastuzumab, and derivatives thereof.
Paragraph 143. The polypeptide linker of paragraph 140, wherein said agent is capable of binding to and killing a tumor cell or inhibiting tumor cell proliferation.
Paragraph 144. The polypeptide linker of paragraph 118 admixed with a pharmaceutically acceptable carrier, excipient, or diluent.
Paragraph 145. The polypeptide linker of paragraph 118, wherein said agent exhibits an *in vivo* half-life of between 6 hours and 7 days.
Paragraph 146. The polypeptide linker of paragraph 118, wherein said agent exhibits an *in vivo* half-life greater than 8 hours.

## Claims

1. An agent for use in treating a breast cancer by administration prior to, concurrent with, or following radiotherapy or surgical intervention, said agent comprising a human serum albumin (HSA) linker comprising an amino acid sequence having at least 95% sequence identity to the sequence set forth in SEQ ID NO: 1 and comprising a first binding moiety bonded to the amino terminus of said HSA linker and a second binding moiety bonded to the carboxy terminus of said HSA linker, wherein each of said first and second binding moieties specifically binds ErbB2 or ErbB3 and wherein:
i) if said first binding moiety specifically binds ErbB3, said second binding moiety specifically binds ErbB2;
ii) if said first binding moiety specifically binds ErbB2, said second binding moiety specifically binds ErbB3; and
iii) wherein the agent is optionally formulated for parenteral administration.

2. The agent for use according to claim 1, wherein said amino acid sequence comprises the amino acid sequence of SEQ ID NO: 1.

3. The agent for use according to claim 1 or claim 2, wherein said first binding moiety has at least 97% sequence identity to the amino acid sequence set forth in SEQ ID NO: 26 and said second binding moiety has at least 97% sequence identity to the amino acid sequence set forth in SEQ ID NO: 27.

4. The agent for use according to claim 1, wherein the agent is sterile filtered.

5. The agent for use according to claim 6, wherein the agent is stored in a sterile aqueous solution.

6. The agent for use according to claim 6, wherein the agent is lyophilized.

7. A kit comprising the agent of any one of claims 1 to 6, wherein the kit includes multiple packages of a single- or multi-dose pharmaceutical composition containing an effective amount of the agent, and instructions for administering said agent to a patient, wherein preferably said patient has a proliferative disease.

8. The kit according to claim 7, wherein the agent is sterile filtered.

9. The kit according to claim 7 or 8, wherein the agent is stored in a sterile aqueous solution.

10. The kit according to claim 7, wherein the agent is lyophilized.


SEQUENCE LISTING

<110>  Merrimack Pharmaceuticals, Inc. et al.

<120>  Human Serum Albumin Linkers and Conjugates thereof

<130>  06727/028WO3

<150>  61/123,942
<151>  2008-04-11

<150>  61/115,797
<151>  2008-11-18

<160>  58

<170>  PatentIn version 3.5

<210>  1
<211>  585
<212>  PRT
<213>  Artificial Sequence

<220>
<223>  Synthetic construct

<400>  1

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Ser Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Gln Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu
            580                 585

<210>  2
<211>  1755
<212>  DNA
<213>  Artificial Sequence

<220>
<223>  Synthetic construct

<400>  2
gacgctcaca agagcgaagt ggcacatagg ttcaaagatc tgggcgaaga gaactttaag      60

gccctcgtcc tgatcgcttt cgcacagtac ctccagcagt ctccctttga agatcacgtg     120

aaactggtca atgaggtgac cgaatttgcc aagacatgcg tggctgatga gagtgcagaa 180

aactgtgaca aatcactgca tactctcttt ggagataagc tgtgcaccgt cgccacactc 240

agagagactt atggggaaat ggctgactgt tgcgcaaaac aggagcctga acggaatgag 300

tgtttcctcc agcacaagga tgacaaccca aatctgcccc gcctcgtgcg acctgaggtc 360

gatgtgatgt gcaccgcctt tcatgacaac gaagagacat tcctgaagaa atacctgtat 420

gaaattgctc gtaggcaccc atacttttat gcccccgagc tcctgttctt tgcaaagaga 480

tacaaagctg ccttcactga atgttgccag gcagctgata aggccgcatg tctcctgcct 540

aaactggacg agctccggga tgaaggtaag gcttccagcg ccaaacagcg cctgaagtgc 600

gcttctctcc agaagtttgg cgagcgagca ttcaaagcct gggctgtggc ccgtctcagt 660

cagaggtttc caaaggcaga atttgctgag gtctcaaaac tggtgaccga cctcacaaag 720

gtccatactg agtgttgcca cggagatctg ctggaatgtg ccgacgatag agcagacctc 780

gctaaatata tctgcgagaa tcaggattcc attagctcta agctgaaaga atgttgcgag 840

aagcccctcc tggaaaagag tcattgtatc gccgaggtgg aaaacgacga gatgccagca 900

gatctgccat cactcgctgc cgactttgtg gaatccaaag atgtctgcaa gaattacgca 960

gaggctaaag acgtgttcct ggggatgttt ctgtatgagt acgcccggcg tcaccccgat 1020

tatagcgtcg tgctcctgct ccgactggca aagacctacg aaacaactct ggagaaatgt 1080

tgcgctgccg cagaccctca tgaatgttat gctaaggtgt tcgatgagtt taagccactc 1140

gtcgaagagc cccagaacct gattaaacag aattgcgaac tgttcgagca gctcggtgaa 1200

tacaagtttc agaacgccct gctcgtgcgt tataccaaaa aggtccctca ggtgtctaca 1260

ccaactctgg tggaggtcag taggaatctg ggcaaagtgg gatcaaagtg ttgcaaacac 1320

cccgaggcaa agagaatgcc ttgtgctgaa gattacctct ccgtcgtgct gaaccagctc 1380

tgcgtgctgc atgaaaagac cccagtcagc gatcgggtga caaaatgttg caccgaatct 1440

ctggtcaatc gccgaccctg tttcagtgcc ctcgaagtgg acgaaactta tgtgcctaag 1500

gagtttcagg ctgaaacatt cacctttcac gccgatatct gcactctgtc cgagaaagaa 1560

aggcagatta agaaacagac agcactggtc gagctcgtga agcataaacc aaaggctacc 1620

aaggagcagc tgaaagccgt catggacgat ttcgcagctt ttgtggaaaa gtgttgcaaa 1680

gccgacgata aggagacttg tttcgcagaa gaggggaaaa agctcgtggc tgccagccag 1740

gcagctctgg gtctg 1755

<210> 3
<211> 585
<212> PRT
<213> Homo sapiens

<400> 3

<210>  4
<211>  1755
<212>  DNA
<213>  Homo sapiens

<400>  4
gatgcacaca agagtgaggt tgctcatcgg tttaaagatt tgggagaaga aaatttcaaa      60

gccttggtgt tgattgcctt tgctcagtat cttcagcagt gtccatttga agatcatgta     120

aaattagtga atgaagtaac tgaatttgca aaaacatgtg tagctgatga gtcagctgaa     180

aattgtgaca aatcacttca tacccttttt ggagacaaat tatgcacagt tgcaactctt     240

cgtgaaacct atggtgaaat ggctgactgc tgtgcaaaac aagaacctga gagaaatgaa     300

tgcttcttgc aacacaaaga tgacaaccca aacctccccc gattggtgag accagaggtt     360

gatgtgatgt gcactgcttt tcatgacaat gaagagacat ttttgaaaaa atacttatat     420

gaaattgcca gaagacatcc ttacttttat gccccggaac tccttttctt tgctaaaagg     480

tataaagctg cttttacaga atgttgccaa gctgctgata aagctgcctg cctgttgcca     540

aagctcgatg aacttcggga tgaagggaag gcttcgtctg ccaaacagag actcaaatgt     600

gccagtctcc aaaaatttgg agaaagagct ttcaaagcat gggcagtggc tcgcctgagc     660

cagagatttc ccaaagctga gtttgcagaa gtttccaagt tagtgacaga tcttaccaaa     720

gtccacacgg aatgctgcca tggagatctg cttgaatgtg ctgatgacag ggcggacctt     780

gccaagtata tctgtgaaaa tcaggattcg atctccagta aactgaagga atgctgtgaa     840

aaacctctgt tggaaaaatc ccactgcatt gccgaagtgg aaaatgatga gatgcctgct     900

gacttgcctt cattagctgc tgattttgtt gaaagtaagg atgtttgcaa aaactatgct     960

gaggcaaagg atgtcttcct gggcatgttt ttgtatgaat atgcaagaag gcatcctgat    1020

tactctgtcg tgctgctgct gagacttgcc aagacatatg aaaccactct agagaagtgc 1080

tgtgccgctg cagatcctca tgaatgctat gccaaagtgt tcgatgaatt taaacctctt 1140

gtggaagagc ctcagaattt aatcaaacaa aactgtgagc tttttaagca gcttggagag 1200

tacaaattcc agaatgcgct attagttcgt tacaccaaga aagtacccca agtgtcaact 1260

ccaactcttg tagaggtctc aagaaaccta ggaaaagtgg gcagcaaatg ttgtaaacat 1320

cctgaagcaa aaagaatgcc ctgtgcagaa gactatctat ccgtggtcct gaaccagtta 1380

tgtgtgttgc atgagaaaac gccagtaagt gacagagtca caaaatgctg cacagagtcc 1440

ttggtgaaca ggcgaccatg cttttcagct ctggaagtcg atgaaacata cgttcccaaa 1500

gagtttaatg ctgaaacatt caccttccat gcagatatat gcacactttc tgagaaggag 1560

agacaaatca agaaacaaac tgcacttgtt gagcttgtga aacacaagcc caaggcaaca 1620

aaagagcaac tgaaagctgt tatggatgat ttcgcagctt ttgtagagaa gtgctgcaag 1680

gctgacgata aggagacctg ctttgccgag gagggtaaaa aacttgttgc tgcaagtcaa 1740

gctgccttag gctta 1755

<210> 5
<211> 4
<212> PRT
<213> Artificial sequence

<220>
<223> Synthetic Construct

<400> 5

Ala Ala Ala Leu
1

<210> 6
<211> 588
<212> PRT
<213> Artificial sequence

<220>
<223> Synthetic Construct

<400> 6

Ala Ala Ser Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Ser Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Gln Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu
            580                 585

<210>  7
<211>  588
<212>  PRT
<213>  Artificial Sequence

<220>
<223>  Synthetic Construct

<400>  7

Ala Ala Gln Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Ser Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
        180                 185                 190

Lys Ala Ser Ser Ala Lys Gln Arg Leu Lys Cys Ala Ser Leu Gln Lys
    195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Gln Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu
            580                 585

<210>  8
<211>  589
<212>  PRT
<213>  Artificial Sequence

<220>
<223>  Synthetic Construct

<400>  8

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Ser Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Gln Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys

545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu Ala Ala Ala Leu
            580                 585

<210>  9
<211>  592
<212>  PRT
<213>  Artificial Sequence

<220>
<223>  Synthetic Construct

<400>  9

Ala Ala Ser Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Ser Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn

420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Gln Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu Ala Ala Leu
            580                 585                 590

<210>  10
<211>  592
<212>  PRT
<213>  Artificial Sequence

<220>
<223>  Synthetic Construct

<400>  10

Ala Ala Gln Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Ser Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp

290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Gln Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu Ala Ala Leu
            580                 585                 590

<210>  11
<211>  588
<212>  PRT
<213>  Artificial Sequence

<220>
<223>  Synthetic Construct

<400>  11

Ala Ala Ser Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Cys Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp

165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Asn Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu
            580                 585

<210>  12
<211>  588
<212>  PRT
<213>  Artificial Sequence

<220>
<223>  Synthetic Construct

<400>  12

Ala Ala Gln Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Cys Pro Phe Glu Asp His Val Lys Leu Val Asn Glu

35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Asn Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu
            580                 585

<210>  13
<211>  589
<212>  PRT
<213>  Artificial Sequence

<220>
<223>  Synthetic Construct

<400>  13

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg Leu Lys Cys Ala Ser Leu Gln Lys Phe Gly Glu
        195                 200                 205

Arg Ala Phe Lys Ala Trp Ala Val Ala Arg Leu Ser Gln Arg Phe Pro
    210                 215                 220

Lys Ala Glu Phe Ala Glu Val Ser Lys Leu Val Thr Asp Leu Thr Lys
225                 230                 235                 240

Val His Thr Glu Cys Cys His Gly Asp Leu Leu Glu Cys Ala Asp Asp
                245                 250                 255

Arg Ala Asp Leu Ala Lys Tyr Ile Cys Glu Asn Gln Asp Ser Ile Ser
            260                 265                 270

Ser Lys Leu Lys Glu Cys Cys Glu Lys Pro Leu Leu Glu Lys Ser His
        275                 280                 285

Cys Ile Ala Glu Val Glu Asn Asp Glu Met Pro Ala Asp Leu Pro Ser
    290                 295                 300

Leu Ala Ala Asp Phe Val Glu Ser Lys Asp Val Cys Lys Asn Tyr Ala
305                 310                 315                 320

Glu Ala Lys Asp Val Phe Leu Gly Met Phe Leu Tyr Glu Tyr Ala Arg
                325                 330                 335

Arg His Pro Asp Tyr Ser Val Val Leu Leu Leu Arg Leu Ala Lys Thr
            340                 345                 350

Tyr Glu Thr Thr Leu Glu Lys Cys Cys Ala Ala Ala Asp Pro His Glu
        355                 360                 365

Cys Tyr Ala Lys Val Phe Asp Glu Phe Lys Pro Leu Val Glu Glu Pro
    370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
            420                 425                 430

Val Gly Ser Lys Cys Cys Lys His Pro Glu Ala Lys Arg Met Pro Cys
        435                 440                 445

Ala Glu Asp Tyr Leu Ser Val Val Leu Asn Gln Leu Cys Val Leu His
    450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
            500                 505                 510

Ile Cys Thr Leu Ser Glu Lys Glu Arg Gln Ile Lys Lys Gln Thr Ala
        515                 520                 525

Leu Val Glu Leu Val Lys His Lys Pro Lys Ala Thr Lys Glu Gln Leu
    530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu Ala Ala Ala Leu
            580                 585

<210>  14
<211>  592
<212>  PRT
<213>  Artificial Sequence

<220>
<223>  Synthetic Construct

<400>  14
   Ala Ala Ser Asp Ala His Lys Ser Glu Val Ala His Arg Phe Lys Asp
   1               5                   10                  15
   Leu Gly Glu Glu Asn Phe Lys Ala Leu Val Leu Ile Ala Phe Ala Gln
               20                  25                  30
   Tyr Leu Gln Gln Cys Pro Phe Glu Asp His Val Lys Leu Val Asn Glu
           35                  40                  45
   Val Thr Glu Phe Ala Lys Thr Cys Val Ala Asp Glu Ser Ala Glu Asn
       50                  55                  60
   Cys Asp Lys Ser Leu His Thr Leu Phe Gly Asp Lys Leu Cys Thr Val
   65                  70                  75                  80
   Ala Thr Leu Arg Glu Thr Tyr Gly Glu Met Ala Asp Cys Cys Ala Lys
                   85                  90                  95
   Gln Glu Pro Glu Arg Asn Glu Cys Phe Leu Gln His Lys Asp Asp Asn
               100                 105                 110
   Pro Asn Leu Pro Arg Leu Val Arg Pro Glu Val Asp Val Met Cys Thr
           115                 120                 125
   Ala Phe His Asp Asn Glu Glu Thr Phe Leu Lys Lys Tyr Leu Tyr Glu
       130                 135                 140
   Ile Ala Arg Arg His Pro Tyr Phe Tyr Ala Pro Glu Leu Leu Phe Phe
   145                 150                 155                 160
   Ala Lys Arg Tyr Lys Ala Ala Phe Thr Glu Cys Cys Gln Ala Ala Asp
                   165                 170                 175
   Lys Ala Ala Cys Leu Leu Pro Lys Leu Asp Glu Leu Arg Asp Glu Gly
               180                 185                 190
   Lys Ala Ser Ser Ala Lys Gln Arg Leu Lys Cys Ala Ser Leu Gln Lys
           195                 200                 205
   Phe Gly Glu Arg Ala Phe Lys Ala Trp Ala Val Ala Arg Leu Ser Gln
       210                 215                 220
   Arg Phe Pro Lys Ala Glu Phe Ala Glu Val Ser Lys Leu Val Thr Asp
   225                 230                 235                 240
   Leu Thr Lys Val His Thr Glu Cys Cys His Gly Asp Leu Leu Glu Cys
                   245                 250                 255
   Ala Asp Asp Arg Ala Asp Leu Ala Lys Tyr Ile Cys Glu Asn Gln Asp
               260                 265                 270
   Ser Ile Ser Ser Lys Leu Lys Glu Cys Cys Glu Lys Pro Leu Leu Glu
           275                 280                 285
   Lys Ser His Cys Ile Ala Glu Val Glu Asn Asp Glu Met Pro Ala Asp
       290                 295                 300
   Leu Pro Ser Leu Ala Ala Asp Phe Val Glu Ser Lys Asp Val Cys Lys
   305                 310                 315                 320
   Asn Tyr Ala Glu Ala Lys Asp Val Phe Leu Gly Met Phe Leu Tyr Glu
                   325                 330                 335
   Tyr Ala Arg Arg His Pro Asp Tyr Ser Val Val Leu Leu Leu Arg Leu
               340                 345                 350
   Ala Lys Thr Tyr Glu Thr Thr Leu Glu Lys Cys Cys Ala Ala Ala Asp
           355                 360                 365
   Pro His Glu Cys Tyr Ala Lys Val Phe Asp Glu Phe Lys Pro Leu Val
       370                 375                 380
   Glu Glu Pro Gln Asn Leu Ile Lys Gln Asn Cys Glu Leu Phe Glu Gln
   385                 390                 395                 400
   Leu Gly Glu Tyr Lys Phe Gln Asn Ala Leu Leu Val Arg Tyr Thr Lys
                   405                 410                 415
   Lys Val Pro Gln Val Ser Thr Pro Thr Leu Val Glu Val Ser Arg Asn
               420                 425                 430
   Leu Gly Lys Val Gly Ser Lys Cys Cys Lys His Pro Glu Ala Lys Arg
           435                 440                 445
   Met Pro Cys Ala Glu Asp Tyr Leu Ser Val Val Leu Asn Gln Leu Cys
       450                 455                 460
   Val Leu His Glu Lys Thr Pro Val Ser Asp Arg Val Thr Lys Cys Cys
   465                 470                 475                 480
   Thr Glu Ser Leu Val Asn Arg Arg Pro Cys Phe Ser Ala Leu Glu Val
                   485                 490                 495
   Asp Glu Thr Tyr Val Pro Lys Glu Phe Asn Ala Glu Thr Phe Thr Phe
               500                 505                 510
   His Ala Asp Ile Cys Thr Leu Ser Glu Lys Glu Arg Gln Ile Lys Lys
           515                 520                 525
   Gln Thr Ala Leu Val Glu Leu Val Lys His Lys Pro Lys Ala Thr Lys
       530                 535                 540
   Glu Gln Leu Lys Ala Val Met Asp Asp Phe Ala Ala Phe Val Glu Lys
   545                 550                 555                 560
   Cys Cys Lys Ala Asp Asp Lys Glu Thr Cys Phe Ala Glu Glu Gly Lys
                   565                 570                 575
   Lys Leu Val Ala Ala Ser Gln Ala Ala Leu Gly Leu Ala Ala Ala Leu
               580                 585                 590

<210>  15
<211>  592
<212>  PRT
<213>  Artificial Sequence

<220>
<223>  Synthethic Construct

<400>  15

Ala Ala Gln Asp Ala His Lys Ser Glu Val Ala His Arg Phe Lys Asp
1               5                   10                  15

Leu Gly Glu Glu Asn Phe Lys Ala Leu Val Leu Ile Ala Phe Ala Gln
            20                  25                  30

Tyr Leu Gln Gln Cys Pro Phe Glu Asp His Val Lys Leu Val Asn Glu
        35                  40                  45

Val Thr Glu Phe Ala Lys Thr Cys Val Ala Asp Glu Ser Ala Glu Asn
    50                  55                  60

Cys Asp Lys Ser Leu His Thr Leu Phe Gly Asp Lys Leu Cys Thr Val
65                  70                  75                  80

Ala Thr Leu Arg Glu Thr Tyr Gly Glu Met Ala Asp Cys Cys Ala Lys
                85                  90                  95

Gln Glu Pro Glu Arg Asn Glu Cys Phe Leu Gln His Lys Asp Asp Asn
            100                 105                 110

Pro Asn Leu Pro Arg Leu Val Arg Pro Glu Val Asp Val Met Cys Thr
        115                 120                 125

Ala Phe His Asp Asn Glu Glu Thr Phe Leu Lys Lys Tyr Leu Tyr Glu
    130                 135                 140

Ile Ala Arg Arg His Pro Tyr Phe Tyr Ala Pro Glu Leu Leu Phe Phe
145                 150                 155                 160

Ala Lys Arg Tyr Lys Ala Ala Phe Thr Glu Cys Cys Gln Ala Ala Asp
                165                 170                 175

Lys Ala Ala Cys Leu Leu Pro Lys Leu Asp Glu Leu Arg Asp Glu Gly
            180                 185                 190

Lys Ala Ser Ser Ala Lys Gln Arg Leu Lys Cys Ala Ser Leu Gln Lys
        195                 200                 205

Phe Gly Glu Arg Ala Phe Lys Ala Trp Ala Val Ala Arg Leu Ser Gln
    210                 215                 220

Arg Phe Pro Lys Ala Glu Phe Ala Glu Val Ser Lys Leu Val Thr Asp
225                 230                 235                 240

Leu Thr Lys Val His Thr Glu Cys Cys His Gly Asp Leu Leu Glu Cys
                245                 250                 255

Ala Asp Asp Arg Ala Asp Leu Ala Lys Tyr Ile Cys Glu Asn Gln Asp
            260                 265                 270

Ser Ile Ser Ser Lys Leu Lys Glu Cys Cys Glu Lys Pro Leu Leu Glu
        275                 280                 285

Lys Ser His Cys Ile Ala Glu Val Glu Asn Asp Glu Met Pro Ala Asp
    290                 295                 300

Leu Pro Ser Leu Ala Ala Asp Phe Val Glu Ser Lys Asp Val Cys Lys
305                 310                 315                 320

Asn Tyr Ala Glu Ala Lys Asp Val Phe Leu Gly Met Phe Leu Tyr Glu
                325                 330                 335

Tyr Ala Arg Arg His Pro Asp Tyr Ser Val Val Leu Leu Leu Arg Leu
            340                 345                 350

Ala Lys Thr Tyr Glu Thr Thr Leu Glu Lys Cys Cys Ala Ala Ala Asp
        355                 360                 365

Pro His Glu Cys Tyr Ala Lys Val Phe Asp Glu Phe Lys Pro Leu Val
    370                 375                 380

Glu Glu Pro Gln Asn Leu Ile Lys Gln Asn Cys Glu Leu Phe Glu Gln
385                 390                 395                 400

Leu Gly Glu Tyr Lys Phe Gln Asn Ala Leu Leu Val Arg Tyr Thr Lys
                405                 410                 415

Lys Val Pro Gln Val Ser Thr Pro Thr Leu Val Glu Val Ser Arg Asn
            420                 425                 430

Leu Gly Lys Val Gly Ser Lys Cys Cys Lys His Pro Glu Ala Lys Arg
        435                 440                 445

Met Pro Cys Ala Glu Asp Tyr Leu Ser Val Val Leu Asn Gln Leu Cys
    450                 455                 460

Val Leu His Glu Lys Thr Pro Val Ser Asp Arg Val Thr Lys Cys Cys
465                 470                 475                 480

Thr Glu Ser Leu Val Asn Arg Arg Pro Cys Phe Ser Ala Leu Glu Val
                485                 490                 495

Asp Glu Thr Tyr Val Pro Lys Glu Phe Asn Ala Glu Thr Phe Thr Phe
            500                 505                 510

His Ala Asp Ile Cys Thr Leu Ser Glu Lys Glu Arg Gln Ile Lys Lys
        515                 520                 525

Gln Thr Ala Leu Val Glu Leu Val Lys His Lys Pro Lys Ala Thr Lys
    530                 535                 540

Glu Gln Leu Lys Ala Val Met Asp Asp Phe Ala Ala Phe Val Glu Lys
545                 550                 555                 560

Cys Cys Lys Ala Asp Asp Lys Glu Thr Cys Phe Ala Glu Glu Gly Lys
                565                 570                 575

Lys Leu Val Ala Ala Ser Gln Ala Ala Leu Gly Leu Ala Ala Leu
            580                 585                 590

<210>  16
<211>  1095
<212>  PRT
<213>  Artificial Sequence

<220>
<223>  Synthetic Construct

<400>  16

Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Arg Asp Gly Ser Ala Ser Tyr Tyr Val Asp Ser Val
    50                  55                  60

Lys Gly Arg Phe Thr Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Val Gly Tyr Phe Asp Leu Trp Gly Arg Gly Thr
            100                 105                 110

Leu Val Thr Val Ser Ser Ala Ser Thr Gly Gly Gly Gly Ser Gly Gly
        115                 120                 125

Gly Gly Ser Gly Gly Gly Gly Ser Gln Ser Ala Leu Thr Gln Pro Ala
    130                 135                 140

Ser Val Ser Gly Ser Pro Gly Gln Ser Ile Thr Ile Ser Cys Thr Gly
145                 150                 155                 160

Thr Ser Ser Asp Val Gly Gly Tyr Asn Phe Val Ser Trp Tyr Gln Gln
                165                 170                 175

His Pro Gly Lys Ala Pro Lys Leu Met Ile Tyr Asp Val Ser Asp Arg
            180                 185                 190

Pro Ser Gly Val Ser Asp Arg Phe Ser Gly Ser Lys Ser Gly Asn Thr
        195                 200                 205

Ala Ser Leu Ile Ile Ser Gly Leu Gln Ala Asp Asp Glu Ala Asp Tyr
    210                 215                 220

Tyr Cys Ser Ser Tyr Gly Ser Ser Ser Thr His Val Ile Phe Gly Gly
225                 230                 235                 240

Gly Thr Lys Val Thr Val Leu Gly Ala Ala Ser Asp Ala His Lys Ser
                245                 250                 255

Glu Val Ala His Arg Phe Lys Asp Leu Gly Glu Glu Asn Phe Lys Ala
            260                 265                 270

Leu Val Leu Ile Ala Phe Ala Gln Tyr Leu Gln Gln Ser Pro Phe Glu
        275                 280                 285

Asp His Val Lys Leu Val Asn Glu Val Thr Glu Phe Ala Lys Thr Cys
    290                 295                 300

Val Ala Asp Glu Ser Ala Glu Asn Cys Asp Lys Ser Leu His Thr Leu
305                 310                 315                 320

Phe Gly Asp Lys Leu Cys Thr Val Ala Thr Leu Arg Glu Thr Tyr Gly
                325                 330                 335

Glu Met Ala Asp Cys Cys Ala Lys Gln Glu Pro Glu Arg Asn Glu Cys
            340                 345                 350

Phe Leu Gln His Lys Asp Asp Asn Pro Asn Leu Pro Arg Leu Val Arg
        355                 360                 365

Pro Glu Val Asp Val Met Cys Thr Ala Phe His Asp Asn Glu Glu Thr
    370                 375                 380

Phe Leu Lys Lys Tyr Leu Tyr Glu Ile Ala Arg Arg His Pro Tyr Phe
385                 390                 395                 400

Tyr Ala Pro Glu Leu Leu Phe Phe Ala Lys Arg Tyr Lys Ala Ala Phe
                405                 410                 415

Thr Glu Cys Cys Gln Ala Ala Asp Lys Ala Ala Cys Leu Leu Pro Lys
            420                 425                 430

Leu Asp Glu Leu Arg Asp Glu Gly Lys Ala Ser Ser Ala Lys Gln Arg
        435                 440                 445

Leu Lys Cys Ala Ser Leu Gln Lys Phe Gly Glu Arg Ala Phe Lys Ala
    450                 455                 460

Trp Ala Val Ala Arg Leu Ser Gln Arg Phe Pro Lys Ala Glu Phe Ala
465                 470                 475                 480

Glu Val Ser Lys Leu Val Thr Asp Leu Thr Lys Val His Thr Glu Cys
                485                 490                 495

Cys His Gly Asp Leu Leu Glu Cys Ala Asp Asp Arg Ala Asp Leu Ala
            500                 505                 510

Lys Tyr Ile Cys Glu Asn Gln Asp Ser Ile Ser Ser Lys Leu Lys Glu
        515                 520                 525

Cys Cys Glu Lys Pro Leu Leu Glu Lys Ser His Cys Ile Ala Glu Val

530                 535                 540

Glu Asn Asp Glu Met Pro Ala Asp Leu Pro Ser Leu Ala Ala Asp Phe
545                 550                 555                 560

Val Glu Ser Lys Asp Val Cys Lys Asn Tyr Ala Glu Ala Lys Asp Val
                565                 570                 575

Phe Leu Gly Met Phe Leu Tyr Glu Tyr Ala Arg Arg His Pro Asp Tyr
            580                 585                 590

Ser Val Val Leu Leu Leu Arg Leu Ala Lys Thr Tyr Glu Thr Thr Leu
        595                 600                 605

Glu Lys Cys Cys Ala Ala Ala Asp Pro His Glu Cys Tyr Ala Lys Val
    610                 615                 620

Phe Asp Glu Phe Lys Pro Leu Val Glu Glu Pro Gln Asn Leu Ile Lys
625                 630                 635                 640

Gln Asn Cys Glu Leu Phe Glu Gln Leu Gly Glu Tyr Lys Phe Gln Asn
                645                 650                 655

Ala Leu Leu Val Arg Tyr Thr Lys Lys Val Pro Gln Val Ser Thr Pro
            660                 665                 670

Thr Leu Val Glu Val Ser Arg Asn Leu Gly Lys Val Gly Ser Lys Cys
        675                 680                 685

Cys Lys His Pro Glu Ala Lys Arg Met Pro Cys Ala Glu Asp Tyr Leu
    690                 695                 700

Ser Val Val Leu Asn Gln Leu Cys Val Leu His Glu Lys Thr Pro Val
705                 710                 715                 720

Ser Asp Arg Val Thr Lys Cys Cys Thr Glu Ser Leu Val Asn Arg Arg
                725                 730                 735

Pro Cys Phe Ser Ala Leu Glu Val Asp Glu Thr Tyr Val Pro Lys Glu
            740                 745                 750

Phe Gln Ala Glu Thr Phe Thr Phe His Ala Asp Ile Cys Thr Leu Ser
        755                 760                 765

Glu Lys Glu Arg Gln Ile Lys Lys Gln Thr Ala Leu Val Glu Leu Val
    770                 775                 780

Lys His Lys Pro Lys Ala Thr Lys Glu Gln Leu Lys Ala Val Met Asp
785                 790                 795                 800

Asp Phe Ala Ala Phe Val Glu Lys Cys Cys Lys Ala Asp Asp Lys Glu
                805                 810                 815

Thr Cys Phe Ala Glu Glu Gly Lys Lys Leu Val Ala Ala Ser Gln Ala
            820                 825                 830

Ala Leu Gly Leu Ala Ala Ala Leu Gln Val Gln Leu Val Gln Ser Gly
        835                 840                 845

Ala Glu Val Lys Lys Pro Gly Glu Ser Leu Lys Ile Ser Cys Lys Gly
    850                 855                 860

Ser Gly Tyr Ser Phe Thr Ser Tyr Trp Ile Ala Trp Val Arg Gln Met
865                 870                 875                 880

Pro Gly Lys Gly Leu Glu Tyr Met Gly Leu Ile Tyr Pro Gly Asp Ser
                885                 890                 895

Asp Thr Lys Tyr Ser Pro Ser Phe Gln Gly Gln Val Thr Ile Ser Val
            900                 905                 910

Asp Lys Ser Val Ser Thr Ala Tyr Leu Gln Trp Ser Ser Leu Lys Pro
        915                 920                 925

Ser Asp Ser Ala Val Tyr Phe Cys Ala Arg His Asp Val Gly Tyr Cys
    930                 935                 940

Thr Asp Arg Thr Cys Ala Lys Trp Pro Glu Trp Leu Gly Val Trp Gly
945                 950                 955                 960

Gln Gly Thr Leu Val Thr Val Ser Ser Gly Gly Gly Gly Ser Ser Gly
                965                 970                 975

Gly Gly Ser Gly Gly Gly Gly Ser Gln Ser Val Leu Thr Gln Pro Pro
            980                 985                 990

Ser Val Ser Ala Ala Pro Gly Gln  Lys Val Thr Ile Ser  Cys Ser Gly
        995                 1000                1005

Ser Ser  Ser Asn Ile Gly Asn  Asn Tyr Val Ser Trp  Tyr Gln Gln
    1010                1015                1020

Leu Pro  Gly Thr Ala Pro Lys  Leu Leu Ile Tyr Asp  His Thr Asn
    1025                1030                1035

Arg Pro Ala Gly Val Pro Asp Arg Phe Ser Gly Ser Lys Ser Gly
    1040                1045                1050

Thr Ser Ala Ser Leu Ala Ile Ser Gly Phe Arg Ser Glu Asp Glu
    1055                1060                1065

Ala Asp Tyr Tyr Cys Ala Ser Trp Asp Tyr Thr Leu Ser Gly Trp
    1070                1075                1080

Val Phe Gly Gly Gly Thr Lys Leu Thr Val Leu Gly
    1085                1090                1095

<210> 17
<211> 1092
<212> PRT
<213> Artificial Sequence

<220>
<223> Synthetic Construct

<400> 17

Gln Val Gln Leu Val Gln Ser Gly Gly Gly Leu Val Lys Pro Gly Gly
1               5                   10                  15

Ser Leu Arg Leu Ser Cys Ala Ala Ser Gly Phe Ser Phe Asn Thr Tyr
            20                  25                  30

Asp Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Val Ala Thr Thr Pro Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gln Ser Val Leu Thr Gln Pro Pro Ser Val
    130                 135                 140

Ser Gly Ala Pro Gly Gln Arg Val Thr Ile Ser Cys Thr Gly Ser Ser
145                 150                 155                 160

Ser Asn Ile Gly Ala Gly Tyr Asp Val His Trp Tyr Gln Gln Leu Pro
                165                 170                 175

Gly Thr Ala Pro Lys Leu Leu Ile Tyr Gly Asn Ser Asn Arg Pro Ser
            180                 185                 190

Gly Val Pro Asp Arg Phe Ser Gly Ser Lys Ser Gly Thr Ser Ala Ser
        195                 200                 205

Leu Ala Ile Thr Gly Leu Gln Ala Glu Asp Glu Ala Asp Tyr Tyr Cys
    210                 215                 220

Gln Ser Tyr Asp Ser Ser Leu Ser Ala Leu Phe Gly Gly Gly Thr Lys
225                 230                 235                 240

Leu Thr Val Leu Gly Ala Ala Ser Asp Ala His Lys Ser Glu Val Ala
                245                 250                 255

His Arg Phe Lys Asp Leu Gly Glu Glu Asn Phe Lys Ala Leu Val Leu
            260                 265                 270

Ile Ala Phe Ala Gln Tyr Leu Gln Gln Ser Pro Phe Glu Asp His Val
        275                 280                 285

Lys Leu Val Asn Glu Val Thr Glu Phe Ala Lys Thr Cys Val Ala Asp
    290                 295                 300

Glu Ser Ala Glu Asn Cys Asp Lys Ser Leu His Thr Leu Phe Gly Asp
305                 310                 315                 320

Lys Leu Cys Thr Val Ala Thr Leu Arg Glu Thr Tyr Gly Glu Met Ala
                325                 330                 335

Asp Cys Cys Ala Lys Gln Glu Pro Glu Arg Asn Glu Cys Phe Leu Gln
            340                 345                 350

His Lys Asp Asp Asn Pro Asn Leu Pro Arg Leu Val Arg Pro Glu Val
        355                 360                 365

Asp Val Met Cys Thr Ala Phe His Asp Asn Glu Glu Thr Phe Leu Lys
    370                 375                 380

Lys Tyr Leu Tyr Glu Ile Ala Arg Arg His Pro Tyr Phe Tyr Ala Pro
385                 390                 395                 400

Glu Leu Leu Phe Phe Ala Lys Arg Tyr Lys Ala Ala Phe Thr Glu Cys
                405                 410                 415

Cys Gln Ala Ala Asp Lys Ala Ala Cys Leu Leu Pro Lys Leu Asp Glu
            420                 425                 430

Leu Arg Asp Glu Gly Lys Ala Ser Ser Ala Lys Gln Arg Leu Lys Cys
        435                 440                 445

Ala Ser Leu Gln Lys Phe Gly Glu Arg Ala Phe Lys Ala Trp Ala Val
    450                 455                 460

Ala Arg Leu Ser Gln Arg Phe Pro Lys Ala Glu Phe Ala Glu Val Ser
465                 470                 475                 480

Lys Leu Val Thr Asp Leu Thr Lys Val His Thr Glu Cys Cys His Gly
                485                 490                 495

Asp Leu Leu Glu Cys Ala Asp Asp Arg Ala Asp Leu Ala Lys Tyr Ile
            500                 505                 510

Cys Glu Asn Gln Asp Ser Ile Ser Ser Lys Leu Lys Glu Cys Cys Glu
        515                 520                 525

Lys Pro Leu Leu Glu Lys Ser His Cys Ile Ala Glu Val Glu Asn Asp
    530                 535                 540

Glu Met Pro Ala Asp Leu Pro Ser Leu Ala Ala Asp Phe Val Glu Ser
545                 550                 555                 560

Lys Asp Val Cys Lys Asn Tyr Ala Glu Ala Lys Asp Val Phe Leu Gly
                565                 570                 575

Met Phe Leu Tyr Glu Tyr Ala Arg Arg His Pro Asp Tyr Ser Val Val
            580                 585                 590

Leu Leu Leu Arg Leu Ala Lys Thr Tyr Glu Thr Thr Leu Glu Lys Cys
        595                 600                 605

Cys Ala Ala Ala Asp Pro His Glu Cys Tyr Ala Lys Val Phe Asp Glu
    610                 615                 620

Phe Lys Pro Leu Val Glu Glu Pro Gln Asn Leu Ile Lys Gln Asn Cys
625                 630                 635                 640

Glu Leu Phe Glu Gln Leu Gly Glu Tyr Lys Phe Gln Asn Ala Leu Leu
                645                 650                 655

Val Arg Tyr Thr Lys Lys Val Pro Gln Val Ser Thr Pro Thr Leu Val
            660                 665                 670

Glu Val Ser Arg Asn Leu Gly Lys Val Gly Ser Lys Cys Cys Lys His
        675                 680                 685

Pro Glu Ala Lys Arg Met Pro Cys Ala Glu Asp Tyr Leu Ser Val Val
    690                 695                 700

Leu Asn Gln Leu Cys Val Leu His Glu Lys Thr Pro Val Ser Asp Arg
705                 710                 715                 720

Val Thr Lys Cys Cys Thr Glu Ser Leu Val Asn Arg Arg Pro Cys Phe
                725                 730                 735

Ser Ala Leu Glu Val Asp Glu Thr Tyr Val Pro Lys Glu Phe Gln Ala
            740                 745                 750

Glu Thr Phe Thr Phe His Ala Asp Ile Cys Thr Leu Ser Glu Lys Glu
        755                 760                 765

Arg Gln Ile Lys Lys Gln Thr Ala Leu Val Glu Leu Val Lys His Lys
    770                 775                 780

Pro Lys Ala Thr Lys Glu Gln Leu Lys Ala Val Met Asp Asp Phe Ala
785                 790                 795                 800

Ala Phe Val Glu Lys Cys Cys Lys Ala Asp Asp Lys Glu Thr Cys Phe
                805                 810                 815

Ala Glu Glu Gly Lys Lys Leu Val Ala Ala Ser Gln Ala Ala Leu Gly
            820                 825                 830

Leu Ala Ala Ala Leu Gln Val Gln Leu Val Gln Ser Gly Ala Glu Val
        835                 840                 845

Lys Lys Pro Gly Glu Ser Leu Lys Ile Ser Cys Lys Gly Ser Gly Tyr
    850                 855                 860

Ser Phe Thr Ser Tyr Trp Ile Ala Trp Val Arg Gln Met Pro Gly Lys
865                 870                 875                 880

Gly Leu Glu Tyr Met Gly Leu Ile Tyr Pro Gly Asp Ser Asp Thr Lys
                885                 890                 895

Tyr Ser Pro Ser Phe Gln Gly Gln Val Thr Ile Ser Val Asp Lys Ser

900                 905                 910

Val Ser Thr Ala Tyr Leu Gln Trp Ser Ser Leu Lys Pro Ser Asp Ser
        915                 920                 925

Ala Val Tyr Phe Cys Ala Arg His Asp Val Gly Tyr Cys Thr Asp Arg
    930                 935                 940

Thr Cys Ala Lys Trp Pro Glu Trp Leu Gly Val Trp Gly Gln Gly Thr
945                 950                 955                 960

Leu Val Thr Val Ser Ser Gly Gly Gly Gly Ser Ser Gly Gly Gly Ser
                965                 970                 975

Gly Gly Gly Gly Ser Gln Ser Val Leu Thr Gln Pro Pro Ser Val Ser
            980                 985                 990

Ala Ala Pro Gly Gln Lys Val Thr  Ile Ser Cys Ser Gly  Ser Ser Ser
        995                 1000                 1005

Asn Ile  Gly Asn Asn Tyr Val  Ser Trp Tyr Gln Gln  Leu Pro Gly
    1010                 1015                 1020

Thr Ala  Pro Lys Leu Leu Ile  Tyr Asp His Thr Asn  Arg Pro Ala
    1025                 1030                 1035

Gly Val  Pro Asp Arg Phe Ser  Gly Ser Lys Ser Gly  Thr Ser Ala
    1040                 1045                 1050

Ser Leu  Ala Ile Ser Gly Phe  Arg Ser Glu Asp Glu  Ala Asp Tyr
    1055                 1060                 1065

Tyr Cys  Ala Ser Trp Asp Tyr  Thr Leu Ser Gly Trp  Val Phe Gly
    1070                 1075                 1080

Gly Gly  Thr Lys Leu Thr Val  Leu Gly
    1085                 1090

<210>  18
<211>  1083
<212>  PRT
<213>  Artificial Sequence

<220>
<223>  Synthetic Construct

<400>  18

Gln Val Gln Leu Val Gln Ser Gly Gly Gly Leu Val Lys Pro Gly Gly
1               5                   10                  15

Ser Leu Arg Leu Ser Cys Ala Ala Ser Gly Phe Ser Phe Asn Thr Tyr
            20                  25                  30

Asp Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Val Ala Thr Thr Pro Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gln Ser Val Leu Thr Gln Pro Pro Ser Val
    130                 135                 140

Ser Gly Ala Pro Gly Gln Arg Val Thr Ile Ser Cys Thr Gly Ser Ser
145                 150                 155                 160

Ser Asn Ile Gly Ala Gly Tyr Asp Val His Trp Tyr Gln Gln Leu Pro
                165                 170                 175

Gly Thr Ala Pro Lys Leu Leu Ile Tyr Gly Asn Ser Asn Arg Pro Ser
            180                 185                 190

Gly Val Pro Asp Arg Phe Ser Gly Ser Lys Ser Gly Thr Ser Ala Ser
        195                 200                 205

Leu Ala Ile Thr Gly Leu Gln Ala Glu Asp Glu Ala Asp Tyr Tyr Cys
    210                 215                 220

Gln Ser Tyr Asp Ser Ser Leu Ser Ala Leu Phe Gly Gly Gly Thr Lys
225                 230                 235                 240

Leu Thr Val Leu Gly Ala Ala Ser Asp Ala His Lys Ser Glu Val Ala
                245                 250                 255

His Arg Phe Lys Asp Leu Gly Glu Glu Asn Phe Lys Ala Leu Val Leu

260                 265                 270

Ile Ala Phe Ala Gln Tyr Leu Gln Gln Ser Pro Phe Glu Asp His Val
        275                 280                 285

Lys Leu Val Asn Glu Val Thr Glu Phe Ala Lys Thr Cys Val Ala Asp
    290                 295                 300

Glu Ser Ala Glu Asn Cys Asp Lys Ser Leu His Thr Leu Phe Gly Asp
305                 310                 315                 320

Lys Leu Cys Thr Val Ala Thr Leu Arg Glu Thr Tyr Gly Glu Met Ala
                325                 330                 335

Asp Cys Cys Ala Lys Gln Glu Pro Glu Arg Asn Glu Cys Phe Leu Gln
            340                 345                 350

His Lys Asp Asp Asn Pro Asn Leu Pro Arg Leu Val Arg Pro Glu Val
        355                 360                 365

Asp Val Met Cys Thr Ala Phe His Asp Asn Glu Glu Thr Phe Leu Lys
    370                 375                 380

Lys Tyr Leu Tyr Glu Ile Ala Arg Arg His Pro Tyr Phe Tyr Ala Pro
385                 390                 395                 400

Glu Leu Leu Phe Phe Ala Lys Arg Tyr Lys Ala Ala Phe Thr Glu Cys
                405                 410                 415

Cys Gln Ala Ala Asp Lys Ala Ala Cys Leu Leu Pro Lys Leu Asp Glu
            420                 425                 430

Leu Arg Asp Glu Gly Lys Ala Ser Ser Ala Lys Gln Arg Leu Lys Cys
        435                 440                 445

Ala Ser Leu Gln Lys Phe Gly Glu Arg Ala Phe Lys Ala Trp Ala Val
    450                 455                 460

Ala Arg Leu Ser Gln Arg Phe Pro Lys Ala Glu Phe Ala Glu Val Ser
465                 470                 475                 480

Lys Leu Val Thr Asp Leu Thr Lys Val His Thr Glu Cys Cys His Gly
                485                 490                 495

Asp Leu Leu Glu Cys Ala Asp Asp Arg Ala Asp Leu Ala Lys Tyr Ile
            500                 505                 510

Cys Glu Asn Gln Asp Ser Ile Ser Ser Lys Leu Lys Glu Cys Cys Glu
        515                 520                 525

Lys Pro Leu Leu Glu Lys Ser His Cys Ile Ala Glu Val Glu Asn Asp
    530                 535                 540

Glu Met Pro Ala Asp Leu Pro Ser Leu Ala Ala Asp Phe Val Glu Ser
545                 550                 555                 560

Lys Asp Val Cys Lys Asn Tyr Ala Glu Ala Lys Asp Val Phe Leu Gly
                565                 570                 575

Met Phe Leu Tyr Glu Tyr Ala Arg Arg His Pro Asp Tyr Ser Val Val
            580                 585                 590

Leu Leu Leu Arg Leu Ala Lys Thr Tyr Glu Thr Thr Leu Glu Lys Cys
        595                 600                 605

Cys Ala Ala Ala Asp Pro His Glu Cys Tyr Ala Lys Val Phe Asp Glu
    610                 615                 620

Phe Lys Pro Leu Val Glu Glu Pro Gln Asn Leu Ile Lys Gln Asn Cys
625                 630                 635                 640

Glu Leu Phe Glu Gln Leu Gly Glu Tyr Lys Phe Gln Asn Ala Leu Leu
                645                 650                 655

Val Arg Tyr Thr Lys Lys Val Pro Gln Val Ser Thr Pro Thr Leu Val
            660                 665                 670

Glu Val Ser Arg Asn Leu Gly Lys Val Gly Ser Lys Cys Cys Lys His
        675                 680                 685

Pro Glu Ala Lys Arg Met Pro Cys Ala Glu Asp Tyr Leu Ser Val Val
    690                 695                 700

Leu Asn Gln Leu Cys Val Leu His Glu Lys Thr Pro Val Ser Asp Arg
705                 710                 715                 720

Val Thr Lys Cys Cys Thr Glu Ser Leu Val Asn Arg Arg Pro Cys Phe
                725                 730                 735

Ser Ala Leu Glu Val Asp Glu Thr Tyr Val Pro Lys Glu Phe Gln Ala
            740                 745                 750

Glu Thr Phe Thr Phe His Ala Asp Ile Cys Thr Leu Ser Glu Lys Glu
        755                 760                 765

Arg Gln Ile Lys Lys Gln Thr Ala Leu Val Glu Leu Val Lys His Lys
    770                 775                 780

Pro Lys Ala Thr Lys Glu Gln Leu Lys Ala Val Met Asp Asp Phe Ala
785                 790                 795                 800

Ala Phe Val Glu Lys Cys Cys Lys Ala Asp Asp Lys Glu Thr Cys Phe
                805                 810                 815

Ala Glu Glu Gly Lys Lys Leu Val Ala Ala Ser Gln Ala Ala Leu Gly
            820                 825                 830

Leu Ala Ala Ala Leu Gln Val Gln Leu Val Glu Ser Gly Gly Gly Leu
        835                 840                 845

Val Gln Pro Gly Gly Ser Leu Arg Leu Ser Cys Ala Ala Ser Gly Phe
    850                 855                 860

Thr Phe Arg Ser Tyr Ala Met Ser Trp Val Arg Gln Ala Pro Gly Lys
865                 870                 875                 880

Gly Leu Glu Trp Val Ser Ala Ile Ser Gly Arg Gly Asp Asn Thr Tyr
                885                 890                 895

Tyr Ala Asp Ser Val Lys Gly Arg Phe Thr Ile Ser Arg Asp Asn Ser
            900                 905                 910

Lys Asn Thr Leu Tyr Leu Gln Met Asn Ser Leu Arg Ala Glu Asp Thr
        915                 920                 925

Ala Val Tyr Tyr Cys Ala Lys Met Thr Ser Asn Ala Val Gly Phe Asp
    930                 935                 940

Tyr Trp Gly Gln Gly Thr Leu Val Thr Val Ser Ser Gly Gly Gly Gly
945                 950                 955                 960

Ser Gly Gly Gly Ser Gly Gly Gly Gly Ser Gly Gln Ser Val Leu Thr
                965                 970                 975

Gln Pro Pro Ser Val Ser Gly Ala Pro Gly Gln Arg Val Thr Ile Ser
            980                 985                 990

Cys Thr Gly Arg His Ser Asn Ile Gly Leu Gly Tyr Gly Val His Trp
        995                 1000                1005

Tyr Gln Gln Leu Pro Gly Thr Ala Pro Lys Leu Leu Ile Tyr Gly
    1010                1015                1020

Asn Thr Asn Arg Pro Ser Gly Val Pro Asp Arg Phe Ser Gly Phe
    1025                1030                1035

Lys Ser Gly Thr Ser Ala Ser Leu Ala Ile Thr Gly Leu Gln Ala
    1040                1045                1050

Glu Asp Glu Ala Asp Tyr Tyr Cys Gln Ser Tyr Asp Arg Arg Thr
    1055                1060                1065

Pro Gly Trp Val Phe Gly Gly Gly Thr Lys Leu Thr Val Leu Gly
    1070                1075                1080

<210> 19
<211> 1092
<212> PRT
<213> Artificial Sequence

<220>
<223> Synthetic Construct

<400> 19


<210>  20
<211>  1096
<212>  PRT
<213>  Artificial Sequence

<220>
<223>  Synthetic Construct

<400>  20

<210>  21
<211>  1086
<212>  PRT
<213>  Artificial Sequence

<220>
<223>  Synthetic Construct

<400>  21

Gln Val Gln Leu Val Gln Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Gly Ile Ser Trp Asn Ser Gly Ser Ile Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Pro Glu Asp Thr Ala Val Tyr Tyr Cys
                85                  90                  95

Ala Arg Asp Leu Gly Ala Lys Gln Trp Leu Glu Gly Phe Asp Tyr Trp
            100                 105                 110

Gly Gln Gly Thr Leu Val Thr Val Ser Ser Ala Ser Thr Gly Gly Gly
        115                 120                 125

Gly Ser Gly Gly Gly Gly Ser Gly Gly Gly Gly Ser Ser Tyr Glu Leu
    130                 135                 140

Thr Gln Asp Pro Ala Val Ser Val Ala Leu Gly Gln Thr Val Arg Ile
145                 150                 155                 160

Thr Cys Gln Gly Asp Ser Leu Arg Ser Tyr Tyr Ala Ser Trp Tyr Gln
                165                 170                 175

Gln Lys Pro Gly Gln Ala Pro Val Leu Val Ile Tyr Gly Lys Asn Asn
            180                 185                 190

Arg Pro Ser Gly Ile Pro Asp Arg Phe Ser Gly Ser Thr Ser Gly Asn
        195                 200                 205

Ser Ala Ser Leu Thr Ile Thr Gly Ala Gln Ala Glu Asp Glu Ala Asp
    210                 215                 220

Tyr Tyr Cys Asn Ser Arg Asp Ser Ser Gly Asn His Trp Val Phe Gly
225                 230                 235                 240

Gly Gly Thr Lys Val Thr Val Leu Gly Ala Ala Ser Asp Ala His Lys
                245                 250                 255

Ser Glu Val Ala His Arg Phe Lys Asp Leu Gly Glu Glu Asn Phe Lys
            260                 265                 270

Ala Leu Val Leu Ile Ala Phe Ala Gln Tyr Leu Gln Gln Ser Pro Phe
        275                 280                 285

Glu Asp His Val Lys Leu Val Asn Glu Val Thr Glu Phe Ala Lys Thr
    290                 295                 300

Cys Val Ala Asp Glu Ser Ala Glu Asn Cys Asp Lys Ser Leu His Thr
305                 310                 315                 320

Leu Phe Gly Asp Lys Leu Cys Thr Val Ala Thr Leu Arg Glu Thr Tyr
                325                 330                 335

Gly Glu Met Ala Asp Cys Cys Ala Lys Gln Glu Pro Glu Arg Asn Glu
            340                 345                 350

Cys Phe Leu Gln His Lys Asp Asp Asn Pro Asn Leu Pro Arg Leu Val
        355                 360                 365

Arg Pro Glu Val Asp Val Met Cys Thr Ala Phe His Asp Asn Glu Glu

370                 375                 380

Thr Phe Lys Lys Tyr Leu Tyr Glu Ile Ala Arg Arg His Pro Tyr Phe
385                 390                 395                 400

Tyr Ala Pro Glu Leu Leu Phe Phe Ala Lys Arg Tyr Lys Ala Ala Phe
                405                 410                 415

Thr Glu Cys Cys Gln Ala Ala Asp Lys Ala Ala Cys Leu Leu Pro Lys
            420                 425                 430

Leu Asp Glu Leu Arg Asp Glu Gly Lys Ala Ser Ser Ala Lys Gln Arg
        435                 440                 445

Leu Lys Cys Ala Ser Leu Gln Lys Phe Gly Glu Arg Ala Phe Lys Ala
    450                 455                 460

Trp Ala Val Ala Arg Leu Ser Gln Arg Phe Pro Lys Ala Glu Phe Ala
465                 470                 475                 480

Glu Val Ser Lys Leu Val Thr Asp Leu Thr Lys Val His Thr Glu Cys
                485                 490                 495

Cys His Gly Asp Leu Leu Glu Cys Ala Asp Asp Arg Ala Asp Leu Ala
            500                 505                 510

Lys Tyr Ile Cys Glu Asn Gln Asp Ser Ile Ser Ser Lys Leu Lys Glu
        515                 520                 525

Cys Cys Glu Lys Pro Leu Leu Glu Lys Ser His Cys Ile Ala Glu Val
    530                 535                 540

Glu Asn Asp Glu Met Pro Ala Asp Leu Pro Ser Leu Ala Ala Asp Phe
545                 550                 555                 560

Val Glu Ser Lys Asp Val Cys Lys Asn Tyr Ala Glu Ala Lys Asp Val
                565                 570                 575

Phe Leu Gly Met Phe Leu Tyr Glu Tyr Ala Arg Arg His Pro Asp Tyr
            580                 585                 590

Ser Val Val Leu Leu Leu Arg Leu Ala Lys Thr Tyr Glu Thr Thr Leu
        595                 600                 605

Glu Lys Cys Cys Ala Ala Ala Asp Pro His Glu Cys Tyr Ala Lys Val
    610                 615                 620

Phe Asp Glu Phe Lys Pro Leu Val Glu Glu Pro Gln Asn Leu Ile Lys
625                 630                 635                 640

Gln Asn Cys Glu Leu Phe Glu Gln Leu Gly Glu Tyr Lys Phe Gln Asn
                645                 650                 655

Ala Leu Leu Val Arg Tyr Thr Lys Lys Val Pro Gln Val Ser Thr Pro
            660                 665                 670

Thr Leu Val Glu Val Ser Arg Asn Leu Gly Lys Val Gly Ser Lys Cys
        675                 680                 685

Cys Lys His Pro Glu Ala Lys Arg Met Pro Cys Ala Glu Asp Tyr Leu
    690                 695                 700

Ser Val Val Leu Asn Gln Leu Cys Val Leu His Glu Lys Thr Pro Val
705                 710                 715                 720

Ser Asp Arg Val Thr Lys Cys Cys Thr Glu Ser Leu Val Asn Arg Arg
                725                 730                 735

Pro Cys Phe Ser Ala Leu Glu Val Asp Glu Thr Tyr Val Pro Lys Glu
            740                 745                 750

Phe Gln Ala Glu Thr Phe Thr Phe His Ala Asp Ile Cys Thr Leu Ser
        755                 760                 765

Glu Lys Glu Arg Gln Ile Lys Lys Gln Thr Ala Leu Val Glu Leu Val
    770                 775                 780

Lys His Lys Pro Lys Ala Thr Lys Glu Gln Leu Lys Ala Val Met Asp
785                 790                 795                 800

Asp Phe Ala Ala Phe Val Glu Lys Cys Cys Lys Ala Asp Asp Lys Glu
                805                 810                 815

Thr Cys Phe Ala Glu Glu Gly Lys Lys Leu Val Ala Ala Ser Gln Ala
            820                 825                 830

Ala Leu Gly Leu Ala Ala Ala Leu Gln Val Gln Leu Val Glu Ser Gly
        835                 840                 845

Gly Gly Leu Val Gln Pro Gly Gly Ser Leu Arg Leu Ser Cys Ala Ala
    850                 855                 860

Ser Gly Phe Thr Phe Arg Ser Tyr Ala Met Ser Trp Val Arg Gln Ala
865                 870                 875                 880

Pro Gly Lys Gly Leu Glu Trp Val Ser Ala Ile Ser Gly Arg Gly Asp
                885                 890                 895

Asn Thr Tyr Tyr Ala Asp Ser Val Lys Gly Arg Phe Thr Ile Ser Arg
            900                 905                 910

Asp Asn Ser Lys Asn Thr Leu Tyr Leu Gln Met Asn Ser Leu Arg Ala
        915                 920                 925

Glu Asp Thr Ala Val Tyr Tyr Cys Ala Lys Met Thr Ser Asn Ala Val
    930                 935                 940

Gly Phe Asp Tyr Trp Gly Gln Gly Thr Leu Val Thr Val Ser Ser Gly
945                 950                 955                 960

Gly Gly Gly Ser Gly Gly Gly Ser Gly Gly Gly Gly Ser Gly Gln Ser
                965                 970                 975

Val Leu Thr Gln Pro Pro Ser Val Ser Gly Ala Pro Gly Gln Arg Val
            980                 985                 990

Thr Ile Ser Cys Thr Gly Arg His Ser Asn Ile Gly Leu Gly Tyr Gly
        995                 1000                1005

Val His Trp Tyr Gln Gln Leu Pro Gly Thr Ala Pro Lys Leu Leu
    1010                1015                1020

Ile Tyr Gly Asn Thr Asn Arg Pro Ser Gly Val Pro Asp Arg Phe
    1025                1030                1035

Ser Gly Phe Lys Ser Gly Thr Ser Ala Ser Leu Ala Ile Thr Gly
    1040                1045                1050

Leu Gln Ala Glu Asp Glu Ala Asp Tyr Tyr Cys Gln Ser Tyr Asp
    1055                1060                1065

Arg Arg Thr Pro Gly Trp Val Phe Gly Gly Gly Thr Lys Leu Thr
    1070                1075                1080

Val Leu Gly
    1085

<210>  22
<211>  1096
<212>  PRT
<213>  Artificial Sequence

<220>

<223>  Synthetic Construct

<400>  22

<210> 23
<211> 1087
<212> PRT
<213> Artificial Sequence

<220>
<223> Synthethic Construct

<400> 23

<210>  24

<211>  1095
<212>  PRT
<213>  Artificial Sequence

<220>
<223>  Synthetic Construct

<400>  24

Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Arg Asp Gly Ser Ala Ser Tyr Tyr Val Asp Ser Val
    50                  55                  60

Lys Gly Arg Phe Thr Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Val Gly Tyr Phe Asp Leu Trp Gly Arg Gly Thr
            100                 105                 110

Leu Val Thr Val Ser Ser Ala Ser Thr Gly Gly Gly Gly Ser Gly Gly
        115                 120                 125

Gly Gly Ser Gly Gly Gly Gly Ser Gln Ser Ala Leu Thr Gln Pro Ala
    130                 135                 140

Ser Val Ser Gly Ser Pro Gly Gln Ser Ile Thr Ile Ser Cys Thr Gly
145                 150                 155                 160

Thr Ser Ser Asp Val Gly Gly Tyr Asn Phe Val Ser Trp Tyr Gln Gln
                165                 170                 175

His Pro Gly Lys Ala Pro Lys Leu Met Ile Tyr Asp Val Ser Asp Arg
            180                 185                 190

Pro Ser Gly Val Ser Asp Arg Phe Ser Gly Ser Lys Ser Gly Asn Thr
        195                 200                 205

Ala Ser Leu Ile Ile Ser Gly Leu Gln Ala Asp Asp Glu Ala Asp Tyr
    210                 215                 220

Tyr Cys Ser Ser Tyr Gly Ser Ser Ser Thr His Val Ile Phe Gly Gly
225                 230                 235                 240

Gly Thr Lys Val Thr Val Leu Gly Ala Ala Ser Asp Ala His Lys Ser
                245                 250                 255

Glu Val Ala His Arg Phe Lys Asp Leu Gly Glu Glu Asn Phe Lys Ala
            260                 265                 270

Leu Val Leu Ile Ala Phe Ala Gln Tyr Leu Gln Gln Ser Pro Phe Glu
        275                 280                 285

Asp His Val Lys Leu Val Asn Glu Val Thr Glu Phe Ala Lys Thr Cys
    290                 295                 300

Val Ala Asp Glu Ser Ala Glu Asn Cys Asp Lys Ser Leu His Thr Leu
305                 310                 315                 320

Phe Gly Asp Lys Leu Cys Thr Val Ala Thr Leu Arg Glu Thr Tyr Gly
                325                 330                 335

Glu Met Ala Asp Cys Cys Ala Lys Gln Glu Pro Glu Arg Asn Glu Cys
            340                 345                 350

Phe Leu Gln His Lys Asp Asp Asn Pro Asn Leu Pro Arg Leu Val Arg
        355                 360                 365

Pro Glu Val Asp Val Met Cys Thr Ala Phe His Asp Asn Glu Glu Thr
    370                 375                 380

Phe Leu Lys Lys Tyr Leu Tyr Glu Ile Ala Arg Arg His Pro Tyr Phe
385                 390                 395                 400

Tyr Ala Pro Glu Leu Leu Phe Phe Ala Lys Arg Tyr Lys Ala Ala Phe
                405                 410                 415

Thr Glu Cys Cys Gln Ala Ala Asp Lys Ala Ala Cys Leu Leu Pro Lys
            420                 425                 430

Leu Asp Glu Leu Arg Asp Glu Gly Lys Ala Ser Ser Ala Lys Gln Arg
        435                 440                 445

Leu Lys Cys Ala Ser Leu Gln Lys Phe Gly Glu Arg Ala Phe Lys Ala
    450                 455                 460

Trp Ala Val Ala Arg Leu Ser Gln Arg Phe Pro Lys Ala Glu Phe Ala

465                 470                 475                 480

Glu Val Ser Lys Leu Val Thr Asp Leu Thr Lys Val His Thr Glu Cys
                485                 490                 495

Cys His Gly Asp Leu Leu Glu Cys Ala Asp Asp Arg Ala Asp Leu Ala
            500                 505                 510

Lys Tyr Ile Cys Glu Asn Gln Asp Ser Ile Ser Ser Lys Leu Lys Glu
        515                 520                 525

Cys Cys Glu Lys Pro Leu Leu Glu Lys Ser His Cys Ile Ala Glu Val
    530                 535                 540

Glu Asn Asp Glu Met Pro Ala Asp Leu Pro Ser Leu Ala Ala Asp Phe
545                 550                 555                 560

Val Glu Ser Lys Asp Val Cys Lys Asn Tyr Ala Glu Ala Lys Asp Val
                565                 570                 575

Phe Leu Gly Met Phe Leu Tyr Glu Tyr Ala Arg Arg His Pro Asp Tyr
            580                 585                 590

Ser Val Val Leu Leu Leu Arg Leu Ala Lys Thr Tyr Glu Thr Thr Leu
        595                 600                 605

Glu Lys Cys Cys Ala Ala Ala Asp Pro His Glu Cys Tyr Ala Lys Val
    610                 615                 620

Phe Asp Glu Phe Lys Pro Leu Val Glu Glu Pro Gln Asn Leu Ile Lys
625                 630                 635                 640

Gln Asn Cys Glu Leu Phe Glu Gln Leu Gly Glu Tyr Lys Phe Gln Asn
                645                 650                 655

Ala Leu Leu Val Arg Tyr Thr Lys Lys Val Pro Gln Val Ser Thr Pro
            660                 665                 670

Thr Leu Val Glu Val Ser Arg Asn Leu Gly Lys Val Gly Ser Lys Cys
        675                 680                 685

Cys Lys His Pro Glu Ala Lys Arg Met Pro Cys Ala Glu Asp Tyr Leu
    690                 695                 700

Ser Val Val Leu Asn Gln Leu Cys Val Leu His Glu Lys Thr Pro Val
705                 710                 715                 720

Ser Asp Arg Val Thr Lys Cys Cys Thr Glu Ser Leu Val Asn Arg Arg
                725                 730                 735

Pro Cys Phe Ser Ala Leu Glu Val Asp Glu Thr Tyr Val Pro Lys Glu
            740                 745                 750

Phe Gln Ala Glu Thr Phe Thr Phe His Ala Asp Ile Cys Thr Leu Ser
        755                 760                 765

Glu Lys Glu Arg Gln Ile Lys Lys Gln Thr Ala Leu Val Glu Leu Val
    770                 775                 780

Lys His Lys Pro Lys Ala Thr Lys Glu Gln Leu Lys Ala Val Met Asp
785                 790                 795                 800

Asp Phe Ala Ala Phe Val Glu Lys Cys Cys Lys Ala Asp Asp Lys Glu
                805                 810                 815

Thr Cys Phe Ala Glu Glu Gly Lys Lys Leu Val Ala Ala Ser Gln Ala
            820                 825                 830

Ala Leu Gly Leu Ala Ala Ala Leu Gln Val Gln Leu Val Gln Ser Gly
        835                 840                 845

Ala Glu Val Lys Lys Pro Gly Glu Ser Leu Lys Ile Ser Cys Lys Gly
    850                 855                 860

Ser Gly Tyr Ser Phe Thr Ser Tyr Trp Ile Ala Trp Val Arg Gln Met
865                 870                 875                 880

Pro Gly Lys Gly Leu Glu Tyr Met Gly Leu Ile Tyr Pro Gly Asp Ser
                885                 890                 895

Asp Thr Lys Tyr Ser Pro Ser Phe Gln Gly Gln Val Thr Ile Ser Val
            900                 905                 910

Asp Lys Ser Val Ser Thr Ala Tyr Leu Gln Trp Ser Ser Leu Lys Pro
        915                 920                 925

Ser Asp Ser Ala Val Tyr Phe Cys Ala Arg His Asp Val Gly Tyr Cys
    930                 935                 940

Thr Asp Arg Thr Cys Ala Lys Trp Pro Glu Trp Leu Gly Val Trp Gly
945                 950                 955                 960

Gln Gly Thr Leu Val Thr Val Ser Ser Gly Gly Gly Gly Ser Ser Gly
                965                 970                 975

Gly Gly Ser Gly Gly Gly Gly Ser Gln Ser Val Leu Thr Gln Pro Pro
            980                 985                 990

Ser Val Ser Ala Ala Pro Gly Gln Lys Val Thr Ile Ser Cys Ser Gly
        995                 1000                1005

Ser Ser Ser Asn Ile Gly Asn Asn Tyr Val Ser Trp Tyr Gln Gln
    1010                1015                1020

Leu Pro Gly Thr Ala Pro Lys Leu Leu Ile Tyr Asp His Thr Asn
    1025                1030                1035

Arg Pro Ala Gly Val Pro Asp Arg Phe Ser Gly Ser Lys Ser Gly
    1040                1045                1050

Thr Ser Ala Ser Leu Ala Ile Ser Gly Phe Arg Ser Glu Asp Glu
    1055                1060                1065

Ala Asp Tyr Tyr Cys Ala Ser Trp Asp Tyr Thr Leu Ser Gly Trp
    1070                1075                1080

Val Phe Gly Gly Gly Thr Lys Leu Thr Val Leu Gly
    1085                1090                1095

<210>  25
<211>  1086
<212>  PRT
<213>  Artificial Sequence

<220>
<223>  Synthetic Construct

<400>  25

Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Arg Asp Gly Ser Ala Ser Tyr Tyr Val Asp Ser Val
    50                  55                  60

Lys Gly Arg Phe Thr Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Val Gly Tyr Phe Asp Leu Trp Gly Arg Gly Thr
            100                 105                 110

Leu Val Thr Val Ser Ser Ala Ser Thr Gly Gly Gly Gly Ser Gly Gly
        115                 120                 125

Gly Gly Ser Gly Gly Gly Gly Ser Gln Ser Ala Leu Thr Gln Pro Ala
    130                 135                 140

Ser Val Ser Gly Ser Pro Gly Gln Ser Ile Thr Ile Ser Cys Thr Gly
145                 150                 155                 160

Thr Ser Ser Asp Val Gly Gly Tyr Asn Phe Val Ser Trp Tyr Gln Gln
                165                 170                 175

His Pro Gly Lys Ala Pro Lys Leu Met Ile Tyr Asp Val Ser Asp Arg
            180                 185                 190

Pro Ser Gly Val Ser Asp Arg Phe Ser Gly Ser Lys Ser Gly Asn Thr
        195                 200                 205

Ala Ser Leu Ile Ile Ser Gly Leu Gln Ala Asp Asp Glu Ala Asp Tyr
    210                 215                 220

Tyr Cys Ser Ser Tyr Gly Ser Ser Ser Thr His Val Ile Phe Gly Gly
225                 230                 235                 240

Gly Thr Lys Val Thr Val Leu Gly Ala Ala Ser Asp Ala His Lys Ser
                245                 250                 255

Glu Val Ala His Arg Phe Lys Asp Leu Gly Glu Glu Asn Phe Lys Ala
            260                 265                 270

Leu Val Leu Ile Ala Phe Ala Gln Tyr Leu Gln Gln Ser Pro Phe Glu
        275                 280                 285

Asp His Val Lys Leu Val Asn Glu Val Thr Glu Phe Ala Lys Thr Cys
    290                 295                 300

Val Ala Asp Glu Ser Ala Glu Asn Cys Asp Lys Ser Leu His Thr Leu
305                 310                 315                 320

Phe Gly Asp Lys Leu Cys Thr Val Ala Thr Leu Arg Glu Thr Tyr Gly
                325                 330                 335

Glu Met Ala Asp Cys Cys Ala Lys Gln Glu Pro Glu Arg Asn Glu Cys
        340                 345                 350

Phe Leu Gln His Lys Asp Asp Asn Pro Asn Leu Pro Arg Leu Val Arg
    355                 360                 365

Pro Glu Val Asp Val Met Cys Thr Ala Phe His Asp Asn Glu Glu Thr
    370                 375                 380

Phe Leu Lys Lys Tyr Leu Tyr Glu Ile Ala Arg Arg His Pro Tyr Phe
385                 390                 395                 400

Tyr Ala Pro Glu Leu Leu Phe Phe Ala Lys Arg Tyr Lys Ala Ala Phe
                405                 410                 415

Thr Glu Cys Cys Gln Ala Ala Asp Lys Ala Ala Cys Leu Leu Pro Lys
            420                 425                 430

Leu Asp Glu Leu Arg Asp Glu Gly Lys Ala Ser Ser Ala Lys Gln Arg
        435                 440                 445

Leu Lys Cys Ala Ser Leu Gln Lys Phe Gly Glu Arg Ala Phe Lys Ala
    450                 455                 460

Trp Ala Val Ala Arg Leu Ser Gln Arg Phe Pro Lys Ala Glu Phe Ala
465                 470                 475                 480

Glu Val Ser Lys Leu Val Thr Asp Leu Thr Lys Val His Thr Glu Cys
                485                 490                 495

Cys His Gly Asp Leu Leu Glu Cys Ala Asp Asp Arg Ala Asp Leu Ala
            500                 505                 510

Lys Tyr Ile Cys Glu Asn Gln Asp Ser Ile Ser Ser Lys Leu Lys Glu
        515                 520                 525

Cys Cys Glu Lys Pro Leu Leu Glu Lys Ser His Cys Ile Ala Glu Val
    530                 535                 540

Glu Asn Asp Glu Met Pro Ala Asp Leu Pro Ser Leu Ala Ala Asp Phe
545                 550                 555                 560

Val Glu Ser Lys Asp Val Cys Lys Asn Tyr Ala Glu Ala Lys Asp Val
                565                 570                 575

Phe Leu Gly Met Phe Leu Tyr Glu Tyr Ala Arg Arg His Pro Asp Tyr
            580                 585                 590

Ser Val Val Leu Leu Leu Arg Leu Ala Lys Thr Tyr Glu Thr Thr Leu
        595                 600                 605

Glu Lys Cys Cys Ala Ala Ala Asp Pro His Glu Cys Tyr Ala Lys Val
    610                 615                 620

Phe Asp Glu Phe Lys Pro Leu Val Glu Glu Pro Gln Asn Leu Ile Lys
625                 630                 635                 640

Gln Asn Cys Glu Leu Phe Glu Gln Leu Gly Glu Tyr Lys Phe Gln Asn
                645                 650                 655

Ala Leu Leu Val Arg Tyr Thr Lys Lys Val Pro Gln Val Ser Thr Pro
            660                 665                 670

Thr Leu Val Glu Val Ser Arg Asn Leu Gly Lys Val Gly Ser Lys Cys
        675                 680                 685

Cys Lys His Pro Glu Ala Lys Arg Met Pro Cys Ala Glu Asp Tyr Leu
    690                 695                 700

Ser Val Val Leu Asn Gln Leu Cys Val Leu His Glu Lys Thr Pro Val
705                 710                 715                 720

Ser Asp Arg Val Thr Lys Cys Cys Thr Glu Ser Leu Val Asn Arg Arg
                725                 730                 735

Pro Cys Phe Ser Ala Leu Glu Val Asp Glu Thr Tyr Val Pro Lys Glu
            740                 745                 750

Phe Gln Ala Glu Thr Phe Thr Phe His Ala Asp Ile Cys Thr Leu Ser
        755                 760                 765

Glu Lys Glu Arg Gln Ile Lys Lys Gln Thr Ala Leu Val Glu Leu Val
    770                 775                 780

Lys His Lys Pro Lys Ala Thr Lys Glu Gln Leu Lys Ala Val Met Asp
785                 790                 795                 800

Asp Phe Ala Ala Phe Val Glu Lys Cys Cys Lys Ala Asp Asp Lys Glu
                805                 810                 815

Thr Cys Phe Ala Glu Glu Gly Lys Lys Leu Val Ala Ala Ser Gln Ala
            820                 825                 830

Ala Leu Gly Leu Ala Ala Ala Leu Gln Val Gln Leu Val Glu Ser Gly

835                 840                 845

Gly Gly Leu Val Gln Pro Gly Gly Ser Leu Arg Leu Ser Cys Ala Ala
    850                 855                 860

Ser Gly Phe Thr Phe Arg Ser Tyr Ala Met Ser Trp Val Arg Gln Ala
865                 870                 875                 880

Pro Gly Lys Gly Leu Glu Trp Val Ser Ala Ile Ser Gly Arg Gly Asp
                885                 890                 895

Asn Thr Tyr Tyr Ala Asp Ser Val Lys Gly Arg Phe Thr Ile Ser Arg
            900                 905                 910

Asp Asn Ser Lys Asn Thr Leu Tyr Leu Gln Met Asn Ser Leu Arg Ala
        915                 920                 925

Glu Asp Thr Ala Val Tyr Tyr Cys Ala Lys Met Thr Ser Asn Ala Val
    930                 935                 940

Gly Phe Asp Tyr Trp Gly Gln Gly Thr Leu Val Thr Val Ser Ser Gly
945                 950                 955                 960

Gly Gly Gly Ser Gly Gly Gly Ser Gly Gly Gly Gly Ser Gly Gln Ser
                965                 970                 975

Val Leu Thr Gln Pro Pro Ser Val Ser Gly Ala Pro Gly Gln Arg Val
            980                 985                 990

Thr Ile Ser Cys Thr Gly Arg His Ser Asn Ile Gly Leu Gly Tyr Gly
        995                 1000                1005

Val His Trp Tyr Gln Gln Leu Pro Gly Thr Ala Pro Lys Leu Leu
    1010                1015                1020

Ile Tyr Gly Asn Thr Asn Arg Pro Ser Gly Val Pro Asp Arg Phe
    1025                1030                1035

Ser Gly Phe Lys Ser Gly Thr Ser Ala Ser Leu Ala Ile Thr Gly
    1040                1045                1050

Leu Gln Ala Glu Asp Glu Ala Asp Tyr Tyr Cys Gln Ser Tyr Asp
    1055                1060                1065

Arg Arg Thr Pro Gly Trp Val Phe Gly Gly Gly Thr Lys Leu Thr
    1070                1075                1080

Val Leu Gly
    1085

<210> 26
<211> 248
<212> PRT
<213> Artificial Sequence

<220>
<223> Synthetic construct

<400> 26

Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Asn Arg Asp Gly Ser Ala Ser Tyr Tyr Val Asp Ser Val
    50                  55                  60

Lys Gly Arg Phe Thr Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Val Gly Tyr Phe Asp Leu Trp Gly Arg Gly Thr
            100                 105                 110

Leu Val Thr Val Ser Ser Ala Ser Thr Gly Gly Gly Gly Ser Gly Gly
        115                 120                 125

Gly Gly Ser Gly Gly Gly Gly Ser Gln Ser Ala Leu Thr Gln Pro Ala
    130                 135                 140

Ser Val Ser Gly Ser Pro Gly Gln Ser Ile Thr Ile Ser Cys Thr Gly
145                 150                 155                 160

Thr Ser Ser Asp Val Gly Gly Tyr Asn Phe Val Ser Trp Tyr Gln Gln
                165                 170                 175

His Pro Gly Lys Ala Pro Lys Leu Met Ile Tyr Asp Val Ser Asp Arg
            180                 185                 190

Pro Ser Gly Val Ser Asp Arg Phe Ser Gly Ser Lys Ser Gly Asn Thr

195                 200                 205

Ala Ser Leu Ile Ile Ser Gly Leu Gln Ala Asp Asp Glu Ala Asp Tyr
    210                 215                 220

Tyr Cys Ser Ser Tyr Gly Ser Ser Ser Thr His Val Ile Phe Gly Gly
225                 230                 235                 240

Gly Thr Lys Val Thr Val Leu Gly
                245

<210>  27
<211>  255
<212>  PRT
<213>  Artificial Sequence

<220>
<223>  Synthetic Construct

<400>  27

Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Ala Trp Val Arg Gln Met Pro Gly Lys Gly Leu Glu Tyr Met
        35                  40                  45

Gly Leu Ile Tyr Pro Gly Asp Ser Asp Thr Lys Tyr Ser Pro Ser Phe
    50                  55                  60

Gln Gly Gln Val Thr Ile Ser Val Asp Lys Ser Val Ser Thr Ala Tyr
65                  70                  75                  80

Leu Gln Trp Ser Ser Leu Lys Pro Ser Asp Ser Ala Val Tyr Phe Cys
                85                  90                  95

Ala Arg His Asp Val Gly Tyr Cys Thr Asp Arg Thr Cys Ala Lys Trp
            100                 105                 110

Pro Glu Trp Leu Gly Val Trp Gly Gln Gly Thr Leu Val Thr Val Ser
        115                 120                 125

Ser Gly Gly Gly Gly Ser Ser Gly Gly Gly Ser Gly Gly Gly Gly Ser
    130                 135                 140

Gln Ser Val Leu Thr Gln Pro Pro Ser Val Ser Ala Ala Pro Gly Gln
145                 150                 155                 160

Lys Val Thr Ile Ser Cys Ser Gly Ser Ser Ser Asn Ile Gly Asn Asn
                165                 170                 175

Tyr Val Ser Trp Tyr Gln Gln Leu Pro Gly Thr Ala Pro Lys Leu Leu
            180                 185                 190

Ile Tyr Asp His Thr Asn Arg Pro Ala Gly Val Pro Asp Arg Phe Ser
        195                 200                 205

Gly Ser Lys Ser Gly Thr Ser Ala Ser Leu Ala Ile Ser Gly Phe Arg
    210                 215                 220

Ser Glu Asp Glu Ala Asp Tyr Tyr Cys Ala Ser Trp Asp Tyr Thr Leu
225                 230                 235                 240

Ser Gly Trp Val Phe Gly Gly Gly Thr Lys Leu Thr Val Leu Gly
                245                 250                 255

<210>  28
<211>  245
<212>  PRT
<213>  Artificial Sequence

<220>
<223>  Synthetic Construct

<400>  28

Gln Val Gln Leu Val Gln Ser Gly Gly Gly Leu Val Lys Pro Gly Gly
1               5                   10                  15

Ser Leu Arg Leu Ser Cys Ala Ala Ser Gly Phe Ser Phe Asn Thr Tyr
            20                  25                  30

Asp Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Val Ala Thr Thr Pro Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gln Ser Val Leu Thr Gln Pro Pro Ser Val
    130                 135                 140

Ser Gly Ala Pro Gly Gln Arg Val Thr Ile Ser Cys Thr Gly Ser Ser
145                 150                 155                 160

Ser Asn Ile Gly Ala Gly Tyr Asp Val His Trp Tyr Gln Gln Leu Pro
                165                 170                 175

Gly Thr Ala Pro Lys Leu Leu Ile Tyr Gly Asn Ser Asn Arg Pro Ser
            180                 185                 190

Gly Val Pro Asp Arg Phe Ser Gly Ser Lys Ser Gly Thr Ser Ala Ser
        195                 200                 205

Leu Ala Ile Thr Gly Leu Gln Ala Glu Asp Glu Ala Asp Tyr Tyr Cys
    210                 215                 220

Gln Ser Tyr Asp Ser Ser Leu Ser Ala Leu Phe Gly Gly Gly Thr Lys
225                 230                 235                 240

Leu Thr Val Leu Gly
                245

<210>  29
<211>  255
<212>  PRT
<213>  Artificial Sequence

<220>
<223>  Synthetic Construct

<400>  29

Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Ala Trp Val Arg Gln Met Pro Gly Lys Gly Leu Glu Tyr Met
        35                  40                  45

Gly Leu Ile Tyr Pro Gly Asp Ser Asp Thr Lys Tyr Ser Pro Ser Phe
    50                  55                  60

Gln Gly Gln Val Thr Ile Ser Val Asp Lys Ser Val Ser Thr Ala Tyr
65                  70                  75                  80

Leu Gln Trp Ser Ser Leu Lys Pro Ser Asp Ser Ala Val Tyr Phe Cys
                85                  90                  95

Ala Arg His Asp Val Gly Tyr Cys Ser Ser Ser Asn Cys Ala Lys Trp
            100                 105                 110

Pro Glu Tyr Phe Gln His Trp Gly Gln Gly Thr Leu Val Thr Val Ser
        115                 120                 125

Ser Gly Gly Gly Gly Ser Ser Gly Gly Gly Ser Gly Gly Gly Gly Ser
    130                 135                 140

Gln Ser Val Leu Thr Gln Pro Pro Ser Val Ser Ala Ala Pro Gly Gln
145                 150                 155                 160

Lys Val Thr Ile Ser Cys Ser Gly Ser Ser Ser Asn Ile Gly Asn Asn
                165                 170                 175

Tyr Val Ser Trp Tyr Gln Gln Leu Pro Gly Thr Ala Pro Lys Leu Leu
            180                 185                 190

Ile Tyr Asp His Thr Asn Arg Pro Ala Gly Val Pro Asp Arg Phe Ser
        195                 200                 205

Gly Ser Lys Ser Gly Thr Ser Ala Ser Leu Ala Ile Ser Gly Phe Arg
    210                 215                 220

Ser Glu Asp Glu Ala Asp Tyr Tyr Cys Ala Ser Trp Asp Tyr Thr Leu
225                 230                 235                 240

Ser Gly Trp Val Phe Gly Gly Gly Thr Lys Leu Thr Val Leu Gly
                245                 250                 255

<210>  30
<211>  249
<212>  PRT
<213>  Artificial Sequence

<220>
<223>  Synthetic Construct

<400>  30

Gln Val Gln Leu Val Gln Ser Gly Gly Gly Leu Val Gln Pro Gly Arg
1               5                   10                  15

Ser Leu Arg Leu Ser Cys Ala Ala Ser Gly Phe Thr Phe Asp Asp Tyr

20                  25                  30

Ala Met His Trp Val Arg Gln Ala Pro Gly Lys Gly Leu Glu Trp Val
        35                  40                  45

Ser Gly Ile Ser Trp Asn Ser Gly Ser Ile Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Pro Glu Asp Thr Ala Val Tyr Tyr Cys
                85                  90                  95

Ala Arg Asp Leu Gly Ala Lys Gln Trp Leu Glu Gly Phe Asp Tyr Trp
            100                 105                 110

Gly Gln Gly Thr Leu Val Thr Val Ser Ser Ala Ser Thr Gly Gly Gly
        115                 120                 125

Gly Ser Gly Gly Gly Gly Ser Gly Gly Gly Gly Ser Ser Tyr Glu Leu
    130                 135                 140

Thr Gln Asp Pro Ala Val Ser Val Ala Leu Gly Gln Thr Val Arg Ile
145                 150                 155                 160

Thr Cys Gln Gly Asp Ser Leu Arg Ser Tyr Tyr Ala Ser Trp Tyr Gln
                165                 170                 175

Gln Lys Pro Gly Gln Ala Pro Val Leu Val Ile Tyr Gly Lys Asn Asn
            180                 185                 190

Arg Pro Ser Gly Ile Pro Asp Arg Phe Ser Gly Ser Thr Ser Gly Asn
        195                 200                 205

Ser Ala Ser Leu Thr Ile Thr Gly Ala Gln Ala Glu Asp Glu Ala Asp
    210                 215                 220

Tyr Tyr Cys Asn Ser Arg Asp Ser Ser Gly Asn His Trp Val Phe Gly
225                 230                 235                 240

Gly Gly Thr Lys Val Thr Val Leu Gly
                245

<210>  31
<211>  249
<212>  PRT
<213>  Artificial Sequence

<220>
<223>  Synthetic construct

<400>  31

Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Thr Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Tyr Ser Ser Ser Trp Ser Glu Val Ala Ser Gly Tyr Trp
            100                 105                 110

Gly Gln Gly Thr Leu Val Thr Val Ser Ser Ala Ser Thr Gly Gly Gly
        115                 120                 125

Gly Ser Gly Gly Gly Gly Ser Gly Gly Gly Gly Ser Ala Ile Val Met
    130                 135                 140

Thr Gln Ser Pro Ser Ser Leu Ser Ala Ser Val Gly Asp Arg Val Thr
145                 150                 155                 160

Ile Thr Cys Arg Ala Ser Gln Gly Ile Arg Asn Asp Leu Gly Trp Tyr
                165                 170                 175

Gln Gln Lys Ala Gly Lys Ala Pro Lys Leu Leu Ile Tyr Ala Ala Ser
            180                 185                 190

Ser Leu Gln Ser Gly Val Pro Ser Arg Phe Ser Gly Ser Gly Ser Gly
        195                 200                 205

Thr Asp Phe Thr Leu Thr Ile Ser Ser Leu Gln Pro Asp Asp Phe Ala
    210                 215                 220

Thr Tyr Phe Cys Gln Gln Ala His Ser Phe Pro Pro Thr Phe Gly Gly

225                 230                 235                 240

Gly Thr Lys Val Glu Ile Lys Arg Gly
                245

<210>  32
<211>  246
<212>  PRT
<213>  Artificial Sequence

<220>
<223>  Synthetic Construct

<400>  32

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Arg Gly Asp Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Met Thr Ser Asn Ala Val Gly Phe Asp Tyr Trp Gly Gln Gly
            100                 105                 110

Thr Leu Val Thr Val Ser Ser Gly Gly Gly Gly Ser Gly Gly Gly Ser
        115                 120                 125

Gly Gly Gly Gly Ser Gly Gln Ser Val Leu Thr Gln Pro Pro Ser Val
    130                 135                 140

Ser Gly Ala Pro Gly Gln Arg Val Thr Ile Ser Cys Thr Gly Arg His
145                 150                 155                 160

Ser Asn Ile Gly Leu Gly Tyr Gly Val His Trp Tyr Gln Gln Leu Pro
                165                 170                 175

Gly Thr Ala Pro Lys Leu Leu Ile Tyr Gly Asn Thr Asn Arg Pro Ser
            180                 185                 190

Gly Val Pro Asp Arg Phe Ser Gly Phe Lys Ser Gly Thr Ser Ala Ser
        195                 200                 205

Leu Ala Ile Thr Gly Leu Gln Ala Glu Asp Glu Ala Asp Tyr Tyr Cys
    210                 215                 220

Gln Ser Tyr Asp Arg Arg Thr Pro Gly Trp Val Phe Gly Gly Gly Thr
225                 230                 235                 240

Lys Leu Thr Val Leu Gly
                245

<210>  33
<211>  10
<212>  PRT
<213>  Homo sapiens

<400>  33

Glu Gln Lys Leu Ile Ser Glu Glu Asp Leu
1               5                   10

<210>  34
<211>  9
<212>  PRT
<213>  Orthomxyoviridue

<400>  34

Tyr Pro Tyr Asp Val Pro Asp Tyr Ala
1               5

<210>  35
<211>  6
<212>  PRT
<213>  Artificial Sequence

<220>
<223>  Synthetic Construct

<400>  35

His His His His His His
1               5

<210>  36
<211>  277
<212>  PRT
<213>  Homo sapiens

<400>  36

<210>  37
<211>  479
<212>  PRT
<213>  Homo sapiens

<400>  37

<210>  38
<211>  247
<212>  PRT
<213>  Homo sapiens

<400>  38

<210>  39
<211>  105
<212>  PRT
<213>  Homo sapiens

<400>  39

Met Gly Asp Val Glu Lys Gly Lys Lys Ile Phe Ile Met Lys Cys Ser
1               5                   10                  15

Gln Cys His Thr Val Glu Lys Gly Gly Lys His Lys Thr Gly Pro Asn
            20                  25                  30

Leu His Gly Leu Phe Gly Arg Lys Thr Gly Gln Ala Pro Gly Tyr Ser
        35                  40                  45

Tyr Thr Ala Ala Asn Lys Asn Lys Gly Ile Ile Trp Gly Glu Asp Thr
    50                  55                  60

Leu Met Glu Tyr Leu Glu Asn Pro Lys Lys Tyr Ile Pro Gly Thr Lys
65                  70                  75                  80

Met Ile Phe Val Gly Ile Lys Lys Lys Glu Glu Arg Ala Asp Leu Ile
                85                  90                  95

Ala Tyr Leu Lys Lys Ala Thr Asn Glu
            100                 105

<210>  40
<211>  233
<212>  PRT
<213>  Homo sapiens

<400>  40

Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe

20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210>  41
<211>  314
<212>  PRT
<213>  Homo sapiens

<400>  41

Met Leu Gly Ile Trp Thr Leu Leu Pro Leu Val Leu Thr Ser Val Ala

1               5                   10                  15

Arg Leu Ser Ser Lys Ser Val Asn Ala Gln Val Thr Asp Ile Asn Ser
            20                  25                  30

Lys Gly Leu Glu Leu Arg Lys Thr Val Thr Thr Val Glu Thr Gln Asn
        35                  40                  45

Leu Glu Gly Leu His His Asp Gly Gln Phe Cys His Lys Pro Cys Pro
    50                  55                  60

Pro Gly Glu Arg Lys Ala Arg Asp Cys Thr Val Asn Gly Asp Glu Pro
65                  70                  75                  80

Asp Cys Val Pro Cys Gln Glu Gly Lys Glu Tyr Thr Asp Lys Ala His
                85                  90                  95

Phe Ser Ser Lys Cys Arg Arg Cys Arg Leu Cys Asp Glu Gly His Gly
            100                 105                 110

Leu Glu Val Glu Ile Asn Cys Thr Arg Thr Gln Asn Thr Lys Cys Arg
        115                 120                 125

Cys Lys Pro Asn Phe Phe Cys Asn Ser Thr Val Cys Glu His Cys Asp
    130                 135                 140

Pro Cys Thr Lys Cys Glu His Gly Ile Ile Lys Glu Cys Thr Leu Thr
145                 150                 155                 160

Ser Asn Thr Lys Cys Lys Glu Glu Val Lys Arg Lys Glu Val Gln Lys
                165                 170                 175

Thr Cys Arg Lys His Arg Lys Glu Asn Gln Gly Ser His Glu Ser Pro
            180                 185                 190

Thr Leu Asn Pro Glu Thr Val Ala Ile Asn Leu Ser Asp Val Asp Leu
        195                 200                 205

Ser Lys Tyr Ile Thr Thr Ile Ala Gly Val Met Thr Leu Ser Gln Val
    210                 215                 220

Lys Gly Phe Val Arg Lys Asn Gly Val Asn Glu Ala Lys Ile Asp Glu
225                 230                 235                 240

Ile Lys Asn Asp Asn Val Gln Asp Thr Ala Glu Gln Lys Val Gln Leu
                245                 250                 255

Leu Arg Asn Trp His Gln Leu His Gly Lys Lys Glu Ala Tyr Asp Thr
            260                 265                 270

Leu Ile Lys Asp Leu Lys Lys Ala Asn Leu Cys Thr Leu Ala Glu Lys
        275                 280                 285

Ile Gln Thr Ile Ile Leu Lys Asp Ile Thr Ser Asp Ser Glu Asn Ser
    290                 295                 300

Asn Phe Arg Asn Glu Ile Gln Ser Leu Val
305                 310

<210>  42
<211>  168
<212>  PRT
<213>  Homo sapiens

<400>  42

Met Phe Gln Ile Pro Glu Phe Glu Pro Ser Glu Gln Glu Asp Ser Ser
1               5                   10                  15

Ser Ala Glu Arg Gly Leu Gly Pro Ser Pro Ala Gly Asp Gly Pro Ser
            20                  25                  30

Gly Ser Gly Lys His His Arg Gln Ala Pro Gly Leu Leu Trp Asp Ala
        35                  40                  45

Ser His Gln Gln Glu Gln Pro Thr Ser Ser Ser His His Gly Gly Ala
    50                  55                  60

Gly Ala Val Glu Ile Arg Ser Arg His Ser Ser Tyr Pro Ala Gly Thr
65                  70                  75                  80

Glu Asp Asp Glu Gly Met Gly Glu Glu Pro Ser Pro Phe Arg Gly Arg
                85                  90                  95

Ser Arg Ser Ala Pro Pro Asn Leu Trp Ala Ala Gln Arg Tyr Gly Arg
            100                 105                 110

Glu Leu Arg Arg Met Ser Asp Glu Phe Val Asp Ser Phe Lys Lys Gly
        115                 120                 125

Leu Pro Arg Pro Lys Ser Ala Gly Thr Ala Thr Gln Met Arg Gln Ser
    130                 135                 140

Ser Ser Trp Thr Arg Val Phe Gln Ser Trp Trp Asp Arg Asn Leu Gly
145                 150                 155                 160

Arg Gly Ser Ser Ala Pro Ser Gln
                165

<210>  43
<211>  279
<212>  PRT
<213>  Homo sapiens

<400>  43

Met Val Asp His Leu Ala Asn Thr Glu Ile Asn Ser Gln Arg Ile Ala
1               5                   10                  15

Ala Val Glu Ser Cys Phe Gly Ala Ser Gly Gln Pro Leu Ala Leu Pro
            20                  25                  30

Gly Arg Val Leu Leu Gly Glu Gly Val Leu Thr Lys Glu Cys Arg Lys
        35                  40                  45

Lys Ala Lys Pro Arg Ile Phe Phe Leu Phe Asn Asp Ile Leu Val Tyr
    50                  55                  60

Gly Ser Ile Val Leu Asn Lys Arg Lys Tyr Arg Ser Gln His Ile Ile
65                  70                  75                  80

Pro Leu Glu Glu Val Thr Leu Glu Leu Leu Pro Glu Thr Leu Gln Ala
                85                  90                  95

Lys Asn Arg Trp Met Ile Lys Thr Ala Lys Lys Ser Phe Val Val Ser
            100                 105                 110

Ala Ala Ser Ala Thr Glu Arg Gln Glu Trp Ile Ser His Ile Glu Glu
        115                 120                 125

Cys Val Arg Arg Gln Leu Lys Ala Thr Gly Arg Pro Pro Ser Thr Glu
    130                 135                 140

His Ala Ala Pro Trp Ile Pro Asp Lys Ala Thr Asp Ile Cys Met Arg
145                 150                 155                 160

Cys Thr Gln Thr Arg Phe Ser Ala Leu Thr Arg Arg His His Cys Arg
                165                 170                 175

Lys Cys Gly Phe Val Val Cys Ala Glu Cys Ser Arg Gln Arg Phe Leu
            180                 185                 190

Leu Pro Arg Leu Ser Pro Lys Pro Val Arg Val Cys Ser Leu Cys Tyr
        195                 200                 205

Arg Glu Leu Ala Ala Gln Gln Arg Gln Glu Glu Ala Glu Glu Gln Gly
    210                 215                 220

Ala Gly Ser Pro Arg Gln Pro Ala His Leu Ala Arg Pro Ile Cys Gly
225                 230                 235                 240

Ala Ser Ser Gly Asp Asp Asp Asp Ser Asp Glu Asp Lys Glu Gly Ser
                245                 250                 255

Arg Asp Gly Asp Trp Pro Ser Ser Val Glu Phe Tyr Ala Ser Gly Val
            260                 265                 270

Ala Trp Ser Ala Phe His Ser
        275

<210>  44
<211>  850
<212>  PRT
<213>  Pieris rapae

<400>  44

Met Ala Asp Arg Gln Pro Tyr Met Thr Asn Gly Ile Gln Ala Ala Val
1               5                   10                  15

Val Glu Trp Ile Arg Ala Leu Asp Leu Glu Ile Ile Ser Leu Leu Leu
            20                  25                  30

Ser Arg Ala Trp Pro Met Ala Leu Leu Ala Thr Ser Glu Leu Arg Trp
        35                  40                  45

Arg Pro Thr Val Leu Thr Asp Thr Asp Asn Val Val Arg Leu Asp Arg
    50                  55                  60

Arg Gln Arg Leu Val Arg Trp Asp Arg Arg Pro Pro Asn Glu Ile Phe
65                  70                  75                  80

Leu Asp Gly Phe Val Pro Ile Val Thr Arg Glu Asn Pro Asp Trp Glu
                85                  90                  95

Glu Thr Asp Leu Tyr Gly Phe Ala Lys Asn Asn His Pro Ser Ile Phe
            100                 105                 110

Val Ser Thr Thr Lys Thr Gln Arg Asn Lys Lys Lys Tyr Val Trp Thr
        115                 120                 125

Pro Arg Asn Ala Asn Arg Gly Ile Val Tyr Gln Tyr Glu Ile Tyr Ala
    130                 135                 140

Pro Gly Gly Val Asp Val Asn Asp Ser Phe Ser Asp Ala Ser Pro Trp
145                 150                 155                 160

Pro Asn Gln Met Glu Val Ala Phe Pro Gly Gly Ile Gln Asn Ile Tyr
                165                 170                 175

Ile Arg Ser Ala Arg Glu Leu His Asn Gly Arg Ile Gln Arg Ile Trp
            180                 185                 190

Ile Asn Pro Asn Phe Leu Asp Pro Gly Asp Leu Glu Pro Ile Val Ser
        195                 200                 205

Ser Ser Arg Thr Pro Gln Val Ile Trp Arg Met Asn His Pro Asp Gly
    210                 215                 220

Gly His Arg Asp Gln Arg Ser Glu Arg Ser Ala Ser Ser Tyr Asp Asp
225                 230                 235                 240

Leu Met Tyr Gly Gly Thr Gly Asn Val Gln Glu Asp Thr Phe Gly Asp
                245                 250                 255

Glu Pro Asn Asn Pro Lys Pro Ile Ala Ala Gly Glu Phe Met Ile Glu
            260                 265                 270

Ser Ile Lys Asp Lys Asn Ser Phe Leu Asp Leu Ser Lys Asn Val Asn
        275                 280                 285

Gly Gly Val Ile His Ser Asn Leu Tyr Ser Gly Gly Asp Asn Gln Ile
    290                 295                 300

Trp Val Phe Ser Tyr Asp Asp Asn Lys Lys Ala Tyr Arg Ile Gln Ser
305                 310                 315                 320

Tyr Gln Asn Ser Tyr Leu Tyr Leu Ser Trp Asp Ser Asn Ala Ser Ser
                325                 330                 335

Lys Glu Met Ile Leu Arg Gly Tyr Thr Asn Ser Gly Ser Asn Asn Gln
            340                 345                 350

Tyr Trp Gln Ile Glu Gln Thr Gly Lys Asn Tyr Arg Leu Arg Asn Leu
        355                 360                 365

Leu Asn Leu Asp Met Ile Ile Thr Ala Gln Asp Lys Pro Ser Ala Phe
    370                 375                 380

Gly Gly Lys Glu Val Ile Val Asn Thr Glu Ile Ser Asn Ser Asn Thr
385                 390                 395                 400

Lys Ile Ser Gln Glu Trp Lys Met Ile Pro Phe Asp Phe Arg Pro Ile
                405                 410                 415

Ile Asp Gly Asp Tyr Asn Ile Phe Asn Val Asp Leu Ser Asn Gln Val
            420                 425                 430

Val Asp Phe Ser Asn Gln Pro Asp Leu Leu Val His Gly His Ile Phe
        435                 440                 445

Cys Asp Asn Glu Asn Gln Thr Trp His Phe Thr Tyr Asn Ser Thr Tyr
    450                 455                 460

His Ala Tyr Lys Ile Trp Ser Gly Arg Lys Ser Asn Leu Leu Leu Thr
465                 470                 475                 480

Trp Asp Ser Asn Ala Ala Ser Lys Glu Met Val Val Arg Ala Tyr Thr
                485                 490                 495

Glu Ser Arg Ser Lys Asn Gln Tyr Trp Arg Ile Glu Gln Thr Gly Ser
            500                 505                 510

Lys Ser Tyr Lys Val Arg Asn Leu Glu Asn Ser Ser Met Ile Leu Gly
        515                 520                 525

Leu Thr Arg Val Ser Thr Pro Tyr Gly Gly Leu Asn Leu Met Val Glu
    530                 535                 540

Asp Asp Ser Asp Gly His Ser Asp Leu His Ser Asp Trp Asp Ile Lys
545                 550                 555                 560

Pro Ile Phe Tyr Gln Asp Ile Pro Asp Gly Asp Tyr Asn Ile Phe Asn
                565                 570                 575

Asp Asn Phe Pro Asn Ile Ala Ile Asp Phe Thr Asn Gln Glu Gly Ser
            580                 585                 590

Leu Ile His Gly His Asn Phe Cys Ser Asn Asn Asn Gln Lys Trp Ser
        595                 600                 605

Phe Val Phe Asp Gly Lys Arg Lys Ala Tyr Arg Ile Lys Ser Gly Val
    610                 615                 620

Arg Ser Asn Leu Trp Leu Ser Trp Asp Ser Asn Ala Ser Ser Lys Glu
625                 630                 635                 640

Met Val Leu Arg Ala Tyr Thr Glu Ser Gly Ser Ser Asn Gln Tyr Trp
                645                 650                 655

Arg Leu Asp Glu Ala Asn Asp Gly Ser Tyr Arg Ile Arg Asn Leu Gln
            660                 665                 670

Asp Tyr Tyr Lys Leu Ile Ala Leu Thr Asn Lys Asn Thr Pro Tyr Gly
        675                 680                 685

Gly Lys Glu Leu Ile Val Ser Asp Asn Lys Glu Ser Gly Asn Thr Trp
    690                 695                 700

Tyr Leu Lys Lys Leu Gly Glu Val Pro Leu Pro Asn Arg Lys Phe Arg
705                 710                 715                 720

Ile Ala Thr Lys Leu Asn Tyr Lys Lys Val Ile Asp Ser Ser Thr Ser
                725                 730                 735

Tyr Asn Leu Ile Ile Thr His Asp Leu Asn Phe Ala Ser Ser Ile Trp
            740                 745                 750

Glu Leu Val Tyr Asp Ser Ser Lys Lys Ala Tyr Asn Ile Tyr Ser Ser
        755                 760                 765

Asp Ile Asn Asn Leu Gly Trp Ile Tyr Gln Asn Lys Asn Phe Phe Val
    770                 775                 780

Lys Leu Gly Asn Ile Asp Gly Pro Asp His Gly Asp Leu Arg Tyr Phe
785                 790                 795                 800

Trp Thr Ile Glu Tyr Ser Met Gln Thr Gly Cys Tyr Leu Ile Arg Ser
                805                 810                 815

Leu His Asp Pro Ala Asn Ala Val Gly Tyr Thr Asp Ser Glu Ser Val
            820                 825                 830

Ile Thr Asp Thr Ser Thr Tyr Ser Asp Asn Gln Leu Phe His Phe Ile
        835                 840                 845

Leu Met
    850

<210> 45
<211> 281
<212> PRT
<213> Homo sapiens

<400> 45

<210>  46
<211>  192
<212>  PRT
<213>  Homo sapiens

<400>  46

Met Asp Gly Ser Gly Glu Gln Pro Arg Gly Gly Gly Pro Thr Ser Ser
1               5                   10                  15

Glu Gln Ile Met Lys Thr Gly Ala Leu Leu Leu Gln Gly Phe Ile Gln
            20                  25                  30

Asp Arg Ala Gly Arg Met Gly Gly Glu Ala Pro Glu Leu Ala Leu Asp
        35                  40                  45

Pro Val Pro Gln Asp Ala Ser Thr Lys Lys Leu Ser Glu Cys Leu Lys
    50                  55                  60

Arg Ile Gly Asp Glu Leu Asp Ser Asn Met Glu Leu Gln Arg Met Ile
65                  70                  75                  80

Ala Ala Val Asp Thr Asp Ser Pro Arg Glu Val Phe Phe Arg Val Ala
                85                  90                  95

Ala Asp Met Phe Ser Asp Gly Asn Phe Asn Trp Gly Arg Val Val Ala
            100                 105                 110

Leu Phe Tyr Phe Ala Ser Lys Leu Val Leu Lys Ala Leu Cys Thr Lys
        115                 120                 125

Val Pro Glu Leu Ile Arg Thr Ile Met Gly Trp Thr Leu Asp Phe Leu
    130                 135                 140

Arg Glu Arg Leu Leu Gly Trp Ile Gln Asp Gln Gly Gly Trp Asp Gly
145                 150                 155                 160

Leu Leu Ser Tyr Phe Gly Thr Pro Thr Trp Gln Thr Val Thr Ile Phe
                165                 170                 175

Val Ala Gly Val Leu Thr Ala Ser Leu Thr Ile Trp Lys Lys Met Gly
            180                 185                 190

<210>  47

<211>  238
<212>  PRT
<213>  Aequorea victoria

<400>  47

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Phe
    50                  55                  60

Ser Tyr Gly Val Gln Cys Phe Ser Arg Tyr Pro Asp His Met Lys Gln
65                  70                  75                  80

His Asp Phe Phe Lys Ser Ala Met Pro Glu Gly Tyr Val Gln Glu Arg
                85                  90                  95

Thr Ile Phe Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Met Ala Asp Lys Gln Lys Asn Gly
145                 150                 155                 160

Ile Lys Val Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr His Gly Met Asp Glu Leu Tyr Lys

225                 230                 235

<210>  48
<211>  194
<212>  PRT
<213>  Aliivibrio fischeri

<400>  48

Met Phe Lys Gly Ile Val Glu Gly Ile Gly Ile Ile Glu Lys Ile Asp
1               5                   10                  15

Ile Tyr Thr Asp Leu Asp Lys Tyr Ala Ile Arg Phe Pro Glu Asn Met
            20                  25                  30

Leu Asn Gly Ile Lys Lys Glu Ser Ser Ile Met Phe Asn Gly Cys Phe
        35                  40                  45

Leu Thr Val Thr Ser Val Asn Ser Asn Ile Val Trp Phe Asp Ile Phe
    50                  55                  60

Glu Lys Glu Ala Arg Lys Leu Asp Thr Phe Arg Glu Tyr Lys Val Gly
65                  70                  75                  80

Asp Arg Val Asn Leu Gly Thr Phe Pro Lys Phe Gly Ala Ala Ser Gly
                85                  90                  95

Gly His Ile Leu Ser Ala Arg Ile Ser Cys Val Ala Ser Ile Ile Glu
            100                 105                 110

Ile Ile Glu Asn Glu Asp Tyr Gln Gln Met Trp Ile Gln Ile Pro Glu
        115                 120                 125

Asn Phe Thr Glu Phe Leu Ile Asp Lys Asp Tyr Ile Ala Val Asp Gly
    130                 135                 140

Ile Ser Leu Thr Ile Asp Thr Ile Lys Asn Asn Gln Phe Phe Ile Ser
145                 150                 155                 160

Leu Pro Leu Lys Ile Ala Gln Asn Thr Asn Met Lys Trp Arg Lys Lys
                165                 170                 175

Gly Asp Lys Val Asn Val Glu Leu Ser Asn Lys Ile Asn Ala Asn Gln
            180                 185                 190

Cys Trp

<210>  49

<211>  225
<212>  PRT
<213>  Montastraea cavernosa

<400>  49

Met Ser Val Ile Lys Ser Val Met Lys Ile Lys Leu Arg Met Asp Gly
1               5                   10                  15

Ile Val Asn Gly His Lys Phe Met Ile Thr Gly Glu Gly Glu Gly Lys
            20                  25                  30

Pro Phe Glu Gly Thr His Thr Ile Ile Leu Lys Val Lys Glu Gly Gly
        35                  40                  45

Pro Leu Pro Phe Ala Tyr Asp Ile Leu Thr Thr Ala Phe Gln Tyr Gly
    50                  55                  60

Asn Arg Val Phe Thr Lys Tyr Pro Lys Asp Ile Pro Asp Tyr Phe Lys
65                  70                  75                  80

Gln Ser Phe Pro Glu Gly Tyr Ser Trp Glu Arg Ser Met Thr Phe Glu
                85                  90                  95

Asp Gln Gly Val Cys Thr Val Thr Ser Asp Ile Lys Leu Glu Gly Asp
            100                 105                 110

Cys Phe Phe Tyr Glu Ile Arg Phe Tyr Gly Val Asn Phe Pro Ser Ser
        115                 120                 125

Gly Pro Val Met Gln Lys Lys Thr Leu Lys Trp Glu Pro Ser Thr Glu
    130                 135                 140

Asn Met Tyr Val Arg Asp Gly Val Leu Leu Gly Asp Val Ser Arg Thr
145                 150                 155                 160

Leu Leu Leu Glu Gly Asp Lys His His Arg Cys Asn Phe Arg Ser Thr
                165                 170                 175

Tyr Gly Ala Lys Lys Gly Val Val Leu Pro Glu Tyr His Phe Val Asp
            180                 185                 190

His Arg Ile Glu Ile Leu Ser His Asp Lys Asp Tyr Asn Thr Val Glu
        195                 200                 205

Val Tyr Glu Asn Ala Val Ala Arg Pro Ser Met Leu Pro Val Lys Ala
    210                 215                 220

Lys

225

<210>  50
<211>  230
<212>  PRT
<213>  Discosoma sp.

<400>  50

Met Ser Cys Ser Lys Asn Val Ile Lys Glu Phe Met Arg Phe Lys Val
1               5                   10                  15

Arg Met Glu Gly Thr Val Asn Gly His Glu Phe Glu Ile Lys Gly Glu
            20                  25                  30

Gly Glu Gly Arg Pro Tyr Glu Gly His Cys Ser Val Lys Leu Met Val
        35                  40                  45

Thr Lys Gly Gly Pro Leu Pro Phe Ala Phe Asp Ile Leu Ser Pro Gln
    50                  55                  60

Phe Gln Tyr Gly Ser Lys Val Tyr Val Lys His Pro Ala Asp Ile Pro
65                  70                  75                  80

Asp Tyr Lys Lys Leu Ser Phe Pro Glu Gly Phe Lys Trp Glu Arg Val
                85                  90                  95

Met Asn Phe Glu Asp Gly Gly Val Val Thr Val Ser Gln Asp Ser Ser
            100                 105                 110

Leu Lys Asp Gly Cys Phe Ile Tyr Glu Val Lys Phe Ile Gly Val Asn
        115                 120                 125

Phe Pro Ser Asp Gly Pro Val Met Gln Arg Arg Thr Arg Gly Trp Glu
    130                 135                 140

Ala Ser Ser Glu Arg Leu Tyr Pro Arg Asp Gly Val Leu Lys Gly Asp
145                 150                 155                 160

Ile His Met Ala Leu Arg Leu Glu Gly Gly Gly His Tyr Leu Val Glu
                165                 170                 175

Phe Lys Ser Ile Tyr Met Val Lys Lys Pro Ser Val Gln Leu Pro Gly
            180                 185                 190

Tyr Tyr Tyr Val Asp Ser Lys Leu Asp Met Thr Ser His Asn Glu Asp
        195                 200                 205

Tyr Thr Val Val Glu Gln Tyr Glu Lys Thr Gln Gly Arg His His Pro

210                 215                 220

Phe Ile Lys Pro Leu Gln
225                 230

<210>  51
<211>  550
<212>  PRT
<213>  Photinus pyralis

<400>  51

Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asn Ile Thr Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Asn Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Ser Pro Lys Gly Val

195                 200                 205

Ala Leu Pro His Arg Thr Ala Cys Val Arg Phe Ser His Ala Arg Asp
    210                 215                 220

Pro Ile Phe Gly Asn Gln Ile Ile Pro Asp Thr Ala Ile Leu Ser Val
225                 230                 235                 240

Val Pro Phe His His Gly Phe Gly Met Phe Thr Thr Leu Gly Tyr Leu
                245                 250                 255

Ile Cys Gly Phe Arg Val Val Leu Met Tyr Arg Phe Glu Glu Glu Leu
            260                 265                 270

Phe Leu Arg Ser Leu Gln Asp Tyr Lys Ile Gln Ser Ala Leu Leu Val
        275                 280                 285

Pro Thr Leu Phe Ser Phe Phe Ala Lys Ser Thr Leu Ile Asp Lys Tyr
    290                 295                 300

Asp Leu Ser Asn Leu His Glu Ile Ala Ser Gly Gly Ala Pro Leu Ser
305                 310                 315                 320

Lys Glu Val Gly Glu Ala Val Ala Lys Arg Phe His Leu Pro Gly Ile
                325                 330                 335

Arg Gln Gly Tyr Gly Leu Thr Glu Thr Thr Ser Ala Ile Leu Ile Thr
            340                 345                 350

Pro Glu Gly Asp Asp Lys Pro Gly Ala Val Gly Lys Val Val Pro Phe
        355                 360                 365

Phe Glu Ala Lys Val Val Asp Leu Asp Thr Gly Lys Thr Leu Gly Val
    370                 375                 380

Asn Gln Arg Gly Glu Leu Cys Val Arg Gly Pro Met Ile Met Ser Gly
385                 390                 395                 400

Tyr Val Asn Asp Pro Glu Ala Thr Asn Ala Leu Ile Asp Lys Asp Gly
                405                 410                 415

Trp Leu His Ser Gly Asp Ile Ala Tyr Trp Asp Glu Asp Glu His Phe
            420                 425                 430

Phe Ile Val Asp Arg Leu Lys Ser Leu Ile Lys Tyr Lys Gly Cys Gln
        435                 440                 445

Val Ala Pro Ala Glu Leu Glu Ser Ile Leu Leu Gln His Pro Asn Ile
    450                 455                 460

Phe Asp Ala Gly Val Ala Gly Leu Pro Gly Asp Asp Ala Gly Glu Leu
465                 470                 475                 480

Pro Ala Ala Val Val Val Leu Glu His Gly Lys Thr Met Thr Glu Lys
                485                 490                 495

Glu Ile Val Asp Tyr Val Ala Ser Gln Val Thr Thr Ala Lys Lys Leu
            500                 505                 510

Arg Gly Gly Val Val Phe Val Asp Glu Val Pro Lys Gly Leu Thr Gly
        515                 520                 525

Lys Leu Asp Ala Arg Lys Ile Arg Glu Ile Leu Ile Lys Ala Lys Lys
    530                 535                 540

Gly Gly Lys Ser Lys Leu
545                 550

<210>  52
<211>  311
<212>  PRT
<213>  Renilla reniformis

<400>  52

Met Thr Ser Lys Val Tyr Asp Pro Glu Gln Arg Lys Arg Met Ile Thr
1               5                   10                  15

Gly Pro Gln Trp Trp Ala Arg Cys Lys Gln Met Asn Val Leu Asp Ser
            20                  25                  30

Phe Ile Asn Tyr Tyr Asp Ser Glu Lys His Ala Glu Asn Ala Val Ile
        35                  40                  45

Phe Leu His Gly Asn Ala Ala Ser Ser Tyr Leu Trp Arg His Val Val
    50                  55                  60

Pro His Ile Glu Pro Val Ala Arg Cys Ile Ile Pro Asp Leu Ile Gly
65                  70                  75                  80

Met Gly Lys Ser Gly Lys Ser Gly Asn Gly Ser Tyr Arg Leu Leu Asp
                85                  90                  95

His Tyr Lys Tyr Leu Thr Ala Trp Phe Glu Leu Leu Asn Leu Pro Lys
            100                 105                 110

Lys Ile Ile Phe Val Gly His Asp Trp Gly Ala Cys Leu Ala Phe His
        115                 120                 125

Tyr Ser Tyr Glu His Gln Asp Lys Ile Lys Ala Ile Val His Ala Glu
    130                 135                 140

Ser Val Val Asp Val Ile Glu Ser Trp Asp Glu Trp Pro Asp Ile Glu
145                 150                 155                 160

Glu Asp Ile Ala Leu Ile Lys Ser Glu Glu Gly Glu Lys Met Val Leu
                165                 170                 175

Glu Asn Asn Phe Phe Val Glu Thr Met Leu Pro Ser Lys Ile Met Arg
            180                 185                 190

Lys Leu Glu Pro Glu Glu Phe Ala Ala Tyr Leu Glu Pro Phe Lys Glu
        195                 200                 205

Lys Gly Glu Val Arg Arg Pro Thr Leu Ser Trp Pro Arg Glu Ile Pro
    210                 215                 220

Leu Val Lys Gly Gly Lys Pro Asp Val Val Gln Ile Val Arg Asn Tyr
225                 230                 235                 240

Asn Ala Tyr Leu Arg Ala Ser Asp Asp Leu Pro Lys Met Phe Ile Glu
                245                 250                 255

Ser Asp Pro Gly Phe Phe Ser Asn Ala Ile Val Glu Gly Ala Lys Lys
            260                 265                 270

Phe Pro Asn Thr Glu Phe Val Lys Val Lys Gly Leu His Phe Ser Gln
        275                 280                 285

Glu Asp Ala Pro Asp Glu Met Gly Lys Tyr Ile Lys Ser Phe Val Glu
    290                 295                 300

Arg Val Leu Lys Asn Glu Gln
305                 310

<210>  53
<211>  197
<212>  PRT
<213>  Artificial Sequence

<220>
<223>  Synthetic Construct

<400>  53

Asp Ala His Lys Ser Glu Val Ala His Arg Phe Lys Asp Leu Gly Glu

1               5                   10                  15

Glu Asn Phe Lys Ala Leu Val Leu Ile Ala Phe Ala Gln Tyr Leu Gln
            20                  25                  30

Gln Ser Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg
        195

<210>  54
<211>  197
<212>  PRT
<213>  Homo sapiens

<400>  54

Gly Lys Ala Ser Ser Ala Lys Gln Arg Leu Lys Cys Ala Ser Leu Gln
1               5                   10                  15

Lys Phe Gly Glu Arg Ala Phe Lys Ala Trp Ala Val Ala Arg Leu Ser

20                  25                  30

Gln Arg Phe Pro Lys Ala Glu Phe Ala Glu Val Ser Lys Leu Val Thr
        35                  40                  45

Asp Leu Thr Lys Val His Thr Glu Cys Cys His Gly Asp Leu Leu Glu
    50                  55                  60

Cys Ala Asp Asp Arg Ala Asp Leu Ala Lys Tyr Ile Cys Glu Asn Gln
65                  70                  75                  80

Asp Ser Ile Ser Ser Lys Leu Lys Glu Cys Cys Glu Lys Pro Leu Leu
                85                  90                  95

Glu Lys Ser His Cys Ile Ala Glu Val Glu Asn Asp Glu Met Pro Ala
            100                 105                 110

Asp Leu Pro Ser Leu Ala Ala Asp Phe Val Glu Ser Lys Asp Val Cys
        115                 120                 125

Lys Asn Tyr Ala Glu Ala Lys Asp Val Phe Leu Gly Met Phe Leu Tyr
    130                 135                 140

Glu Tyr Ala Arg Arg His Pro Asp Tyr Ser Val Val Leu Leu Leu Arg
145                 150                 155                 160

Leu Ala Lys Thr Tyr Glu Thr Thr Leu Glu Lys Cys Cys Ala Ala Ala
                165                 170                 175

Asp Pro His Glu Cys Tyr Ala Lys Val Phe Asp Glu Phe Lys Pro Leu
            180                 185                 190

Val Glu Glu Pro Gln
        195

<210>  55
<211>  205
<212>  PRT
<213>  Artificial sequence

<220>
<223>  Synthetic construct

<400>  55

Val Glu Glu Pro Gln Asn Leu Ile Lys Gln Asn Cys Glu Leu Phe Glu
1               5                   10                  15

Gln Leu Gly Glu Tyr Lys Phe Gln Asn Ala Leu Leu Val Arg Tyr Thr
            20                  25                  30

Lys Lys Val Pro Gln Val Ser Thr Pro Thr Leu Val Glu Val Ser Arg
        35                  40                  45

Asn Leu Gly Lys Val Gly Ser Lys Cys Cys Lys His Pro Glu Ala Lys
    50                  55                  60

Arg Met Pro Cys Ala Glu Asp Tyr Leu Ser Val Val Leu Asn Gln Leu
65                  70                  75                  80

Cys Val Leu His Glu Lys Thr Pro Val Ser Asp Arg Val Thr Lys Cys
                85                  90                  95

Cys Thr Glu Ser Leu Val Asn Arg Arg Pro Cys Phe Ser Ala Leu Glu
            100                 105                 110

Val Asp Glu Thr Tyr Val Pro Lys Glu Phe Gln Ala Glu Thr Phe Thr
        115                 120                 125

Phe His Ala Asp Ile Cys Thr Leu Ser Glu Lys Glu Arg Gln Ile Lys
    130                 135                 140

Lys Gln Thr Ala Leu Val Glu Leu Val Lys His Lys Pro Lys Ala Thr
145                 150                 155                 160

Lys Glu Gln Leu Lys Ala Val Met Asp Asp Phe Ala Ala Phe Val Glu
                165                 170                 175

Lys Cys Cys Lys Ala Asp Asp Lys Glu Thr Cys Phe Ala Glu Glu Gly
            180                 185                 190

Lys Lys Leu Val Ala Ala Ser Gln Ala Ala Leu Gly Leu
        195                 200                 205


<210>  56
<211>  197
<212>  PRT
<213>  Homo sapiens

<400>  56

Asp Ala His Lys Ser Glu Val Ala His Arg Phe Lys Asp Leu Gly Glu
1               5                   10                  15

Glu Asn Phe Lys Ala Leu Val Leu Ile Ala Phe Ala Gln Tyr Leu Gln
            20                  25                  30

Gln Cys Pro Phe Glu Asp His Val Lys Leu Val Asn Glu Val Thr Glu
        35                  40                  45

Phe Ala Lys Thr Cys Val Ala Asp Glu Ser Ala Glu Asn Cys Asp Lys
    50                  55                  60

Ser Leu His Thr Leu Phe Gly Asp Lys Leu Cys Thr Val Ala Thr Leu
65                  70                  75                  80

Arg Glu Thr Tyr Gly Glu Met Ala Asp Cys Cys Ala Lys Gln Glu Pro
                85                  90                  95

Glu Arg Asn Glu Cys Phe Leu Gln His Lys Asp Asp Asn Pro Asn Leu
            100                 105                 110

Pro Arg Leu Val Arg Pro Glu Val Asp Val Met Cys Thr Ala Phe His
        115                 120                 125

Asp Asn Glu Glu Thr Phe Leu Lys Lys Tyr Leu Tyr Glu Ile Ala Arg
    130                 135                 140

Arg His Pro Tyr Phe Tyr Ala Pro Glu Leu Leu Phe Phe Ala Lys Arg
145                 150                 155                 160

Tyr Lys Ala Ala Phe Thr Glu Cys Cys Gln Ala Ala Asp Lys Ala Ala
                165                 170                 175

Cys Leu Leu Pro Lys Leu Asp Glu Leu Arg Asp Glu Gly Lys Ala Ser
            180                 185                 190

Ser Ala Lys Gln Arg
        195

<210>  57
<211>  205
<212>  PRT
<213>  Homo sapiens

<400>  57

<210>  58
<211>  609
<212>  PRT
<213>  Homo sapiens

<400>  58